# EUROPEAN PATENT SPECIFICATION

(11) **EP 4 423 068 B1**
(45) Date of publication and mention of the grant of the patent: **03.12.2025**
(21) Application number: 22813850.9
(22) Date of filing: 28.10.2022
(51) Int. Cl.: C07D 213/82, C07D 215/54, C07D 217/26, C07D 231/20, C07D 237/24, C07D 239/34, C07D 241/24, C07D 311/24, C07D 333/38, C07D 401/06, C07D 405/12, C07D 413/04, C07D 413/12, C07D 471/04, C07D 491/20, C07D 498/04, A61K 31/4412, A61P 9/10

(54) **RXFP1 AGONISTS**
RXFP1-AGONISTEN
AGONISTES DE RXFP1

(30) Priority: 29.10.2021 US 202163273242 P
(43) Date of publication of application: 04.09.2024
(73) Proprietor: Bristol-Myers Squibb Company, Princeton, NJ 08543 (US)
(72) Inventor: CLARKE, Adam James, Princeton, New Jersey 08543 (US); FRIENDS, Todd J., Chesterfield, New Jersey 08515 (US); MATHUR, Arvind, Princeton, New Jersey 08543 (US); MYERS, Michael C., Princeton, New Jersey 08543 (US); LI, Jianqing, Cambridge, Massachusetts 02142 (US); PINTO, Donald J.P., Princeton, New Jersey 08543 (US); PABBISETTY, Kumar Balashanmuga, Princeton, New Jersey 08543 (US); SU, Shun, San Diego, California 92130 (US); VOKITS, Benjamin P., Princeton, New Jersey 08543 (US); BARRE, Durga Buchi Raju, Andhra Pradesh 533437 (IN); SMITH, II, Leon M., Somerset, NJ 08873 (US); ORWAT, Michael J., Princeton, 08543 (US); SHAW, Scott A., Princeton, 08543 (US); KUMAR, Sreekantha Ratna, Bangalore 560099 (IN); POTTURI, Hima Kiran, Bangalore 560099 (IN)
(74) Representative: Dehns
(86) International application number: PCT/US2022/078879
(87) International publication number: WO 2023/077070

(56) References cited:
- WO-A1-2013/165606
- WO-A1-2022/122773

## Description

### CROSS-REFERENCE TO RELATED APPLICATIONS

This application claims the benefit of U.S. Provisional Application No. 63/273,242, filed October 29, 2021.

### BACKGROUND OF THE INVENTION

The present disclosure relates to novel compounds which are relaxin family peptide receptor 1 (RXFP1) agonists, compositions containing them, and the compounds for use in, for example, the treatment of heart failure, fibrotic diseases, and related diseases such as lung disease (e.g., idiopathic pulmonary fibrosis), kidney disease (e.g., chronic kidney disease), and hepatic disease (e.g., non-alcoholic steatohepatitis and portal hypertension).

The human relaxin hormone (also called relaxin or H2 relaxin) is a 6-kDa peptide composed of 53 amino acids whose activity was initially discovered when Frederick Hisaw in 1926 injected crude extracts from swine corpus luteum into virgin guinea pigs and observed a relaxation of the fibrocartilaginous pubic symphysis joint (Hisaw FL., Proc. Soc. Exp. Biol. Med., 1926, 23, 661-663)*.* The relaxin receptor was previously known as Lgr7 but is now officially termed the relaxin family peptide receptor 1 (RXFP1) and was deorphanized as a receptor for relaxin in 2002 (Hsu SY., et al., Science, 2002, 295, 671-674). RXFP1 is reasonably well conserved between mouse and human with 85% amino acid identity and is essentially ubiquitously expressed in humans and in other species *(*Halls ML., et al., Br. J. Pharmacol., 2007, 150, 677-691). The cell signaling pathways for relaxin and RXFP1 are cell type dependent and quite complex (Halls ML., et al., Br. J. Pharmacol., 2007, 150, 677-691; Halls ML., et al. Ann. N Y Acad. Sci., 2009, 1160, 108-111; Halls ML., Ann N Y Acad. Sci., 2007, 1160, 117-120). The best studied pathway is the relaxin-dependent increase in cellular levels of cAMP in which relaxin functions as an RXFP1 agonist to promote G□S coupling and activation of adenylate cyclase (Halls ML., et al., Mol. Pharmacol., 2006, 70, 214-226).

Since the initial discovery of relaxin much experimental work has focused on delineating the role relaxin has played in female reproductive biology and the physiological changes that occur during mammalian pregnancy (Sherwood OD., Endocr. Rev., 2004, 25, 205-234). During human gestation, in order to meet the nutritional demands imposed upon it by the fetus, the female body undergoes a significant ~30% decrease in systemic vascular resistance (SVR) and a concomitant ~50% increase in cardiac output (Jeyabalan AC., K.P., Renal and Electolyte Disorders. 2010, 462-518), (Clapp JF. & Capeless E., Am. J. Cardio., 1997, 80, 1469-1473). Additional vascular adaptations include an ~30% increase in global arterial compliance that is important for maintaining efficient ventricular-arterial coupling, as well as an ~50% increase in both renal blood flow (RBF) and glomerular filtration rate (GFR), important for metabolic waste elimination (Jeyabalan AC., K.P., Renal and Electolyte Disorders. 2010, 462-518), (Poppas A., et al., Circ., 1997, 95, 2407-2415). Both pre-clinical studies in rodents as well as clinical studies performed in a variety of patient settings, provide evidence that relaxin is involved, at least to some extent, in mediating these adaptive physiological changes (Conrad KP., Regul. Integr. Comp. Physiol., 2011, 301, R267-275), (Teichman SL., et al., Heart Fail. Rev., 2009, 14, 321-329). Importantly, many of these adaptive responses would likely be of benefit to HF patients in that excessive fibrosis, poor arterial compliance, and poor renal function are all characteristics common to heart failure patients (Mohammed SF., et al., Circ., 2015, 131, 550-559), (Wohlfahrt P., et al., Eur. J. Heart Fail., 2015, 17, 27-34), (Damman K., et al., Prog. Cardiovasc. Dis., 2011, 54, 144-153).

Heart failure (HF), defined hemodynamically as "systemic perfusion inadequate to meet the body's metabolic demands as a result of impaired cardiac pump function", represents a tremendous burden on today's health care system with an estimated United States prevalence of 5.8 million and greater than 23 million worldwide (Roger VL., et al., Circ. Res., 2013, 113, 646-659). It is estimated that by 2030, an additional 3 million people in the United States alone will have HF, a 25% increase from 2010. The estimated direct costs (2008 dollars) associated with HF for 2010 was $25 billion, projected to grow to $78 B by 2030 (Heidenreich PA., et al., Circ., 2011, 123, 933-944). Astoundingly, in the United States, 1 in 9 deaths has HF mentioned on the death certificate (Roger VL., et al., Circ., 2012, 125, e2-220) and, while survival after HF diagnosis has improved over time (Matsushita K., et al., Diabetes, 2010, 59, 2020-2026), (Roger VL., et al., JAMA, 2004, 292, 344-350), the death rate remains high with ~50% of people with HF dying within 5 years of diagnosis (Roger VL., et al., Circ., 2012, 125, e2-220), (Roger VL., et al., JAMA, 2004, 292, 344-350).

The symptoms of HF are the result of inadequate cardiac output and can be quite debilitating depending upon the advanced stage of the disease. Major symptoms and signs of HF include: 1) dyspnea (difficulty in breathing) resulting from pulmonary edema due to ineffective forward flow from the left ventricle and increased pressure in the pulmonary capillary bed; 2) lower extremity edema occurs when the right ventricle is unable to accommodate systemic venous return; and 3) fatigue due to the failing heart's inability to sustain sufficient cardiac output (CO) to meet the body's metabolic needs (Kemp CD., & Conte JV., Cardiovasc. Pathol., 2011, 21, 365-371). Also, related to the severity of symptoms, HF patients are often described as "compensated" or "decompensated". In compensated heart failure, symptoms are stable, and many overt features of fluid retention and pulmonary edema are absent. Decompensated heart failure refers to a deterioration, which may present as an acute episode of pulmonary edema, a reduction in exercise tolerance, and increasing breathlessness upon exertion (Millane T., et al., BMJ, 2000, 320, 559-562).

In contrast to the simplistic definition of poor cardiac performance not being able to meet metabolic demands, the large number of contributory diseases, multitude of risk factors, and the many pathological changes that ultimately lead to heart failure make this disease exceedingly complex (Jessup M. & Brozena S., N. Engl. J. Med., 2003, 348, 3007-2018). Injurious events thought to be involved in the pathophysiology of HF range from the very acute such as myocardial infarction to a more chronic insult such as lifelong hypertension. Historically, HF was primarily described as "systolic HF" in which decreased left-ventricular (LV) contractile function limits the expulsion of blood and hence results in a reduced ejection fraction (EF is stroke volume/end diastolic volume), or "diastolic HF" in which active relaxation is decreased and passive stiffness is increased limiting LV filling during diastole, however overall EF is maintained (Borlaug BA. & Paulus WJ., Eur Heart J., 2011, 32, 670-679). More recently, as it became understood that diastolic and systolic LV dysfunction was not uniquely specific to these two groups, new terminology was employed: "heart failure with reduced ejection fraction" (HFrEF), and "heart failure with preserved ejection fraction" (HFpEF) ( Borlaug BA. & Paulus WJ., Eur Heart J., 2011, 32, 670-679). Although these two patient populations have very similar signs and symptoms, whether HFrEF and HFpEF represent two distinct forms of HF or two extremes of a single spectrum sharing a common pathogenesis is currently under debate within the cardiovascular community (Borlaug BA. & Redfield MM., Circ., 2011, 123, 2006-2013), (De Keulenaer GW., & Brutsaert DL., Circ., 2011, 123, 1996-2004).

Serelaxin, an intravenous (IV) formulation of the recombinant human relaxin peptide with a relatively short first-phase pharmacokinetic half-life of 0.09 hours, is currently being developed for the treatment of HF (Novartis, 2014). Serelaxin has been given to normal healthy volunteers (NHV) and demonstrated to increase RBF (Smith MC., et al., J. Am. Soc. Nephrol. 2006, 17, 3192-3197) and estimated GFR (Dahlke M., et al., J. Clin. Pharmacol., 2015, 55, 415-422). Increases in RBF were also observed in stable compensated HF patients (Voors AA., et al., Cir. Heart Fail., 2014, 7, 994-1002). In large clinical studies, favorable changes in worsening renal function, worsening HF, as well as fewer deaths, were observed in acute decompensated HF (ADHF) patients in response to an in-hospital 48 hour IV infusion of serelaxin (Teerlink JR., et al., Lancet, 2013, 381, 29-39), (Ponikowski P., et al., Eur. Heart, 2014, 35, 431-441). Suggesting that chronic dosing of serelaxin could provide sustained benefit to HF patients, improvement in renal function based on serum creatinine levels was observed in scleroderma patients given serelaxin continuously for 6 months using a subcutaneous pump (Teichman SL., et al., Heart Fail. Rev., 2009, 14, 321-329). In addition to its potential as a therapeutic agent for the treatment of HF, continuous subcutaneous administration of relaxin has also been demonstrated to be efficacious in a variety of animal models of lung (Unemori EN., et al., J. Clin. Invet., 1996, 98, 2739-2745), kidney (Garber SL., et al., Kidney Int., 2001, 59, 876-882), and liver injury (Bennett RG., Liver Int., 2014, 34, 416-426).

WO2013/165606 discloses compounds which are modulators of the relaxin receptor 1.

In summary, a large body of evidence supports a role for relaxin-dependent agonism of RXFP1 mediating the adaptive changes that occur during mammalian pregnancy, and that these changes translate into favorable physiological effects and outcomes when relaxin is given to HF patients. Additional preclinical animal studies in various disease models of lung, kidney, and liver injury provide evidence that relaxin, when chronically administered, has the potential to provide therapeutic benefit for multiple indications in addition to HF. More specifically, chronic relaxin administration could be of benefit to patients suffering from lung disease (e.g., idiopathic pulmonary fibrosis), kidney disease (e.g., chronic kidney disease), or hepatic disease (e.g., non-alcoholic steatohepatitis and portal hypertension).

### SUMMARY OF THE INVENTION

The present invention provides novel substituted norbornyl compounds, their analogues, including stereoisomers, tautomers, pharmaceutically acceptable salts, or solvates thereof, which are useful as RXFP1 receptor agonists.

Also disclosed are processes and intermediates for making the compounds of the present invention.

The present invention also provides pharmaceutical compositions comprising a pharmaceutically acceptable carrier and at least one of the compounds of the present invention or stereoisomers, tautomers, pharmaceutically acceptable salts, or solvates thereof.

The compounds of the invention may be used, for example, in the treatment and/or prophylaxis of heart failure, fibrotic diseases, and related diseases, such as; lung disease (e.g., idiopathic pulmonary fibrosis), kidney disease (e.g., chronic kidney disease), or hepatic disease (e.g., non-alcoholic steatohepatitis and portal hypertension).

The compounds of the present invention may be used in therapy.

The compounds of the present invention may be used for the manufacture of a medicament for the treatment and/or prophylaxis of heart failure.

The compounds of the invention can be used alone, in combination with other compounds of the present invention, or in combination with one or more, preferably one to two other agent(s).

These and other features of the invention will be set forth in expanded form as the disclosure continues.

### DESCRIPTION OF THE INVENTION

The invention encompasses compounds of Formula (I), which are RXFP1 receptor agonists, and compositions containing them.

In a first aspect, the present invention provides, *inter alia*, compounds of Formula (I): 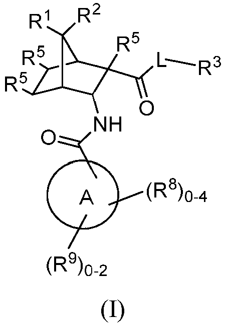 or pharmaceutically acceptable salts thereof, wherein:
L is -O- or -NH-;
Ring A is 5- to 15-membered heterocyclyl comprising 1-5 heteroatoms selected from O, S(=O)ₚ, N, and NR¹⁶;
R¹ is C₁₋₃ alkyl substituted with 1 aryl or C₃₋₆ cycloalkyl substituent;
R² is H; or R¹ and R² are taken together to be =CR⁶R⁷, wherein "=" is a double bond;
or R¹ and R² together with the carbon atom to which they are both attached form a dioxolanyl substituted with 0-1 aryl substituent;
R³ is C₁₋₈ alkyl substituted with 0-5 R⁴, -(CR^{d}R^{d})ₙ-C₃₋₁₀-carbocyclyl substituted with 0-5 R⁴ or -(CR^{d}R^{d})ₙ-3- to 12-membered heterocyclyl comprising 1-4 heteroatoms selected from O, S(=O)ₚ, N, NR^{4a}, and substituted with 0-5 R⁴;
R⁴ is halo, CN, -OH, -SF₅, -S(=O)ₚR^{c}, C₁₋₄ alkyl substituted with 0-5 halo -OH, or -OC₁₋₄ alkyl substituents, -OC₁₋₄ alkyl substituted with 0-5 halo substituents, -(CR^{d}R^{d})ₙ-C₃₋₁₀ carbocyclyl substituted with 0-5 R^{e}, or -(CR^{d}R^{d})ₙ-4- to 9-membered heterocyclyl comprising 1-4 heteroatoms selected from O, S(=O)ₚ, N, and NR^{4a};
R^{4a} is H C₁₋₄ alkyl, or -S(=O)₂CF₃;
R⁵ is H, halo, -OH, C₁₋₄ alkyl substituted with 0-5 halo substituents, or -OC₁₋₄ alkyl substituted with 0-5 halo substituents;
R⁶ is H, halo, CN, C₁₋₇ alkyl substituted with 0-3 R^{6a}, C₂₋₇ alkenyl substituted with 0-3 R^{6a}, C₂₋₇ alkynyl substituted with 0-3 R^{6a}, -C(=O)OR^{6b}, -CONR^{6b}R^{6b}, -(CH₂)ₙ-C₃₋₁₀ carbocyclyl substituted with 0-5 R¹⁴, or 3- to 12-membered heterocyclyl comprising 1-4 heteroatoms selected from O, S(=O)ₚ, N, or NR^{14a}, and substituted with 0-5 R¹⁴;
R^{6a} is halo, -OH, -OC₁₋₄ alkyl, C₁₋₄ alkyl, aryl, or C₃₋₆ cycloalkyl substituted with 0-4 halo substituents;
R^{6b} is H, C₁₋₄ alkyl substituted with 0-1 aryl, or C₃₋₆ cycloalkyl substituted with 0-4 halo substituents;
R⁷ is H or C₁₋₄ alkyl;
R⁸ is =O, C₁₋₄ alkyl, or -OC₁₋₆ alkyl substituted with 0-5 halo, -OH, -OC₁₋₄ alkyl, C₃₋₆ cycloalkyl, aryl, or 3- to 6-membered heterocyclyl substituents;
R⁹ is halo, CN, -C(=O)OR^{b}, -C(=O)NR¹⁷R¹⁷, C₁₋₈ alkyl substituted with 0-4 R¹⁰ and 0-2 R¹¹, C₂₋₈ alkenyl substituted with 0-2 R¹⁰ and 0-2 R¹¹, C₂₋₈ alkynyl substituted with 0-2 R¹⁰ and 0-2 R¹¹, -(A)₀₋₁-C₃₋₆ carbocyclyl substituted with 0-3 R¹⁰ and 0-2 R¹¹, -(A)₀₋₁-C₆₋₉ spirocycloalkyl substituted with 0-2 R¹⁰ and 0-2 R¹¹, or -(A)₀₋₁-3-to 12-membered heterocyclyl comprising 1-5 heteroatoms selected from O, S(=O)ₚ, N, and NR^{11a}, and substituted with 0-3 R¹⁰ and 0-2 R¹¹;
A is -O-, -S-, -CH₂O-, or -OCH₂-;
R¹⁰ is halo, CN, C₁₋₄ alkyl, =O, -OH, or -OC₁₋₄ alkyl;
R¹¹ is C₁₋₄ alkyl substituted with 0-5 R¹² and 0-2 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₉ carbocyclyl substituted with 0-5 R^{e}, aryl substituted with 0-5 R^{e}, 3- to 12-membered heterocyclyl comprising 1-4 heteroatoms selected from O, S(=O)ₚ, N and NR¹⁵, and substituted with 0-5 R^{e};
R^{11a} is H, C₁₋₄ alkyl substituted with 0-4 R^{11b}, -C(=O)R^{b}, -C(=O)OR^{b}, -C(=O)NR^{a}R^{a}, C₃₋₆ cycloalkyl substituted with 0-5 R^{e}, aryl substituted with 0-5 R^{e}, 4- to 6-membered heterocyclyl comprising 1-4 heteroatoms selected from O, S(=O)ₚ, N and NR¹⁵ and substituted with 0-5 R^{e};
R^{11b} is halo, -OH, -C(=O)OH, -C(=O)OC₁₋₄ alkyl, or aryl;
R¹² is halo, -C(=O)OR^{b}, -C(=O)NR^{a}R^{a}, -C(=O)NR^{a}OR^{b}, C₁₋₄ alkyl substituted with 0-3 halo or -OH substituents, or C₃₋₆ cycloalkyl;
R¹³ is -OR^{b}, -NR^{a}R^{a}, -NR^{a}C(=O)R^{b}, -NR^{a}C(=O)OR^{b}, -NR^{a}C(=O)NR^{a}R^{a}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, - S(=O)ₚR^{c}, -(CH₂)ₙ-C₃₋₁₀ carbocyclyl substituted with 0-3 R^{e}, or -(CH₂)ₙ-3- to 12-membered heterocyclyl comprising 1-4 heteroatoms selected from O, S(=O)ₚ, and N, and substituted with 0-3 R^{e};
R¹⁴ is halo, CN, C₁₋₄ alkyl substituted with 0-3 halo substituents, -OC₁₋₄ alkyl substituted with 0-3 halo substituents, -(CH₂)ₙ-NR^{a}R^{a}, -(CH₂)ₙ-aryl substituted with 0-3 R^{e}, -O-aryl substituted with 0-3 R^{e}, or -(CH₂)ₙ- 3- to 12-membered heterocyclyl comprising 1-4 heteroatoms selected from O, S(=O)ₚ, and N, and substituted with 0-3 R^{e};
R^{14a} is H, C(=O)C₁₋₄ alkyl, C₁₋₃ alkyl substituted with 0-3 Si(C₁₋₃ alkyl)₃ or aryl substituted with 0-2 halo substituents;
R¹⁵ is H, C₁₋₄ alkyl, or aryl;
R¹⁶ is H, -C(=O)R^{b}, -C(=O)OR^{b}, -C(=O)NR^{a}R^{a}, -S(=O)ₚR^{c}, C₁₋₄ alkyl substituted with 0-4 R^{16a}, or aryl substituted with 0-4 R^{16a};
R^{16a} is halo, C₁₋₄ alkyl, OR^{b}, C(=O)OR^{b}, or -S(=O)ₚR^{c};
R¹⁷ is H or C₁₋₄ alkyl substituted with 0-3 R¹⁰ and 0-2 R¹¹; or R¹⁷ and R¹⁷ together with the nitrogen atom to which they are both attached form a 3- to 12-membered heterocyclyl comprising 1-5 heteroatoms selected from O, S(=O)ₚ, N, and NR^{11a}, and substituted with 0-3 R¹⁰ and 0-2 R¹¹;
R^{a} is H, -OC₁₋₆ alkyl, C₁₋₆ alkyl substituted with 0-5 R^{e}, C₂₋₆ alkenyl substituted with 0-5 R^{e}, C₂₋₆ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-5 heteroatoms selected from O, S(=O)ₚ, and N, and substituted with 0-5 R^{e}; or R^{a} and R^{a} together with the nitrogen atom to which they are both attached form a 3- to 12-membered heterocyclyl selected from O, S(=O)ₚ, and N, and substituted with 0-5 R^{e};
R^{b} is H, C₁₋₆ alkyl substituted with 0-5 R^{e}, C₂₋₆ alkenyl substituted with 0-5 R^{e}, C₂₋₆ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl selected from O, S(=O)ₚ, and N, and substituted with 0-5 R^{e};
R^{c} is C₁₋₆ alkyl substituted with 0-5 R^{e}, C₂₋₆ alkenyl substituted with 0-5 R^{e}, C₂₋₆ alkynyl substituted with 0-5 R^{e}, C₃₋₆ carbocyclyl, or 3- to 12-membered heterocyclyl comprising 1-5 heteroatoms selected from O, S(=O)ₚ, and N;
R^{d} is H, C₁₋₄ alkyl, or C₃₋₆ cycloalkyl;
R^{e} is halo, CN, NO₂, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₁₀ carbocyclyl substituted with 0-5 R^{g}, -(CH₂)ₙ-3- to 12-membered heterocyclyl comprising 1-5 heteroatoms selected from O, S(=O)ₚ, and N, and substituted with 0-5 R^{g}, -(CH₂)ₙOR^{f}, - C(=O)OR^{f}, -C(=O)NR^{f}R^{f}, -NR^{f}C(=O)R^{f}, -S(=O)ₚR^{f}, -S(=O)ₚNR^{f}R^{f}, - NR^{f}S(=O)ₚR^{f}, -NR^{f}C(=O)OR^{f}, -OC(=O)NR^{f}R^{f}, or -(CH₂)ₙNR^{f}R^{f};
R^{f} is H, C₁₋₆ alkyl substituted with 0-2 OH or -OC₁₋₄ alkyl substituents, C₃₋₆ cycloalkyl, aryl, or 3- to 12-membered heterocyclyl comprising 1-5 heteroatoms selected from O, S(=O)ₚ, and N; or R^{f} and R^{f} together with the nitrogen atom to which they are both attached form a 3- to 12-membered heterocyclyl comprising 1-5 heteroatoms selected from O, S(=O)ₚ, and N;
R^{g} is halo, CN, -OH, C₁₋₆ alkyl, C₃₋₆ cycloalkyl, or aryl;
n is zero, 1, 2, or 3; and
p is zero, 1, or 2.

In a second aspect within the scope of the first aspect, the present invention provides compounds of Formula (II): 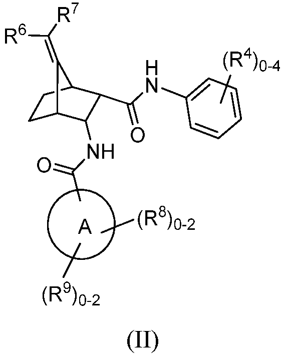 or pharmaceutically acceptable salts thereof, wherein:
Ring A is 5- to 14-membered heterocyclyl comprising 1-4 heteroatoms selected from O, S(=O)ₚ, N, and NR¹⁶;
R⁴ is halo, C₁₋₄ alkyl substituted with 0-4 halo substituents, -OC₁₋₄ alkyl substituted with 0-4 halo, or -S(=O)ₚC₁₋₄ alkyl substituted with 0-4 halo substituents;
R⁶ is halo, C₁₋₇ alkyl substituted with 0-3 R^{6a}, C₃₋₆ cycloalkyl substituted with 0-3 R¹⁴, C₃₋₆ cycloalkenyl substituted with 0-3 R¹⁴, C₆₋₁₀ aryl substituted with 0-3 R¹⁴, or 4- to 6-membered heterocyclyl comprising 1-3 heteroatoms selected from O, S(=O)ₚ, N, and NR^{14a}, and substituted with 0-3 R¹⁴;
R^{6a} is halo, -OH, C₃₋₆ cycloalkyl, or aryl;
R⁷ is H or C₁₋₃ alkyl;
R⁸ is =O, or -OC₁₋₄ alkyl substituted with 0-5 halo, -OH, -OC₁₋₄ alkyl, or aryl substituents;
R⁹ is halo, CN, -C(=O)OR^{b}, -C(=O)NR¹⁷R¹⁷, C₁₋₇ alkyl substituted with 0-3 R¹⁰ and 0-2 R¹¹, C₂₋₇ alkenyl substituted with 0-2 R¹⁰ and 0-2 R¹¹, C₂₋₇ alkynyl substituted with 0-2 R¹⁰ and 0-2 R¹¹, phenyl substituted with 0-3 R¹⁰ and 0-2 R¹¹ or 3- to 12-membered heterocyclyl comprising 1-5 heteroatoms selected from O, S(=O)ₚ, and substituted with 0-3 R¹⁰ and 0-2 R¹¹;
R¹⁰ is halo, CN, C₁₋₄ alkyl, -OH, or -OC₁₋₄ alkyl;
R¹¹ is C₁₋₃ alkyl substituted with 0-1 R¹² and 0-1 R¹³, -OR^{b}, -NR^{a}R^{a}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl substituted with 0-5 R^{e}, 4- to 6-membered heterocyclyl comprising 1-4 heteroatoms selected from O, S(=O)ₚ, N and NR¹⁵ and substituted with 0-5 R^{e};
R¹² is halo, -C(=O)OR^{b}, -C(=O)NHR^{a}, or C₁₋₄ alkyl substituted with 0-3 halo or OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, or -S(=O)ₚR^{c};
R¹⁴ is halo, CN, C₁₋₄ alkyl substituted with 0-3 halo substituents, -OC₁₋₄ alkyl substituted with 0-3 halo substituents;
R^{14a} is H, -C(=O)C₁₋₄ alkyl, or C₁₋₃ alkyl substituted with 0-3 aryl substituted with 0-2 halo substituents;
R¹⁶ is H, C₁₋₃ alkyl substituted with 0-4 R^{16a}, or aryl substituted with 0-4 R^{16a};
R^{16a} is halo, C₁₋₃ alkyl, OR^{b}, C(=O)OR^{b} or -S(=O)ₚR^{c};
R¹⁷ is H or C₁₋₃ alkyl; or R¹⁷ and R¹⁷ together with the nitrogen atom to which they are both attached form a 3- to 10-membered heterocyclyl comprising 1-5 heteroatoms selected from O, S(=O)ₚ, N, and NR^{11a}, and substituted with 0-3 R¹⁰ and 0-2 R¹¹;
R^{a} is H, C₁₋₅ alkyl substituted with 0-5 R^{e}, C₂₋₅ alkenyl substituted with 0-5 R^{e}, C₂₋₅ alkynyl substituted with 0-5 R^{e}, -(CH₂)ₙ-C₃₋₁₀ carbocyclyl substituted with 0-5 R^{e}, or -(CH₂)ₙ-3- to 10-membered heterocyclyl comprising 1-5 heteroatoms selected from O, S(=O)ₚ, and N, and substituted with 0-5 R^{e}; or R^{a} and R^{a} together with the nitrogen atom to which they are both attached form a 3- to 10-membered heterocyclyl comprising 1-5 heteroatoms selected from O, S(=O)ₚ, and N, and substituted with 0-5 R^{e};
R^{b} is H, C₁₋₅ alkyl substituted with 0-5 R^{e}, C₂₋₅ alkenyl substituted with 0-5 R^{e}, C₂₋₅ alkynyl substituted with 0-5 R^{e}, -(CH₂)ₙ-C₃₋₁₀ carbocyclyl substituted with 0-5 R^{e}, or -(CH₂)ₙ- 3- to 10-membered heterocyclyl comprising 1-5 heteroatoms selected from O, S(=O)ₚ, and N, and substituted with 0-5 R^{e};
R^{c} is C₁₋₅ alkyl substituted with 0-5 R^{e}, C₂₋₅ alkenyl substituted with 0-5 R^{e}, C₂₋₅ alkynyl substituted with 0-5 R^{e}, C₃₋₆ carbocyclyl, or 3- to 10-membered heterocyclyl comprising 1-5 heteroatoms selected from O, S(=O)ₚ, and N;
R^{e} is halo, CN, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₆ carbocyclyl substituted with 0 5 R^{g}, -(CH₂)ₙ- 4- to 6-membered heterocyclyl comprising 1-4 heteroatoms selected from O, S(=O)ₚ, and N, and substituted with 0-5 R^{g}, -(CH₂)ₙOR^{f}, - C(=O)OR^{f}, -C(=O)NR^{f}R^{f}, -NR^{f}C(=O)R^{f}, -S(=O)ₚR^{f}, -NR^{f}C(=O)OR^{f}, - OC(=O)NR^{f}R^{f}, or -(CH₂)ₙNR^{f}R^{f};
R^{f} is H, C₁₋₅ alkyl, C₃₋₆ cycloalkyl, or aryl; or R^{f} and R^{f} together with the nitrogen atom to which they are both attached form a heterocyclyl;
R^{g} is halo, CN, -OH, C₁₋₆ alkyl, C₃₋₆ cycloalkyl, or aryl;
n is zero, 1, 2, or 3; and
p is zero, 1, or 2.

In a third aspect within the scope of the second aspect, the present invention provides compounds of Formula (III): 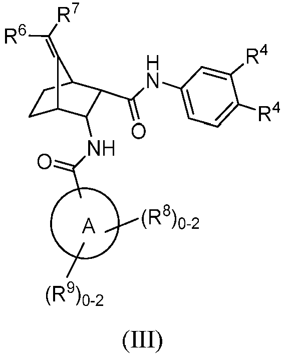 or pharmaceutically acceptable salts thereof, wherein: 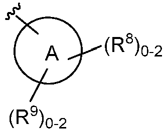 is 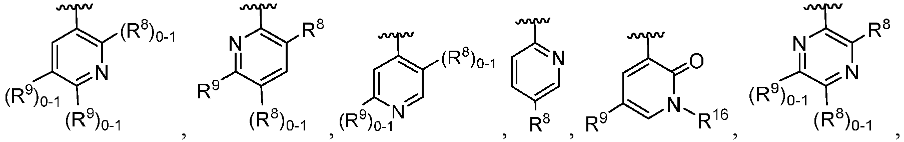 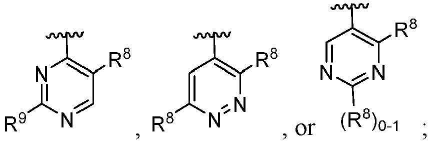
R⁴ is halo or C₁₋₃ alkyl substituted with 0-4 halo substituents;
R⁶ is halo, C₁₋₄ alkyl substituted with 0-3 R^{6a}, C₃₋₆ cycloalkyl, phenyl substituted with 0-3 R¹⁴, naphthyl, or 5- to 6-membered heterocyclyl comprising 1-3 heteroatoms selected from O, S, and N;
R^{6a} is halo, -OH, or C₃₋₆ cycloalkyl;
R¹ is H;
R⁸ is -OC₁₋₄ alkyl substituted with 0-5 halo or -OH substituents;
R⁹ is halo, CN, -C(=O)OR^{b}, -C(=O)NR¹⁷R¹⁷, C₁₋₅ alkyl substituted with 0-3 R¹⁰ and 0-2 R¹¹, C₂₋₄ alkynyl substituted with 0-2 R¹⁰ and 0-2 R¹¹, or phenyl substituted with 0-3 R¹⁰ and 0-2 R¹¹, or 3- to 9-membered heterocyclyl comprising 1-5 heteroatoms selected from O, S(=O)ₚ, and substituted with 0-3 R¹⁰ and 0-2 R¹¹;
R¹⁰ is halo, CN, C₁₋₄ alkyl, or -OH;
R¹¹ is -OR^{b}, -C(=O)OR^{b}, -C(=O)NR^{a}R^{a}, C₁₋₃ alkyl substituted with 0-1 R¹² and 0-1 R¹³ or C₃₋₆ cycloalkyl substituted with 0-5 R^{e};
R¹² is halo, -C(=O)OR^{b}, or C₁₋₃ alkyl substituted with 0-3 halo or OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -OC(=O)NR^{a}R^{a}, - OC(=O)NR^{a}OR^{b}, or -S(=O)ₚR^{c};
R¹⁷ and R¹⁷ together with the nitrogen atom to which they are both attached form a 3- to 10-membered heterocyclyl comprising 1-5 heteroatoms selected from O, S(=O)ₚ, N, and NR^{11a}, and substituted with 0-3 R¹⁰ and 0-2 R¹¹;
R^{a} is H, C₁₋₆ alkyl substituted with 0-5 R^{e}, -(CH₂)ₙ-phenyl substituted with 0-5 R^{e}, or -(CH₂)ₙ-heterocyclyl substituted with 0-5 R^{e}; or R^{a} and R^{a} together with the nitrogen atom to which they are both attached form a heterocyclyl substituted with 0-5 R^{e};
R^{b} is H, C₁₋₆ alkyl substituted with 0-5 R^{e}, -(CH₂)₀₋₁-C₃₋₆ cycloalkyl substituted with 0-5 R^{e}, -(CH₂)₀₋₁-phenyl substituted with 0-5 R^{e}, or -(CH₂)ₙ-heterocyclyl substituted with 0-5 R^{e};
R^{e} is halo, CN, =O, C(=O)OH, C₁₋₆ alkyl, (CH₂)ₙOR^{f} or -S(=O)ₚR^{f};
R^{f} is H or C₁₋₃ alkyl and
n is zero, 1, 2, or 3.

In a fourth aspect within the scope of the third aspect, the present invention provides compounds of Formula (III), or pharmaceutically acceptable salts thereof, wherein:
R⁴ is halo or C₁₋₄ alkyl substituted with 0-4 F substituents;
R⁶ is C₁₋₃ alkyl substituted with 0-3 R^{6a}, C₃₋₆ cycloalkyl, phenyl, or 5- to 6-membered heteroaryl comprising 1-3 heteroatoms selected from O, S, and N;
R^{6a} is halo;
R⁷ is H;
R⁸ is -OC₁₋₃ alkyl substituted with 0-4 halo or OH substituents;
R⁹ is 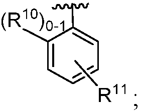
R¹⁰ is halo;
R¹¹ is -C(=O)OR^{b} or C₁₋₂ alkyl substituted with 0-1 R¹² and 0-1 R¹³;
R¹² is halo, -C(=O)OR^{b}, or C₁₋₂ alkyl substituted with 0-3 halo or OH substituents;
R¹³ is -OR^{b}, -NR^{a}R^{a}, -OC(=O)NR^{a}R^{a}, or -S(=O)ₚR^{c};
R^{a} is H, C₁₋₅ alkyl substituted with 0-4 R^{e}, -(CH₂)ₙ-phenyl substituted with 0-4 R^{e}, or -(CH₂)ₙ-heterocyclyl substituted with 0-4 R^{e}; or R^{a} and R^{a} together with the nitrogen atom to which they are both attached form a heterocyclyl substituted with 0-4 R^{e};
R^{b} is H, C₁₋₅ alkyl substituted with 0-4 R^{e}, -(CH₂)ₙ-phenyl substituted with 0-4 R^{e}, or -(CH₂)ₙ-heterocyclyl substituted with 0-4 R^{e};
R^{c} is C₁₋₅ alkyl substituted with 0-4 R^{e};
R^{e} is halo, CN, =O, C₁₋₅ alkyl; and
n is zero, 1, or 2.

In a fifth aspect within the scope of the fourth aspect, the present invention provides compounds of Formula (III), or pharmaceutically acceptable salts thereof, wherein:
R⁴ is F or CF₃;
R⁶ is CF₃, cyclopropyl, phenyl, or 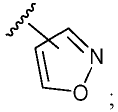
R⁸ is -OC₁₋₄alkyl substituted with 0-2 OH substituents;
R⁹ is 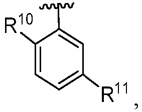 R¹⁰ is F;
R¹¹ is -C(=O)OR^{b} or 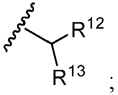
R¹² is halo, -C(=O)OR^{b}, or CF₃;
R¹³ is -OH or -OC(=O)NR^{a}R^{a};
R^{a} is H, C₁₋₄ alkyl, C₃₋₆ cycloalkyl, or phenyl; and
R^{b} is H or C₁₋₃ alkyl.

In a sixth aspect within the scope of the second aspect, the present invention provides compounds of Formula (III): 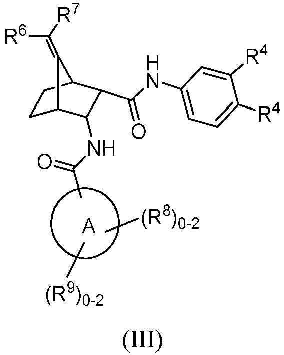 or pharmaceutically acceptable salts thereof, wherein: 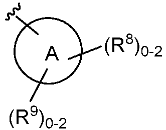 is 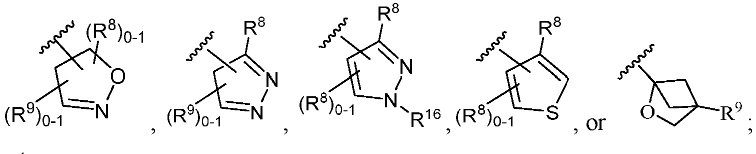
R⁴ is halo or C₁₋₄ alkyl substituted with 0-4 halo;
R⁶ is C₁₋₄ alkyl substituted with 0-3 R^{6a}, C₃₋₆ cycloalkyl, phenyl substituted with 0-3 R¹⁴, or 5- to 6-membered heterocyclyl comprising 1-3 heteroatoms selected from O, S, and N;
R^{6a} is halo;
R⁷ is H;
R⁸ is -OC₁₋₄ alkyl substituted with 0-5 halo or OH substituents;
R⁹ is halo, CN, C₁₋₄ alkyl substituted with 0-3 R¹⁰, or phenyl substituted with 0-3 R¹⁰ and 0-2 R¹¹;
R¹⁰ is halo, CN, or C₁₋₄ alkyl;
R¹¹ is -C(=O)OR^{b};R¹⁶ is H, C₁₋₄ alkyl substituted with 0-4 R^{16a}, or phenyl substituted with 0-4 R^{16a};
R^{16a} is halo, -OR^{b}, -C(=O)OR^{b} or -S(=O)ₚR^{c};
R^{b} is H or C₁₋₄ alkyl; and
R^{c} is C₁₋₃ alkyl.

In a seventh aspect within the scope of the second aspect, the present invention provides compounds of Formula (III): 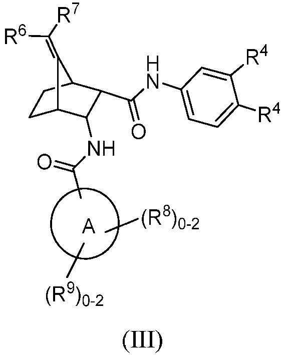 or pharmaceutically acceptable salts thereof, wherein: 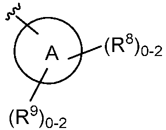 is 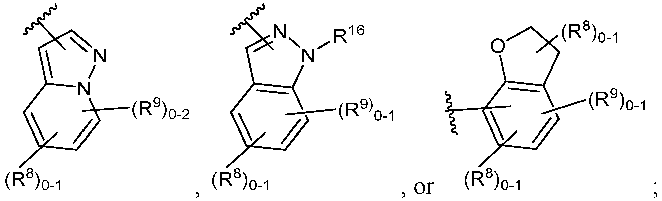
R⁴ is halo or C₁₋₄ alkyl substituted with 0-4 halo substituents;
R⁶ is C₁₋₄ alkyl substituted with 0-3 R^{6a}, C₃₋₆ cycloalkyl, phenyl substituted with 0-3 R¹⁴, or 5- to 6-membered heterocyclyl comprising 1-3 heteroatoms selected from O, S, and N;
R⁷ is H;
R⁸ is -OC₁₋₄ alkyl substituted with 0-5 halo or OH substituents;
R⁹ is halo, CN, or C₁₋₄ alkyl substituted with 0-3 R¹⁰;
R¹⁰ is halo;
R¹⁶ is H, C₁₋₄ alkyl substituted with 0-4 R^{16a}, or aryl substituted with 0-4 R^{16a}; and
R^{16a} is halo, C₁₋₄ alkyl, or C(=O)OR^{b}.

In an eighth aspect within the scope of the second aspect, the present invention provides compounds of Formula (III): 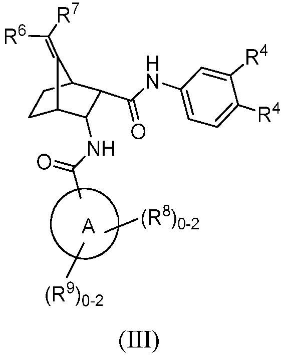 or pharmaceutically acceptable salts thereof, wherein: 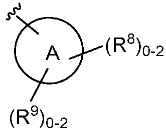 is 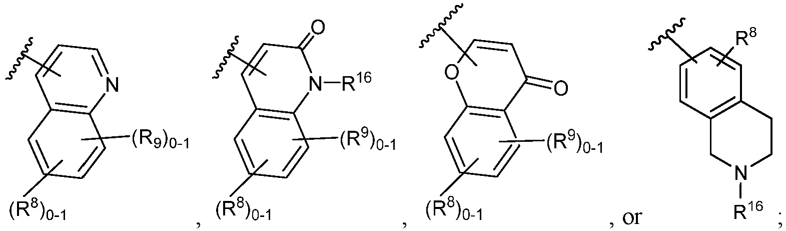
R⁴ is halo or C₁₋₄ alkyl substituted with 0-4 halo substituents;
R⁶ is C₁₋₄ alkyl substituted with 0-3 R^{6a}, C₃₋₆ cycloalkyl, phenyl substituted with 0-3 R¹⁴, or 5- to 6-membered heterocyclyl comprising 1-3 heteroatoms selected from O, S, and N;
R⁷ is H;
R⁸ is -OC₁₋₄ alkyl substituted with 0-5 halo or OH substituents;
R⁹ is halo or CN;
R¹⁶ is H, C₁₋₄ alkyl, -S(=O)ₚR^{c}, or C₁₋₄ alkyl substituted with 0-4 R^{16a}; and
R^{16a} is halo, C₁₋₄ alkyl, -OH, OC₁₋₃ alkyl, or -S(=O)ₚC₁₋₃ alkyl.

In a ninth aspect within the scope of the second aspect, the present invention provides compounds of Formula (III): 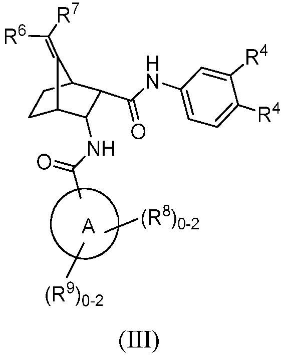 or pharmaceutically acceptable salts thereof, wherein: 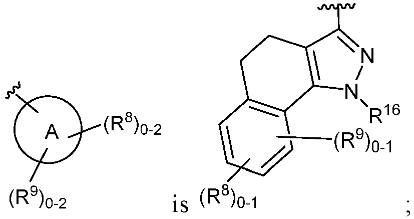
R⁴ is halo or C₁₋₄ alkyl substituted with 0-4 halo;
R⁶ is C₁₋₄ alkyl substituted with 0-3 R^{6a}, C₃₋₆ cycloalkyl, phenyl substituted with 0-3 R¹⁴, or 5- to 6-membered heterocyclyl comprising 1-3 heteroatoms selected from O, S, and N;
R⁷ is H;
R⁸ is -OC₁₋₄ alkyl substituted with 0-5 halo or OH substituents;
R⁹ is halo or CN;
R¹⁶ is H or C₁₋₄ alkyl substituted with 0-4 R^{16a}; and
R^{16a} is halo.

In a tenth aspect within the scope of the third aspect, the present invention provides compounds of Formula (IV): 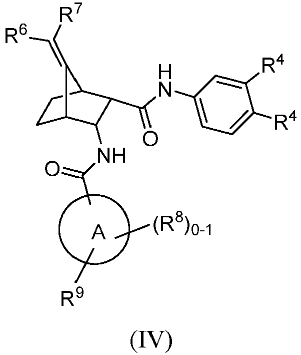 or pharmaceutically acceptable salts thereof, wherein: 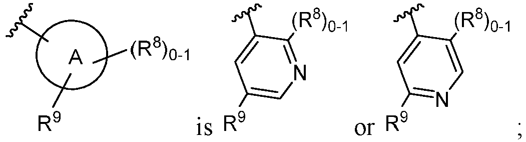
R⁴ is For CF₃;
R⁶ is C₃₋₆ cycloalkyl;
R⁷ is H;
R⁸ is -OC₁₋₃ alkyl;
R⁹ is -C(=O)OR^{b}, -C(=O)NR¹⁷R¹⁷, C₁₋₃ alkyl substituted with 0-1 R¹¹, 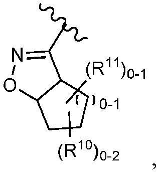 or 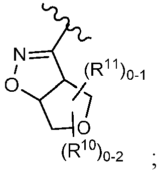
R¹⁰ is halo, CN, C₁₋₄ alkyl, or -OH;
R¹¹ is -OR^{b}, -C(=O)OR^{b}, -C(=O)NR^{a}R^{a}, C₁₋₃ alkyl substituted with 0-1 R¹³, or C₃₋₆ cycloalkyl substituted with 0-5 R^{e};
R¹³ is -OH;
R¹⁷ is H or C₁₋₂ alkyl substituted with 0-3 R¹⁰ and 0-2 R¹¹; or R¹⁷ and R¹⁷ together with the nitrogen atom to which they are both attached form 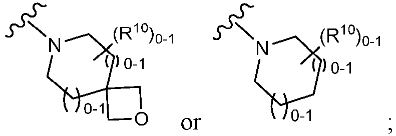
R^{a} is H or C₁₋₃ alkyl;
R^{b} is H, C₁₋₃ alkyl substituted with 0-5 R^{e}, or -(CH₂)₀₋₁-C₃₋₆ cycloalkyl substituted with 0-5 R^{e}; and
R^{e} is halo, CN, =O, C(=O)OH, C₁₋₆ alkyl, CH₂OH, or -S(=O)₂C₁₋₃ alkyl.

In an eleventh aspect within the scope of the first aspect, the present invention provides compounds of Formula (V): 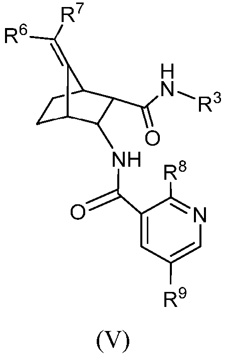 or pharmaceutically acceptable salts thereof, wherein:
R³ is C₁₋₄ alkyl substituted with 0-3 R⁴, -(CHR^{d})ₙ-C₃₋₆-carbocyclyl substituted with 0-3 R⁴;
R⁴ is halo, CN, or C₁₋₄ alkyl substituted with 0-5 halo substituents;
R⁶ is halo, C₁₋₄ alkyl substituted with 0-3 R^{6a}, C₃₋₆ cycloalkyl, or 5- to 6-membered heterocyclyl comprising 1-3 heteroatoms selected from O, S, and N, and substituted with 0-5 R¹⁴;
R^{6a} is halo or OH;
R⁷ is H;
R⁸ is -OC₁₋₃ alkyl;
R⁹ is 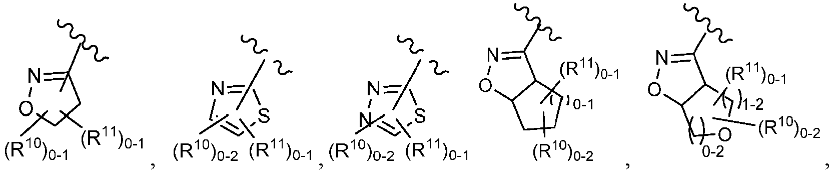 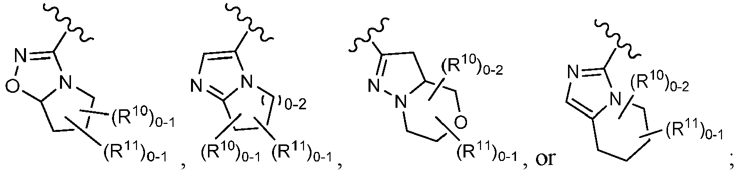
R¹⁰ is halo, CN, C₁₋₄ alkyl, or -OH;
R¹¹ is C₁₋₃ alkyl substituted with 0-3 R¹² and 0-2 R¹³, CN, or OR^{b};
R¹² is halo;
R¹³ is -OR^{b} or C₃₋₆ carbocyclyl;
R¹⁴ is halo, CN, or C₁₋₄ alkyl substituted with 0-3 halo substituents;
R^{b} is H or C₁₋₃ alkyl substituted with 0-5 R^{e};
R^{d} is H or C₁₋₄ alkyl;
R^{e} is halo or OH; and
n is zero or 1.

In a twelfth aspect within the scope of the first aspect, the present invention provides compounds of Formula (VI): 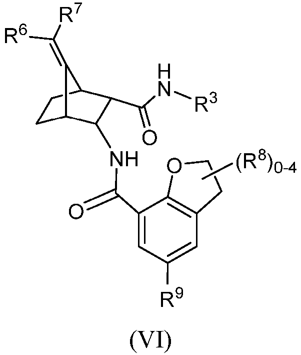 or pharmaceutically acceptable salts thereof, wherein:
R³ is C₁₋₄ alkyl substituted with 0-3 R⁴, -(CHR^{d})ₙ-C₃₋₆-carbocyclyl substituted with 0-3 R⁴;
R⁴ is halo, CN, or C₁₋₄ alkyl substituted with 0-5 halo substituents;
R⁶ is halo, C₁₋₄ alkyl substituted with 0-3 R^{6a}, C₃₋₆ cycloalkyl, or 5- to 6-membered heterocyclyl comprising 1-3 heteroatoms selected from O, S, and N, and substituted with 0-5 R¹⁴;
R^{6a} is halo or -OH;
R⁷ is H;
R⁸ is C₁₋₃ alkyl;
R⁹ is 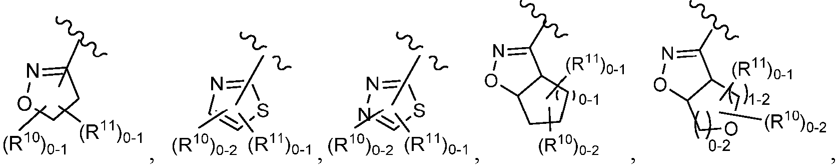 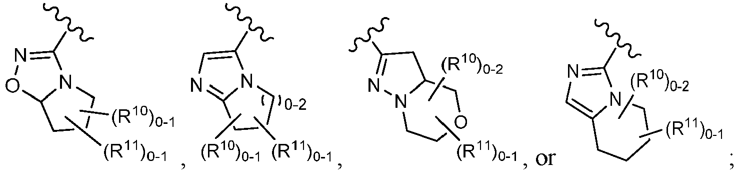
R¹⁰ is halo, CN, C₁₋₄ alkyl, or -OH;
R¹¹ is C₁₋₃ alkyl substituted with 0-3 R¹² and 0-2 R¹³, CN, or OR^{b};
R¹² is halo;
R¹³ is -OR^{b} or C₃₋₆ carbocyclyl;
R¹⁴ is halo, CN, or C₁₋₄ alkyl substituted with 0-3 halo substituents;
R^{b} is H or C₁₋₃ alkyl substituted with 0-5 R^{e};
R^{d} is H or C₁₋₄ alkyl;
R^{e} is halo or OH; and
n is zero or 1.

In a thirteenth aspect within the scope of the first aspect, the present invention provides compounds of Formula (VII): 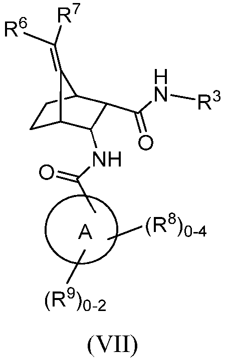 or pharmaceutically acceptable salts thereof, wherein: 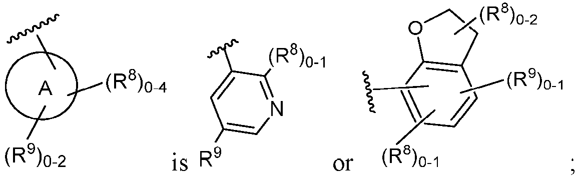
R³ is -(CHR^{d})ₙ-C₃₋₁₀-carbocyclyl substituted with 0-5 R⁴;
R⁴ is halo, CN, C₁₋₄ alkyl substituted with 0-5 halo or OH substituents, -OC₁₋₄ alkyl substituted with 0-5 halo substituents, or -S(=O)ₚR^{c};
R⁶ is C₁₋₄ alkyl substituted with 0-3 R^{6a} or C₃₋₆ cycloalkyl substituted with 0-5 R¹⁴;
R^{6a} is halo;
R⁷ is H;
R⁸ is C₁₋₃ alkyl or -OC₁₋₃ alkyl;
R⁹ is -C(=O)NR¹⁷R¹⁷, C₃₋₆ carbocyclyl substituted with 0-3 R¹⁰ and 0-2 R¹¹, or 5- to 12-membered heterocyclyl comprising 1-5 heteroatoms selected from O, S(=O)ₚ, N, and NR^{11a}, and substituted with 0-3 R¹⁰ and 0-2 R¹¹;
R¹⁰ is halo, CN, C₁₋₄ alkyl, =O, -OH, or -OC₁₋₄ alkyl;
R¹¹ is C₁₋₄ alkyl substituted with 0-5 R¹² and 0-2 R¹³, -S(=O)ₚR^{c}, C₃₋₆ cycloalkyl substituted with 0-5 R^{e};
R^{11a} is H, C₁₋₄ alkyl substituted with 0-4 R^{11b}, -C(=O)R^{b}, -C(=O)OR^{b}, -C(=O)NR^{a}R^{a}, C₃₋₆ cycloalkyl substituted with 0-5 R^{e}, aryl substituted with 0-5 R^{e}, 4- to 6-membered heterocyclyl comprising 1-4 heteroatoms selected from O, S(=O)ₚ, N and NR¹⁵ and substituted with 0-5 R^{e};
R^{11b} is halo, -OH, -C(=O)OH, -C(=O)OC₁₋₄ alkyl, or aryl;
R¹² is halo, -C(=O)OR^{b}, C₁₋₄ alkyl substituted with 0-3 halo or OH substituents, or C₃₋₆ cycloalkyl;
R¹³ is -OR^{b}, -NR^{a}R^{a}, -OC(=O)NR^{a}R^{a},;
R¹⁴ is halo;
R¹⁷ is H or C₁₋₄ alkyl substituted with 0-3 R¹⁰ and 0-2 R¹¹; or R¹⁷ and R¹⁷ together with the nitrogen atom to which they are both attached form a 3- to 9-membered heterocyclyl comprising 1-4 heteroatoms selected from O, S(=O)ₚ, N, and NR^{11a}, and substituted with 0-3 R¹⁰ and 0-2 R¹¹;
R^{a} is H, C₁₋₆ alkyl substituted with 0-5 R^{e}, C₃₋₁₀ carbocyclyl substituted with 0-5 R^{e}; or R^{a} and R^{a} together with the nitrogen atom to which they are both attached form a heterocyclyl substituted with 0-5 R^{e};
R^{b} is H or C₁₋₄ alkyl substituted with 0-5 R^{e};
R^{c} is C₁₋₄ alkyl;
R^{d} is H or C₁₋₃ alkyl;
R^{e} is halo, CN, NO₂, =O, C₁₋₆ alkyl substituted with 0-5 R^{g}, C₃₋₆ cycloalkyl, or -S(=O)ₚR^{f},
R^{f} is H or C₁₋₄ alkyl,
R^{g} is halo, CN, -OH, or C₁₋₄ alkyl;
n is zero or 1; and
p is zero, 1, or 2.

In a fourteenth aspect within the scope of the twelfth aspect, the present invention provides compounds of Formula (VII) or pharmaceutically acceptable salts thereof, wherein:
R³ is -CHR^{d}-C₃₋₆ cycloalkyl substituted with 0-2 R⁴ or phenyl substituted with 0-2 R⁴;
R⁴ is F, CH₃, or CF₃;
R⁶ is C₁₋₄ alkyl substituted with 0-3 halo substituents or C₃₋₆ cycloalkyl substituted with 0-3 halo substituents;
R⁷ is H;
R⁸ is -OCH₃;
R⁹ is -C(=O)NR¹⁷R¹⁷, 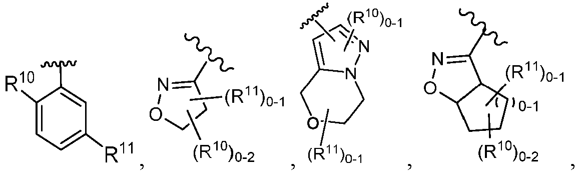 or 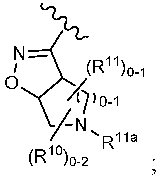
R¹⁰ is halo;
R¹¹ is C₁₋₄ alkyl substituted with 0-3 R¹² and 0-2 R¹³, -S(=O)ₚR^{c}, or C₃₋₆ cycloalkyl substituted with 0-3 R^{e};
R^{11a} is H or C₁₋₄ alkyl substituted with 0-3 R^{11b};
R^{11b} is -OH;
R¹² is C₁₋₄ alkyl substituted with 0-3 halo substituents;
R¹³ is -OC(=O)NR^{a}R^{a};
R¹⁴ is halo;
R¹⁷ is H or C₁₋₄ alkyl substituted with 0-2 R¹⁰ and 0-2 R¹¹; or R¹⁷ and R¹⁷ together with the nitrogen atom to which they are both attached form a 3- to 9-membered heterocyclyl comprising 1-4 heteroatoms selected from O, S(=O)ₚ, N, and NR^{11a}, and substituted with 0-2 R¹⁰ and 0-2 R¹¹;
R^{a} is H, C₁₋₄ alkyl substituted with 0-4 R^{e}, C₃₋₁₀ carbocyclyl substituted with 0-4 R^{e}; or R^{a} and R^{a} together with the nitrogen atom to which they are both attached form a heterocyclyl substituted with 0-4 R^{e};
R^{b} is H or C₁₋₃ alkyl;
R^{c} is C₁₋₃ alkyl;
R^{d} is C₁₋₂ alkyl;
R^{e} is C₁₋₄ alkyl, C₃₋₆ cycloalkyl, or -S(=O)ₚR^{f};
R^{f} is C₁₋₄ alkyl; and
p is zero, 1, or 2.

In one embodiment of Formula (I), R¹ and R² combined are =CR⁶R⁷, R⁶ and R⁷ are both methyl.

In another embodiment of Formula (I), R¹ and R² combined are =CR⁶R⁷; R⁶ is CF₃; R⁷ is H.

In another embodiment of Formula (I), R¹ and R² combined are =CR⁶R⁷; R⁶ is halo; R⁷ is H.

In another embodiment of Formula (I), R¹ and R² combined are =CR⁶R⁷; R⁶ is phenyl substituted with 0-1 R¹⁴; R⁷ is H; R¹⁴ is halo, -OC₁₋₄ alkyl, or phenyl.

In another embodiment of Formula (I), R¹ and R² combined are =CR⁶R⁷; R⁶ is 5-membered heterocyclyl comprising 1-3 heteroatoms selected from O and N; R⁷ is H.

In another embodiment of Formula (I), R¹ and R² combined are =CR⁶R⁷; R⁶ is C₃₋₆ cycloalkyl; R⁷ is H.

In another embodiment of Formula (I), R¹ and R² combined are =CR⁶R⁷; R⁶ is - CH₂-C₃₋₆ cycloalkyl substituted with halo; R⁷ is H.

In another embodiment of Formula (I), R¹ and R² combined are =CR⁶R⁷; R⁶ is cyclopropyl; R⁷ is H.

In another embodiment of Formula (I), R³ is C₁₋₆ alkyl.

In another embodiment of Formula (I), R³ is methyl, ethyl, propyl, or butyl, or pentyl.

In another embodiment of Formula (I), R³ is

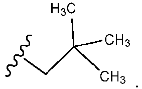

In another embodiment of Formula (I), R³ is C₃₋₆ cycloalkyl substituted with 0-2 R⁴.

In another embodiment of Formula (I), R³ is C₃₋₆ cycloalkenyl substituted with 0-2 R⁴.

In another embodiment of Formula (I), R³ is

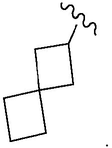

In another embodiment of Formula (I), R³ is -(CR^{d}R^{d})₁₋₂-phenyl substituted with 0-2 R⁴; R⁴ is halo, CF₃ or OCF₃; R^{d} is H or methyl.

In another embodiment of Formula (I), R³ is -(CHR^{d})-C₃₋₆ cycloalkyl substituted with 0-2 R⁴; R⁴ is halo or C₁₋₂ alkyl; R_{d} is H or C₁₋₂ alkyl .

In another embodiment of Formula (I), R³ is alkyl. 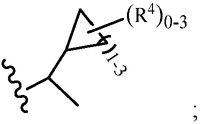 R⁴ is halo or C₁₋₃

In another embodiment of Formula (I), R³ is 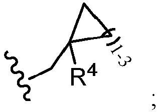 R⁴ is C₁₋₂ alkyl.

In another embodiment of Formula (I), R³ is 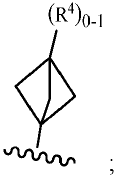 R⁴ is halo or CN.

In another embodiment of Formula (I), R³ is -(CR^{d}R^{d})₁₋₂-5-membered heterocyclyl comprising 1-2 heteroatoms selected from O and N; R^{d} is H or methyl.

In another embodiment of Formula (I), R⁴ is halo, CN, C₁₋₂ alkyl substituted with 0-3 halo substituents.

In another embodiment of Formula (I), R³ is cyclopropyl, cyclobutyl, cyclopentyl substituted with 0-1 R⁴, or cyclohexyl; R⁴ is CN or C₁₋₂ alkyl.

In one embodiment of Formula (I), R⁵ is H, halo, or OH.

In one embodiment of Formula (I), R⁶ is CH₃ or CF₃

In another embodiment of Formula (I), R⁶ is C₃₋₆ cycloalkyl substituted with 0-3 halo substituents.

In another embodiment of Formula (I), R⁶ is 5- to 6-membered heterocyclyl comprising 1-3 heteroatoms selected from O, S(=O)ₚ, N, and NR¹³, and substituted with 0-3 R¹⁴; R¹⁴ is C₁₋₃ alkyl substituted with 0-3 halo substituents.

In one embodiment of Formula (I), R⁷ is H or CH₃.

In one embodiment of Formula (I), R⁸ is halo or -OCH₃.In one embodiment of Formula (V), R³ is 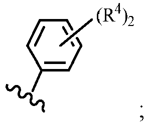 R⁴ is halo, CF₃, or -OCF₃; R⁶ is 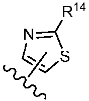 C₃₋₆ cycloalkyl or C₁₋₃ alkyl substituted with 0-3 R^{6a}; R^{6a} is halo; R¹⁴ is C₁₋₂ alkyl substituted with 0-3 halo substituents; R⁷ is H; R⁸ is -OC₁₋₃ alkyl substituted with 0-1 CF₃ or -OCH₃ substituent; R⁹ is 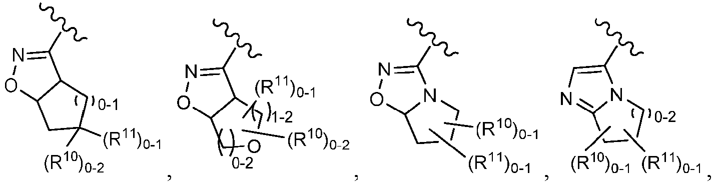 or 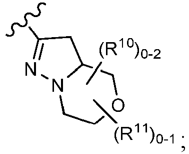 R¹⁰ is C₁₋₄ alkyl, CN, or OH; R¹¹ is C₁₋₃ alkyl substituted with 0-3 R¹² and 0-2 R¹³; R¹² is halo; and R¹³ is OH or C₃₋₆ cycloalkyl.

In one embodiment of Formula (V), R³ is 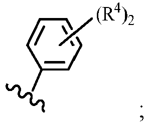 R⁴ is halo, CF₃, or - OCF₃; R⁶ is 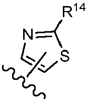 C₃₋₆ cycloalkyl or C₁₋₃ alkyl substituted with 0-3 R^{6a}; R^{6a} is halo; R¹⁴ is C₁₋₂ alkyl substituted with 0-3 halo substituents; R⁷ is H; R⁸ is -OC₁₋₃ alkyl substituted with 0-1 CF₃ or -OCH₃ substituents; R⁹ is 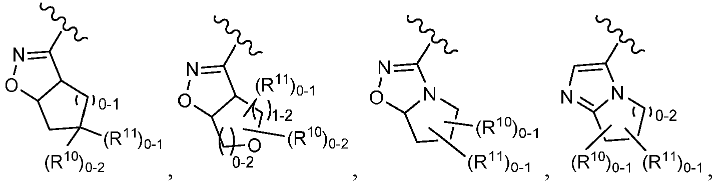 or 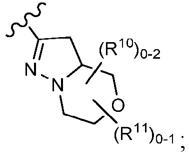 R¹⁰ is C₁₋₄ alkyl, CN, or OH; R¹¹ is C₁₋₃ alkyl substituted with 0-3 R¹² and 0-2 R¹³; R¹² is halo; and R¹³ is OH or C₃₋₆ cycloalkyl.

In another embodiment of Formula (V), R³ is 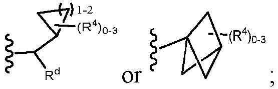 R⁴ is halo, CN, or C₁₋₂ alkyl substituted with 0-3 halo; R^{d} is C₁₋₂ alkyl; R⁶ is 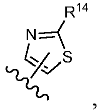 C₃₋₆ cycloalkyl substituted with 0-3 R^{6a}, or C₁₋₃ alkyl substituted with 0-3 R^{6a}; R^{6a} is halo or OH; R¹⁴ is C₁₋₂ alkyl substituted with 0-3 halo substituents; R⁷ is H; R⁸ is -OC₁₋₂ alkyl substituted with 0-1 C₃₋₆ cycloalkyl substituent; R⁹ is 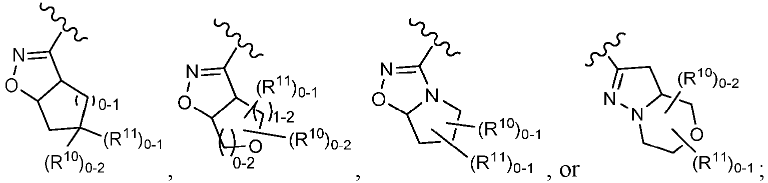 R¹⁰ is C₁₋₄ alkyl or OH; R¹¹ is C₁₋₃ alkyl substituted with 0-3 R¹² and 0-2 R¹³; R¹² is halo; and R¹³ is OH or C₃₋₆ cycloalkyl.

For a compound of Formula (I), the scope of any instance of a variable substituent, including R¹, R², R³, R⁴, R^{4a}, R⁵, R⁶, R^{6a}, R^{6b}, R⁷, R⁸, R⁹, R¹⁰, R¹¹, R^{11a}, R^{11b}, R¹², R¹³, R¹⁴, R^{14a}, R¹⁵, R¹⁶, R^{16a}, R¹⁷, R^{a}, R^{b}, R^{c}, R^{d}, R^{e}, R^{f}, and R^{g} can be used independently with the scope of any other instance of a variable substituent. As such, the invention includes combinations of the different aspects.

Unless specified otherwise, these terms have the following meanings.

"Halo" includes fluoro, chloro, bromo, and iodo.

"Alkyl" or "alkylene" is intended to include both branched and straight-chain saturated aliphatic hydrocarbon groups having the specified number of carbon atoms. For example, "C₁ to C₁₀ alkyl" or "C₁₋₁₀ alkyl" (or alkylene), is intended to include C₁, C₂, C₃, C₄, C₅, C₆, C₇, C₈, C₉, and C₁₀ alkyl groups. Additionally, for example, "C₁ to C₆ alkyl" or "C₁-C₆ alkyl" denotes alkyl having 1 to 6 carbon atoms. Alkyl group can be unsubstituted or substituted with at least one hydrogen being replaced by another chemical group. Example alkyl groups include, but are not limited to, methyl (Me), ethyl (Et), propyl (*e.g.*, n-propyl and isopropyl), butyl (*e.g.*, n-butyl, isobutyl, *t*-butyl), and pentyl (*e.g.*, n-pentyl, isopentyl, neopentyl). When "C₀ alkyl" or "C₀ alkylene" is used, it is intended to denote a direct bond. "Alkyl" also includes deuteroalkyl such as CD₃.

"Alkenyl" or "alkenylene" is intended to include hydrocarbon chains of either straight or branched configuration having one or more, preferably one to three, carbon-carbon double bonds that may occur in any stable point along the chain. For example, "C₂ to C₆ alkenyl" or "C₂₋₆ alkenyl" (or alkenylene), is intended to include C₂, C₃, C₄, C₅, and C₆ alkenyl groups; such as ethenyl, propenyl, butenyl, pentenyl, and hexenyl.

"Alkynyl" or "alkynylene" is intended to include hydrocarbon chains of either straight or branched configuration having one or more, preferably one to three, carbon-carbon triple bonds that may occur in any stable point along the chain. For example, "C₂ to C₆ alkynyl" or "C₂₋₆ alkynyl" (or alkynylene), is intended to include C₂, C₃, C₄, C₅, and C₆ alkynyl groups; such as ethynyl, propynyl, butynyl, pentynyl, and hexynyl.

"Carbocycle", "carbocyclyl", or "carbocyclic residue" is intended to mean any stable 3-, 4-, 5-, 6-, 7-, or 8-membered monocyclic or bicyclic or 7-, 8-, 9-, 10-, 11-, 12-, or 13-membered bicyclic or tricyclic hydrocarbon ring, any of which may be saturated, partially unsaturated, unsaturated or aromatic. Examples of such carbocyclyls include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, [3.3.0]bicyclooctane, [4.3.0]bicyclononane, [4.4.0]bicyclodecane (decalin), [2.2.2]bicyclooctane, fluorenyl, phenyl, naphthyl, indanyl, adamantyl, anthracenyl, and tetrahydronaphthyl (tetralin). As shown above, bridged rings are also included in the definition of carbocyclyl (e.g., [2.2.2]bicyclooctane). A bridged ring occurs when one or more carbon atoms link two non-adjacent carbon atoms. Preferred bridges are one or two carbon atoms. It is noted that a bridge always converts a monocyclic ring into a tricyclic ring. When a ring is bridged, the substituents recited for the ring may also be present on the bridge. When the term "carbocyclyl" is used, it is intended to include "aryl," "cycloalkyl," "spirocycloalkyl", and "cycloalkenyl." Preferred carbocyclyls, unless otherwise specified, are cyclopropyl, cyclobutyl, cyclopentyl, cyclohexyl, phenyl, and indanyl.

"Cycloalkyl" is intended to mean cyclized alkyl groups, including mono-, bi- or multicyclic ring systems. "C₃ to C₇ cycloalkyl" or "C₃₋₇ cycloalkyl" is intended to include C₃, C₄, C₅, C₆, and C₇ cycloalkyl groups. Non-limiting examples of monocyclic cycloalkyls include cyclopropyl, cyclobutyl, cyclopentyl, cyclohexyl, cycloheptyl and cyclooctyl. Non-limiting examples of multicyclic cycloalkyls include 1-decalinyl, norbornyl and adamantyl.

"Cycloalkenyl" is intended to mean cyclized alkenyl groups, including mono- or multi-cyclic ring systems that contain one or more double bonds in at least one ring; although, if there is more than one, the double bonds cannot form a fully delocalized pi-electron system throughout all the rings (otherwise the group would be "aryl," as defined herein). "C₃ to C₇ cycloalkenyl" or "C₃₋₇ cycloalkenyl" is intended to include C₃, C₄, C₅, C₆, and C₇ cycloalkenyl groups.

"Spirocycloalkyl" is intended to mean hydrocarbon bicyclic ring systems with both rings connected through a single atom. The ring can be different in size and nature, or identical in size and nature. Examples include spiropentane, spirohexane, spiroheptane, spirooctane, spirononane, or spirodecane.

"Bicyclic carbocyclyl" or "bicyclic carbocyclic group" is intended to mean a stable 9- or 10-membered carbocyclic ring system that contains two fused rings and consists of carbon atoms. Of the two fused rings, one ring is a benzo ring fused to a second ring; and the second ring is a 5- or 6-membered carbon ring which is saturated, partially unsaturated, or unsaturated. The bicyclic carbocyclic group may be attached to its pendant group at any carbon atom which results in a stable structure. The bicyclic carbocyclic group described herein may be substituted on any carbon if the resulting compound is stable. Examples of a bicyclic carbocyclic group are, but not limited to, naphthyl, 1,2-dihydronaphthyl, 1,2,3,4-tetrahydronaphthyl, and indanyl.

"Aryl" groups refer to monocyclic or polycyclic aromatic hydrocarbons, including, for example, phenyl, naphthyl, and phenanthranyl. Aryl moieties are well known and described, for example, in Lewis, R.J., ed., Hawley's Condensed Chemical Dictionary, 13th Edition, John Wiley & Sons, Inc., New York (1997).

"Benzyl" is intended to mean a methyl group on which one of the hydrogen atoms is replaced by a phenyl group, wherein said phenyl group may optionally be substituted with 1 to 5 groups, preferably 1 to 3 groups.

"Heterocycle", "heterocyclyl" or "heterocyclic ring" is intended to mean a stable 3-, 4-, 5-, 6-, or 7-membered monocyclic or bicyclic or 7-, 8-, 9-, 10-, 11-, 12-, 13-, 14-., or 15-membered polycyclic heterocyclic ring that is saturated, partially unsaturated, or fully unsaturated, and that contains carbon atoms and 1, 2, 3, 4, or 5 heteroatoms independently selected from the group consisting of N, O and S; and including any polycyclic group in which any of the above-defined heterocyclic rings is fused to a benzene ring. The nitrogen and sulfur heteroatoms may optionally be oxidized (*i.e.*, N→O and S(O)ₚ, wherein p is 0, 1 or 2). The nitrogen atom may be substituted or unsubstituted (*i.e.,* N or NR wherein R is H or another substituent, if defined). The heterocyclic ring may be attached to its pendant group at any heteroatom or carbon atom that results in a stable structure. The heterocyclic rings described herein may be substituted on carbon or on a nitrogen atom if the resulting compound is stable. A nitrogen in the heterocyclyl may optionally be quaternized. It is preferred that when the total number of S and O atoms in the heterocyclyl exceeds 1, then these heteroatoms are not adjacent to one another. It is preferred that the total number of S and O atoms in the heterocyclyl is not more than 1. Bridged rings are also included in the definition of heterocyclyl. When the term "heterocyclyl" is used, it is intended to include heteroaryl.

Examples of heterocyclyls include, but are not limited to, acridinyl, azetidinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H,6H-1,5,2-dithiazinyl, dihydrofuro[2,3-b]tetrahydrofuran, furanyl, furazanyl, imidazolidinyl, imidazolinyl, imidazolyl, 1H-indazolyl, imidazolopyridinyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridinyl, isoxazolyl, isoxazolopyridinyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinylperimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridooxazolyl, pyridoimidazolyl, pyridothiazolyl, pyridinyl, pyrimidinyl, pyrrolidinyl, pyrrolinyl, 2-pyrrolidonyl, 2H-pyrrolyl, pyrrolyl, quinazolinyl, quinolinyl, 4H-quinolizinyl, quinoxalinyl, quinuclidinyl, tetrazolyl, tetrahydrofuranyl, tetrahydroisoquinolinyl, tetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridinyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl. Also included are fused ring and spiro compounds containing, for example, the above heterocyclyls.

"Bicyclic heterocyclyl" "bicyclic heterocyclyl" or "bicyclic heterocyclic group" is intended to mean a stable 9- or 10-membered heterocyclic ring system which contains two fused rings and consists of carbon atoms and 1, 2, 3, or 4 heteroatoms independently selected from the group consisting of N, O and S. Of the two fused rings, one ring is a 5-or 6-membered monocyclic aromatic ring comprising a 5-membered heteroaryl ring, a 6-membered heteroaryl ring or a benzo ring, each fused to a second ring. The second ring is a 5- or 6-membered monocyclic ring which is saturated, partially unsaturated, or unsaturated, and comprises a 5-membered heterocyclyl, a 6-membered heterocyclyl or a carbocyclyl (provided the first ring is not benzo when the second ring is a carbocyclyl).

The bicyclic heterocyclic group may be attached to its pendant group at any heteroatom or carbon atom which results in a stable structure. The bicyclic heterocyclic group described herein may be substituted on carbon or on a nitrogen atom if the resulting compound is stable. It is preferred that when the total number of S and O atoms in the heterocyclyl exceeds 1, then these heteroatoms are not adjacent to one another. It is preferred that the total number of S and O atoms in the heterocyclyl is not more than 1.

Examples of a bicyclic heterocyclic group are, but not limited to, quinolinyl, isoquinolinyl, phthalazinyl, quinazolinyl, indolyl, isoindolyl, indolinyl, 1H-indazolyl, benzimidazolyl, 1,2,3,4-tetrahydroquinolinyl, 1,2,3,4-tetrahydroisoquinolinyl, 5,6,7,8-tetrahydroquinolinyl, 2,3-dihydrobenzofuranyl, chromanyl, 1,2,3,4-tetrahydroquinoxalinyl, and 1,2,3,4-tetrahydroquinazolinyl.

"Heteroaryl" is intended to mean stable monocyclic and polycyclic aromatic hydrocarbons that include at least one heteroatom ring member such as sulfur, oxygen, or nitrogen. Heteroaryl groups include, without limitation, pyridyl, pyrimidinyl, pyrazinyl, pyridazinyl, triazinyl, furyl, quinolyl, isoquinolyl, thienyl, imidazolyl, thiazolyl, indolyl, pyrroyl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, purinyl, carbazolyl, benzimidazolyl, indolinyl, benzodioxolanyl, and benzodioxane. Heteroaryl groups are substituted or unsubstituted. The nitrogen atom is substituted or unsubstituted (*i.e.,* N or NR wherein R is H or another substituent, if defined). The nitrogen and sulfur heteroatoms may optionally be oxidized (*i.e.,* N→O and S(O)ₚ, wherein p is 0, 1 or 2).

As referred to herein, the term "substituted" means that at least one hydrogen atom is replaced with a non-hydrogen group, provided that normal valencies are maintained and that the substitution results in a stable compound. When a substituent is keto (*i.e.,* =O), then 2 hydrogens on the atom are replaced. Keto substituents are not present on aromatic moieties. When a ring system (*e.g.*, carbocyclic or heterocyclic) is said to be substituted with a carbonyl group or a double bond, it is intended that the carbonyl group or double bond be part (*i.e.,* within) of the ring. Ring double bonds, as used herein, are double bonds that are formed between two adjacent ring atoms (*e.g.*, C=C, C=N, or N=N).

In cases wherein there are nitrogen atoms (*e.g.*, amines) on compounds of the present invention, these may be converted to N-oxides by treatment with an oxidizing agent (e.g., mCPBA and/or hydrogen peroxides).

Thus, shown and claimed nitrogen atoms are considered to disclose both the shown nitrogen and its N-oxide (N-O) derivative.

When any variable occurs more than one time in any constituent or formula for a compound, its definition at each occurrence is independent of its definition at every other occurrence. Thus, for example, if a group is shown to be substituted with 0-3 R groups, then said group may optionally be substituted with up to three R groups, and at each occurrence R is selected independently from the definition of R. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom in which such substituent is bonded to the rest of the compound of a given formula, then such substituent may be bonded via any atom in such substituent. Combinations of substituents and/or variables are permissible only if such combinations result in stable compounds.

The invention includes all pharmaceutically acceptable salt forms of the compounds. Pharmaceutically acceptable salts are those in which the counter ions do not contribute significantly to the physiological activity or toxicity of the compounds and as such function as pharmacological equivalents. These salts can be made according to common organic techniques employing commercially available reagents. Some anionic salt forms include acetate, acistrate, besylate, bromide, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc.

Throughout the specification and the appended claims, a given chemical formula or name shall encompass all stereo and optical isomers and racemates thereof where such isomers exist. Unless otherwise indicated, all chiral (enantiomeric and diastereomeric) and racemic forms are within the scope of the invention. Enantiomers and diastereomers are examples of stereoisomers. The term "enantiomer" refers to one of a pair of molecular species that are mirror images of each other and are not superimposable. The term "diastereomer" refers to stereoisomers that are not mirror images. The term "racemate" or "racemic mixture" refers to a composition composed of equimolar quantities of two enantiomeric species, wherein the composition is devoid of optical activity.

The invention includes all tautomeric forms of the compounds, atropisomers and rotational isomers.

All processes used to prepare compounds of the present invention and intermediates made therein are considered to be part of the present invention.

The symbols "R" and "S" represent the configuration of substituents around a chiral carbon atom(s). The isomeric descriptors "R" and "S" are used as described herein for indicating atom configuration(s) relative to a core molecule and are intended to be used as defined in the literature (IUPAC Recommendations 1996, Pure and Applied Chemistry, 68:2193-2222 (1996)).

The term "chiral" refers to the structural characteristic of a molecule that makes it impossible to superimpose it on its mirror image. The term "homochiral" refers to a state of enantiomeric purity. The term "optical activity" refers to the degree to which a homochiral molecule or nonracemic mixture of chiral molecules rotates a plane of polarized light.

The invention is intended to include all isotopes of atoms occurring in the compounds. Isotopes include those atoms having the same atomic number but different mass numbers. By way of general example and without limitation, isotopes of hydrogen include deuterium and tritium. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties.

### BIOLOGICAL METHODS

RXFP1 Cyclic Adenosine Monophosphate (cAMP) Assays. Human embryonic kidney cells 293 (HEK293) cells and HEK293 cells stably expressing human RXFP1, were cultured in MEM medium supplemented with 10% qualified FBS, and 300 □g/ml hygromycin (Life Technologies). Cells were dissociated and suspended in assay buffer. The assay buffer was HBSS buffer (with calcium and magnesium) containing 20 mM HEPES, 0.05% BSA, and 0.5 mM IBMX. Cells (3000 cells per well, except 1500 cell per well for HEK293 cells stably expressing human RXFP1) were added to 384-well Proxiplates (Perkin-Elmer). Cells were immediately treated with test compounds in DMSO (2% final) at final concentrations in the range of 0.010 nM to 50 □M. Cells were incubated for 30 min at room temperature. The level of intracellular cAMP was determined using the HTRF HiRange cAMP assay reagent kit (Cisbio) according to manufacturer's instructions. Solutions of cryptate conjugated anti-cAMP and d2 fluorophore-labelled cAMP were made in a supplied lysis buffer separately. Upon completion of the reaction, the cells were lysed with equal volume of the d2-cAMP solution and anti-cAMP solution. After a 1 h room temperature incubation, time-resolved fluorescence intensity was measured using the Envision (Perkin-Elmer) at 400 nm excitation and dual emission at 590 nm and 665 nm. A calibration curve was constructed with an external cAMP standard at concentrations ranging from 2.7 □M to 0.1 pM by plotting the fluorescent intensity ratio from 665 nm emission to the intensity from the 590 nm emission against cAMP concentrations. The potency and activity of a compound to inhibit cAMP production was then determined by fitting to a 4-parametric logistic equation from a plot of cAMP level versus compound concentrations.

The examples disclosed below were tested in the human RXFP1 (hRXFP1) HEK293 cAMP assay described above and found to have agonist activity. Table 1 lists EC50 values in the hRXFP1 HEK293 cAMP assay measured for the examples.

**Table 1**

| Example | cAMP hRXFP 1 HEK293 Assay EC50 (nM) |
|---|---|
| 1 | 710 |
| 2 | 660 |
| 3 | 440 |
| 4 | 5,000 |
| 5 | 1,100 |
| 6 | 1,100 |
| 7 | 1,200 |
| 8 | 1,300 |
| 9 | 1,400 |
| 10 | 5,000 |
| 11 | 940 |
| 12 | 4,600 |
| 13 | 1,700 |
| 14 | 2,900 |
| 15 | 3,500 |
| 16 | 3,800 |
| 17 | 4,500 |
| 18 | 2,100 |
| 19 | 4,000 |
| 20 | 250 |
| 21 | 80 |
| 22 | 1,700 |
| 23 | 2,400 |
| 24 | 1,000 |
| 25 | 33 |
| 26 | 76 |
| 27 | 290 |
| 28 | 34 |
| 29 | 136 |
| 30 | 109 |
| 31 | 162 |
| 32 | 166 |
| 33 | 4,400 |
| 34 | 3,000 |
| 35 | 42 |
| 36 | 63 |
| 37 | 95 |
| 38 | 63 |
| 39 | 110 |
| 40 | 39 |
| 41 | 770 |
| 42 | 8.1 |
| 43 | 9.4 |
| 44 | 360 |
| 45 | 560 |
| 46 | 65 |
| 47 | 200 |
| 48 | 880 |
| 49 | 153 |
| 50 | 129 |
| 51 | 1,300 |
| 52 | 1,300 |
| 53 | 1,600 |
| 54 | 1,200 |
| 55 | 360 |
| 56 | 2,800 |
| 57 | 3,900 |
| 58 | 3,000 |
| 59 | 870 |
| 60 | 210 |
| 61 | 3.7 |
| 62 | 2,400 |
| 63 | 120 |
| 65 | 21 |
| 66 | 1.3 |
| 67 | 300 |
| 68 | 130 |
| 69 | 1.5 |
| 70 | 2.3 |
| 71 | 28 |
| 72 | 160 |
| 73 | 24 |
| 74 | 3.5 |
| 75 | 8.1 |
| 76 | 65 |
| 77 | 71 |
| 78 | 140 |
| 79 | 230 |
| 80 | 260 |
| 81 | 2,300 |
| 82 | 660 |
| 83 | 2,400 |
| 84 | 330 |
| 85 | 200 |
| 86 | 240 |
| 87 | 630 |
| 88 | 110 |
| 89 | 230 |
| 90 | 2,400 |
| 91 | 4,700 |
| 92 | 4,800 |
| 93 | 1,700 |
| 94 | 1,200 |
| 95 | 1,600 |
| 96 | 1.4 |
| 97 | 3.1 |
| 99 | 3.2 |
| 100 | 3.4 |
| 101 | 1.6 |
| 102 | 2.5 |
| 103 | 3.4 |
| 104 | 4.0 |
| 105 | 1.2 |
| 106 | 1.8 |
| 107 | 2.6 |
| 108 | 3.5 |
| 109 | 2.7 |
| 110 | 3.5 |
| 111 | 3.6 |
| 112 | 3.9 |
| 113 | 4.3 |
| 114 | 1.2 |
| 115 | 2.1 |
| 116 | 2.7 |
| 117 | 3.5 |
| 118 | 4.0 |
| 119 | 3.9 |
| 120 | 4.8 |
| 121 | 1.3 |
| 122 | 2.6 |
| 123 | 3.1 |
| 124 | 3.6 |
| 125 | 3.6 |
| 126 | 4.2 |
| 127 | 4.3 |
| 128 | 4.9 |
| 129 | 5.0 |
| 130 | 1.2 |
| 131 | 1.6 |
| 132 | 1.8 |
| 133 | 2.1 |
| 134 | 3.2 |
| 135 | 4.0 |
| 136 | 2.5 |
| 137 | 3.0 |
| 138 | 4.8 |
| 139 | 2.9 |
| 140 | 3.2 |
| 141 | 3.4 |
| 142 | 2.2 |
| 143 | 2.9 |
| 144 | 2.0 |
| 145 | 3.0 |
| 146 | 3.5 |
| 147 | 3.8 |
| 148 | 0.5 |
| 149 | 2.7 |
| 150 | 5.3 |
| 151 | 3.5 |
| 152 | 9.0 |
| 153 | 4.6 |
| 154 | 4.1 |
| 155 | 6.8 |
| 156 | 1.3 |
| 157 | 5.6 |
| 158 | 7.8 |
| 159 | 6.1 |
| 160 | 8.4 |
| 161 | 9.0 |
| 162 | 4.6 |
| 163 | 1.0 |
| 164 | 1.4 |
| 165 | 1.2 |
| 166 | 2.5 |
| 167 | 1.7 |
| 168 | 5.3 |
| 169 | 7.9 |
| 170 | 3.3 |
| 171 | 7.9 |
| 172 | 4.3 |
| 173 | 0.6 |
| 174 | 1.9 |
| 175 | 7.8 |
| 176 | 6.0 |
| 177 | 3.3 |
| 178 | 1.6 |
| 179 | 6.5 |
| 180 | 4.3 |
| 181 | 1.4 |
| 182 | 2.6 |
| 183 | 0.8 |
| 184 | 4.8 |
| 185 | 2.5 |
| 186 | 0.8 |
| 187 | 5.3 |
| 188 | 0.5 |

### PHARMACEUTICAL COMPOSITIONS AND METHODS OF USE

The compounds of Formula (I) are RXFP1 receptor agonists and may find use in the treatment of medical indications such as heart failure (e.g., HF_{R}EF and HFpEF ), fibrotic diseases, and related diseases such as lung disease (e.g., idiopathic pulmonary fibrosis or pulmonary hypertension), kidney disease (e.g., chronic kidney disease), or hepatic disease (e.g., non-alcoholic steatohepatitis and portal hypertension). The compounds of Formular (I) can also be used to treat disorders that are a result of or a cause of arterial stiffness, reduced arterial elasticity, reduced arterial compliance and distensibility including hypertension, kidney disease, peripheral arterial disease, carotid and cerebrovascular disease (i.e stroke and dementia), diabetes, microvascular disease resulting in end organ damage, coronary artery disease, and heart failure. The compounds described herein may also be used in the treatment of pre-eclampsia.

Another aspect of the invention is a pharmaceutical composition comprising a compound of Formula (I) and a pharmaceutically acceptable carrier.

Another aspect of the invention is a pharmaceutical composition comprising a compound of Formula (I) and a pharmaceutically acceptable carrier, for use in for the treatment of a relaxin-associated disorder.

Another aspect of the invention is a compound of Formula (I) for use in a method of treating a cardiovascular disease, the method comprising administering an effective amount of a compound of Formula (I) to a patient in need thereof.

Another aspect of the invention is a compound of Formula (I) for use in a method of treating heart failure, the method comprising administering an effective amount of a compound of Formula (I) to a patient in need thereof.

Another aspect of the invention is a compound of Formula (I) for use in a method of treating fibrosis, the method comprising administering a therapeutically effective amount of a compound of Formula (I) to a patient in need thereof.

Another aspect of the invention is a compound of Formula (I) for use in a method of treating a disease associated with fibrosis, the method comprising administering a therapeutically effective amount of a compound of Formula (I) to a patient in need thereof.

Another aspect of the invention is a compound of Formula (I) for use in a method of treating idiopathic pulmonary fibrosis, the method comprising administering a therapeutically effective amount of a compound of Formula (I) to a patient in need thereof.

Another aspect of the invention is a compound of Formula (I) for use in a method of treating a kidney disease (e.g., chronic kidney disease), the method comprising administering a therapeutically effective amount of a compound of Formula (I) to a patient in need thereof.

Another aspect of the invention is a compound of Formula (I) for use in a method of treating or preventing kidney failure, the method comprising administering a therapeutically effective amount of a compound of Formula (I) to a patient in need thereof.

Another aspect of the invention is a compound of Formula (I) for use in a method of improving, stabilizing or restoring renal function in a patient in need thereof, the method comprising administering a therapeutically effective amount of a compound of Formula (I) to the patient.

Another aspect of the invention is a compound of Formula (I) for use in a method of treating a hepatic disease, the method comprising administering a therapeutically effective amount of a compound of Formula (I) to a patient in need thereof.

Another aspect of the invention is a compound of Formula (I) for use in a method of treating non-alcoholic steatohepatitis and portal hypertension, the method comprising administering a therapeutically effective amount of a compound of Formula (I) to a patient in need thereof.

Another aspect of the invention is a compound of Formula (I) for use in the prophylaxis and/or treatment of a relaxin-associated disorder.

Unless otherwise specified, the following terms have the stated meanings.

The term "patient" or "subject" refers to any human or non-human organism that could potentially benefit from treatment with a RXFP1 agonist as understood by practitioners in this field. Exemplary subjects include human beings of any age with risk factors for cardiovascular disease. Common risk factors include, but are not limited to, age, sex, weight, family history, sleep apnea, alcohol or tobacco use, physical inactivity, arrhythmia, or signs of insulin resistance such as acanthosis nigricans, hypertension, dyslipidemia, or polycystic ovary syndrome (PCOS).

"Treating" or "treatment" cover the treatment of a disease-state as understood by practitioners in this field and include the following: (a) inhibiting the disease-state, *i.e.*, arresting it development; (b) relieving the disease-state, *i.e.*, causing regression of the disease state; and/or (c) preventing the disease-state from occurring in a mammal, in particular, when such mammal is predisposed to the disease-state but has not yet been diagnosed as having it.

"Preventing" or "prevention" cover the preventive treatment *(i.e.,* prophylaxis and/or risk reduction) of a subclinical disease-state aimed at reducing the probability of the occurrence of a clinical disease-state as understood by practitioners in this field. Patients are selected for preventative therapy based on factors that are known to increase risk of suffering a clinical disease state compared to the general population. "Prophylaxis" therapies can be divided into (a) primary prevention and (b) secondary prevention. Primary prevention is defined as treatment in a subject that has not yet presented with a clinical disease state, whereas secondary prevention is defined as preventing a second occurrence of the same or similar clinical disease state. "Risk reduction" or "reducing risk" covers therapies that lower the incidence of development of a clinical disease state. As such, primary and secondary prevention therapies are examples of risk reduction.

"Therapeutically effective amount" is intended to include an amount of a compound of the present invention that is effective when administered alone or in combination with other agents to treat disorders as understood by practitioners in this field. When applied to a combination, the term refers to combined amounts of the active ingredients that result in the preventive or therapeutic effect, whether administered in combination, serially, or simultaneously.

"Disorders of the cardiovascular system" or "cardiovascular disorders" include for example the following disorders: hypertension (high blood pressure), peripheral and cardiac vascular disorders, coronary heart disease, stable and unstable angina pectoris, heart attack, myocardial insufficiency, abnormal heart rhythms (or arrhythmias), persistent ischemic dysfunction ("hibernating myocardium"), temporary postischemic dysfunction ("stunned myocardium"), heart failure, disturbances of peripheral blood flow, acute coronary syndrome, heart failure, heart muscle disease (cardiomyopathy), myocardial infarction and vascular disease (blood vessel disease).

"Heart failure" includes both acute and chronic manifestations of heart failure, as well as more specific or related types of disease, such as advanced heart failure, post-acute heart failure, cardio-renal syndrome, heart failure with impaired kidney function, chronic heart failure, chronic heart failure with mid-range ejection fraction (HFmEF), compensated heart failure, decompensated heart failure, right heart failure, left heart failure, global failure, ischemic cardiomyopathy, dilated cardiomyopathy, heart failure associated with congenital heart defects, heart valve defects, heart failure associated with heart valve defects, mitral stenosis, mitral insufficiency, aortic stenosis, aortic insufficiency, tricuspid stenosis, tricuspid insufficiency, pulmonary stenosis, pulmonary valve insufficiency, heart failure associated with combined heart valve defects, myocardial inflammation (myocarditis), chronic myocarditis, acute myocarditis, viral myocarditis, diabetic heart failure, alcoholic cardiomyopathy, heart failure associated with cardiac storage disorders, diastolic heart failure, systolic heart failure, acute phases of worsening heart failure, heart failure with preserved ejection fraction (HFpEF), heart failure with reduced ejection fraction (HFrEF), chronic heart failure with reduced ejection fraction (HFrEF), chronic heart failure with preserved ejection fraction (HFpEF), post myocardial remodeling, angina, hypertension, pulmonary hypertension and pulmonary artery hypertension.

"Fibrotic disorders" encompasses diseases and disorders characterized by fibrosis, including among others the following diseases and disorders: hepatic fibrosis, cirrhosis of the liver, NASH, pulmonary fibrosis or lung fibrosis, cardiac fibrosis, endomyocardial fibrosis, nephropathy, glomerulonephritis, interstitial renal fibrosis, fibrotic damage resulting from diabetes, bone marrow fibrosis and similar fibrotic disorders, scleroderma, morphea, keloids, hypertrophic scarring (also following surgical procedures), naevi, diabetic retinopathy, proliferative vitreoretinopathy and disorders of the connective tissue (for example sarcoidosis).

Relaxin-associated disorders include but are not limited to disorders of the cardiovascular system and fibrotic disorders.

The compounds of this invention can be administered by any suitable means, for example, orally, such as tablets, capsules (each of which includes sustained release or timed release formulations), pills, powders, granules, elixirs, tinctures, suspensions (including nanosuspensions, microsuspensions, spray-dried dispersions), syrups, and emulsions; sublingually; bucally; parenterally, such as by subcutaneous, intravenous, intramuscular, or intrasternal injection, or infusion techniques (e.g., as sterile injectable aqueous or non-aqueous solutions or suspensions); nasally, including administration to the nasal membranes, such as by inhalation spray; topically, such as in the form of a cream or ointment; or rectally such as in the form of suppositories. They can be administered alone, but generally will be administered with a pharmaceutical carrier selected on the basis of the chosen route of administration and standard pharmaceutical practice.

"Pharmaceutical composition" means a composition comprising a compound of the invention in combination with at least one additional pharmaceutically acceptable carrier. A "pharmaceutically acceptable carrier" refers to media generally accepted in the art for the delivery of biologically active agents to animals, in particular, mammals, including, *i.e.*, adjuvant, excipient or vehicle, such as diluents, preserving agents, fillers, flow regulating agents, disintegrating agents, wetting agents, emulsifying agents, suspending agents, sweetening agents, flavoring agents, perfuming agents, anti-bacterial agents, anti-fungal agents, lubricating agents and dispensing agents, depending on the nature of the mode of administration and dosage forms.

Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, e.g., stabilization of the active agent, binders, etc., well known to those of ordinary skill in the art. Descriptions of suitable pharmaceutically acceptable carriers, and factors involved in their selection, are found in a variety of readily available sources such as, for example, Allen, L.V., Jr. et al., Remington: The Science and Practice of Pharmacy (2 Volumes), 22nd Edition, Pharmaceutical Press (2012).

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired.

By way of general guidance, the daily oral dosage of each active ingredient, when used for the indicated effects, will range between about 0.01 to about 5000 mg per day, preferably between about 0.1 to about 1000 mg per day, and most preferably between about 0.1 to about 250 mg per day. Intravenously, the most preferred doses will range from about 0.01 to about 10 mg/kg/minute during a constant rate infusion. Compounds of this invention may be administered in a single daily dose, or the total daily dosage may be administered in divided doses of two, three, or four times daily.

The compounds are typically administered in admixture with suitable pharmaceutical diluents, excipients, or carriers (collectively referred to herein as pharmaceutical carriers) suitably selected with respect to the intended form of administration, *e.g.*, oral tablets, capsules, elixirs, and syrups, and consistent with conventional pharmaceutical practices.

Dosage forms (pharmaceutical compositions) suitable for administration may contain from about 1 milligram to about 2000 milligrams of active ingredient per dosage unit. In these pharmaceutical compositions the active ingredient will ordinarily be present in an amount of about 0.1-95% by weight based on the total weight of the composition. A typical capsule for oral administration contains at least one of the compounds of the present invention (250 mg), lactose (75 mg), and magnesium stearate (15 mg). The mixture is passed through a 60 mesh sieve and packed into a No. 1 gelatin capsule. A typical injectable preparation is produced by aseptically placing at least one of the compounds of the present invention (250 mg) into a vial, aseptically freeze-drying and sealing. For use, the contents of the vial are mixed with 2 mL of physiological saline, to produce an injectable preparation.

The compounds may be employed in combination with other suitable therapeutic agents useful in the treatment of diseases or disorders including: anti-atherosclerotic agents, anti-dyslipidemic agents, anti-diabetic agents, anti-hyperglycemic agents, anti-hyperinsulinemic agents, anti-thrombotic agents, anti-retinopathic agents, anti-neuropathic agents, anti-nephropathic agents, anti-ischemic agents, anti-hypertensive agents, anti-obesity agents, anti-hyperlipidemic agents, anti-hypertriglyceridemic agents, anti-hypercholesterolemic agents, anti-restenotic agents, anti-pancreatic agents, lipid lowering agents, anorectic agents, memory enhancing agents, anti-dementia agents, cognition promoting agents, appetite suppressants, agents for treating heart failure, agents for treating peripheral arterial disease, agents for treating malignant tumors, and anti-inflammatory agents.

The additional therapeutic agents may include ACE inhibitors, β-blockers, diuretics, mineralocorticoid receptor antagonists, ryanodine receptor modulators, SERCA2a activators, renin inhibitors, calcium channel blockers, adenosine A1 receptor agonists, partial adenosine A1 receptor, dopamine β-hydroxylase inhibitors, angiotensin II receptor antagonists, angiotensin II receptor antagonists with biased agonism for select cell signaling pathways, combinations of angiotensin II receptor antagonists and neprilysin enzyme inhibitors, neprilysin enzyme inhibitors, soluble guanylate cyclase activators, myosin ATPase activators, rho-kinase 1 inhibitors, rho-kinase 2 inhibitors, apelin receptor agonists, nitroxyl donating compounds, calcium-dependent kinase II inhibitors, antifibrotic agents, galectin-3 inhibitors, vasopressin receptor antagonists, FPR2 receptor modulators, natriuretic peptide receptor agonists, transient receptor potential vanilloid-4 channel blockers, anti-arrhythmic agents, If "funny current" channel blockers, nitrates, digitalis compounds, inotropic agents and β-receptor agonists, cell membrane resealing agents for example Poloxamer 188, anti-hyperlipidemic agents, plasma HDL-raising agents, anti-hypercholesterolemic agents, cholesterol biosynthesis inhibitors (such as HMG CoA reductase inhibitors), LXR agonist, FXR agonist, probucol, raloxifene, nicotinic acid, niacinamide, cholesterol absorption inhibitors, bile acid sequestrants, anion exchange resins, quaternary amines, cholestyramine, colestipol, low density lipoprotein receptor inducers, clofibrate, fenofibrate, bezafibrate, ciprofibrate, gemfibrizol, vitamin B6, vitamin B12, anti-oxidant vitamins, anti-diabetes agents, platelet aggregation inhibitors, fibrinogen receptor antagonists, aspirin and fibric acid derivatives, PCSK9 inhibitors, aspirin, and P2Y12 Inhibitors such as Clopidogrel.

The additional therapeutic agents may also include nintedanib, Pirfenidone, LPA1 antagonists, LPA1 receptor antagonists, GLP1 analogs, tralokinumab (IL-13, AstraZeneca), vismodegib (hedgehog antagonist, Roche), PRM-151 (pentraxin-2, TGF beta-1, Promedior), SAR-156597 (bispecific Mab IL-4&IL-13, Sanofi), simtuzumab ((anti-lysyl oxidase-like 2 (anti-LOXL2) antibody, Gilead), CKD-942, PTL-202 (PDE inh./pentoxifylline/NAC oral control. release, Pacific Ther.), omipalisib (oral PI3K/mTOR inhibitor, GSK), IW-001 (oral sol. bovine type V collagen mod., ImmuneWorks), STX-100 (integrin alpha V/ beta-6 ant, Stromedix/ Biogen), Actimmune (IFN gamma), PC-SOD (midismase; inhaled, LTT Bio-Pharma / CKD Pharm), lebrikizumab (anti-IL-13 SC humanized mAb, Roche), AQX-1125 (SHIP1 activator, Aquinox), CC-539 (JNK inhibitor, Celgene), FG-3019 (FibroGen), SAR-100842 (Sanofi), and obeticholic acid (OCA or INT-747, Intercept).

The above other therapeutic agents, when employed in combination with the compounds of the present invention may be used, for example, in those amounts indicated in the *Physicians' Desk Reference*, as in the patents set out above, or as otherwise determined by practitioners in the art.

Particularly when provided as a single dosage unit, the potential exists for a chemical interaction between the combined active ingredients. For this reason, when the compound of the present invention and a second therapeutic agent are combined in a single dosage unit they are formulated such that although the active ingredients are combined in a single dosage unit, the physical contact between the active ingredients is minimized (that is, reduced). For example, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. One of the active ingredients may also be coated with a material that affects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low viscosity grade of hydroxypropyl methylcellulose (HPMC) or other appropriate materials as known in the art, in order to further separate the active components. The polymer coating serves to form an additional barrier to interaction with the other component.

The compounds of the present invention are also useful as standard or reference compounds, for example as a quality standard or control, in tests or assays involving RXFP1. Such compounds may be provided in a commercial kit, for example, for use in pharmaceutical research involving RXFP1. For example, a compound of the present invention could be used as a reference in an assay to compare its known activity to a compound with an unknown activity. This would ensure the experimenter that the assay was being performed properly and provide a basis for comparison, especially if the test compound was a derivative of the reference compound. When developing new assays or protocols, compounds according to the present invention could be used to test their effectiveness. The compounds of the present invention may also be used in diagnostic assays involving RXFP1.

The present invention also encompasses an article of manufacture. As used herein, article of manufacture is intended to include, but not be limited to, kits and packages. The article of manufacture of the present invention, comprises: (a) a first container; (b) a pharmaceutical composition located within the first container, wherein the composition, comprises a first therapeutic agent, comprising a compound of the present invention or a pharmaceutically acceptable salt form thereof; and, (c) a package insert stating that the pharmaceutical composition can be used for the treatment of dyslipidemias and the sequelae thereof. In another embodiment, the package insert states that the pharmaceutical composition can be used in combination (as defined previously) with a second therapeutic agent for the treatment of dyslipidemias and the sequelae thereof. The article of manufacture can further comprise: (d) a second container, wherein components (a) and (b) are located within the second container and component (c) is located within or outside of the second container. Located within the first and second containers means that the respective container holds the item within its boundaries.

The first container is a receptacle used to hold a pharmaceutical composition. This container can be for manufacturing, storing, shipping, and/or individual/bulk selling. First container is intended to cover a bottle, jar, vial, flask, syringe, tube *(e.g.,* for a cream preparation), or any other container used to manufacture, hold, store, or distribute a pharmaceutical product.

The second container is one used to hold the first container and, optionally, the package insert. Examples of the second container include, but are not limited to, boxes (*e.g.,* cardboard or plastic), crates, cartons, bags (*e.g.,* paper or plastic bags), pouches, and sacks. The package insert can be physically attached to the outside of the first container via tape, glue, staple, or another method of attachment, or it can rest inside the second container without any physical means of attachment to the first container. Alternatively, the package insert is located on the outside of the second container. When located on the outside of the second container, it is preferable that the package insert is physically attached via tape, glue, staple, or another method of attachment. Alternatively, it can be adjacent to or touching the outside of the second container without being physically attached.

The package insert is a label, tag, marker, etc. that recites information relating to the pharmaceutical composition located within the first container. The information recited will usually be determined by the regulatory agency governing the area in which the article of manufacture is to be sold *(e.g.,* the United States Food and Drug Administration). Preferably, the package insert specifically recites the indications for which the pharmaceutical composition has been approved. The package insert may be made of any material on which a person can read information contained therein or thereon. Preferably, the package insert is a printable material (e.g., paper, plastic, cardboard, foil, adhesive-backed paper or plastic, etc.) on which the desired information has been formed (e.g., printed or applied).

### CHEMICAL METHODS

The compounds of this invention can be made by various methods known in the art including those of the following schemes and in the specific embodiments section. The structure numbering and variable numbering shown in the synthetic schemes are distinct from, and should not be confused with, the structure or variable numbering in the claims or the rest of the specification. The variables in the schemes are meant only to illustrate how to make some of the compounds of this invention.

It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene, T.W. et al., Protecting Groups in Organic Synthesis, 4th Edition, Wiley (2007)).

Abbreviations are defined as follows: "1 x" for once, "2 x" for twice, "3 x" for thrice, " °C" for degrees Celsius, "aq" for aqueous, "eq" or "equiv." for equivalent or equivalents, "g" for gram or grams, "mg" for milligram or milligrams, "L" for liter or liters, "mL" for milliliter or milliliters, "µL" for microliter or microliters, "N" for normal, "M" for molar, "nM" for nanomolar, "pM" for picomolar, "mol" for mole or moles, "mmol" for millimole or millimoles, "min" for minute or minutes, "h" for hour or hours, "rt" for room temperature, "RT" for retention time, "atm" for atmosphere, "psi" for pounds per square inch, "conc." for concentrate, "aq" for "aqueous", "sat." for saturated, "MW" for molecular weight, "MS" or "Mass Spec" for mass spectrometry, "ESI" for electrospray ionization mass spectroscopy, "LC-MS" for liquid chromatography mass spectrometry, "HPLC" for high pressure liquid chromatography, "RP HPLC" for reverse phase HPLC, "NMR" for nuclear magnetic resonance spectroscopy, "SFC" for super critical fluid chromatography, "¹H" for proton, "δ " for delta, "s" for singlet, "d" for doublet, "t" for triplet, "q" for quartet, "m" for multiplet, "br" for broad, "Hz" for hertz, "MHz" for megahertz, and "α", "β", "R", "S", "E", and "Z" are stereochemical designations familiar to one skilled in the art.

| | |
|---|---|
| AcCl | acetyl chloride |
| AcOH | acetic acid |
| AIBN | Azobisisobutyronitrile |
| Boc | tert-butyloxycarbonyl |
| BuLi | butyl lithium |
| DCE | Dichloroethane |
| DCM | Dichloromethane |
| DIEA | diispropyl ethylamine |
| DMAP | 4-dimethylamino pyridine |
| DMF | Dimethylformamide |
| DPPA | Diphenyl phosphoryl azide |
| Et₂O | diethyl ether |
| EtOAc | Ethylacetate |
| EtOH | Ethanol |
| HATU | (1-[Bis(dimethylamino)methylene]-1H-1,2,3-triazolo[4,5-b]pyridinium 3-oxid hexafluorophosphate) |
| HMPA | hexamethylphosphramide |
| IPA | isopropanol |
| i-Pr | Isopropyl |
| KHMDS | potassium bis(trimethylsilyl)amide\ |
| LDA | lithium diisopropyl amide |
| MeCN | Acetonitrile |
| MeOH | Methanol |
| Me | Methyl |
| NBS | N-bromosuccinimide |
| NCS | N-chlorosuccinimide |
| Pd/C | palladium on carbon |
| pTsOH | p-toluenesulfonic acid |
| PyBroP | Bromotripyrrolidinophosphonium hexafluorophosphate |
| T3P | 2,4,6-Tripropyl-1,3,5,2,4,6-trioxatriphosphorinane-2,4,6-trioxide |
| TBAF | tetra-n-butyl ammonium fluoride |
| t-Bu | tert-butyl |
| Teoc | 2-(trimethyl silyl)ethyl |
| TFA | trifluoro acetic acid |
| TFAA | trifluoro acetic anhydride |
| THF | Tetrahydrofuran |
| XPhos-Pd-G2 | 2nd generation XPhos precatalyst CAS no. 1310584-14-5 |
| XPhos-Pd-G3 | 3rd generation XPhos precatalyst CAS no. 14445085-55-1 |

The following general methods were used in the exemplified examples, except where noted otherwise. Purification of intermediates and final products was carried out via either normal or reverse phase chromatography. Normal phase chromatography was carried out using prepacked SiO₂ cartridges eluting with either gradients of hexanes and ethyl acetate or DCM and MeOH unless otherwise indicated. Reverse phase preparative HPLC was carried out using C18 columns with UV 220 nm or prep LCMS detection eluting with gradients of Solvent A (90% water, 10% MeOH, 0.1% TFA) and Solvent B (10% water, 90% MeOH, 0.1% TFA) or with gradients of Solvent A (95% water, 5% ACN, 0.1% TFA) and Solvent B (5% water, 95% ACN, 0.1% TFA) or with gradients of Solvent A (95% water, 2% ACN, 0.1% HCOOH) and Solvent B (98% ACN, 2% water, 0.1% HCOOH) or with gradients of Solvent A (95% water, 5% ACN, 10 mM NH₄OAc) and Solvent B (98% ACN, 2% water, 10 mM NH₄OAc) or with gradients of Solvent A (98% water, 2% ACN, 0.1% NH₄OH) and Solvent B (98% ACN, 2% water, 0.1% NH₄OH). Stereoisomer separations were achieved in > 95% ee or de.

LC/MS methods employed in characterization of examples are listed below.

### Method A:

Instrument: Waters Acquity coupled with a Waters MICROMASS^{®} ZQ Mass Spectrometer
Linear gradient of 2 to 98% B over 1 min, with 0.5 min hold time at 98% B UV visualization at 220 nm
Column: Waters BEH C18, 2.1 x 50 mm
Flow rate: 0.8 mL/min (Method A)
Mobile Phase A: 0.05% TFA, 100% water
Mobile Phase B: 0.05% TFA, 100% acetonitrile

### Method B:

Instrument: Shimadzu Prominence HPLC coupled with a Shimadzu LCMS-2020 Mass Spectrometer
Linear gradient of 0 to 100% B over 3 min, with 0.75 min hold time at 100% B UV visualization at 220 nm
Column: Waters Xbridge C18, 2.1 x 50 mm, 1.7 um particles
Flow rate: 1 mL/min
Mobile Phase A: 10 mM ammonium acetate, 95:5 water:acetonitrile
Mobile Phase B: 10 mM ammonium acetate, 5:95 water:acetonitrile

### Method C:

Instrument: Shimadzu Prominence HPLC coupled with a Shimadzu LCMS-2020 Mass Spectrometer
Linear gradient of 0 to 100% B over 3 min, with 0.75 min hold time at 100% B UV visualization at 220 nm
Column: Waters Xbridge C18, 2.1 x 50 mm, 1.7 um particles
Flow rate: 1 mL/min
Mobile Phase A: 0.1% TFA, 95:5 water:acetonitrile
Mobile Phase B: 0.1% TFA, 5:95 water:acetonitrile

### Method D:

Instrument: Waters Acquity coupled with a Waters MICROMASS^{®} ZQ Mass Spectrometer
Linear gradient of 10% B to 98% B over 1 min, with 0.5 min hold time at 98% B UV visualization at 220 nm
Column: Waters Acquity GEN C18, 2.1 x 50 mm, 1.7 um particles
Flow rate: 1 mL/min
Mobile Phase A: 0.05% TFA, 100% water
Mobile Phase B: 0.05% TFA, 100% acetonitrile

NMR Employed in Characterization of Examples. ¹H NMR spectra were obtained with Bruker or JEOL^{®} Fourier transform spectrometers operating at frequencies as follows: ¹H NMR: 400 MHz (Bruker or JEOL^{®}) or 500 MHz (Bruker or JEOL^{®}). Spectra data are reported in the format: chemical shift (multiplicity, coupling constants, number of hydrogens). Chemical shifts are specified in ppm downfield of a tetramethylsilane internal standard (δ units, tetramethylsilane = 0 ppm) and/or referenced to solvent peaks, which in ¹H NMR spectra appear at 2.51 ppm for DMSO-d₆, 3.30 ppm for CD₃OD, 1.94 ppm for CD₃CN, and 7.24 ppm for CDCl₃.

Syntheses of key norbornyl intermediates are outlined in Schemes I-XI. Norbornyl intermediates can be made with isopropylidene bridgehead substitution as described in Scheme 1, starting from **I-1**. Diels-Alder cyclization with maleic anhydride furnished compound **I-2**, which was reduced and anhydride opened with methanol to yield **I-3**. Curtius reaction with DPPA on the free acid in the presence of trimethylsilylethanol produced **I-4**. Enatiomers of **I-4** were separated and the trimethylsilylcarbamate was cleaved with TFA and the amine reprotected as the trifluoroacetamide **I-5.** The methyl ester was converted to the amide via treatment with 4-fluoro-3-trifluoromethylaniline and trimethyl aluminum to furnish **I-6**. Deprotection of the trifluoroacetamide using K₂CO₃ and MeOH produced amine **I-7**.

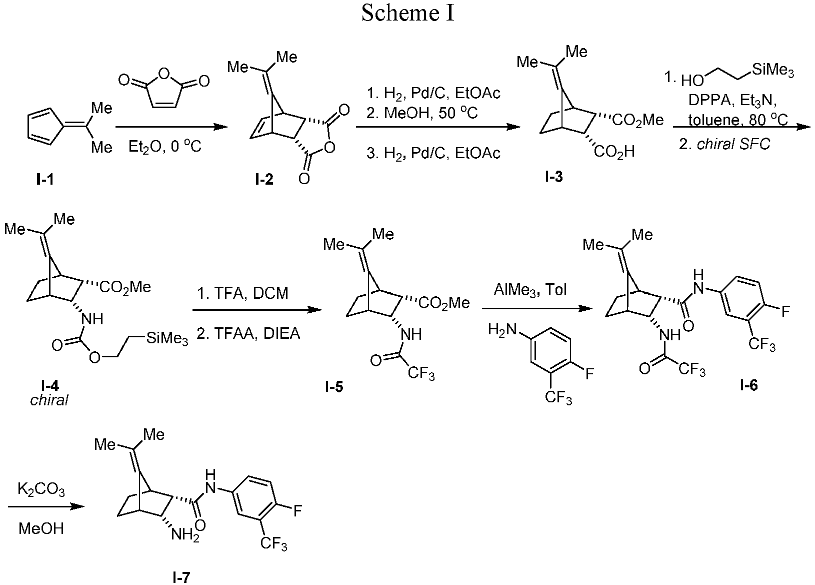

Intermediate **I-2:** At 0 °C, into the reaction vessel was added Et₂O (100 mL), 5-(propan-2-ylidene)cyclopenta-1,3-diene (10 g, 94 mmol), and furan-2,5-dione (10 g, 100 mmol). The reaction mixture was stirred at 0 °C for 18 h, concentrated under reduced pressure and purified via silica gel chromatography to provide **I-2** (3.74 g, 18.3 mmol, 19.0% yield). Intermediate **I-2** is a known compound; please see: PCT Int. Appl., 2011163502, 29 Dec 2011.

Intermediate **I-3:** Into the reaction vessel was added **I-2** (2.74 g, 13.4 mmol), EtOAc (100 mL), pyridine (0.540 mL, 6.71 mmol), and Pd/C (70 mg, 0.070 mmol). The reaction mixture was stirred at 23 °C under 1 atm H₂ (H₂ balloon) for 60 min filtered through Celite, and concentrated under reduced pressure. The residue was dissolved in MeOH (50 mL) and heated at 50 °C for 12 h. The solution was concentrated under reduced pressure (azeotroped with toluene 3 x 15 mL) to produce **I-3** (3.21 g, 13.5 mmol, 100% yield) that was used without further purification.

Intermediate **I-4**: Into the reaction vessel was added **I-3** (3.21 g, 13.5 mmol), Et₃N (3.38 mL, 24.2 mmol), toluene (75 mL), and diphenylphosphoryl azide (4.35 mL, 20.2 mmol). The reaction mixture was stirred at 23 °C for 1 h and subsequently heated at 85 °C for 30 min. 2-(trimethylsilyl)ethanol (4.83 mL, 33.7 mmol) was added to the reaction mixture and, after stirring at 85 °C for 66 h, the reaction mixture was allowed to cool to 23 °C and purified via silica gel chromatography to provide racemic **I-4** (3.71 g, 10.5 mmol, 78% yield) LC-MS RT = 1.25 min; (M+H) = 354.1. Method A. Racemic **I-4** was separated into individual enantiomers using chiral SFC. Preparative chromatographic conditions: Instrument: Thar 350 SFC; Column: Whelko-RR, 5 x 50 cm, 10 micron; Mobile phase: 13% IPA/87% CO₂; Flow conditions: 300 mL/min, 100 Bar, 35 °C; Detector wavelength: 220 nm; Injections details: 4 injections of 3.5 mL of 59 g / 490 mL MeOH:DCM (4:1) 120 mg/mL in IPA. Analytical chromatographic conditions: Instrument: Thar analytical SFC; Column: Whelko-RR (0.46 x 25 cm, 5 micron; Mobile phase: 5% IPA/95% CO₂; Flow conditions: 3 mL/min, 140 Bar, 40 °C; Detector wavelength: 200-400 nm UV; RT = 3.50 Peak #1, 4.42 Peak #2. Intermediate **I-4** product Peak #1 was collected and carried forward to produce chiral **I-5**.

Intermediate **I-5**: Peak #1 of intermediate **I-4** (2.87 g, 8.12 mmol) was dissolved in 10:1 DCM/TFA and stirred at rt for 72 h. The reaction mixture was concentrated under reduced pressure to generate (1R,2S,3R,4R)-methyl 3-amino-7-(propan-2-ylidene)bicyclo[2.2.1]heptane-2-carboxylate (1.699 g, 8.120 mmol, 100 % yield) which was used without further purification. To (1R,2S,3R,4R)-methyl 3-amino-7-(propan-2-ylidene)bicyclo[2.2.1]heptane-2-carboxylate (1.7 g, 8.1 mmol) was added DCM (41 mL) and the flask was cooled to 0 °C via an ice bath. TFAA (1.26 mL, 8.90 mmol) and DIEA (5.7 mL, 33 mmol) were added. The reaction mixture was allowed to warm to 23 °C and stirred for 30 min. Saturated NaHCO₃ (50 mL) was added to the reaction mixture and the solution extracted with EtOAc (3 x 50 mL). The combined organic portions were dried over Na₂SO₄ filtered and concentrated under reduced pressure to afford **I-5** (2.48 g, 8.12 mmol, 100% yield) that was used without further purification. LC-MS RT = 1.11 min; MS (ESI) m/z = 306.1 (M+H)⁺; Method A.

Intermediate **I-6**: To a solution of intermediate **I-5** (2.7 g, 8.8 mmol) in toluene (88 ml) was added trimethylaluminum (26.5 mmol) premixed with 4-fluoro-3(trifluoromethyl)aniline (29.2 mmol) as a solution in toluene (0.275 M in amine, 0.25 M in trimethylaluminum). The reaction mixture was stirred at 60 °C for 30 min. On cooling to rt, the reaction mixture was diluted with EtOAc (100 mL) and sat. Rochelle's salt (100 mL) added. The aqueous portion was extracted with EtOAc (3 x 75 mL). The combined organic portion was dried over Na₂SO₄, filtered, concentrated under reduced pressure, and subjected to silica gel chromatography purification and the residue further purified by preparative reverse phase HPLC to yield (1R,2S,3R,4R)-N-(4-fluoro-3-(trifluoromethyl)phenyl)-7-(propan-2-ylidene)-3-(2,2,2-trifluoroacetamido)bicyclo[2.2.1]heptane-2-carboxamide **I-6** (2.5 g, 5.5 mmol, 63 % yield) as a yellow foam. LC-MS RT = 1.20 min; MS (ESI) m/z = 453.0 (M+H)⁺; Method A.

Intermediate **I-7**: Intermediate **I-6** (133 mg, 0.290 mmol) was dissolved in water (2.9 mL) and MeOH (2.9 mL), then K₂CO₃ (2.03 g, 1.47 mmol) was added. The reaction mixture was stirred at 40 °C for 4 h, then partitioned between water (5 mL) and extracted with EtOAc (3 x 10 mL). The combined organic extracts were dried over Na₂SO₄ filtered and concentrated under reduced pressure to afford 1-7 (105 mg, 0.290 mmol, 100% yield) that was used without further purification. LC-MS RT = 0.82 min; MS (ESI) m/z = 357.1 (M+H)⁺; Method A.

### Scheme Ia

The norbornyl intermediate IIa-8 could also be prepared by the general route shown in Scheme Ia from furan-2,5-dione and ferrocenium hexafluorophosphate. Diels Alder condensation, followed by hydrolysis to IIa-2, Curtius rearrangement to the intermediate amine which was reduced under hydrogenation conditions and subsequently protected to generate intermediate IIa- 3. Cleavage of the benzyl ester and cross coupling to NHR₁R₂ generated intermediates with the general structure IIa-5. Conversion of the C7 hydroxy group to the ketone followed by Wittig olefination generated major isomer intermediate IIa-8. The major isomer was separated from the minor isomer by chromatography and the racemate separated into enantiopure IIa-8 (-).

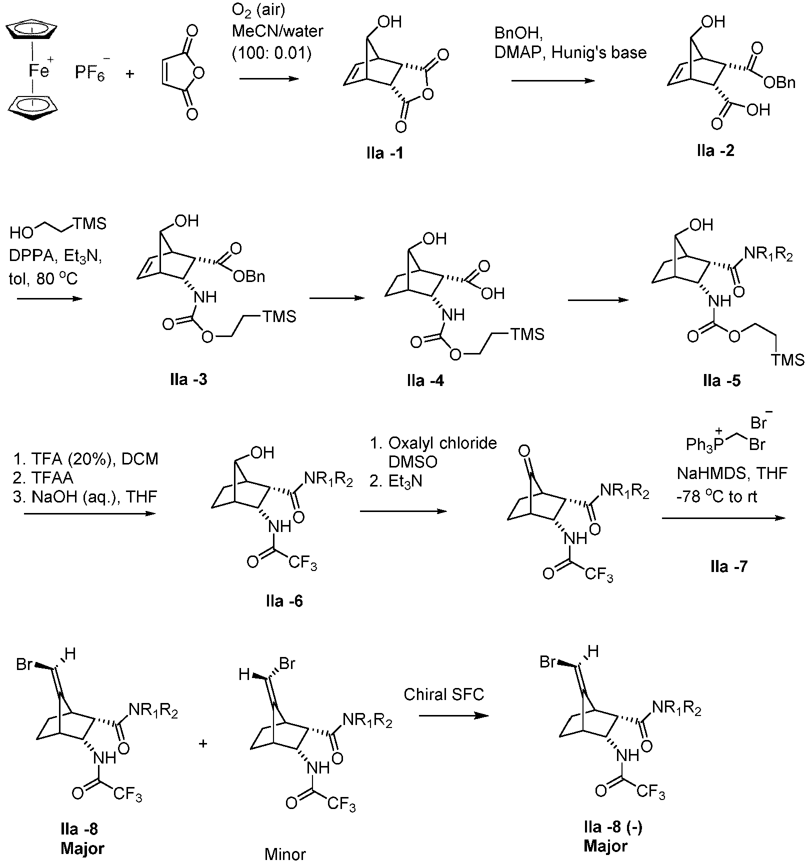

Scheme II utilizes elaboration via ozonolysis and Horner-Wadsworth Emmons (although functionalizations could proceed with a variety of reagents such as alkyl lithium, alkyl magnesium, Wittig reaction, not limited to these) and a bridgehead substituted olefinic bromide that allows for Suzuki cross-coupling reactions on more advanced intermediates. This scheme demonstrates this process on more advanced intermediates that contain substituted heteroaryl functionality at the R³ position.

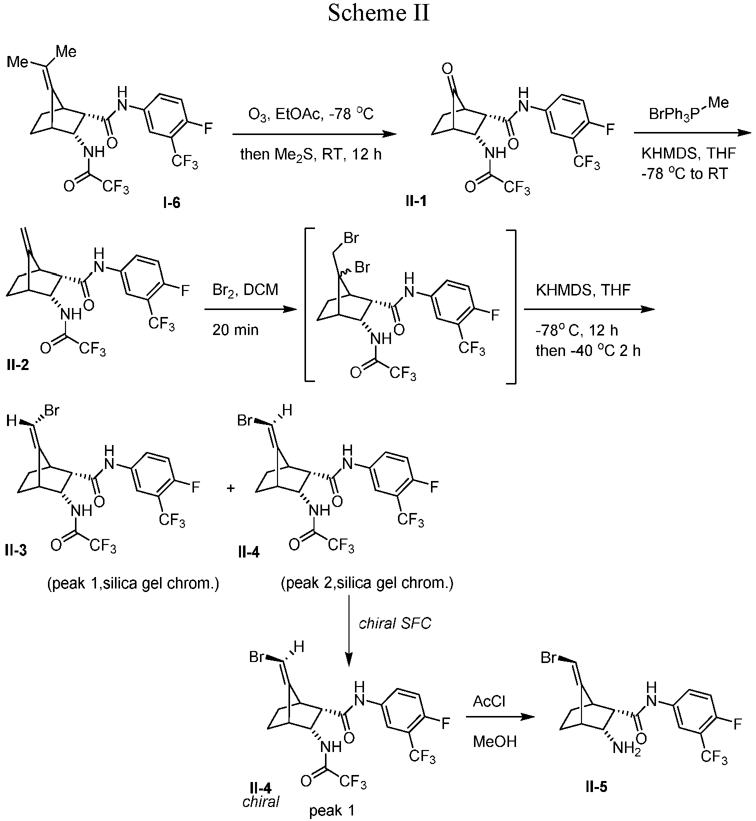

Intermediate **II-1** Into the reaction vessel was added intermediate **I-6** (110 mg, 0.243 mmol) and EtOAc (2 mL). The reaction mixture was cooled to -78 °C and O₃ was bubbled through the solution until the solution became pale purple/blue. N₂ was subsequently bubbled through the solution at -78 °C to remove excess O₃ (solution became colorless). Dimethyl sulfide (0.43 mL, 4.8 mmol) was subsequently added at -78 °C and the reaction mixture was allowed to warm to rt and stirred at rt for 12h. After concentrating under reduced pressure, the residue was dissolved in EtOAc and filtered through silica gel to generate(1R,2S,3R,4S)-N-(4-fluoro-3-(trifluoromethyl)phenyl)-7-oxo-3-(2,2,2-trifluoroacetamido)bicyclo[2.2.1]heptane-2-carboxamide after removal of solvent under reduced pressure, (**II-1,** 101 mg, 0.237 mmol, 97.0 % yield). ¹H NMR (500 MHz, CDCl₃) δ 9.61 (br d, J=6.3 Hz, 1H), 7.76 (dd, J=5.9, 2.6 Hz, 1H), 7.71 (dt, J=8.9, 3.4 Hz, 1H), 7.66 (s, 1H), 7.23 (t, J=9.4 Hz, 1H), 4.70 (dt, J=10.3, 5.3 Hz, 1H), 3.33 (dd, J=10.5, 4.4 Hz, 1H), 2.54 (t, J=4.3 Hz, 1H), 2.42 (t, J=4.1 Hz, 1H), 2.20 - 2.10 (m, 1H), 2.06 - 1.99 (m, 1H), 1.96 - 1.81 (m, 2H).

Intermediate **II-2**: Into the reaction vessel was added bromo(methyl)triphenylphosphorane (419 mg, 1.17 mmol) (fine powder by grinding the commercial material) and THF (7 mL). The reaction mixture was cooled to -78 °C and KHMDS (1.2 mL, 1.2 mmol) was added. The reaction mixture was allowed to stir vigorously at -78 °C for 30 min and **II-1** (100 mg, 0.24 mmol) was added at -78 °C. After stirring at -78 °C for a further 10 min, the reaction mixture was allowed to warm to 23 °C and stirred for a further 1.5 h. The reaction mixture was cooled to -40 °C and quenched by the addition of sat. NaHCO₃. The resulting solution was extracted with EtOAc. The combined organic portion was dried over Na₂SO₄, filtered, concentrated under reduced pressure, and purified via silica gel chromatography to produce **II-2** (71 mg, 0.17 mmol, 71% yield). LCMS RT = 1.16 min; (M+H) = 425.0; Method A.

Intermediates **II-3** and **II-4:** Into the reaction vessel was added **II-2** (71 mg, 0.17 mmol), DCM (3 mL), and Br₂ (0.03 mL, 0.6 mmol). The reaction mixture was stirred at 23 °C for 20 min and concentrated under reduced pressure utilizing a trap with sat. Na₂S₂O₃ to quench excess Br₂. The resulting dibromide was dissolved in THF (3 mL). After cooling to -78 °C, KHMDS (1.0 mL, 1.0 mmol) was added. The reaction mixture was kept at -78 °C for 12 h and -40 °C for 2 h, quenched by the addition of sat. NaHCO₃ at -40 °C, and the resulting solution extracted with EtOAc. The organic phase was collected, dried over Na₂SO₄, filtered, concentrated under reduced pressure, and purified via silica gel chromatography to produce **II-4** (27 mg, 0.050 mmol, 32% yield) (peak2). LCMS RT = 1.19 min; (M+H) = 504.9; Method A. and the corresponding E-isomer **II-3** (28 mg, 0.06 mmol, 33% yield) (peak 1).

Racemic **II-4** (4 grams) was produced as outlined above and separated into individual enantiomers using chiral SFC. Preparative chromatographic conditions: Instrument: Thar 350 SFC; Column: Chiralcel OD-H, 5 x 50 cm, 5 micron; Mobile phase: 20% MeOH/80% CO₂; Flow conditions: 340 mL/min, 100 Bar, 35 °C; Detector wavelength: 220 nm; Injections details: 3.75 mL of 30 mg/mL in MeOH. Peak # 1 RT = 7.81 min, Peak #2 RT = 10.97 min. Peak #1 of **II-4** (1.9 grams) was collected and carried forward to produce chiral **II-5.**

Intermediate **II-5:** Into the reaction was added MeOH (3 mL) and AcCl (0.3 mL, 4.2 mmol). After stirring for 5 min, chiral Peak #1 **II-4** (75 mg, 0.15 mmol) was added and the reaction mixture was stirred at 40 °C for 48 h. The reaction mixture was concentrated under reduced pressure produced **II-5** (67 mg, 0.16 mmol, 100%) that was used without further purification. LC-MS RT = 0.78 min; (M+H) = 408.9; Method A.

Scheme III demonstrates the diversification possible with a variety of reagents for example, alkyl lithium, alkyl magnesium, Wittig reaction, Horner-Wadsworth Emmons, but not limited to these.

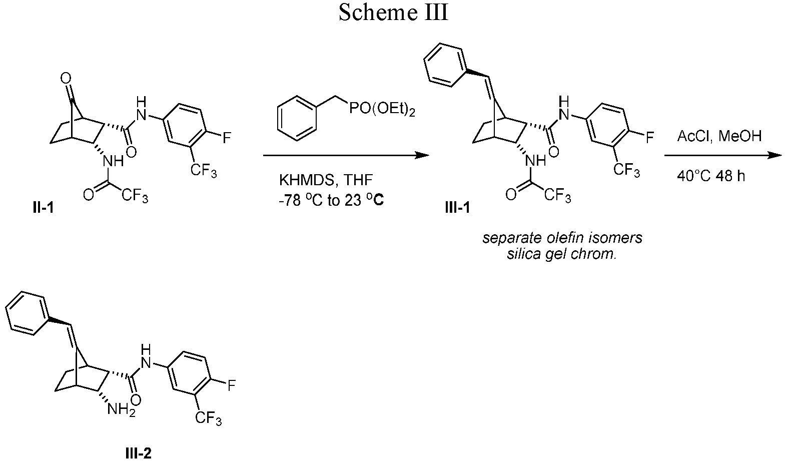

Intermediate **III-1:** In to the reaction vessel was added diethyl benzylphosphonate (375 mg, 1.64 mmol) and THF (10 mL). The reaction mixture was cooled to -78 °C and KHMDS (1.6 mL, 1.6 mmol) was added. This reaction mixture was stirred at -78 °C for 10min and intermediate **II-1** (140 mg, 0.328 mmol) was added. After 20 min, the reaction mixture was allowed to warm to rt and stirred at rt for 2h. The reaction mixture was quenched by the addition of sat NaHCO₃ and the solution extracted with EtOAc. The combined organic portion was dried over Na₂SO₄, filtered, concentrated, and subjected to silica gel chromatography purification to the E-isomer byproduct (111 mg, 0.222 mmol, 67.5 % yield) and (1R,2S,3R,4R,Z)-7-benzylidene-N-(4-fluoro-3-(trifluoromethyl)phenyl)-3-(2,2,2-trifluoroacetamido)bicyclo[2.2.1]heptane-2-carboxamide (**III-1,** 49 mg, 0.098 mmol, 30 % yield). RT = 1.23 min; MS (ESI) m/z = *501.1* (M+H)⁺; Method A.

Intermediate **III-2**: To a vial containing MeOH (3 mL) cooled to 0 °C (ice/water bath) was added acetyl chloride (0.3 mL, 4 mmol) dropwise. The resulting solution was stirred at rt for 10 min, then was added to **III-1** (94 mg, 0.19 mmol). The reaction mixture was stirred at 40 °C for 48 h and concentration under reduced pressure afforded (1R,2S,3R,4R)-3-amino-7-((Z)-benzylidene)-N-(4-fluoro-3-(trifluoromethyl)phenyl)bicyclo[2.2.1]heptane-2-carboxamide (**III-2,** 73 mg, 0.18 mmol, 96 % yield). RT = 0.87 min; MS (ESI) m/z = 405.1 (M+H)⁺; Method A which was used without further purification

Scheme IV demonstrates installation of cycloalkyl and heterocycle bridgehead functionality in similar manner as scheme II, in this example, isoxazole and cyclopropyl groups, but not limited to these groups.

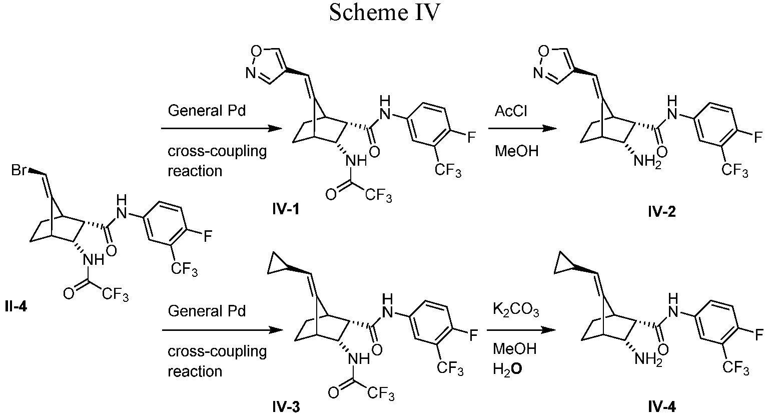

Intermediate **IV-1**: Into the reaction vessel containing **II-4** (125 mg, 0.250 mmol) was added 4-(4,4,5,5-tetramethyl-1,3,2-dioxaborolan-2-yl)isoxazole (125 mg, 0.610 mmol), PdCl₂(dppf)-CH₂Cl₂ adduct (50.7 mg, 0.0620 mmol), and Na₂CO₃ (1.5 mL, 3.00 mmol). The reaction mixture was degassed with nitrogen for 3 min, sealed, and stirred at 60 °C for 2 h. After cooling to 23 °C, the reaction mixture was extracted with EtOAc, dried over Na₂SO₄, concentrated under reduced pressure, and purified via silica gel chromatography to produce **IV-1** (100 mg, 0.21 mmol, 83% yield). LC-MS RT = 1.07 min; MS (ESI) m/z = 492.1 (M+H)⁺; Method A.

Intermediate **IV-2**: Intermediate **IV-2** was prepared from **IV-1** in the same manner as intermediate **III-2** in scheme III (67 mg, 0.16 mmol, 100% yield). RT = 0.76 min; MS (ESI) m/z = 396.0 (M+H)⁺; Method A.

Intermediate **IV-3**: Intermediate **IV-3** was prepared from **II-4** in the same manner as intermediate **IV-1** in scheme IV (5.1 mg, 0.01 mmol, 23% yield). RT = 1.21 min; MS (ESI) m/z = 465.1 (M+H)⁺; Method A.

Intermediate **IV-4**: Intermediate **IV-4** was prepared from **IV-3** in the same manner as intermediate **I-7** in scheme I (4.0 mg, 0.01 mmol, 100% yield). RT = 0.84 min; MS (ESI) m/z = 369.1 (M+H)⁺; Method A.

Scheme V demonstrates functionalizing the C7 position with a trifluoromethyl group.

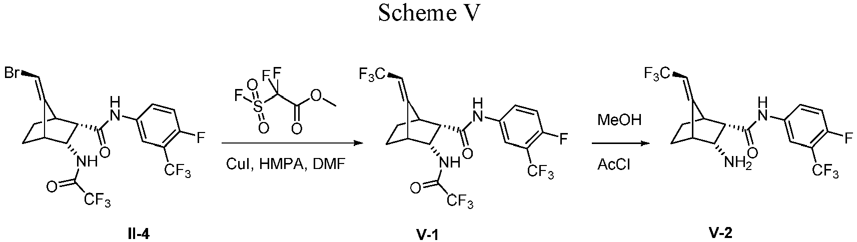

Intermediate **V-1**: Intermediate **V-1** was prepared from **II-4**. To a 250 mL round bottom flask charged with methyl 2,2-difluoro-2-(fluorosulfonyl)acetate (2.5 mL, 20 mmol) in anhydrous DMF (50 mL) was added dropwise via dropping funnel to a suspension of **II-4** and CuI (2.3 g, 12 mmol) in anhydrous DMF (100 mL) and HMPA (8.0 mL, 19 mmol) and the reaction mixture heated at 75 °C under an inert nitrogen atmosphere for 16 h. The cooled reaction mixture was filtered and purified by silica gel chromatography to produce **V-1** (3.0 g, 6.1 mmol, 77% yield). 1H NMR (500 MHz, CDCl₃) δ 9.38 (br d, J=6.1 Hz, 1H), 7.77 - 7.69 (m, 2H), 7.46 (s, 1H), 7.24 (t, J=9.1 Hz, 1H), 5.62 (q, J=7.2 Hz, 1H), 4.50 (dt, J=10.5, 5.3 Hz, 1H), 3.50 - 3.42 (m, 1H), 3.13 - 3.04 (m, 1H), 2.89 (t, J=4.0 Hz, 1H), 2.02 - 1.90 (m, 2H), 1.76 - 1.60 (m, 2H).

Intermediate **V-2**: Intermediate **V-2** was prepared from **V-1**. MeOH (1.5 mL) and acetyl chloride (2.1 mmol) were charged into a 2 dram vial and stirred at 23 °C for 5 min. **V-1** was added to the reaction vial and the contents heated at 40 °C for 24 h. Concentration with a stream of nitrogen gave **V-2** as the HCl salt which was used without further purification. LC-MS RT = 0.75 min; MS (ESI) m/z = 397.1 (M+H)⁺; Method A.

Scheme VI demonstrates a general method of preparing heterocyclic carboxylic acids for amide to coupling with intermediates for example, **V-2** utilizing coupling agents such as HATU (not limited to). Additionally the carboxylic acids can be assembled in an alternate order or using an alternate protection strategy.

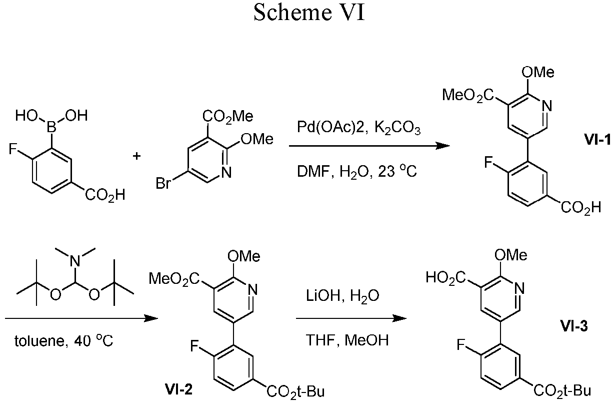

Intermediate **VI-1**: A 20 mL vial was charged with methyl 5-bromo-2-methoxynicotinate (0.150 g, 0.610 mmol), 3-borono-4-fluorobenzoic (0.168 g, 0.910 mmol), Pd(OAc)₂ (0.013 g, 0.061 mmol), K₂CO₃ (0.253 g, 1.80 mmol), followed by water (0.120 mL), and DMF (6 mL). The reaction mixture was stirred for 18 h at 23 °C. After quenching the reaction mixture by the addition of HCl (20 mL, 1.0 M) and extracting with ethyl acetate (3 x 20 mL), the combined organic portion was dried over Na₂SO₄ filtered and concentrated under reduced pressure. The residue was purified via preparative RP-HPLC to produce intermediate **VI-1** (120 mg, 0.41 mmol, 67% yield). LC-MS RT = 0.82 min; MS (ESI) m/z = 306.1 (M+H)⁺; Method A.

Intermediate **VI-2**: To a 20 mL vial charged with **VI-1** (0.120 mg 0.41 mmol) in toluene (4 mL), was added 1,1-di-tert-butyoxy-N,N-dimethylmethanamine (0.49 mL, 2.0 mmol). The reaction mixture was heated at 40 °C for 2 days. The reaction mixture was concentrated under reduced pressure onto silica gel and purified by normal phase column chromatography to give **VI-2** (110 mg, 0.31 mmol, 76%). LC-MS RT = 1.15 min; MS (ESI) m/z = 362.1 (M+H)⁺; Method A.

Intermediate **VI-3**: To a vial containing **VI-2** (56 mg, 0.16 mmol) in THF (0.89 mL)/water (0.44 mL)/MeOH (0.22 mL) was added a 1N aqueous solution of lithium hydroxide (1N, 0.78 mL, 0.78 mmol) and the reaction mixture was stirred at rt for 18 hr, then diluted with 1N HCl (5 mL) and the solution extracted with EtOAc (3 x 5 mL). The combined organic portions were dried over Na₂SO₄ filtered and concentrated under reduced pressure to afford 5-(5-(tert-butoxycarbonyl)-2-fluorophenyl)-2-methoxynicotinic acid (54 mg, 0.16 mmol, 100 % yield). LC-MS RT = 1.02 min; MS (ESI) m/z = 348.1 (M+H)⁺; Method A.

Alternatively various heteroaryl halides and aryl boronic acid or ester could be cross coupled by the general route shown in Scheme VI to yield aryl-pyrimidine and isomeric pyridines, for example but not limited to. In addition, cross coupling may be accomplished with aliphatic groups via Sonogashira conditions employing the appropriate alkyne, Pd(PPh₃)₄, CuI, and triethylamine followed by subsequent reduction with Pd/C and hydrogen and hydrolysis with lithium hydroxide as demonstrated in scheme VII

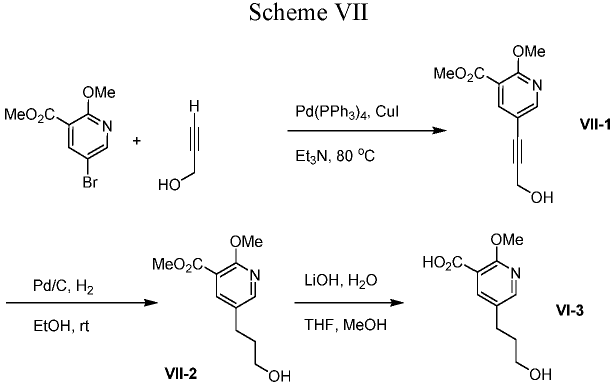

Intermediate **VII-1**: To a slurry of methyl 5-bromo-2-methoxynicotinate (0.50 g, 2.0 mmol) in TEA (20 mL) was added propargyl alcohol (0.15 mL, 2.5 mmol), Pd(PPh₃)₄ (0.047 g, 0.041 mmol), and CuI (3.9 mg, 0.020 mmol). The reaction mixture was degassed, and heated at 80 °C under N₂ for 16 h. The reaction solution was partitioned between EtOAc and water and the organic layer was separated and dried over Na₂SO₄. The fluid was decanted and concentrated under reduced pressure and the residue purified by silica gel chromatography to furnish methyl 5-(3-hydroxyprop-1-yn-1-yl)-2-methoxynicotinate (300 mg, 1.4 mmol, 68 % yield): ¹H NMR (500 MHz, CDCl₃) δ 8.40 (d, J=2.3 Hz, 1H), 8.22 (d, J=2.4 Hz, 1H), 4.52 (d, J=6.3 Hz, 2H), 4.08 (s, 3H), 3.93 (s, 3H), 1.69 (t, J=6.2 Hz, 1H). MS (ESI) *m*/*z* 222.2 (M+H).

Intermediate **VII-2**: To a solution of **VII-1** (0.16 g, 0.72 mmol) dissolved in ethanol (7 mL) was added Pd-C 10% wt. (0.077 g, 0.072 mmol) and hydrogen introduced at ambient pressure via balloon for 16 h. The reaction solution was filtered and concentrated under reduced pressure to furnish methyl 5-(4-hydroxypropyl)-2-methoxynicotinate which is contaminated with over reduction product methyl 2-methoxy-5-propylnicotinate which used without further purification (0.65 g, 2.7 mmol, 65 % yield). MS (ESI) *m*/*z* 226.1 (M+H).

Intermdiate **VII-3**: To a solution of methyl **VII-2** (0.022 g, 0.099 mmol) dissolved in THF (0.8 mL) and water (0.2 mL) was added lithium hydroxide monohydrate (4 mg, 0.1 mmol) and stirred 16 h. The reaction was neutralized by the addition of 1M HCl and extracted into EtOAc. The organic layer was separated, dried over Na₂SO₄, and the fluid was decanted and concentrated under reduced pressure. The residue was used without further purification: 5-(3-hydroxypropyl)-2-methoxynicotinic acid (0.02 g, 0.1 mmol, 100 % yield). MS (ESI) *m*/*z* 222.2 (M+H).

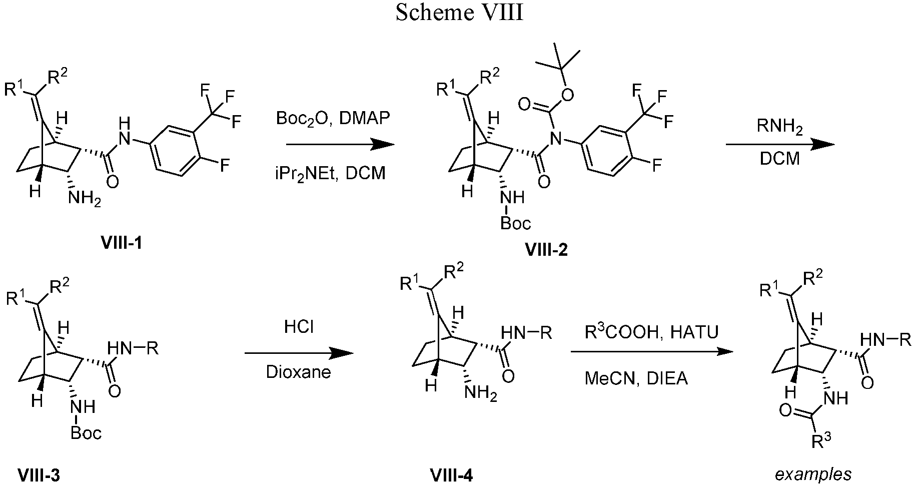

Scheme VIII demonstrates a general route to amides from generic aniline amide **VIII-1**. Analogs were prepared by activating amine **VIII-1** with Boc₂O in the presence of DMAP, and subsequently displacing the activated amide, **VIII-2** with an amine. Further derivitizes are prepared by treatment of **VIII-3** with acid, and subsequent amide bond formation is prepared between amine **VIII-4** and a carboxyllic acid with cross coupling reagents, for example HATU, but not limited to.

### Examples

### Example 1

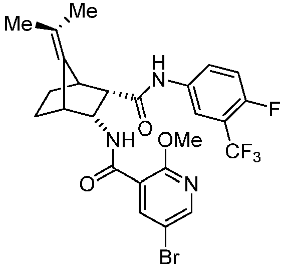

5-bromo-N-[(2R,3S)-3-{[4-fluoro-3-(trifluoromethyl)phenyl]carbamoyl}-7-(propan-2-ylidene)bicyclo[2.2.1]heptan-2-yl]-2-methoxypyridine-3-carboxamide: Into the reaction vessel was added intermediate **I-7** (12 mg, 0.077 mmol), 5-bromo-2-methoxynicotinic acid (18 mg, 0.077 mmol), MeCN (2.3 mL), DIEA (0.04 mL, 0.3 mmol), and HATU (53 mg, 0.14 mmol). The reaction mixture was stirred at RT for 3 hr, concentrated under reduced pressure, and subjected to HPLC purification to afford 1 (24 mg, 0.042 mmol, 60 % yield). ¹H NMR (500 MHz, DMSO-d6) δ 10.57 (br s, 1H), 10.01 (br d, J=6.7 Hz, 1H), 8.44 (br d, J=2.4 Hz, 1H), 8.32 (br d, J=2.1 Hz, 1H), 8.23 - 8.17 (m, 1H), 7.83 - 7.76 (m, 1H), 7.48 (br t, J=9.6 Hz, 1H), 4.34 - 4.25 (m, 1H), 4.05 (s, 3H), 3.12 - 3.06 (m, 1H), 3.06 - 3.00 (m, 1H), 2.95 - 2.89 (m, 1H), 1.80 - 1.63 (m, 8H), 1.40 - 1.28 (m, 2H). LC-MS RT: 2.89 min; MS (ESI) m/z = 570.2 (M+H)+; Method B.

### Example 20

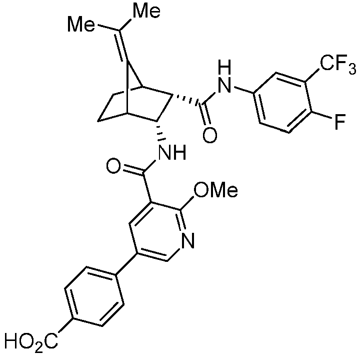

Procedure for 4-(5-{[(2R,3S)-3-{[4-fluoro-3-(trifluoromethyl)phenyl]carbamoyl}-7-(propan-2-ylidene)bicyclo[2.2.1]heptan-2-yl]carbamoyl}-6-methoxypyridin-3-yl)benzoic acid: To a vial was added example **1** (4.4 mg, 0.026 mmol), 4-boronobenzoic acid (4.4 mg, 0.026 mmol), 0.5 M aqueous K₃PO₄ (0.07 mL, 0.04 mmol), THF (0.3 mL), and Xphos Pd G2 (1.4 mg, 1.8 µmol). The reaction mixture was heated under microwave irradiation at 100 °C for 30 min, then cooled to RT, partitioned between water (5 mL) and extracted with EtOAc (3 x 5 mL). The combined organic portions were dried over Na₂SO₄, filtered, concentrated and purified by HPLC to afford **20** (7.8 mg, 0.013 mmol, 71 % yield). ¹H NMR (500 MHz, DMSO-d6) δ 10.58 (br s, 1H), 10.04 (br d, J=6.9 Hz, 1H), 8.72 (br s, 1H), 8.57 (br s, 1H), 8.24 (br d, J=3.9 Hz, 1H), 8.03 (br d, J=8.1 Hz, 2H), 7.82 (br d, J=7.7 Hz, 3H), 7.49 (br t, J=9.7 Hz, 1H), 4.36 (br s, 1H), 4.13 (s, 3H), 3.12 (br d, J=10.9 Hz, 1H), 3.08 - 3.03 (m, 1H), 2.98 - 2.93 (m, 1H), 1.85 - 1.78 (m, 1H), 1.77 - 1.70 (m, 7H), 1.41 - 1.31 (m, 2H). LC-MS RT: 2.11 min; MS (ESI) m/z = 612.2 (M+H)+; Method C.

### Example 21

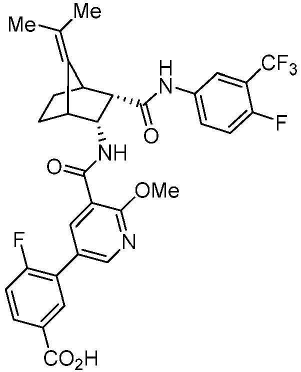

4-fluoro-3-(5-{[(2R,3S)-3-{[4-fluoro-3-(trifluoromethyl)phenyl]carbamoyl}-7-(propan-2-ylidene)bicyclo[2.2.1]heptan-2-yl]carbamoyl }-6-methoxypyridin-3-yl)benzoic acid was prepared in a similar manner to example **20** substituting 3-borono-4-fluorobenzoic acid for 4-boronobenzoic acid. **21** (4.1 mg, 5.80 µmol, 30.1 % yield).¹H NMR (500 MHz, DMSO-d6) δ 10.59 (br s, 1H), 10.06 (br d, J=6.6 Hz, 1H), 8.56 (br s, 1H), 8.45 (br s, 1H), 8.23 (br d, J=4.5 Hz, 1H), 8.13 - 8.04 (m, 1H), 8.03 - 7.98 (m, 1H), 7.87 - 7.79 (m, J=2.9 Hz, 1H), 7.53 - 7.43 (m, 2H), 4.41 - 4.29 (m, 1H), 4.14 (s, 3H), 3.21 - 3.09 (m, 1H), 3.08 - 3.03 (m, 1H), 2.98 - 2.92 (m, 1H), 1.85 - 1.78 (m, 1H), 1.77 - 1.68 (m, 7H), 1.41 - 1.32 (m, 2H). LC-MS RT: 2.11 min; MS (ESI) m/z = 630.5 (M+H)+; Method B.

### Example 26

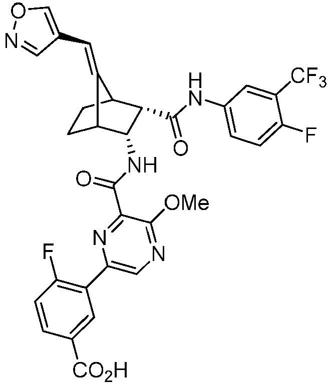

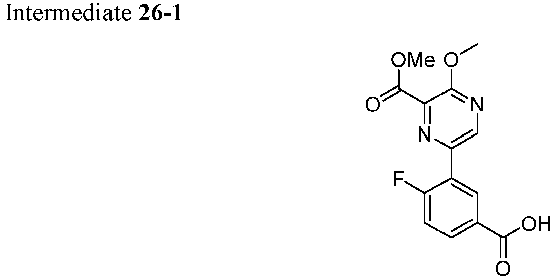

To a vial was added methyl 6-bromo-3-methoxypyrazine-2-carboxylate (0.10 g, 0.41 mmol), 3-borono-4-fluorobenzoic acid (0.10 g, 0.61 mmol), Pd(OAc)₂ (9 mg, 0.04 mmol), K₂CO₃ (0.170 g, 1.2 mmol) followed by H₂O (0.080 mL, 4.5 mmol), and DMF (4 mL). The reaction was degassed with nitogren for 2 min, then stirred for 18 h at rt. The reaction mixture was diluted with water and extracted with EtOAc (3 x 10 mL). The combined organic layers were dried over Na₂SO₄, concentrated under reduced pressure, and the resulting residue was purified by HPLC to afford 4-fluoro-3-(5-methoxy-6-(methoxycarbonyl)pyrazin-2-yl)benzoic acid (37 mg, 0.12 mmol, 30 % yield). MS (ESI) *m*/*z* 307.0 (M+H)⁺.

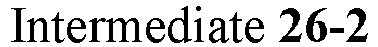

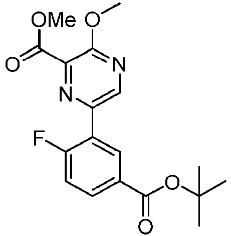

To a vial containing **26-1** (0.038 g, 0.12 mmol) suspended in toluene (1.3 mL) was added 1,1-di-tert-butoxy-N,N-dimethylmethanamine (0.15 mL, 0.62 mmol). The reaction mixture was heated to 40 °C for 5 hr. The reaction was concentrated onto silica gel, then purified by silica gel chromatography to afford methyl 6-(5-(tert-butoxycarbonyl)-2-fluorophenyl)-3-methoxypyrazine-2-carboxylate (29 mg, 0.080 mmol, 65 % yield). MS (ESI) *m*/*z* 363.1 (M+H)⁺.

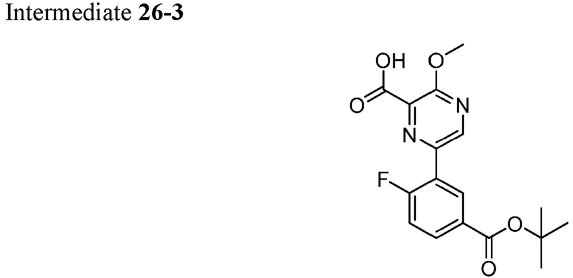

To a vial containing **26-2** (0.029 6g, 0.080 mmol) dissolved in THF (0.45 mL)/Water (0.2 mL)/MeOH (0.1 mL) was added lithium hydroxide (0.4 mL, 1M, 0.4 mmol), and the reaction mixture was stirred at rt for 18 hrs, then partitioned between 1N HCl (5 mL) and extracted with EtOAc (3 x 5 mL). The combined organic layers were dried over Na₂SO₄, filtered, and concentrated under reduced pressure to produce 6-(5-(tert-butoxycarbonyl)-2-fluorophenyl)-3-methoxypyrazine-2-carboxylic acid (28 mg, 0.080 mmol, 100 % yield). MS (ESI) *m*/*z* 349.1 (M+H)⁺.

Example **26**: To a solution of **IV-2** (10 mg, 0.025 mmol), **26-3** (11 mg, 0.030 mmol), MeCN (0.25 mL), and DIEA (0.02 mL, 0.09 mmol) was added HATU (11 mg, 0.028 mmol). The reaction mixture was stirred at rt for 3 h, concentrated under reduced pressure and redissolved in 20% TFA in DCM (1 mL). The reaction was stirred at rt for 5 h, then concentrated under reduced pressure and purified by HPLC to afford 4-fluoro-3-(6-(((1R,2R,3S,4R,Z)-3-((4-fluoro-3-(trifluoromethyl)phenyl)carbamoyl)-7-(isoxazol-4-ylmethylene)bicyclo[2.2.1]heptan-2-yl)carbamoyl)-5-methoxypyrazin-2-yl)benzoic acid (2.8 mg, 4.0 µmol, 16 % yield). 1H NMR (500 MHz, DMSO-d6) δ 10.45 (s, 1H), 9.80 (br d, J=7.3 Hz, 1H), 8.96 (s, 1H), 8.75 (d, J=1.6 Hz, 1H), 8.70 (s, 1H), 8.53 (dd, J=7.6, 1.8 Hz, 1H), 8.07 - 8.01 (m, 1H), 8.00 - 7.96 (m, 1H), 7.86 - 7.79 (m, 1H), 7.45 - 7.33 (m, 2H), 6.13 (s, 1H), 4.53 - 4.45 (m, 1H), 4.01 (s, 3H), 3.49 (br s, 1H), 3.30 - 3.24 (m, 1H), 2.92 (br s, 1H), 1.99 - 1.87 (m, 2H), 1.61 - 1.47 (m, 2H) LC-MS RT: 2.27 min; MS (ESI) m/z = 670.3 (M+H)+; Method C.

### Example 33

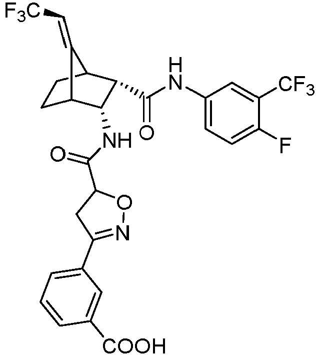

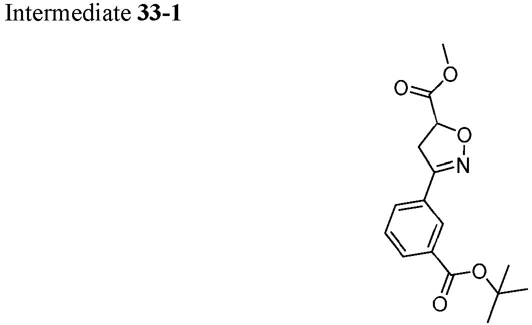

Tert-butyl 3-formylbenzoate (0.10 g, 0.49 mmol) was dissolved in DCM (5 mL). To this solution was added hydroxylamine hydrochloride (34 mg, 0.49 mmol), followed by TEA (1 mL) and the reaction mixture was stirred at rt for 18h. The reaction mixture was diluted with water and the DCM layer was separated, dried (MgSO₄) filtered and concentrated under reduced pressure to produce tert-butyl (E)-3-((hydroxyimino)methyl)benzoate as an oil (126 mg, quant). ¹H NMR (500 MHz, CHLOROFORM-d) δ 8.17 (m, 2H), 8.03 (d, J=7.7 Hz, 1H), 7.80 (dt, J=7.7, 1.4 Hz, 1H), 7.57 - 7.42 (m, 2H), 1.70 - 1.48 (m, 9H). LCMS m/z 222.08 (M+H). The oil was was dissolved in DCM (5 mL) and to this solution was added NCS (0.066 g, 0.49 mmol) and the reaction mixture was stirred at rt for 18h. To this solution was added excess methylacrylate (2 mL) and followed by a sat. solution of NaHCO₃ (5 mL) and stirred at rt for 18h. The reaction mixture was diluted with water (50 mL), and the solution extracted with EtOAc (2 x 25 mL). The combined organic portions were dried (MgSO₄), filtered and concentrated under reduced pressure to produce an oil which was purified via a 12g silica gel chromatography produce methyl 3-(3-(tert-butoxycarbonyl)phenyl)-4,5-dihydroisoxazole-5-carboxylate (140 mg, 0.46 mmol, 94%). ¹H NMR (500 MHz, CHLOROFORM-d) δ 8.20 (s, 1H), 8.06 (dt, J=7.8, 1.4 Hz, 1H), 7.94 (d, J=7.8 Hz, 1H), 7.49 (t, J=7.5 Hz, 1H), 5.24 (dd, J=11.0, 7.2 Hz, 1H), 3.90 - 3.80 (m, 3H), 3.78 - 3.65 (m, 2H), 1.76 - 1.55 (m, 9H). LCMS m/z = 306.1 (M+H).

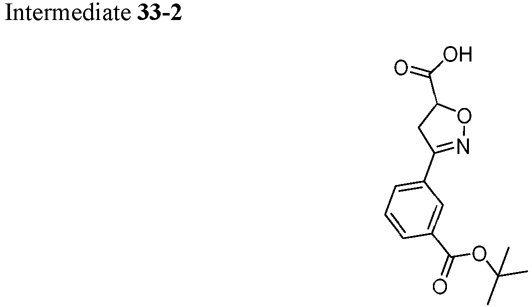

To a solution of 33-1 (0.085 g, 0.28 mmol) dissolved in MeOH (3 mL) was added LiOH (14 mg, 0.56 mmol) followed by the addition of water (3 mL) and the reaction mixture was stirred at rt for 18h. The reaction mixture was diluted with water (50 mL) and acidified (HCl, 1N) and the solution extracted with EtOAc (2 x 25 mL). The combined organic portions were dried (MgSO₄), filtered and evaporated under reduced pressure to produce 3-(3-(tert-butoxycarbonyl)phenyl)-4,5-dihydroisoxazole-5-carboxylic acid which was used without further purification, (79 mg, 0.27 mmol, 97%). ¹H NMR (500 MHz, CHLOROFORM-d) δ 8.20 (t, J=1.6 Hz, 1H), 8.07 (dt, J=7.8, 1.4 Hz, 1H), 7.93 (dt, J=8.0, 1.4 Hz, 1H), 7.49 (t, J=7.8 Hz, 1H), 5.37 - 5.23 (m, 1H), 3.84 - 3.67 (m, 2H), 1.68 - 1.47 (m, 9H). LCMS m/z = 292.3 (M+H).

Example **33:** 3-(5-{[(2R,3S,7Z)-3-{[4-fluoro-3-(trifluoromethyl)phenyl]carbamoyl}-7-(2,2,2-trifluoroethylidene)bicyclo[2.2.1]heptan-2-yl carbamoyl }-4,5-dihydro-1,2-oxazol - 3-yl)benzoic acid was prepared by the coupling method described for example 1 using the trifluoromethyl norbornyl intermediate **V-2** and **33-2** followed by deprotection with TFA as in the procedure to prepare example **26.** (4.7 mg, 7.7 mmol, 20 % yield). ¹H NMR (500 MHz, DMSO-d6) δ 9.19 (br d, *J*=7.0 Hz, 1H), 8.23 (s, 1H), 8.05 (br d, *J*=6.4 Hz, 1H), 8.03 (br d, *J*=7.9 Hz, 1H), 7.88 (br d, *J*=7.3 Hz, 2H), 7.58 (br t, *J*=7.8 Hz, 1H), 7.51 (br t, J*=*9.6 Hz, 1H), 5.90 (q, *J*=7.6 Hz, 1H), 5.23 (dd, *J*=11.7, 5.6 Hz, 1H), 4.30 (br s, 1H), 3.78 (br dd, *J*=17.2, 11.7 Hz, 1H), 3.22 (br d, *J*=10.4 Hz, 1H), 3.05 (br s, 1H), 2.96 (br s, 1H), 2.74 (s, 1H), 1.92 - 1.83 (m, 1H), 1.74 - 1.68 (m, 1H), 1.52 - 1.35 (m, 1H). MS (ESI) *m*/*z* = 614.0 (M+H). HPLC Purity: 100.0 %; RT= 2.13 min. Method C.

### Example 34

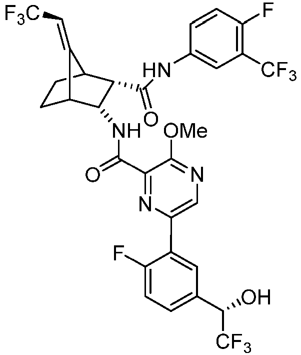

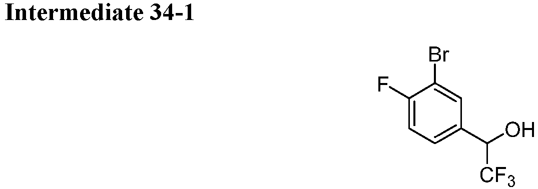

Into the reaction vessel was added 3-bromo-4-fluorobenzaldehyde (240 mg, 1.2 mmol), DMF (3.5 mL), (trifluoromethyl)trimethylsilane (0.34 mL, 2.3 mmol), and K₂CO₃ (8 mg, 0.06 mmol). The reaction mixture was stirred at rt for 60 min and 2N HCl (3 mL) was added. After strirring at rt for 1h, the reaction mixture was diluted with EtOAc (15 mL), and the solution washed with sat NH₄Cl. The aqueous phase was extracted with additional EtOAc (10 mL x2). The organic portions were combined, dried over Na₂SO₄, filtered, concentrated under reduced pressure, and purified by silica gel chromatography to produce 1-(3-bromo-4-fluorophenyl)-2,2,2-trifluoroethan-1-ol (205 mg, 0.751 mmol, 64.9 % yield). ¹H NMR (500 MHz, CDCl₃) δ 7.74 (dd, *J*=6.5, 2.1 Hz, 1H), 7.43 (ddd, J=8.4, 4.8, 2.2 Hz, 1H), 7.19 (t, J=8.4 Hz, 1H), 5.11 - 4.98 (m, 1H), 2.69 (d, *J*=4.4 Hz, 1H).

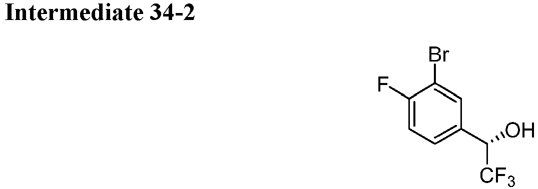

A solution of (S)-2-phenyl-2,3-dihydrobenzo[d]imidazo[2,1-b]thiazole (0.41 g, 1.6 mmol) and **34-1** (11 g, 40 mmol) in diisopropyl ether (130 mL) was cooled to -20 °C. The resulting solution was treated with isobutyric anhydride (4.0 mL, 24 mmol) and transferred to a freezer (-20 °C < t < 0 °C) for 16 h. The reaction mixture was diluted with MeOH (~ 1 mL) and the solution was extracted from phosphate buffer with EtOAc and the organic layer was concentrated under reduced pressure. The residue was purified by silica gel chromatography to furnish (S)-1-(3-bromo-4-fluorophenyl)-2,2,2-trifluoroethan-1-ol (4.9 g, 18 mmol, 44 % yield).

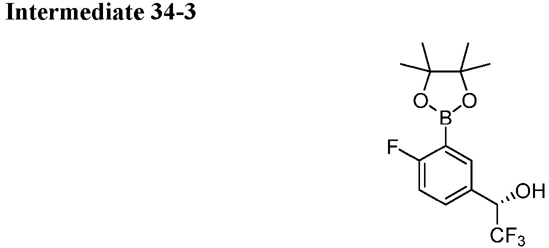

To a solution of **34-2** (0.50 g, 1.8 mmol) dissolved in 1,4-dioxane (4.6 ml) was added bis(pinacolato)diboron (0.93 g, 3.7 mmol), potassium acetate (0.54 g, 5.5 mmol), and PdCl₂(dppf)-DCM adduct (0.15 g, 0.18 mmol). The reaction mixture was heated at 110 °C for 2 h, allowed to cool and partitioned between EtOAc and water. The organic phase was separated, concentrated under reduced pressure, and purified via silica gel chromatography to furnish (S)-2,2,2-trifluoro-1-(4-fluoro-3-(4,4,5,5-tetramethyl-1,3,2-dioxaborolan-2-yl)phenyl)ethan-1-ol (0.41 g, 1.3 mmol, 70 % yield) ¹H NMR (500 MHz, CDCl₃) δ 7.84 (dd, *J*=5.4*,* 2.4 Hz, 1H), 7.67 - 7.56 (m, 1H), 7.11 (t, *J*=8.7 Hz, 1H), 5.05 (q, J=6.6 Hz, 1H), 1.39 (s, 12H). MS (ESI) *m*/*z* 251.0 (M+H-pinacol).

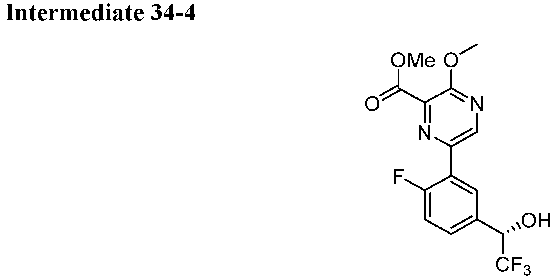

To methyl 6-bromo-3-methoxypyrazine-2-carboxylate (40 mg, 0.16 mmol), **34-3** (57 mg, 0.18 mmol), 0.5 M aqueous K₃PO₄ (0.65 mL, 0.32 mmol), and THF (0.32 mL) in a flask was added Xphos-Pd-G2 (2.7 mg, 3.2 µmol) solution. The reaction mixture was stirred at rt for 16 h, then partitioned between EtOAc and water. The organic layer was separated, dried over Na₂SO₄ decanted and concentrated under reduced pressure. The residue was purified via silica gel chromatography to furnish methyl (S)-6-(2-fluoro-5-(2,2,2-trifluoro-1-hydroxyethyl)phenyl)-3-methoxypyrazine-2-carboxylate (11 mg, 0.031 mmol, 19 % yield). MS (ESI) *m*/*z* 360.9 (M+H).

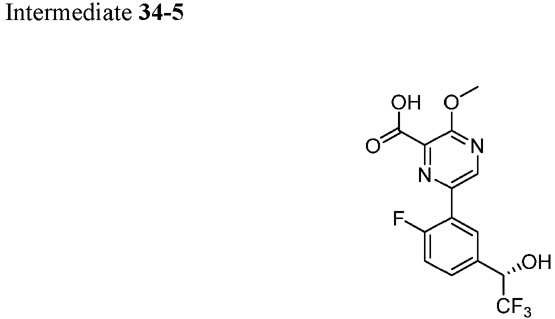

To a solution of **34-4** (11 mg, 0.031 mmol) dissolved in THF (0.120 mL) was added water (0.030 mL) and lithium hydroxide monohydrate (1.3 mg, 0.031 mmol) and the reaction mixture stirred 16 h. The reaction mixture was diluted with water and EtOAc and neutralized by the addition of ~ 0.2 mL of 1N HCl. The organic layer was separated and concentrated under reduced pressure to furnish (S)-6-(2-fluoro-5-(2,2,2-trifluoro-1-hydroxyethyl)phenyl)-3-methoxypyrazine-2-carboxylic acid (12 mg, quant.) which was used without further purification. MS (ESI) *m*/*z* 346.8 (M+H).

Example **34** was prepared from coupling **34-5** with **V-2** under HATU conditions similar to example **1** to produce N-[(2R,3 S,7Z)-3-{[4-fluoro-3-(trifluoromethyl)phenyl]carbamoyl}-7-(2,2,2-trifluoroethylidene)bicyclo[2.2.1]heptan-2-yl]-6-{2-fluoro-5-[(1S)-2,2,2-trifluoro-1-hydroxyethyl]phenyl}-3-methoxypyrazine-2-carboxamide (17 mg, 0.023 mmol, 68 % yield). ¹H NMR (500 MHz, DMSO-d₆) δ 9.85 - 9.73 (m, 1H), 8.84 - 8.71 (m, 1H), 8.09 (br d, *J*=7.2 Hz, 1H), 8.02 (br d, *J*=5.6 Hz, 1H), 7.93 - 7.82 (m, 1H), 7.69 - 7.59 (m, 1H), 7.53 - 7.39 (m, 1H), 7.32 - 7.21 (m, 1H), 6.95 (br t, *J*=7.3 Hz, 1H), 6.03 - 5.91 (m, 1H), 5.30 - 5.16 (m, 1H), 4.60 - 4.47 (m, 1H), 4.10 - 3.94 (m, 3H), 3.30 - 3.21 (m, 1H), 3.07 - 2.96 (m, 1H), 2.04 - 1.91 (m, 2H), 1.58 - 1.44 (m, 2H). One aliphatic proton under solvent. LC-MS RT: 2.59 min; MS (ESI) m/z = 724.94 (M+H)+; Method C.

### Example 35

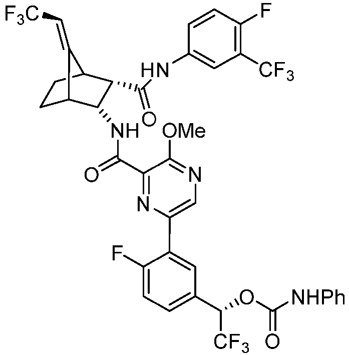

(1S)-2,2,2-trifluoro-1-[4-fluoro-3-(6-{[(2R,3S,7Z)-3-{[4-fluoro-3-(trifluoromethyl)phenyl]carbamoyl}-7-(2,2,2-trifluoroethylidene)bicyclo[2.2.1]heptan-2-yl]carbamoyl }-5-methoxypyrazin-2-yl)phenyl]ethyl N-phenylcarbamate was prepared from carbamate formation with phenyl isocyanate: To a solution of example 35 (0.017 g, 0.023 mmol) dissolved in DCM (2.3 mL) was added pyridine (0.038 mL, 0.47 mmol) and phenyl isocyanate (0.013 mL, 0.117 mmol) and the reaction mixure stirred at rt for 16 h. The reaction mixture was concentrated under reduced pressure and purified via reverse phase HPLC to furnish (S)-2,2,2-trifluoro-1-(4-fluoro-3-(6-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-5-methoxypyrazin-2-yl)phenyl)ethyl phenylcarbamate (6.4 mg, 7.6 µmol, 32 % yield). ¹H NMR (500 MHz, DMSO-d6) δ 10.60 (s, 1H), 10.25 (br s, 1H), 9.77 (br d, J=7.3 Hz, 1H), 8.79 (d, J=2.1 Hz, 1H), 8.23 - 8.14 (m, 1H), 7.99 (dd, J=6.3, 2.3 Hz, 1H), 7.74 (br t, J=9.2 Hz, 2H), 7.52 (dd, J=10.8, 8.7 Hz, 1H), 7.42 (br d, J=7.6 Hz, 2H), 7.37 (br t, J=9.8 Hz, 1H), 7.28 (br t, J=7.9 Hz, 2H), 7.03 (br t, J=7.3 Hz, 1H), 6.42 (q, J=7.1 Hz, 1H), 5.93 (q, J=7.6 Hz, 1H), 4.61 - 4.46 (m, 1H), 4.00 (s, 3H), 3.29 (br dd, J=11.0, 4.0 Hz, 1H), 3.24 (br s, 1H), 2.99 (br s, 1H), 2.05 - 1.87 (m, 2H), 1.48 (br d, J=8.2 Hz, 2H). LC-MS: 2.83 min; MS (ESI) *m*/*z* 844.28 (M+H); Method C.

### Example 40

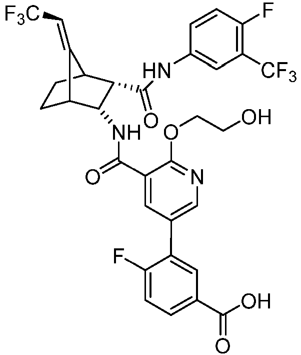

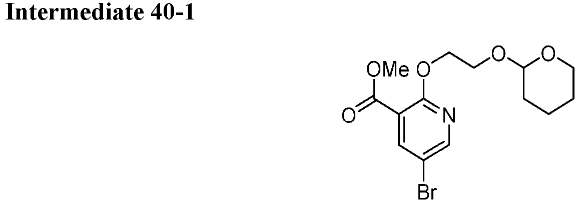

To a solution of 2-((tetrahydro-2H-pyran-2-yl)oxy)ethan-1-ol (0.17 mL, 1.3 mmol) dissolved in 1 mL of THF at 0 °C was added sodium hydride (0.051 g, 1.3 mmol) and the reaction mixture stirred for 45 min. The resulting solution was added to a precooled solution of methyl 5-bromo-2-fluoronicotinate (0.30 g, 1.3 mmol) dissolved in THF (26 mL) at 0 °C and the reaction mixture was allowed to warm to room temperature over 72 h. The reaction mixture was concentrated under reduced pressure and purified via silica gel chromatography to furnish methyl 5-bromo-2-(2-((tetrahydro-2H-pyran-2-yl)oxy)ethoxy)nicotinate (0.20 g, 0.56 mmol, 43 % yield) MS (ESI) *m*/*z* 383.7 (M+Na).

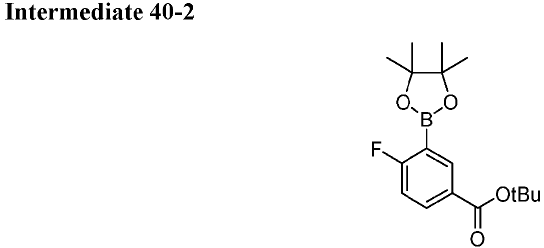

To a solution of **40-1** (1.0 g, 3.6 mmol) dissolved 1,4-dioxane (9.1 mL) was added bis(pinacolato)diboron (1.8 g, 7.3 mmol), potassium acetate (1.1 g, 11 mmol), and PdCl₂(dppf)-DCM adduct (0.30 g, 0.36 mmol) and the reaction was heated at 110 °C for 2 h. The reaction mixture was partitioned between EtOAc and water, and the organic phase separated, concentrated under reduced pressure and purified via silica gel chromatography to furnish tert-butyl 4-fluoro-3-(4,4,5,5-tetramethyl-1,3,2-dioxaborolan-2-yl)benzoate (1.0 g, 3.2 mmol, 88 % yield) ¹H NMR (500 MHz, CDCl₃) δ 8.40 (dd, J=5.7, 2.4 Hz, 1H), 8.08 (ddd, J=8.6, 5.3, 2.4 Hz, 1H), 7.07 (t, J=8.7 Hz, 1H), 1.62 (s, 9H), 1.39 (s, 12H)

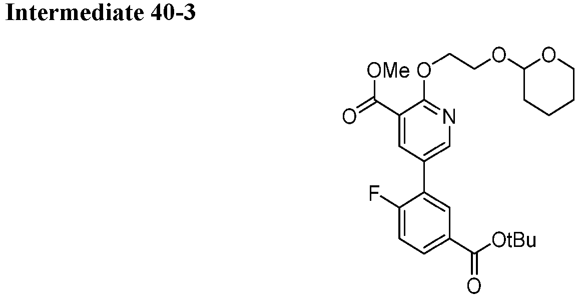

To **40-1** (0.053 g, 0.15 mmol), **40-2** (0.05 g, 0.2 mmol), 0.5 M aqueous K₃PO₄ (0.59 mL, 0.30 mmol), and THF (0.3 mL) in a flask was added Xphos-Pd-G2 (3 mg, 3 µmol) and the reaction mixture was stirred at rt for 16 h. The reaction mixture was partitioned between EtOAc and water, and the organic layer separated, dried over Na₂SO₄ decanted and concentrated under reduced pressure. The residue was purified via silica gel chromatography to furnish methyl 5-(5-(tert-butoxycarbonyl)-2-fluorophenyl)-2-(2-((tetrahydro-2H-pyran-2-yl)oxy)ethoxy)nicotinate (47 mg, 0.099 mmol, 67 % yield). MS (ESI) *m*/*z* 498.0 (M+Na).

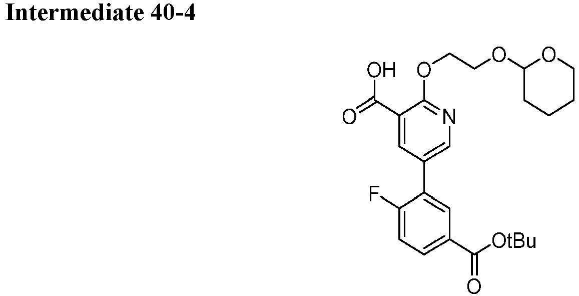

To a solution of **40-3** (0.047 g, 0.099 mmol) dissolved in THF (0.4 mL) was added water (0.1 mL) and lithium hydroxide monohydrate (4.2 mg, 0.10 mmol) and the reaction mixture stirred for 16 h. The reaction mixture was diluted with water and EtOAc neutralized by the addition of ~ 0.2 mL of 1N HCl. The organic layer was separated and concentrated under reduced pressure to furnish 5-(5-(tert-butoxycarbonyl)-2-fluorophenyl)-2-(2-((tetrahydro-2H-pyran-2-yl)oxy)ethoxy)nicotinic acid (46 mg, 0.10 mmol, 100 % yield) which was used without further purification. MS (ESI) *m*/*z* 462.1 (M+H).

To a mixture of **V-2** (0.043 g, 0.100 mmol), DIEA (0.052 mL, 0.299 mmol), and **40-4** (0.046 g, 0.10 mmol) slurried in MeCN (1 mL) was added HATU (0.038 g, 0.100 mmol) and the reaction mixture stirred for 16 h. Water (0.1 mL) and TFA (0.23 mL) was added to the reaction mixture and the resulting solution stirred for 16 h. The reaction mixture concetrated under reduced pressure and the residue purified via preparative HPLC to furnish 4-fluoro-3-(5-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-6-(2-hydroxyethoxy)pyridin-3-yl)benzoic acid (5 mg, 7 µmol, 7 % yield): ¹H NMR (500 MHz, DMSO-d₆) δ 10.65 (s, 1H), 9.82 (br d, *J=*7.3 Hz, 1H), 8.54 (s, 1H), 8.43 (s, 1H), 8.12 (br d, *J*=4.3 Hz, 1H), 8.09 - 8.04 (m, 1H), 8.04 - 7.98 (m, 1H), 7.82 - 7.72 (m, 1H), 7.52 - 7.39 (m, 2H), 5.94 (q, *J*=7.6 Hz, 1H), 4.75 - 4.64 (m, 1H), 4.65 - 4.53 (m, 2H), 4.03 - 3.83 (m, 2H), 3.29 (br dd, *J*=10.7, 4.3 Hz, 1H), 3.22 (br s, 1H), 3.01 (br s, 1H), 2.05 (br d, *J*=8.5 Hz, 2H), 1.53 (br d, *J*=7.9 Hz, 2H). LC-MS: 2.27 min; MS (ESI) *m*/*z* 699.97 (M+H); Method C.

### Example 42

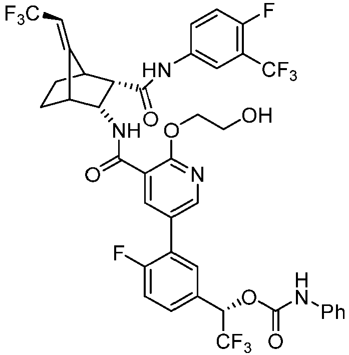

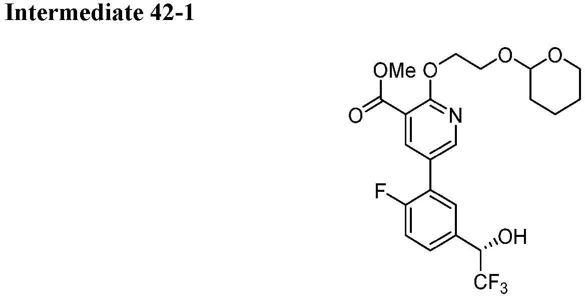

To a mixture of **40-1** (0.051 g, 0.14 mmol), **34-3** (0.050 g, 0.16 mmol), 0.5 M aqueous K₃PO₄ solution (0.57 mL, 0.284 mmol), and THF (0.28 mL) in a flask was added XPhos-Pd-G2 (2.4 mg, 2.8 µmol) and the reaction mixture was stirred at rt for 16 h. The reaction mixture was partitioned between EtOAc and water, and the organic layer was separated, dried over Na₂SO₄ decanted and concentrated under reduced pressure. The residue was purified via silica gel chromatography to furnish methyl 5-(2-fluoro-5-((S)-2,2,2-trifluoro-1-hydroxyethyl)phenyl)-2-(2-((tetrahydro-2H-pyran-2-yl)oxy)ethoxy)nicotinate (0.031 g, 0.065 mmol, 46 % yield). MS (ESI) *m*/*z* 360.8 (M+H).

Intermediate **42-1** was hydrolyzed with aqueous LiOH in THF and coupled with amine as described in the general procedure for example **1** which was subsequently dissolved in DCM (1.6 mL). To this solution was added pyridine (0.025 mL, 0.31 mmol) and phenyl isocyanate (9 µL, 0.08 mmol) and the reaction mixture stirred for 16 h. The reaction solution was concentrated under reduced pressure and the residue dissolved in a mixture of 0.8 mL MeCN and 0.2 mL water with 0.1 mL TFA. After 1 h, the reaction solution was concentrated under reduced pressure and the residue purified by HPLC prep to furnish (S)-2,2,2-trifluoro-1-(4-fluoro-3-(5-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-6-(2-hydroxyethoxy)pyridin-3-yl)phenyl)ethyl phenylcarbamate (3.2 mg, 3.7 µmol, 23 % yield): ¹H NMR (500 MHz, DMSO-d₆) δ 10.64 (s, 1H), 10.29 (br s, 1H), 9.85 (br d, *J*=7.3 Hz, 1H), 8.54 (br s, 1H), 8.46 (s, 1H), 8.13 (br d, *J*=4.0 Hz, 1H), 7.85 (br d, *J*=7.0 Hz, 1H), 7.82 - 7.74 (m, 1H), 7.69 (br d, *J*=4.9 Hz, 1H), 7.57 - 7.42 (m, 4H), 7.31 (br t, *J*=7.8 Hz, 2H), 7.05 (br t, *J*=7.3 Hz, 1H), 6.65 - 6.51 (m, 1H), 5.95 (q, *J*=8.1 Hz, 1H), 4.81 (t, *J*=5.6 Hz, 1H), 4.74 - 4.65 (m, 1H), 4.61 (dt, *J*=10.8, 5.3 Hz, 2H), 4.05 - 3.84 (m, 2H), 3.22 (br s, 1H), 3.02 (br s, 1H), 2.04 (br d, *J*=11.3 Hz, 2H), 1.53 (br d, *J*=7.9 Hz, 2H). Analytical LC-MS: 2.95 min; MS (ESI) *m*/*z* 873.3 (M+H); Method C.

### Example 49

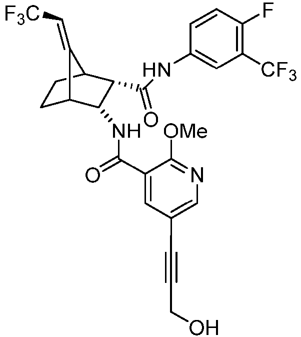

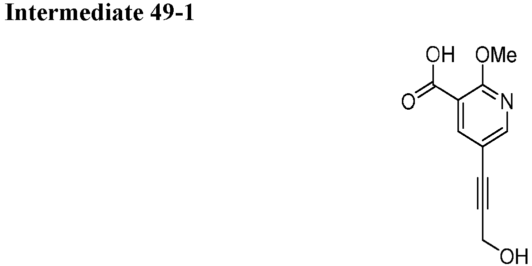

To a solution of **VII-1** (0.022 g, 0.10 mmol) dissolved in THF (0.8 mL) and water (0.2 mL) was added lithium hydroxide monohydrate (4 mg, 0.1 mmol) and the reaction mixture stirred for 16 h. The reaction was neutralized to pH 6 by the addition of 1M HCl and extracted into EtOAc. The organic layer was separated, dried over Na₂SO₄, and the fluid was decanted and concentrated under reduced pressure. The residue was used without further purification, : 5-(3-hydroxyprop-1-yn-1-yl)-2-methoxynicotinic acid (0.02 g, 0.1 mmol, 100 % yield). MS (ESI) *m*/*z* 208.0 (M+H).

N-[(2R,3S,7Z)-3-{[4-fluoro-3-(trifluoromethyl)phenyl]carbamoyl }-7-(2,2,2-trifluoroethylidene)bicyclo[2.2.1]heptan-2-yl]-5-(3-hydroxyprop-1-yn-1-yl)-2-methoxypyridine-3-carboxamide was prepared via HATU coupling between **5-2** and **49-2** as described in the general procedure for example **1.** (17 mg, 0.029 mmol, 29 % yield).1H NMR (500 MHz, DMSO-d6) δ 10.67 (s, 1H), 10.03 (d, J=6.7 Hz, 1H), 8.42 (d, J=2.4 Hz, 1H), 8.27 - 8.17 (m, 2H), 7.84 - 7.76 (m, 1H), 7.50 (t, J=9.8 Hz, 1H), 5.94 (q, J=7.9 Hz, 1H), 4.55 - 4.43 (m, 1H), 4.32 (d, J=5.8 Hz, 2H), 4.10 (s, 3H), 3.28 (dd, J=11.0, 4.0 Hz, 1H), 3.23 (br s, 1H), 3.00 (br s, 1H), 2.00 - 1.83 (m, 2H), 1.58 - 1.42 (m, 2H). Analytical LC-MS: 1.16 min; MS (ESI) *m*/*z* 586.20 (M+H); Method C.

### Example 50

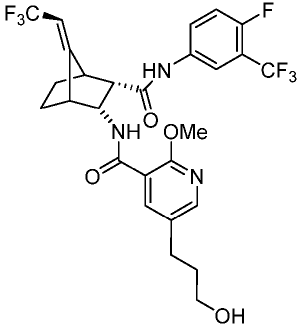

N-[(2R,3S,7Z)-3-{[4-fluoro-3-(trifluoromethyl)phenyl]carbamoyl }-7-(2,2,2-trifluoroethylidene)bicyclo[2.2.1]heptan-2-yl]-5-(3-hydroxypropyl)-2-methoxypyridine-3-carboxamide was prepared via HATU coupling between **V-2** and **VII-3** as described by the general procedure for example **1.** (21 mg, 0.035 mmol, 31 % yield). ¹H NMR (500 MHz, DMSO-d6) δ 10.66 (s, 1H), 9.98 (d, J=7.0 Hz, 1H), 8.21 (dd, J=6.3, 2.3 Hz, 1H), 8.14 (dd, J=13.6, 2.3 Hz, 2H), 7.86 - 7.73 (m, 1H), 7.49 (t, J=9.6 Hz, 1H), 5.92 (q, J=7.6 Hz, 1H), 4.56 - 4.43 (m, 1H), 4.04 (s, 3H), 3.39 (t, J=6.3 Hz, 1H), 3.26 (dd, J=11.0, 4.3 Hz, 1H), 3.22 (br s, 1H), 2.99 (br s, 1H), 2.61 (t, J=7.6 Hz, 2H), 2.03 - 1.93 (m, 1H), 1.90 - 1.79 (m, 1H), 1.73 - 1.63 (m, 2H), 1.49 (br d, J=7.9 Hz, 2H). Analytical LC-MS: 2.36 min; MS (ESI) *m*/*z* 590.25 (M+H); Method C.

### Example 51

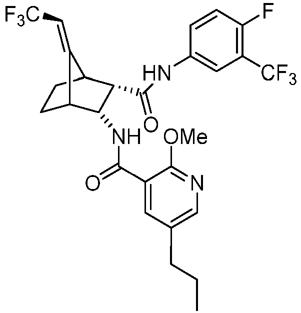

N-[(2R,3S,7Z)-3-{[4-fluoro-3-(trifluoromethyl)phenyl]carbamoyl}-7-(2,2,2-trifluoroethylidene)bicyclo[2.2.1]heptan-2-yl]-2-methoxy-5-propylpyridine-3-carboxamide was isolate from **example 50** as byproduct from over reduction product from the hydrogenation in the preparation of intermediate **50-1.** (2.1 mg, 0.0032 mmol, 2.8 % yield). ¹H NMR (500 MHz, DMSO-d6) δ 10.66 (s, 1H), 9.98 (br d, J=7.0 Hz, 1H), 8.23 (br dd, J=6.3, 2.6 Hz, 1H), 8.18 - 8.06 (m, 2H), 7.84 - 7.72 (m, 1H), 7.50 (br t, J=9.9 Hz, 1H), 5.93 (q, J=7.8 Hz, 1H), 4.58 - 4.41 (m, 1H), 4.05 (s, 3H), 3.33 - 3.17 (m, 2H), 3.00 (s, 2H), 2.05 - 1.76 (m, 2H), 1.64 - 1.40 (m, 4H), 0.86 (t, J=7.3 Hz, 3H). Analytical LC-MS: 2.87 min; MS (ESI) *m*/*z* 574.12 (M+H); Method C.

### Example 53

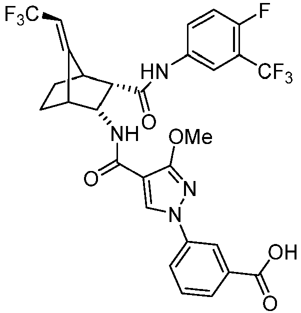

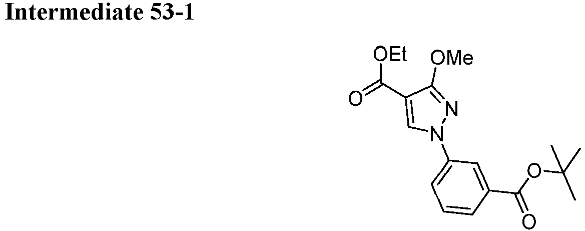

To a solution of tert-butyl 3-iodobenzoate (0.21 g, 0.71 mmol) dissolved in DMSO (4.7 mL) was added proline (11 mg, 0.094 mmol), ethyl 3-methoxy-1H-pyrazole-4-carboxylate (0.08 g, 0.5 mmol), K₂CO₃ (0.13 g, 0.94 mmol), and copper(I) iodide (9 mg, 0.05 mmol). The reaction mixture was heated at 80 °C for 16 h. The reaction mixture was partitioned between water and EtOAc and the organic layer was separated and dried over Na₂SO₄. The fluid was decanted and concentrated under reduced pressure and the residue purified via silica gel chromatography to produce ethyl 1-(3-(tert-butoxycarbonyl)phenyl)-3-methoxy-1H-pyrazole-4-carboxylate (92 mg, 0.27 mmol, 57 % yield). MS (ESI) *m*/*z* 347.3 (M+H).

3-(4-{[(2R,3S,7Z)-3-{[4-fluoro-3-(trifluoromethyl)phenyl]carbamoyl}-7-(2,2,2-trifluoroethylidene)bicyclo[2.2.1]heptan-2-yl]carbamoyl}-3-methoxy-1H-pyrazol-1-yl)benzoic acid was prepared via hydrolysis of the methyl ester **53-1** with LiOH followed by amide formation with **V-2** under HATU conditions and deprotection as outlined example **26** to produce example **53.** (8.8 mg, 0.14 mmol, 50 % yield). ¹H NMR (500 MHz, DMSO-d6) δ 10.66 (s, 1H), 8.99 (br d, J=7.0 Hz, 1H), 8.77 (br s, 1H), 8.46 - 8.16 (m, 2H), 8.04 (br d, J=7.3 Hz, 1H), 7.95 - 7.75 (m, 2H), 7.74 - 7.58 (m, 1H), 7.51 (br t, J=9.6 Hz, 1H), 5.93 (q, J=7.6 Hz, 1H), 4.57 - 4.43 (m, 1H), 4.10 (s, 3H), 3.26 (br dd, J=11.1, 3.5 Hz, 1H), 3.18 (br s, 1H), 3.04 - 2.93 (m, 1H), 1.97 (br d, J=9.5 Hz, 1H), 1.92 - 1.83 (m, 1H), 1.57 - 1.42 (m, 2H). Analytical LC-MS: 2.44 min; MS (ESI) *m*/*z* 641.23 (M+H); Method C.

### Example 55

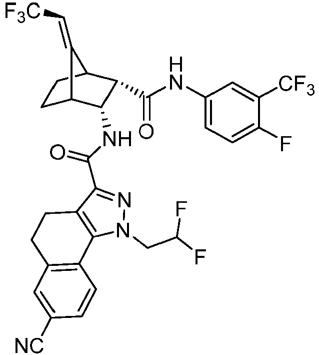

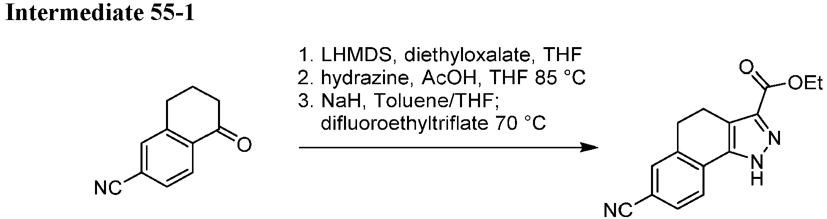

Intermediate **55-1.** To a solution of 5-oxo-5,6,7,8-tetrahydronaphthalene-2-carbonitrile (0.47 g, 2.8 mM) dissolved in THF (5 mL) was added LHMDS (4.1 mL, 4.1 mM) and diethyloxalate (0.40 g, 2.8 mM). The reaction mixture was stirred at rt 16 h, followed by the addition of AcOH (10 mL), and hydrazine (66 mg, 2.1 mmol). The reaction mixture was heated at 85 °C for 1 h. The reaction mixture was concentrated under reduced pressure to afford 7-cyano-4,5-dihydro-1H-benzo[g]indazole-3-carboxylic acid which was redissolved in a mixture of toluene/THF (8:3, 15 mL) and NaH (60% wt in dispersion oil) (83 mg, 2.07 mmol) was added. The reaction mixture was stirred at rt for 1 h followed by the addition of the difluoroethyltriflate (440 mg, 2.1 mmol). The reaction mixture was heated at 70 °C for 18h, allowed to cool. Water (25 mL) was added to the reaction mixture and the solution was extracted with EtOAc (2 x 25mL), the combined organic portions dried over MgSO₄, filtered, and concentrated under reduced pressure. The residue was purified via silica gel chromatography to afford ethyl 2-(6-cyano-1-oxo-1,2,3,4-tetrahydronaphthalen-2-yl)-2-oxoacetate (520 mg, 1.6 mmol 76 %). ¹H NMR (400 MHz, CDCl₃) δ 7.76 - 7.69 (m, 1H), 7.68 - 7.62 (m, 2H), 6.38 - 6.14 (tt, J=55.3, 4.6 Hz, 1H), 4.92 - 4.79 (m, 2H), 4.69 - 4.41 (m, 2H), 3.11 - 2.87 (m, 4H), 1.75 - 1.23 (m, 3H). MS (ESI) *m*/*z* = 332.08 (M+H).

Intermediate **55-2.** The intermediate **55-1** (520 mg, 1.6 mmol) was dissolved in a solution of MeOH/THF (5:1, 25 mL and water 5 mL). To this solution was added LiOH (75 mg, 3.2 mmol) and the stirred at rt for 16 h. The reaction was diluted with HCl and extracted with EtOAc (2 x 100 mL), The conmbined organic portions were dried over MgSO₄, filtered, and concetrated under reduced pressure to afford **55-2** (420 mg, 88 %). ¹H NMR (400 MHz, CD₃OD) δ 7.90 (d, J=8.1 Hz, 1H), 7.80 - 7.71 (m, 2H), 6.40 (tt, J=54.9, 4.0 Hz, 1H), 5.08 - 4.90 (m, 2H), 3.11 - 2.94 (m, 4H). MS (ESI) *m*/*z* = 304.08 (M+H).

**Example 55.** 7-cyano-1-(2,2-difluoroethyl)-N-((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)-4,5-dihydro-1H-benzo[g]indazole-3-carboxamide was prepared by the general coupling method described for example **1** using the trifluoromethyl norbornyl intermediate **V-2** and intermediate **55-2.** (2.1 mg, 3.1 mmol, 48 % yield) ¹H NMR (500 MHz, DMSO-d6) δ 10.74 - 10.65 (m, 1H), 9.33 (br d, *J*=7.2 Hz, 1H), 8.16 (br s, 1H), 7.86 (br s, 2H), 7.81 (br s, 1H), 7.76 (br s, 1H), 7.57 - 7.45 (m, 1H), 6.49 (br s, 1H), 5.95 (br d, *J*=6.5 Hz, 1H), 5.05 (br t, *J*=14.5 Hz, 2H), 4.47 (br s, 1H), 3.27 (br d, *J*=9.8 Hz, 1H), 3.02 - 2.92 (m, 2H), 2.89 (br s, 2H), 1.99 - 1.80 (m, 2H), 1.52 (br d, *J*=7.7 Hz, 2H). MS (ESI) *m*/*z* = 682.3 (M+H). HPLC Purity: 99.2 %; Retention Time: 2.54 min. Method B.

### Example 56

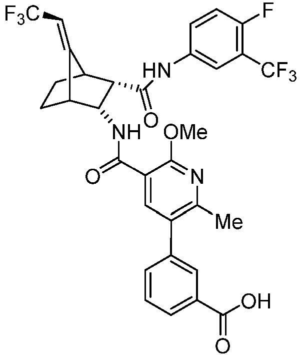

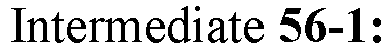

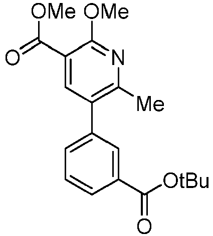

Ethyl 5-(3-(tert-butoxycarbonyl)phenyl)-2-hydroxy-6-methylnicotinate (50 mg, 0.19 mmol), (3-(tert-butoxycarbonyl)phenyl)boronic acid (130 mg, 0.58 mmol) and Pd-XPhos G3 (12 mg, 0.014 mmol) were placed in a pressure vial. Then THF (2 mL) and phosphoric acid, potassium salt (0.5 M aq.) (0.77 mL, 0.38 mmol) were added, and the reaction mixture was degassed (3X, reduced pressure/Ar). The pressure vial was capped, and the reaction mixture was stirred at 120 °C for 30 min. The reaction mixture was filtered, diluted with EtOAc, washed with water, concetrated under reduced pressure and purified via silica gel chromatography to afford intermediate **56-1** (40 mg, 58%) as a white powder. MS (ESI) *m*/*z* = 358.2 (M+H).

A solution of **56-1** (40 mg, 0.34 mmol) in THF (1.0 mL) and water (0.34 mL) was treated with LiOH solution (1 M, 0.37 mL, 0.37 mmol). The solution was stirred at rt for 24 h. The reaction mixture was acidified by the addition of HCl (1.0 M) to pH 1 then extracted with EtOAc. The organic layer was dried over Na₂SO₄ decanted and concentrated under reduced pressure to afford a clear oil which was dissolved in DMF (1.0 mL) and treated with **V-2** (25 mg, 0.06 mmol), HATU (26 mg, 0.07 mmol), and DIEA (0.03 mL, 0.2 mmol). After 18 h, The reaction mixture was concentrated under reduced pressure then purified by prep HPLC to give 3-(5-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-6-hydroxy-2-methylpyridin-3-yl)benzoic acid, example **56** (11.0 mg, 16.9 mmol, 24%). ¹H NMR (500 MHz, CD₃OD) δ 10.95 (d, *J*=8.5 Hz, 1H), 8.28 (s, 1H), 8.06 (d, *J*=7.4 Hz, 1H), 7.99 - 7.91 (m, 2H), 7.78 - 7.69 (m, 1H), 7.63 - 7.52 (m, 2H), 7.24 (t, *J*=9.6 Hz, 1H), 5.76 (d, *J*=7.7 Hz, 1H), 4.79 (dd, *J*=10.3, 4.8 Hz, 1H), 3.31 - 3.25 (m, 1H), 3.25 - 3.19 (m, 1H), 2.94 (t, *J*=3.9 Hz, 1H), 2.52 (t, *J*=9.5 Hz, 1H), 2.36 (s, 3H), 2.17 (d, *J*=4.1 Hz, 1H), 1.65 (t, *J*=13.6 Hz, 2H). LCMS (Method: XBridge Phenyl 3.5 µm, 3.0x150 mm: Solvent A: 5% ACN, 95% water, 0.05% TFA; Solvent B: 5% water, 95% ACN, 0.05% TFA; 10 to 100% B over 15 min; flow rate: 1.0 mL/min; detection at 220 nm and 254 nm.) Rt = 13.2 min, *m*/*z* = 652.08 (M+H).

### Example 59

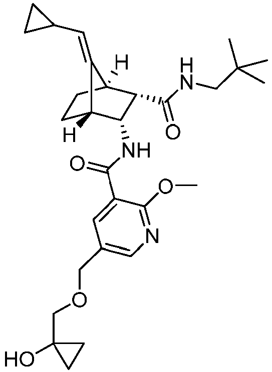

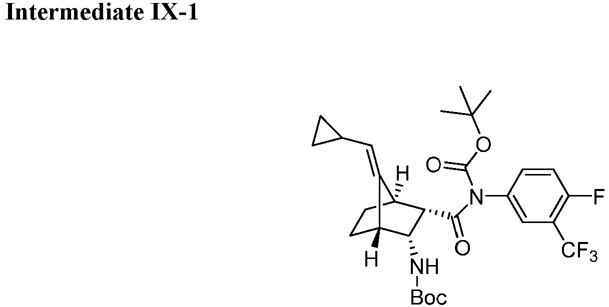

To a solution of **IV-4** (0.10 g, 0.27 mmol) dissolved in DCM (2.7 mL) was added Boc₂O (0.32 mL, 1.4 mmol), DIEA (0.24 mL, 1.4 mmol), and DMAP (17 mg, 0.14 mmol). The reaction mixture was stirred for 14h, concentrated under reduced pressure andthe residue purified with silica gel chromatography to afford tert-butyl ((1R,2S,3R,4R,Z)-3-((tert-butoxycarbonyl)amino)-7-(cyclopropylmethylene)bicyclo[2.2.1]heptane-2-carbonyl)(4-fluoro-3-(trifluoromethyl)phenyl)carbamate (0.11 g, 0.19 mmol, 71 % yield) LC-MS RT: 1.37 min; MS (ESI) m/z = 569.3 (M+H)⁺; Method A.

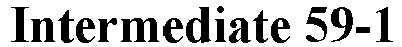

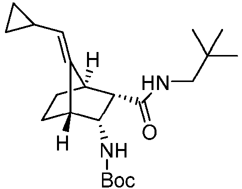

To a solution of **IX-1** (0.11 g, 0.19 mmol) dissolved in DCM (1.3 mL) was added 2,2-dimethylpropan-1-amine (0.14 g, 1.5 mmol). The reaction mixture was stirred overnight and concentrated under reduced pressure, then the residue was purified with silica gel chromatography to afford tert-butyl ((1R,2R,3S,4R,Z)-7-(cyclopropylmethylene)-3-(neopentylcarbamoyl)bicyclo[2.2.1]heptan-2-yl)carbamate (69 mg, 0.18 mmol, 95 % yield). LC-MS RT: 1.21 min; MS (ESI) m/z = 377.3 (M+H)⁺; Method A.

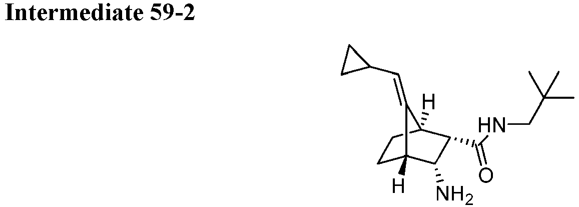

To a flask containing **59-1** (69 mg, 0.18 mmol) was added a solution of HCl (4 M in dioxane) (1.5 mL, 6.0 mmol) and the reaction mixture was stirred 1 h. The reaction mixture was concentrated under reduced pressure and azeotroped with hexanes under reduced pressure to afford (1R,2S,3R,4R,Z)-3-amino-7-(cyclopropylmethylene)-N-neopentylbicyclo[2.2.1]heptane-2-carboxamide (67 mg, 0.18 mmol, 100 % yield) as a hydrochloride salt, which was used without further manipulation. LC-MS RT: 0.87 min; MS (ESI) m/z = 277.1 (M+H)⁺; Method A.

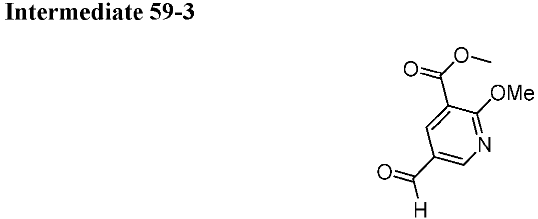

To a solution of methyl 5-bromo-2-methoxynicotinate (1.2 g, 4.9 mmol) dissolved in DMF (24 mL) was added 2-isocyano-2-methylpropane (0.66 mL, 5.9 mmol), triethylsilane (0.78 mL, 4.9 mmol), 2-(dicyclohexylphosphino)biphenyl (68 mg, 0.20 mmol) and Na₂CO₃ (520 mg, 4.9 mmol). The reaction mixture was degassed with nitrogen for 5 mins before Pd(OAc)₂ (33 mg, 0.15 mmol) was added and the reaction mixture was heated to 65 °C for 18h. The reaction mixture was allowed to cool to room temperature, diluted with 10% LiCl aqueous solution and ethyl acetate, then extracted with ethyl acetate. The organic portions were dried over sodium sulfate, filtered and partially concentrated (~5 mL solvent left) under reduced pressure. To this solution was added 1 M HCl (50 mL) and the resulting solution stirred for 2 hours, then quenched with 1 M sodium carbonate to pH 9. The reaction mixture was extracted with ethyl acetate (3 x 30 mL), the organic portions dried over sodium sulfate, filtered and concentrated under reduced pressure. The residue was purified with column chromatography to methyl 5-formyl-2-methoxynicotinate (390 mg, 2.0 mmol, 41% yield) as a white solid. ¹H NMR (500 MHz, CDCl₃) δ 10.04 (s, 1H), 8.81 (d, J=2.5 Hz, 1H), 8.64 (d, J=2.2 Hz, 1H), 4.18 (s, 3H), 3.96 (s, 3H). LC-MS RT: 0.73 min; MS (ESI) m/z = 196.1 (M+H)⁺; Method A.

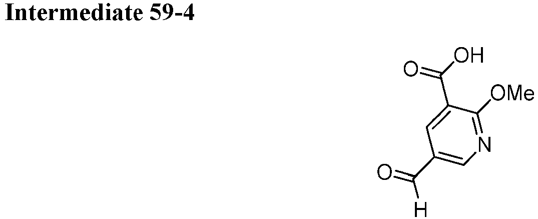

**59-3** (390 mg, 2.0 mmol) was dissolved in THF (8 mL) and water (2 mL) and lithium hydroxide monohydrate (110 mg, 2.6 mmol) were added and the reaction mixture stirred for 30 hours. The reaction mixture was neutralized with 1N HCl and ethyl acetate added The resulting solution was extracted 3x with ethyl acetate. The combined organic portions were washed with brine, dried over Na₂SO₄ and concentrated under reduced pressure to afford 5-formyl-2-methoxynicotinic acid (340 mg, 1.9 mmol, 96 % yield), which was used without further purification. LC-MS RT: 0.57 min; MS (ESI) m/z = 182.1 (M+H)⁺; Method A.

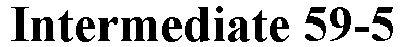

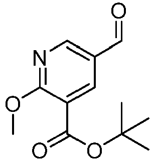

N,N-dimethylformamide di-tert-butyl acetal (2.5 mL, 11 mmol) was added dropwise to a solution of **59-4** (340 mg, 1.9 mmol) dissolved in toluene (0.5 mL). The solution was heated to 80 °C for 30h. The reaction mixture was diluted with water (10 mL) and extracted with ethyl acetate (3 x 10 mL). The combined organic phases were washed with brine (30 mL), dried over Na₂SO₄ , filtered and then concentrated under reduced pressure. The residue was then purified by silica gel chromatography to afford tert-butyl 5-formyl-2-methoxynicotinate (200 mg, 0.85 mmol, 45 % yield). ¹H NMR (500 MHz, CDCl₃) δ 10.03 (s, 1H), 8.77 (d, J=2.2 Hz, 1H), 8.54 (d, J=2.2 Hz, 1H), 4.17 (s, 3H), 1.63 (s, 9H). LC-MS RT: 0.89 min; MS (ESI) m/z = 238.1 (M+H)⁺; Method A.

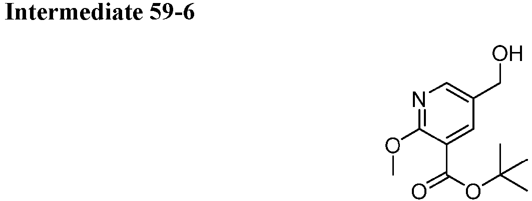

To a solution of **59-5** (200 mg, 0.85 mmol) dissolved in ethanol (8.5 mL) was added NaBH₄ (34 mg, 0.89 mmol) and the reaction mixture was stirred at rt for 4 hours. The reaction mixture was partitioned between ethyl acetate & water and the organic portion was washed with brine, dried over Na₂SO₄ and concentrated under reduced pressure to afford tert-butyl tert-butyl 5-(hydroxymethyl)-2-methoxynicotinate (170 mg, 0.72 mmol, 85 % yield), which was used without further purification. ¹H NMR (500 MHz, CDCl₃) δ 8.28 (d, J=2.5 Hz, 1H), 8.13 (d, J=2.2 Hz, 1H), 4.70 (d, J=5.5 Hz, 2H), 4.07 (s, 3H), 1.69 (t, J=5.6 Hz, 1H), 1.62 (s, 9H). LC-MS RT: 0.81 min; MS (ESI) m/z = 240.3 (M+H)⁺; Method A.

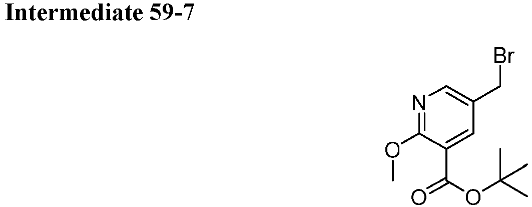

To a solution of **59-6** (170 mg, 0.72 mmol) dissolved in DCM (3.6 mL) was added triphenylphosphine (280 mg, 1.1 mmol) and CBr₄ (36 mg, 1.1 mmol). The reaction mixture was stirred at rt for 18h. The reaction mixture was concentrated under reduced pressure, then purified by silica gel chromatography to afford tert-butyl 5-(hydroxymethyl)-2-methoxynicotinate (180 mg, 0.60 mmol, 82 % yield). ¹H NMR (500 MHz, CDCl₃) δ 8.31 (d, J=2.5 Hz, 1H), 8.12 (d, J=2.5 Hz, 1H), 4.50 (s, 2H), 4.06 (s, 3H), 1.62 (s, 9H). LC-MS RT: 1.06 min; MS (ESI) m/z = 302.1 (M+H)⁺; Method A.

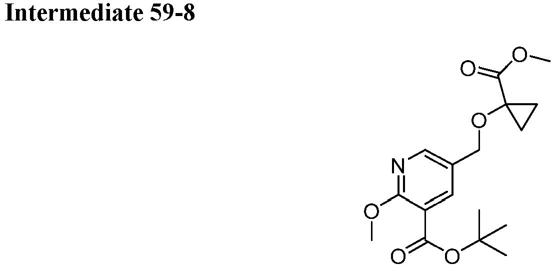

To a solution of methyl 1-hydroxycyclopropane-1-carboxylate (52 mg, 0.45 mmol) dissolved in THF (1.3 mL) cooled to 0 °C was added tetrabutylammonium iodide (10 mg, 0.03 mmol) and NaH (18 mg, 0.45 mmol). The reaction mixture was warmed to rt and stirred for another 15 min before a solution of **59-7** (80 mg, 0.27 mmol) dissolved in THF (1.3 mL) was added dropwise. The reaction was stirred at rt for 3 d. The reaction was diluted water, and the aqueous solution was extracted 3x with ethyl acetate. The organic portions were washed with brine, dried over Na₂SO₄ and concentrated under reduced pressure to afford tert-butyl 2-methoxy-5-((1-(methoxycarbonyl)cyclopropoxy)methyl)nicotinate (87 mg, 0.26 mmol, 98% yield) which was used without further purification. LC-MS RT: 1.03 min; MS (ESI) m/z = 338.3 (M+H)⁺; Method A.

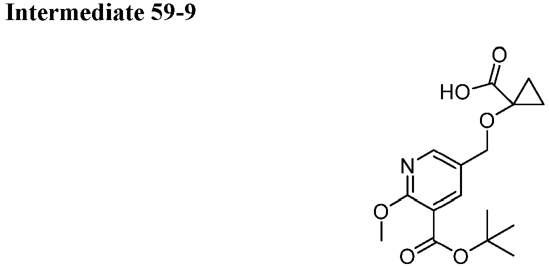

To a solution of **59-8** (87 mg, 0.26 mmol) dissolved in a mixture of THF (2 mL) and water (0.67 mL) was added lithium hydroxide monohyrdate (16 mg, 0.36 mmol) and the reaction mixture was stirred at rt for 24 h. The reaction mixture was neutralized with 1N HCl and extracted with ethyl acetate (3x). The organic portions were washed with brine, dried over Na₂SO₄ and concentrated under reduced pressure to afford 1-((5-(tert-butoxycarbonyl)-6-methoxypyridin-3-yl)methoxy)cyclopropane-1-carboxylic acid (76 mg, 0.24 mmol, 92 % yield) which was used without further purification. LC-MS RT: 0.91 min; MS (ESI) m/z = 324.2 (M+H)⁺; Method A.

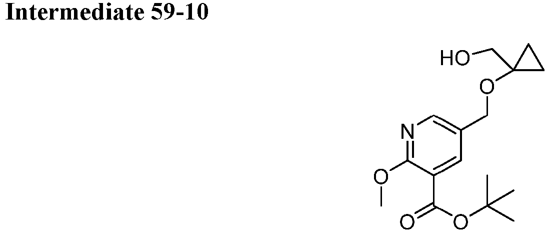

To a solution of **59-9** (76 mg, 0.24 mmol) dissolved in an THF (2.0 ml) and cooled to 0 °C was added borane (0.47 mL, 0.47 mmol, 1M in THF) and stirred at rt for 18h. The reaction mixture was quenched with 1N HCl (0.5 mL) and then extracted with ethyl acetate (3x). The organic portions were washed with brine, dried over Na₂SO₄ and concentrated under reduced pressure to afford tert-butyl 5-((1-(hydroxymethyl)cyclopropoxy)methyl)-2-methoxynicotinate (72 mg, 0.23 mmol, 99 % yield), which was used without purification. LC-MS RT: 0.89 min; MS (ESI) m/z = 310.3 (M+H)⁺; Method A.

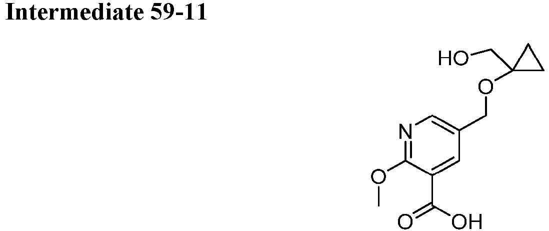

To a solution of **59-10** dissolved in DCM (1.7 mL) was added TFA (0.6 mL) and stirred at rt for 18h. The reaction mixture was concentrated under reduced pressure and the residue was diluted with ethyl acetate, washed with 1M potassium phosphate solution (pH 7.4) followed by brine, dried over Na₂SO₄. The aqueous portion was then brought to pH 1 with 1N HCl, and extracted 3x with ethyl acetate and the organic portions were washed with brine, dried over Na₂SO₄ and combined with the other organics portions and concentrated to afford 5-((1-(hydroxymethyl)cyclopropoxy)methyl)-2-methoxynicotinic acid (57 mg, 0.22 mmol, 97% yield) which was used without further purification. LC-MS RT: 0.62 min; MS (ESI) m/z = 254.1 (M+H)⁺; Method A.

**Example 59:** To a solution of **59-11** (13 mg, 0.051 mmol) and 59-2 hydrogen chloride salt (9.7 mg, 0.031 mmol) dissolved in DMF (0.4 mL) was added BOP (16 mg, 0.037 mmol) and DIEA (0.027 mL, 0.15 mmol) and stirred for 1.5 h. The reaction mixture was diluted with MeOH and filtered through a syringe filter and purified with HPLC to afford N-[(1R,2R,3S,4R,7Z)-7-(cyclopropylmethylidene)-3-[(2,2-dimethylpropyl)carbamoyl]bicyclo[2.2.1]heptan-2-yl]-5-{[(1-hydroxycyclopropyl)methoxy]methyl}-2-methoxypyridine-3-carboxamide (6.9 mg, 0.013 mmol, 43%). ¹H NMR (500 MHz, DMSO-d₆) δ 10.03 (br d, *J*=7.0 Hz, 1H), 8.22 (d, *J*=2.1 Hz, 1H), 8.18 (d, *J*=2.1 Hz, 1H), 8.00 - 7.92 (m, 1H), 4.77 (t, *J*=5.8 Hz, 1H), 4.63 (d, *J*=9.8 Hz, 1H), 4.57 (s, 2H), 4.33 - 4.23 (m, 1H), 4.02 (s, 3H), 3.58 (d, *J*=5.8 Hz, 1H), 3.07 - 2.95 (m, 2H), 2.81 (br dd, *J*=13.0*,* 5.6 Hz, 1H), 1.90 - 1.81 (m, 1H), 1.76 - 1.67 (m, 1H), 1.51 - 1.42 (m, 1H), 1.33 (br t, *J*=9.8 Hz, 2H), 0.81 (s, 9H), 0.75 - 0.66 (m, 4H), 0.60 - 0.52 (m, 2H), 0.32 (br d, *J*=2.7 Hz, 2H). LC-MS RT: 2.20 min; MS (ESI) m/z = 512.5 (M+H)⁺; Method B.

### Example 61

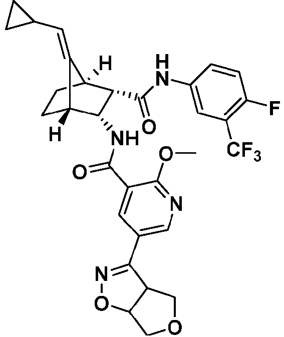

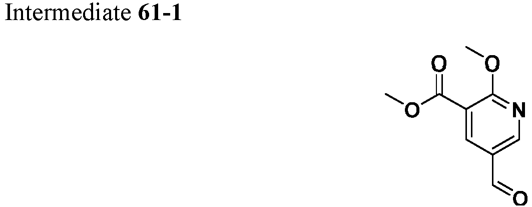

A mixture of 5-bromo-6-methoxynicotinaldehyde (0.85 g, 3.9 mmol), TEA (0.5 mL, 3.9 mmol), Pd(OAc)₂ (0.18 g, 0.80 mmol), dppf (0.65 g, 1.2 mmol) was slurried in DMSO (10 mL) and MeOH (7 mL) in steel reaction vessel. The reaction mixture was placed under CO (g) at 482633 Pa (70 psi) and heated to 80 °C for 18h. The reaction mixture was allowed to cool, then partitioned with water (20 mL) and ethyl acetate (50 mL). The aqueous layer was extracted with ethyl acetate (2 x 20 mL). The combined organic portions were washed with brine (15 mL) and dried (MgSO₄) and concetrated under reduced pressure. The resudue was purified with silica gel chromatography to afford methyl 5-formyl-2-methoxynicotinate (0.74 g, 3.8 mmol, 96 % yield). ¹H NMR (500 MHz, CDCl₃) δ 10.03 (s, 1H), 8.81 (d, *J*=2.3 Hz, 1H), 8.64 (d, *J*=2.3 Hz, 1H), 4.18 (s, 3H), 3.96 (s, 3H). LCMS (ESI) *m*/*z*: 196.2 (M+H)⁺

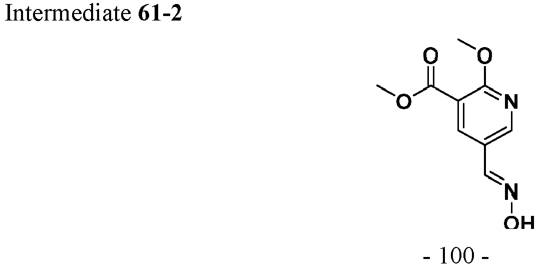

To a solution of **61-1** (0.59 g, 3.0 mmol) dissolved in DCM (15 mL) was added hydroxylamine hydrochloride (0.25 g, 3.6 mmol) followed by TEA (1.3 mL, 9.0 mmol) and the reaction mixture was stirred overnight. The reaction mixture was concetrated under reduced pressure and water (50 mL) was added. The resulting solid was collected by filtration and dried to afford methyl (*E*)-5-((hydroxyimino)methyl)-2-methoxynicotinate (0.43 g, 2.0 mmol, 68%) which was used without further purification. ¹H NMR (500 MHz, CDCl₃) δ 8.45 (s, 2H), 8.14 (s, 1H), 4.10 (s, 3H), 3.94 (s, 3H). LCMS (ESI) *m*/*z:* 211.1 (M+H)⁺

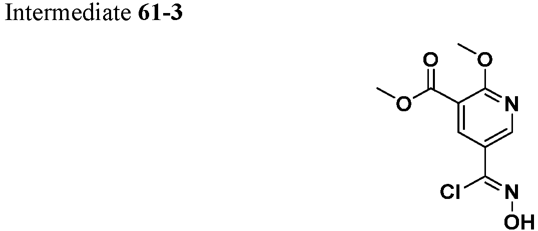

To a solution of **61-2** (0.24 g, 1.1 mmol) dissolved in DMF (3 mL) was added NCS (0.18 mg, 1.4 mmol) and the mixture was stirred overnight. The reaction mixture was diluted with water and the resultant solid was collected by filtration to afford methyl (Z)-5-(chloro(hydroxyimino)methyl)-2-methoxynicotinate (0.24 g, 1.0 mmol, 86%) which was used without purification.¹H NMR (500 MHz, CDCl₃) δ 8.80 (d, *J*=2.6 Hz, 1H), 8.60 (d, *J=*2.6 Hz, 1H), 7.88 (s, 1H), 4.12 (s, 3H), 3.95 (s, 3H). LCMS(ESI) *m*/*z:* 245.1 (M+H)⁺

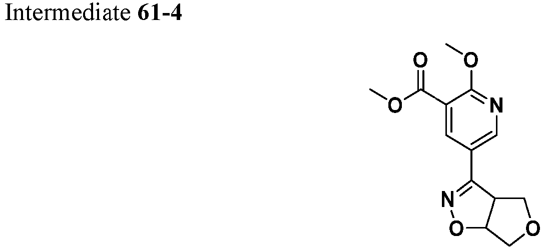

To **61-3** (200 mg, 0.82 mmol) in DCM (5 mL) was added 2,5-dihydrofuran (0.57 g, 8.2 mmol) and TEA (0.34 mL, 2.5 mmol). After 24h, the solvent was removed under vacuum and the residue was purified by normal phase silica gel chromatography, using hexanes/EtOAc as eluents, to afford methyl 2-methoxy-5-(3a,4,6,6a-tetrahydrofuro[3,4-d]isoxazol-3-yl)nicotinate (160 mg, 0.56 mmol, 69 % yield). LCMS(ESI) *m*/*z: 279.1* (M+H)⁺

### Intermediate 61-5 (isomer 1), and 61-6 (isomer 2):

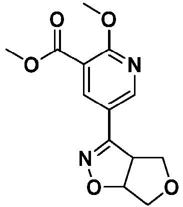

**61-4** was subjected to chiral separtion SFC using the following conditions: Column: Chiralpak IA, 21 x 250 mm, 5 micron, Mobile Phase: 40%-60% MeOH / 60%-40% CO₂, Flow Conditions: 90 mL/min, 150 Bar, 40°C,; Analytical method: Column Chiralpak IA, 4.6 x 250 mm, 3 micron, Mobile Phase: 25% MeOH / 75% CO₂, Flow Conditions: 2 mL/min, 150 Bar, 40°C, to afford chiral peak-1, (RT=1.91 min., >99% ee), **61-5** (30 mg, 0.11 mmol, 13 % yield). ¹H NMR (500 MHz, CDCl₃) δ 8.53 (d, *J*=2.4 Hz, 1H), 8.50 (d, *J*=2.4 Hz, 1H), 5.55 - 5.29 (m, 1H), 4.36 (d, *J*=10.8 Hz, 1H), 4.31 (s, 1H), 4.16 (dd, *J=*9.5, 1.1 Hz, 1H), 4.11 (s, 3H), 3.94 (s, 3H), 3.90 (dd, *J*=9.5, 6.7 Hz, 1H), 3.81 (dd, *J=*11.0*,* 3.8 Hz, 1H) and chiral peak-2, (RT=7.51 min., >99% ee), 61-6 (38 mg, 0.14 mmol, 17% yield). ¹H NMR (500 MHz, CDCl₃) δ 8.53 (d, *J*=2.4 Hz, 1H), 8.50 (d, *J*=2.6 Hz, 1H), 5.46 - 5.41 (m, 1H), 4.36 (d, *J*=10.8 Hz, 1H), 4.31 (s, 1H), 4.16 (dd, *J*=9.5, 1.2 Hz, 1H), 4.11 (s, 3H), 3.94 (s, 3H), 3.91 (d, *J*=6.7 Hz, 1H), 3.81 (dd, *J*=10.8*,* 4.0 Hz, 1H).

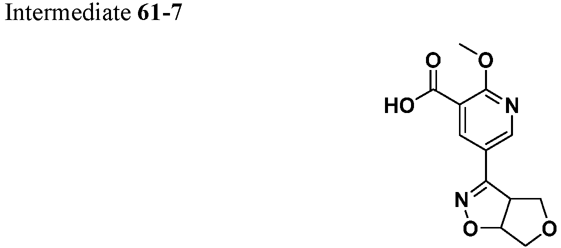

To a solution of **61-6** (38 mg, 0.14 mmol) dissolved in THF (1 mL) and MeOH (0.5 mL) cooled to 0 °C was added 1 M aq. LiOH (0.41 mL, 0.41 mmol) and the reaction mixture was stirred for 14h. The reaction mixture was neutralized with 1N HCl and solvent volume reduced under reduced pressure. The resdiue was freeze-dried to afford 2-methoxy-5-(3a,4,6,6a-tetrahydrofuro[3,4-*d*]isoxazol-3-yl)nicotinic acid (36 mg, 0.14 mmol, quant.) which was used without purification. ¹H NMR (500 MHz, DMSO-d₆) δ 8.20 (s, 1H), 7.84 - 7.68 (m, 1H), 5.32 (dd, *J*=9.2, 3.5 Hz, 1H), 4.61 - 4.37 (m, 1H), 4.09 (d, *J*=10.7 Hz, 1H), 3.91 (d, *J*=8.9 Hz, 1H), 3.83 (s, 3H), 3.77 (dd, *J*=9.4, 6.9 Hz, 1H), 3.65 (dd, *J*=10.5*,* 3.7 Hz, 1H). LCMS(ESI) *m*/*z:* 265.1 (M+H)⁺

**Example 61.** To a solution of **IV-4** (9.8 mg, 26 □mol), **61-7** (7 mg, 26 □mol), BOP (13 mg, 29 □mol) dissolved in DMF (1 mL) was added DIEA (14 □L, 79 □mol. After 24h, the reaction mixture was purified by reverse phase HPLC to afford example 5-{3aH,4H,6H,6aH-furo[3,4-d][1,2]oxazol-3-y1 }-N-[(1R,2R,3S,4R,7Z)-7-(cyclopropylmethylidene)-3-{[4-fluoro-3-(trifluoromethyl)phenyl]carbamoyl}bicyclo[2.2.1]heptan-2-yl]-2-methoxypyridine-3-carboxamide (12 mg, 17 □mol, 64 % yield). ¹H NMR (500 MHz, DMSO-d₆) δ 10.66 - 10.49 (m, 1H), 10.04 (d, *J*=7.0 Hz, 1H), 8.68 - 8.46 (m, 2H), 8.23 (dd, *J*=6.5, 2.5 Hz, 1H), 7.80 (br dd, *J*=7.8, 3.8 Hz, 1H), 7.49 (t, *J*=9.8 Hz, 1H), 5.39 (dd, *J*=9.2, 3.6 Hz, 1H), 4.70 (d, *J*=9.5 Hz, 1H), 4.55 (br t, *J*=7.8 Hz, 1H), 4.47 - 4.39 (m, 1H), 4.14 (s, 3H), 4.11 (d, *J*=10.7 Hz, 1H), 3.96 (br d, *J*=9.2 Hz, 1H), 3.77 (dd, *J*=9.5, 6.8 Hz, 1H), 3.67 (dd, *J*=10.9, 3.7 Hz, 1H), 3.17 (br dd, *J*=11.1*,* 3.9 Hz, 1H), 3.12 (br s, 1H), 2.74 (br s, 1H), 1.86 - 1.67 (m, 2H), 1.59 - 1.47 (m, 1H), 1.44 - 1.29 (m, 2H), 0.89 - 0.66 (m, 2H), 0.45 - 0.21 (m, 2H). LCMS(ESI) *m*/*z:* 615.3 (M+H)⁺ RT= 2.54 min., method B.

### Example 65:

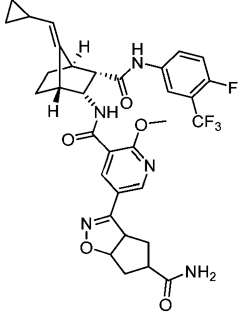

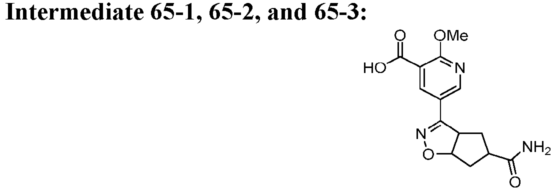

Cyclopent-3-ene-1-carboxamide (1.2 g, 11 mmol) was added dropwise to a solution of methyl (Z)-5-(chloro(hydroxyimino)methyl)-2-methoxynicotinate **59-3** (0.88 g, 3.6 mmol) dissolved in DCM (36 mL) followed by addition of TEA (1.5 mL, 11 mmol). After 12h, the reaction mixture was concentrated under reduced pressure, then purified by normal phase chromatography. The residue was dissolved in THF/MeOH (1:1, 10 mL) and treated with LiOH monohydrate (450 mg, 11 mmol) dissolved in H₂O (3 mL). After 3h, the reaction mixture was made slightly acidic with 1.0N HCl solution and the solution was concentrated under reduced pressure. The remaining aqueous layer was extracted with EtOAc (3 x 20 mL), washed with brine, dried over sodium sulfate, filtered, and concentrated under reduced pressure. The benzoic acid **65-1** (85 mg, 0.28 mmol, 8.0 % yield) and was used withouth further purification. LC-MS RT = 1.079 min; (M+H)⁺ = 329.1; Method B.

Individual chiral diastereomer nicotinic acid intermediates **65-2** and **65-3** were obtained by chiral SFC of diasteremeric mixture intermediate **65-1** (85 mg, 0.28 mmol). Chiral SFC Preparative chromatographic conditions: Instrument: PIC Solution SFC Prep-200; Column: Chiralpak AD-H, 21 x 250 mm, 5 micron; Mobile Phase: 35% MeOH / 65% CO2; Flow Conditions: 45 mL/min, 150 Bar, 40°C; Detector Wavelength: 220 nm; Injection Details: 1.0 mL of ~16.25mg/mL. Analytical Chromatographic Conditions: Instrument: Shimadzu Nexera SFC; Column: Chiralpak AD-H, 4.6 x 100 mm, 3 micron; Mobile Phase: 35% MeOH / 65% CO2; Flow Conditions: 2.0 mL/min, 150 Bar, 40°C; Detector Wavelength: 220 nm; Injection Details: 5 µL of ~1mg/mL in MeOH.

**Intermediate 65-2** (Peak 1; >95%ee; chiral analytical RT = 1.70 min) was obtained as a film (25 mg, 0.080 mmol, 29%). ¹H NMR (500 MHz, DMSO-d6) δ 13.28 (br s, 1H), 8.56 (d, J=2.3 Hz, 1H), 8.30 (d, J=2.1 Hz, 1H), 7.33 (br s, 1H), 6.80 (br s, 1H), 5.16 (dd, J=8.7, 5.0 Hz, 1H), 4.31 (t, J=8.8 Hz, 1H), 3.94 (s, 3H), 2.08 (dd, J=13.8, 6.0 Hz, 1H), 2.04 - 1.86 (m, 4H).

**Intermediate 65-3** (Peak 2; >95%ee; chiral analytical RT = 2.80 min) was obtained as a film (25 mg, 0.080 mmol, 29%). ¹H NMR (500 MHz, DMSO-d6) δ 8.50 (br s, 1H), 8.21 (br s, 1H), 7.36 (br s, 1H), 6.82 (br s, 1H), 5.18 (dd, J=8.5, 5.0 Hz, 1H), 4.32 (t, J=8.9 Hz, 1H), 3.94 (s, 3H), 2.60 - 2.53 (m, 1H), 2.10 (dd, J=13.8, 6.0 Hz, 1H), 2.06 - 1.89 (m, 4H).

**Example 65** was prepared as described in example **59** using **65-2** (23 mg, 0.075 mmol) and **IV-4** to afford 3-(5-(((1R,2R,3S,4R,Z)-7-(cyclopropylmethylene)-3-((4-fluoro-3-(trifluoro-methyl)phenyl)carbamoyl)bicyclo[2.2.1]heptan-2-yl)carbamoyl)-6-methoxypyridin-3-yl)-3a,5,6,6a-tetrahydro-4H-cyclopenta[d]isoxazole-5-carboxamide (isomer-1) (26 mg, 0.038 mmol, 50 % yield). ¹H NMR (500 MHz, DMSO-d6) δ 10.54 (s, 1H), 10.05 (d, J=7.0 Hz, 1H), 8.61 - 8.57 (m, 2H), 8.23 (dd, J=6.6, 2.6 Hz, 1H), 7.83 - 7.78 (m, 1H), 7.50 (t, J=9.8 Hz, 1H), 7.34 (s, 1H), 6.82 (s, 1H), 5.19 (dd, J=8.7, 5.0 Hz, 1H), 4.70 (d, J=9.6 Hz, 1H), 4.43 (ddd, J=10.2, 6.3, 4.0 Hz, 1H), 4.33 (t, J=8.9 Hz, 1H), 4.14 (s, 3H), 3.17 (dd, J=10.6, 4.3 Hz, 1H), 3.12 (t, J=3.5 Hz, 1H), 2.74 (t, J=3.4 Hz, 1H), 2.57 - 2.52 (m, 1H), 2.13 - 1.88 (m, 4H), 1.84 - 1.75 (m, 2H), 1.54 - 1.47 (m, 1H), 1.44 - 1.40 (m, 1H), 0.79 - 0.70 (m, 2H), 0.38 - 0.32 (m, 2H). LC-MS (M+H) = 656.3; HPLC RT = 1.34 min; Method A.

### Example 71:

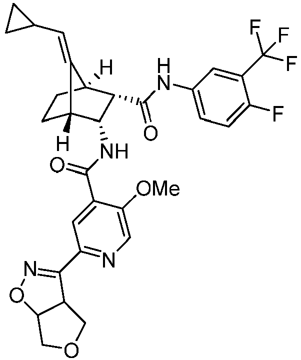

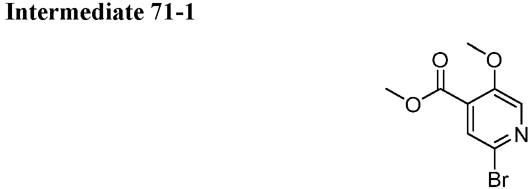

To a solution of sodium methoxide, 0.5 M in MeOH (3.4 mL, 1.7 mmol) was added methyl 2-bromo-5-fluoroisonicotinate (0.33 g, 1.4 mmol) and the reaction mixture was stirred at rt for 18h. The reaction mixture was partitioned between EtOAc and water, and the organic portion was washed with brine, dried over Na₂SO₄, filtered and concentrated under reduced pressure to afford methyl 2-bromo-5-methoxyisonicotinate (240 mg, 0.97 mmol, 69 % yield), which was used without further purification. ¹H NMR (500 MHz, CDCl₃) δ 8.21 (s, 1H), 7.76 (s, 1H), 4.01 (s, 3H), 3.96 (s, 3H). LC-MS RT: 0.82 min; MS (ESI) m/z = 248.1 (M+H)⁺; Method A.

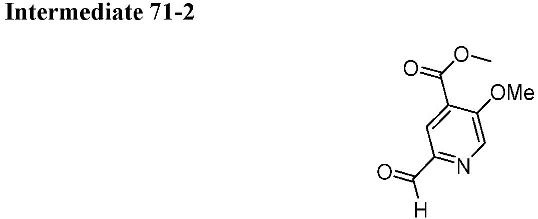

Intermediate **71-2** was prepared from **71-1** according to the general method described for the preparation of **61-1** to afford methyl 2-formyl-5-methoxyisonicotinate (24 mg, 0.13 mmol, 27 % yield) LC-MS RT: 0.65 min; MS (ESI) m/z = 196.1 (M+H)+; Method A.

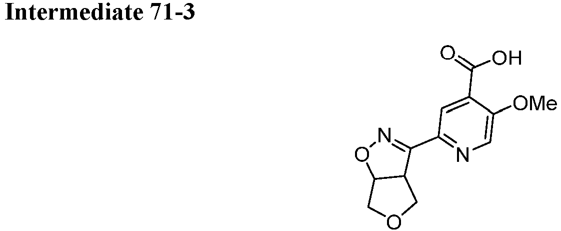

Intermediate **71-3** was prepared from **71-2** according to the general method described for the preparation of **61-7** to afford 5-methoxy-2-(3a,4,6,6a-tetrahydrofuro[3,4-d]isoxazol-3-yl)isonicotinic acid (18 mg, 0.068 mmol, 54 % yield over 4 steps). LC-MS RT: 0.58 min; MS (ESI) m/z = 265.1 (M+H)⁺; Method A.

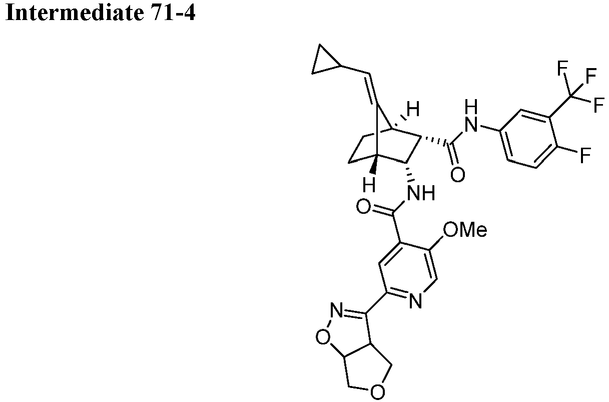

**Intermediate 71-4** was prepared from **71-3** and **IV-4** according to the general method described forthe preparation of **example 59** to afford a mixture of diastereomers N-((1R,2R,3 S,4R,Z)-7-(cyclopropylmethylene)-3-((4-fluoro-3-(trifluoromethyl)phenyl)carbamoyl)bicyclo[2.2.1]heptan-2-yl)-5-methoxy-2-(3a,4,6,6a-tetrahydrofuro[3,4-d]isoxazol-3-yl)isonicotinamide (30 mg, 0.048 mmol. 70 % yield) ¹H NMR (500 MHz, DMSO-d6) δ 10.55 (s, 1H), 10.01 (dd, J=6.8, 3.7 Hz, 1H), 8.65 (s, 1H), 8.32 (s, 1H), 8.23 (br d, J=6.1 Hz, 1H), 7.78 (br dd, J=8.3, 4.1 Hz, 1H), 7.49 (t, J=9.8 Hz, 1H), 5.38 (dt, J=9.2, 2.9 Hz, 1H), 4.70 (d, J=9.5 Hz, 1H), 4.51 - 4.39 (m, 2H), 4.17 (s, 3H), 4.14 - 4.06 (m, 2H), 3.83 - 3.74 (m, 1H), 3.66 (dd, J=10.8, 3.5 Hz, 1H), 3.17 (br dd, J=11.0, 4.3 Hz, 1H), 3.13 (br s, 1H), 2.74 (br s, 1H), 1.86 - 1.73 (m, 2H), 1.58 - 1.48 (m, 1H), 1.47 - 1.33 (m, 2H), 0.81 - 0.66 (m, 2H), 0.35 (br d, J=4.0 Hz, 2H). LC-MS RT: 2.53 min; MS (ESI) m/z = 615.05 (M+H)⁺; Method B.

### Example 71 (isomer 1) and 72 (isomer 2)

**71-4** was subjected to SFC-chiral chromatography with the following conditions: Column: Chiral AS 30 x 250 mm. 5 micron; Mobile phase: 65% CO2/ 35% MeOH w/0.1%DEA; Flow conditions: 100 mL/min; Analytical chromatographic conditions: Column: Chiral AS, 4.6 x 100 mm, 5 micron; Mobile phase: 65% CO2/ 35% MeOH w/0.1%DEA; Flow conditions: 2 mL/min; Peak 1 **(71),** RT = 1.2 min, >95% ee, 1H NMR (500 MHz, DMSO-d6) δ 10.56 (s, 1H), 10.01 (br d, J=6.7 Hz, 1H), 8.66 (s, 1H), 8.32 (s, 1H), 8.27 - 8.19 (m, 1H), 7.85 - 7.74 (m, 1H), 7.49 (br t, J=9.5 Hz, 1H), 5.39 (br dd, J=9.3, 3.2 Hz, 1H), 4.71 (br d, J=9.8 Hz, 1H), 4.52 - 4.37 (m, 2H), 4.17 (s, 3H), 4.15 - 4.03 (m, 2H), 3.79 (br dd, J=9.0, 7.2 Hz, 1H), 3.67 (br dd, J=10.4, 3.1 Hz, 1H), 3.18 (br dd, J=10.7, 3.4 Hz, 1H), 3.13 (br s, 1H), 2.74 (br s, 1H), 1.87 - 1.71 (m, 2H), 1.59 - 1.48 (m, 1H), 1.46 - 1.32 (m, 2H), 0.84 - 0.61 (m, 2H), 0.36 (br s, 2H), LC-MS RT: 2.44 min; MS (ESI) m/z = 614.9 (M+H)⁺; Method B.; Peak 2 **(72),** RT = 3.0 min, > 95% ee, 1H NMR (500 MHz, DMSO-d6) δ 10.57 (s, 1H), 10.02 (br d, J=6.7 Hz, 1H), 8.66 (s, 1H), 8.32 (s, 1H), 8.23 (br d, J=3.7 Hz, 1H), 7.79 (br d, J=7.9 Hz, 1H), 7.50 (br t, J=9.6 Hz, 1H), 5.39 (br dd, J=9.0, 2.9 Hz, 1H), 4.71 (br d, J=10.1 Hz, 1H), 4.50 - 4.40 (m, 2H), 4.17 (s, 3H), 4.16 - 4.05 (m, 2H), 3.85 - 3.74 (m, 1H), 3.71 - 3.63 (m, 1H), 3.18 (br dd, J=10.2, 2.9 Hz, 1H), 3.13 (br s, 1H), 2.75 (br s, 1H), 1.80 (br dd, J=10.4, 9.2 Hz, 2H), 1.53 (br d, J=5.8 Hz, 1H), 1.43 (br s, 2H), 0.84 - 0.64 (m, 2H), 0.36 (br s, 2H), LC-MS RT: 2.43 min; MS (ESI) m/z = 614.9 (M+H)⁺; Method B.

### Example 74:

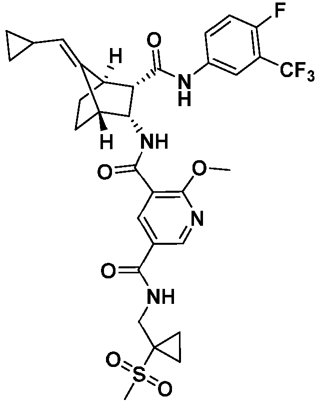

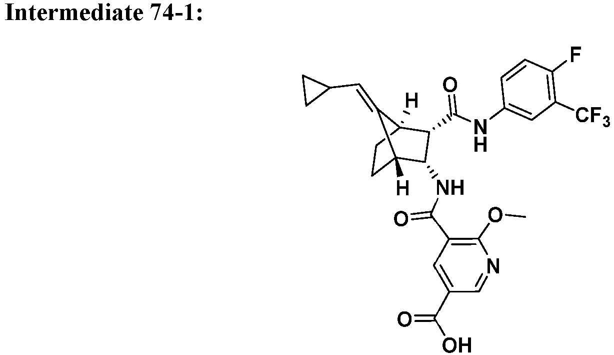

To a solution of example **78** (0.10 g, 0.17 mmol) dissolved in DCM (6 mL) was added TFA (0.26 mL, 3.3 mmol) at 0°C. The resulting reaction mixture was stirred at room temperature for 2h. The reaction mixture was concentrated under reduced pressure to afford 5-(((1R,2R,3R,4R,Z)-7-(cyclopropylmethylene)-3-((4-fluoro-3-(trifluoromethyl)phenyl)carbamoyl)bicyclo[2.2.1]heptan-2-yl)carbamoyl)-6-methoxynicotinic acid (80 mg, 0.15 mmol, 88 % yield) which was used without further purification. LC-MS RT: 0.46 min, method E; MS (ESI) m/z = 548.2 (M+H)⁺.

**Example 74** was prepared by the general procedure described for example **1** using **74-1** (20. mg, 0.037 mmol) and (1-(methylsulfonyl)cyclopropyl)methanamine hydrochloride (10. mg, 0.055 mmol) to afford N3-((1R,2R,3R,4R,Z)-7-(cyclopropylmethylene)-3-((4-fluoro-3-(trifluoromethyl)phenyl)carbamoyl)bicyclo[2.2.1]heptan-2-yl)-2-methoxy-N5-((1-(methylsulfonyl)cyclopropyl)methyl)pyridine-3,5-dicarboxamide (6.5 mg, 9.2 µmol, 25 % yield). LC-MS RT: 2.3 min, method A. MS (ESI) m/z = 679.2 (M+H)⁺. ¹H NMR (400 MHz, DMSO-d6) δ 10.54 (s, 1H), 10.03 (d, J=7.1 Hz, 1H), 8.87 (t, J=6.1 Hz, 1H), 8.81 - 8.76 (m, 1H), 8.76 - 8.66 (m, 1H), 8.23 (dd,J=6.7, 2.6 Hz, 1H), 7.84 - 7.74 (m, 1H), 7.49 (t, J=10.0 Hz, 1H), 4.70 (d, J=9.5 Hz, 1H), 4.48 - 4.40 (m, 1H), 4.14 (s, 3H), 3.83 (d, J=6.1 Hz, 2H),3.20 - 3.13 (m, 1H), 3.13 - 3.09 (m, 1H), 3.06 (s, 3H), 2.78 - 2.71 (m, 1H), 1.85 - 1.72 (m, 2H), 1.56 - 1.44 (m, 1H), 1.44 - 1.34 (m, 2H), 1.29 - 1.21(m, 3H), 1.17 - 1.11 (m, 2H), 0.79 - 0.68 (m, 2H), 0.36 (dd, J=4.4, 2.0 Hz, 2H).

### Example 77:

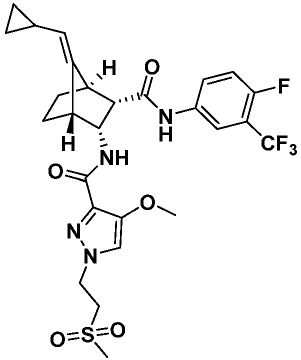

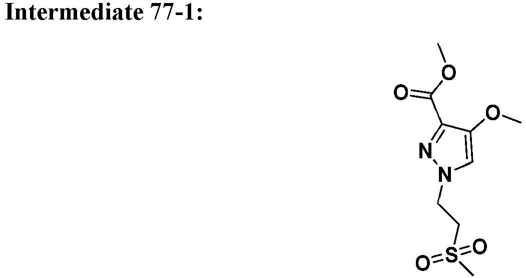

To a solution of methyl 4-methoxy-1H-pyrazole-5-carboxylate (250 mg, 1.6 mmol) dissolved in DMF (10 mL) was added K₂CO₃ (660 mg, 4.8 mmol) followed by 1-chloro-2-(methylsulfonyl)ethane (340 mg, 2.4 mmol). The reaction mixture was stirred at 70 °C for 5 h. The reaction mixture was diluted with EtOAc (20ml), filtered through celite, then the filtrate was concentrated under reduced pressure to afford mixture of regioisomers (250 mg, 0.95 mmol, 60 % yield). The mixture was used without further purification. LCMS RT: 0.53 min; method E; MS (ESI) m/z = 263.2 (M+H)⁺.

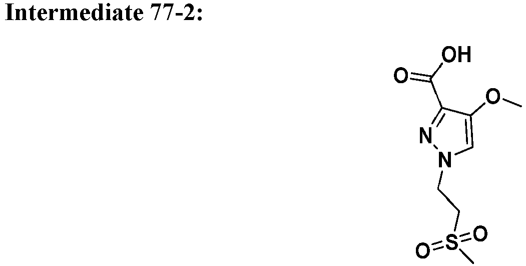

To a solution of **77-1** (0.2 g, 0.763 mmol) dissolved in MeOH (10 mL) cooled to 0 °C was added a solution of 2N aq LiOH (0.037 g, 1.5 mmol). The reaction mixture was allowed to warm to rt over 3 h thenconcentrated under reduced pressure and the residue was dissolved in water (5 mL). The aqueous portion was acidified with 1.5 N HCl and the resulting precipitate was filtered and dried under reduced pressure to yield a mixture of regioisomers (0.12 g, 0.37 mmol, 48 % yield). The mixture was used without further purification. LC-MS RT: 0.31 min; method E; MS (ESI) m/z = 249.2 (M+H)⁺.

**Example 77** was prepared by the general procedure described for example **1** using **77-2** and **IV-4** afford N-[(1R,2R,3 S,4R,7Z)-7-(cyclopropylmethylidene)-3-{[4-fluoro-3-(trifluoromethyl)phenyl]carbamoyl }bicyclo[2.2.1]heptan-2-yl]-1-(2-methanesulfonylethyl)-4-methoxy-1H-pyrazole-3-carboxamide (5 mg, 8 µmol, 6 % yield). LC-MS RT: 3.47 min; method C; MS (ESI) m/z = 599.2 (M+H)⁺. 1H NMR (400 MHz, DMSO-d6) δ ppm 10.51 (s, 1 H) 9.24 (d, J=7.53 Hz, 1 H) 8.22 (dd, J=6.53, 2.51 Hz, 1 H) 7.75 - 7.81 (m, 1 H) 7.58 (s, 1 H) 7.49 (t, J=9.79 Hz, 1 H) 4.76 - 4.95 (m, 1 H) 4.42 (br s, 1 H) 3.92 (s, 3 H) 3.57 (t, J=7.03 Hz, 1 H) 3.36 - 3.39 (m, 1 H) 3.22 - 3.29 (m, 1 H) 3.14 (dd, J=10.79, 4.27 Hz, 2 H) 3.06 (br s, 1 H) 2.96 (s, 2 H) 2.68 - 2.73 (m, 1 H) 2.52 - 2.56 (m, 2 H) 2.34 - 2.47 (m, 2 H) 1.63 - 1.89 (m, 1 H) 1.46 - 1.58 (m, 2 H) 1.42 (br s, 2 H) 1.24 (s, 1 H).

### Example 78:

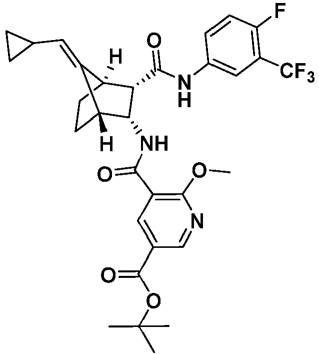

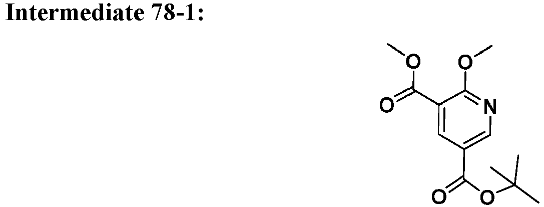

A solution of 6-methoxy-5-(methoxycarbonyl)nicotinic acid (3.5 g, 17 mmol) was dissolved in toluene (100 mL) and heated to 80 °C. To the solution was added 1,1-di-tert-butoxy-N,N-dimethylmethanamine (40 mL, 170 mmol) and the resulting solution stirred at 80 °C for 16 h. The reaction mixture was concentrated under reduced pressure and the resulting residue was dissolved in EtOAc (2x75 ml), and the combined organic portion washed with water (2 x 50 mL). The organic portion was dried over Na₂SO₄, filtered and concentrated under reduced pressure, then purified with silica gel chromatography eluted with 0 - 30 % EtOAc in petroleum ether to afford 5-(tert-butyl) 3-methyl 2-methoxypyridine-3,5-dicarboxylate which was used without further purification (3.7 g, 14 mmol, 84 % yield).LCMS RT = 1.90 min, method E, LCMS(ESI) m/z = 268.2 (M+H)⁺.

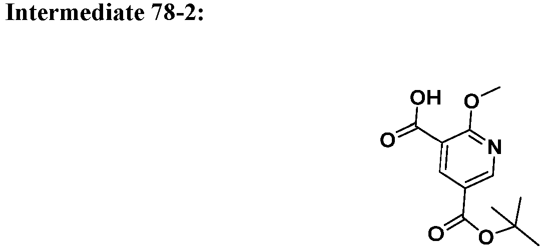

A solution of **78-1** (400 mg, 1.5 mmol) dissolved in THF (25 mL) was cooled to 0°C and 1N aqueous solution of NaOH (3.0 mL, 3.0 mmol ) was added. The reaction mixture was allowed to warm to rt over 3 hr. The reaction mixture was concentrated under reduced pressure, the residue was dissolved in water (2ml) and the aqueous phase washed with diethyl ether (10ml). The aqueous portion was acidified with 1.5 N HCl to pH 4 and the resultant precipitate was filtered and dried under reduced pressure to afford 5-(tert-butoxycarbonyl)-2-methoxynicotinic acid (210 mg, 0.83 mmol, 55 % yield) which was used without further purification. LCMS RT = 0.80 min, method E. LCMS(ESI) m/z = 254.2 (M+H)⁺.

**Example 78** was prepared by the general coupling procedures described for example **1** using **IV-4** (10 mg, 0.027 mmol) and **78-2** (6.87 mg, 0.027 mmol) to afford tert-butyl 5-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-6-methoxynicotinate (9.2 mg, 0.014 mmol, 53 % yield). LC-MS RT: 2.92 min; [A] MS (ESI) m/z = 604.2 (M+H). 1H NMR (400MHz, DMSO-d6) δ = 10.54 (s, 1H), 10.02 (d, J=6.8 Hz, 1H), 8.80 (d, J=2.4 Hz, 1H), 8.68 (d, J=2.2 Hz, 1H), 8.22 (dd, J=2.7, 6.6 Hz, 1H), 7.84 -7.75 (m, 1H), 7.49 (t, J=9.7 Hz, 1H), 4.70 (d, J=9.5 Hz, 1H), 4.45 - 4.37 (m, 1H), 4.15 (s, 3H), 3.20 - 3.14 (m, 1H), 3.13 - 3.09 (m, 1H), 2.76 - 2.71 (m, 1H),1.83 - 1.73 (m, 2H), 1.60 - 1.45 (m, 13H), 1.44 - 1.37 (m, 2H)

### Example 81:

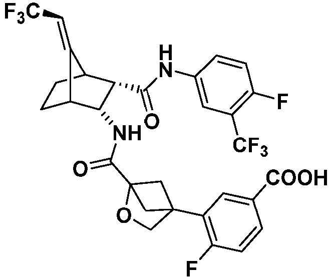

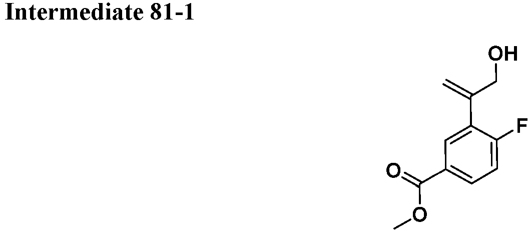

A solution of 2-bromoprop-2-en-1-ol (1.4 g, 10 mmol) dissolved in DMF (15 mL) was treated with (2-fluoro-5-(methoxycarbonyl)phenyl)boronic acid (2.0 g, 10 mmol), potassium phosphate (3.4 mL, 10 mmol), and chloro(2-dicyclohexylphosphino-2',4',6'-triisopropyl-1,1'-biphenyl)[2-(2'-amino-1,1'-biphenyl)]palladium(II) (0.08 g, 0.10 mmol) and the reaction mixture sparged with nitrogen for 10 min, sealed, and stirred at 65 °C for 3h. The reaction mixture was neutralized with 1N HCl, extracted with ethyl acetate, dried over sodium sulfate, and concentrated under reduced pressure, then purified with reverse phase purification to afford methyl 4-fluoro-3-(3-hydroxyprop-1-en-2-yl)benzoate (1.0 g, 4.7 mmol, 47 % yield). MS (ESI) *m*/*z:* 211.1 (M+H).

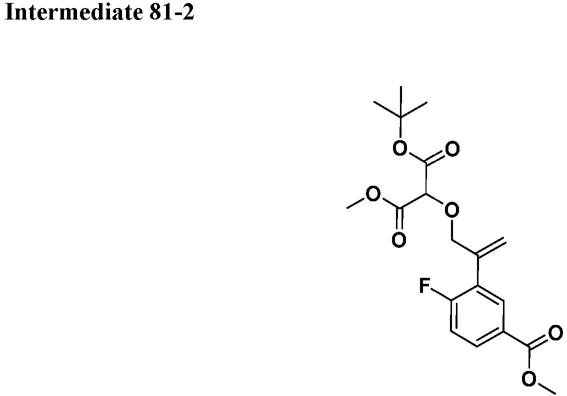

To a round bottom flask containing **81-1** (0.50 g, 2.4 mmol) and rhodium(II) acetate dimer (48 mg, 0.11 mmol) was added toluene (10 mL) and the reaction mixture heated at reflux. To the reaction mixture was added a solution of 1-(tert-butyl) 3-methyl 2-diazomalonate (0.43 g, 2.2 mmol) dissolved in Toluene (2 mL) over a period of 5 minutes and the resulting solution stirred for 30 min. The reaction mixture was concentrated under reduced pressure then purified with silica gel chromatography to afford 1-(tert-butyl) 3-methyl 2-((2-(2-fluoro-5-(methoxycarbonyl)phenyl)allyl)oxy)malonate (610 mg, 1.6 mmol, 71 % yield). MS (ESI) *m*/*z:* 211.1 (M+H)⁺.

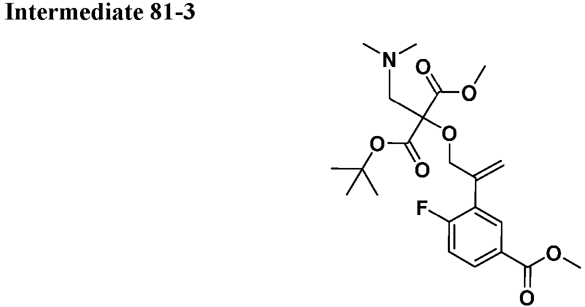

To a solution of **81-2** (612 mg, 1.6 mmol) dissolved in DCM (10 mL) was added dimethylmethylideneammonium iodide (440 mg, 2.4 mmol) followed by TEA (0.34 mL, 2.4 mmol) and the reaction mixture stirred for 14h. The reaction mixture was concentrated under reduced pressure then purified with silica gel chromatography to afford 1-(tert-butyl) 3-methyl 2-((dimethylamino)methyl)-2-((2-(2-fluoro-5-(methoxycarbonyl)phenyl)allyl)oxy)malonate (420 mg, 0.96 mmol, 57% yield). MS (ESI) *m*/*z:* 440.2 (M+H)⁺.

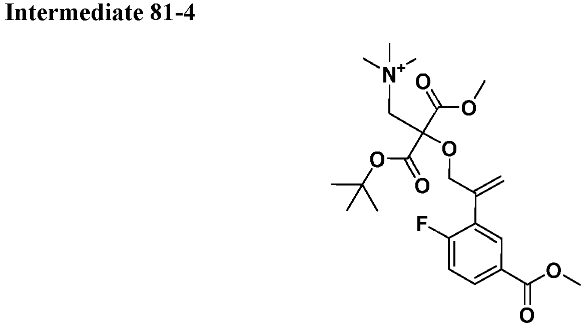

To a solution of **81-3** (420 mg, 0.94 mmol) dissolved in acetone (10 mL) was added methyl iodide (0.089 mL, 1.4 mmol) and the reaction mixture stirred for 14h. The reaction mixture was concentrated under reduced pressure to afford 3-(tert-butoxy)-2-((2-(2-fluoro-5-(methoxycarbonyl)phenyl)allyl)oxy)-2-(methoxycarbonyl)-N,N,N-trimethyl-3-oxopropan-1-aminium (430 mg, 0.94 mmol, quant.) which was used without further purification. ¹H NMR (400MHz, DMSO-d₆) δ 8.02 - 7.94 (m, 2H), 7.45 - 7.36 (m, 1H), 5.70 (s, 1H), 5.54 (s, 1H), 4.63 (s, 2H), 4.14 - 4.01 (m, 2H), 3.87 (s, 3H), 3.84 (s, 3H), 3.05 (s, 9H), 1.46 (s, 9H).

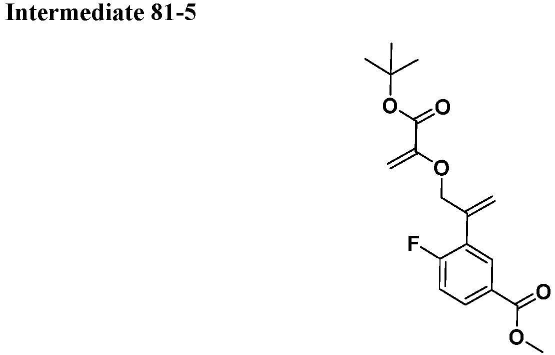

To a solution of **81-4** (430 mg, 0.94 mmol) dissolved in DMSO (6 mL) was added a 1M solution of sodium hydroxide (1.1 mL, 1.1 mmol) and the reaction mixture stirred for 3 h at rt. The reaction mixture was concentrated under reduced pressure then purified using silica gel chromatography to afford methyl 3-(3-((3-(tert-butoxy)-3-oxoprop-1-en-2-yl)oxy)prop-1-en-2-yl)-4-fluorobenzoate (210 mg, 0.62 mmol, 66% yield). MS (ESI) *m*/*z:* 337.3 (M+H)⁺.

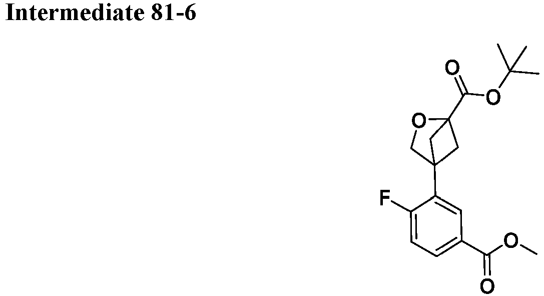

To a solution of **81-5** (200 mg, 0.6 mmol) dissolved in DMSO (30 mL) was added (Ir[dF(CF₃)ppy]₂(dtbpy))-PF₆ (7 mg, 6 µmol) and the reaction mixture was degassed under vacuum and backfilled with N₂ 3x. The reaction mixture was irradiated with blue LED for 60 h. The reaction mixture was diluted with brine, extracted with ethyl acetate, the organic portion concentrated under reduced pressure, then purified with silica gel chromatography to afford tert-butyl 4-(2-fluoro-5-(methoxycarbonyl)phenyl)-2-oxabicyclo[2.1.1]hexane-1-carboxylate (120 mg, 0.36 mmol 57 % yield). MS (ESI) *m*/*z:* 337.0 (M+H).

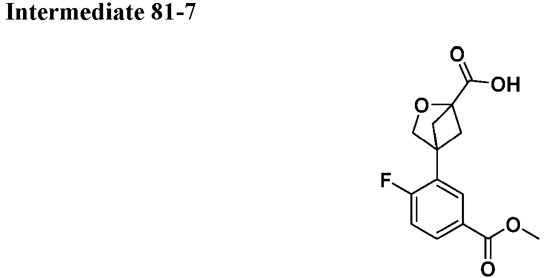

To a solution of **81-6** (120 mg, 0.36 mmol) dissolved in DCM (2.4 mL) was added TFA (0.6 mL, 8 mmol) and the reaction mixture stirred at room temperature for 30 min. The reaction mixture was concentrated under reduced pressure to afford 4-(2-fluoro-5-(methoxycarbonyl)phenyl)-2-oxabicyclo[2.1.1]hexane-1-carboxylic acid (100 mg, 0.36 mmol, quant.) which was used without further purification. MS (ESI) *m*/*z:* 280.0 (M+H)⁺.

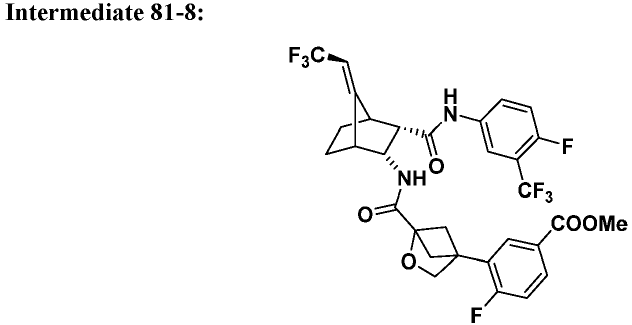

**81-8** was prepared from intermediate **V-2** and intermediate **81-7** according to the general procedure outlined for **Example 1** to afford methyl 4-fluoro-3-(1-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-2-oxabicyclo[2.1.1]hexan-4-yl)benzoate (16 mg, 0.020 mmol, 38 % yield). MS (ESI) *m*/*z:* 659.3 (M+H)⁺.

**Example 81** wasprepared from intermediate **81-8** as outlined in **Example 26,** then purified with HPLC to afford 4-fluoro-3-(1-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-2-oxabicyclo[2.1.1]hexan-4-yl)benzoic acid (6.9 mg, 0.01 mmol, 59% yield). ¹H NMR (500MHz, DMSO-d₆) δ 10.65 (s, 1H), 9.05 (d, *J*=7.2 Hz, 1H), 8.04 - 7.97 (m, 1H), 7.95 - 7.88 (m, 1H), 7.87 - 7.79 (m, 2H), 7.51 (t, *J*=9.7 Hz, 1H), 7.35 - 7.28 (m, 1H), 5.93 (q, *J*=7.8 Hz, 1H), 4.38 - 4.27 (m, 1H), 4.03 (d, *J=5.5* Hz, 1H), 3.98 (d, *J=5.4* Hz, 1H), 3.23 (dd, *J*=10.7*,* 4.2 Hz, 1H), 3.14 (br. s., 1H), 2.97 (br. s., 1H), 2.37 (dd, *J*=14.9, 7.0 Hz, 2H), 2.19 - 2.12 (m, 1H), 2.11 - 2.03 (m, 1H), 1.88 - 1.77 (m, 2H), 1.49 (d, *J*=7.8 Hz, 2H); LC-MS (M+H) = 645.2; RT = 2.03 min; Method B.

### Example 82:

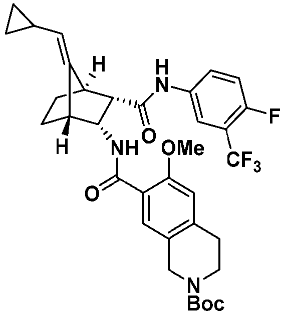

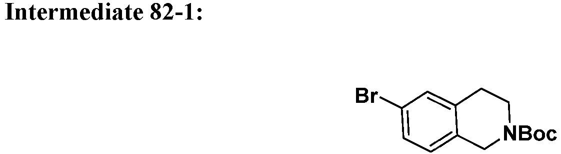

To a solution of 6-bromo-1,2,3,4-tetrahydroisoquinoline hydrochloride (5.0 g, 20 mmol) in DCM (100 mL) were added TEA (14 mL, 100 mmol), DMAP (0.25 g, 2.0 mmol) and Boc-anhydride (5.6 mL, 24 mmol) at 0 °C, then the reaction mixture was stirred at rt for 12h. The volatiles were removed under reduced pressure, the residue was diluted with EtOAc (2 x 100 mL), the organic portion washed with water, dried (Na₂SO₄), concentrated under reduced pressure, then purified with silica chromatography to afford tert-butyl 6-bromo-3,4-dihydroisoquinoline-2(1H)-carboxylate (4.5 g, 14 mmol, 72 % yield). LCMS: MS (ES):*m*/*z* = 214.1 [M+2H-Boc].

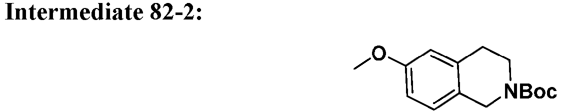

Pd₂(dba)₃ (1.5 g, 1.6 mmol) and 2-di-tert-butylphosphino-2',4',6'-triisopropylbiphenyl (1.0 g, 2.4 mmol) were added to **82-2** (5.0 g, 16 mmol), methanol (6.5 mL, 160 mmol) and Cs₂CO₃ (26 g, 80 mmol) dissolved in dioxane (80 mL) at rt, degassed with nitrogen for 5 min, then stirred at 85 °C for 12 h. The reaction mixture was filtered and the filtrate was concentrated under reduced pressure, then purified with silica gel chromatography to afford tert-butyl 6-methoxy-3,4-dihydroisoquinoline-2(1H)-carboxylate (4.0 g, 15 mmol, 95 % yield). LCMS: MS (ES):*m*/*z* = 164.1 [M+H-Boc].

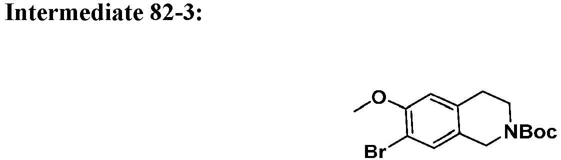

NBS (4.1 g, 23 mmol) was added to a solution of **82-2** (6.0 g, 23 mmol) dissolved in acetonitrile (100 mL) at rt and the reaction mixture was stirred for 5h. The reaction mixture was concentrated under reduced pressure and the residue was extracted with EtOAc. The organic portion was washed with water, dried over Na₂SO₄, concentrated, then purified with silica gel chromatography, elution with 25-45 % EtOAc in petroleum ether to afford tert-butyl 7-bromo-6-methoxy-3,4-dihydroisoquinoline-2(1H)-carboxylate (5.0 g, 15 mmol, 64 % yield). LCMS: MS (ES):*m*/*z* = 288.0 [M+2H-tBu].

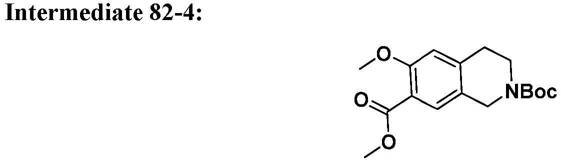

To a solution of **82-3** dissolved in methanol and degassed with bubbling nitrogen (70 mL) and DMF (70 mL) was added TEA (4.5 mL, 32 mmol), Pd(OAc)₂ (0.14 g, 0.64 mmol) and dppf (0.54 g, 0.96 mmol) at rt. The reactionmixture was stirred at under CO (g) at 10 kg pressure and 100 °C in an autoclave for 16 h. The reaction mixture was concentrated under reduced pressure, the residue diluted with EtOAc, filtered through celite and the filter cakewashed with EtOAc. The filtrate was concentrated under reduced pressure, then purified by with silica chromatography, elution with 30-35 % EtOAc in petroleum ether to afford 2-(tert-butyl) 7-methyl 6-methoxy-3,4-dihydroisoquinoline-2,7(1H)-dicarboxylate (520 mg, 1.6 mmol, 25% yield). LCMS: MS (ES):*m*/*z* = 322.2 [M+H].

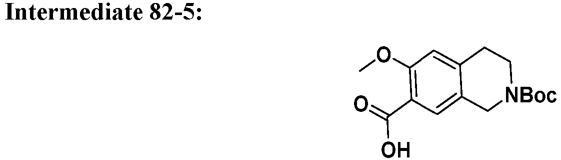

To a solution of **82-4** (350 mg, 1.1 mmol) dissolved in MeOH (5.0 mL) and THF (5.0 mL) was added a solution of LiOH (260 mg, 11 mmol) dissolved in water (3.0 mL) and the reaction mixture was stirred at rt for 5 h. The solution was concentrated under reduced pressure, the residue diluted with water (10 mL), and washed with 20% EtOAc in petroleum ether (20 mL).The aqueous layer was acidified with 0.1N HCl and the resultant precipitate was filtered, washed with water and dried under reduced pressure to afford 2-(tert-butoxycarbonyl)-6-methoxy-1,2,3,4-tetrahydroisoquinoline-7-carboxylic acid (260 mg, 0.85 mmol, 78 % yield) which was used without further purification. LCMS: MS (ES):*m*/*z* = 306.1 [M-H].

### Example 82

Prepared from intermediate **82-5** and **IV-4** according to the general procedure described for **Example** 1 to afford tert-butyl 7-(((1R,2R,3S,4R,Z)-7-(cyclopropylmethylene)-3-((4-fluoro-3-(trifluoromethyl)phenyl) carbamoyl)bicyclo[2.2.1]heptan-2-yl)carbamoyl)-6-methoxy-3,4-dihydroisoquinoline-2(1H)-carboxylate (180 mg, 67 % yield). ¹H NMR (400 MHz, DMSO-d6) δ = 10.49 (s, 1H), 9.78 (d, *J* = 7.1 Hz, 1H), 8.21 (dd, *J=* 2.7, 6.6 Hz, 1H), 7.83 - 7.74 (m, 1H), 7.69 (s, 1H), 7.47 (t, *J=* 9.8 Hz, 1H), 6.97 (s, 1H), 4.67 (d, *J* = 9.5 Hz, 1H), 4.50 - 4.33 (m, 3H), 3.96 (s, 3H), 3.53 (br t, *J=* 5.7 Hz, 2H), 3.14 (dd, *J* = 4.4, 10.8 Hz, 1H), 3.07 (t, *J* = 3.4 Hz, 1H), 2.80 (t*, J =* 5.9 Hz, 2H), 2.70 (t, *J* = 3.9 Hz, 1H), 1.85 (br d, *J* = 9.8 Hz, 1H), 1.80 - 1.72 (m, 1H), 1.49 (br dd, *J* = 4.6*,* 9.5 Hz, 1H), 1.46 - 1.31 (m, 12H), 0.81 - 0.64 (m, 2H), 0.34 (dd, *J* = 2.1, 4.5 Hz, 2H); LCMS: RT = 2.788 min, MS (ES):*m*/*z* = 658.4 [M+H⁺] method B.

### Example 83:

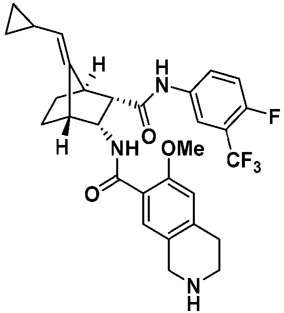

**Example 83:** To a solution of **Example 82** (200 mg, 0.30 mmol) dissolved in DCM (5.0 mL) was added TFA (0.11 mL, 1.5 mmol) at 0°C, then the reaction mixture was stirred at rt for 4 h. The reaction mixture was concentrated under vacuum and was triturated with diethyl ether to generate N-((1R,2R,3S,4R,Z)-7-(cyclopropylmethylene)-3-((4-fluoro-3-(trifluoromethyl)phenyl)carbamoyl) bicyclo[2.2.1]heptan-2-yl)-6-methoxy-1,2,3,4-tetrahydroisoquinoline-7-carboxamide (160 mg, 0.29 mmol, 97 % yield) as an off white solid. LCMS: MS (ES):*m*/*z* = 558.3 [M+H]. 1H NMR (400 MHz, DMSO-d6) δ = 10.48 (s, 1H), 9.75 (d, J = 7.1 Hz, 1H), 8.22 (dd, J = 2.4, 6.6 Hz, 1H), 7.82 - 7.71 (m, 1H), 7.58 (s, 1H), 7.47 (t, J = 9.8Hz, 1H), 6.86 (s, 1H), 4.67 (d, J = 9.8 Hz, 1H), 4.47 - 4.34 (m, 1H), 3.94 (s, 3H), 3.80 (s, 2H), 3.13 (dd, J = 4.2, 10.8 Hz, 1H), 3.09 - 3.04 (m, 1H), 2.99 - 2.89(m, 2H), 2.76 - 2.66 (m, 3H), 1.89 - 1.81 (m, 1H), 1.79 - 1.70 (m, 1H), 1.55 - 1.44 (m, 1H), 1.44 - 1.30 (m, 2H), 0.82 - 0.66 (m, 2H), 0.34 (dd, J = 2.0, 4.6 Hz,2H)

### Example 84:

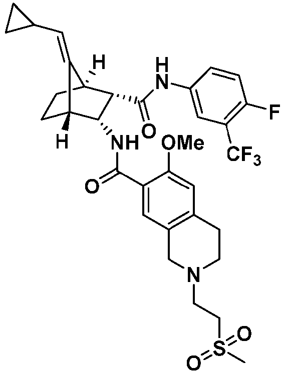

To a solution of **Example 83** (20.0 mg, 0.036 mmol) and 1-chloro-2-(methylsulfonyl)ethane (5.1 mg, 0.036 mmol) in DMF (1.0 mL) and THF (1.0 mL) solvent mixture was added TEA (0.015 mL, 0.11 mmol) at rt, and the reaction mixture stirred for 14h. The reaction mixture was purified via preparative HPLC to afford N-((1R,2R,3 S,4R,Z)-7-(cyclopropylmethylene)-3-((4-fluoro-3-(trifluoromethyl)phenyl)carbamoyl) bicyclo[2.2.1]heptan-2-yl)-6-methoxy-2-(2-(methylsulfonyl)ethyl)-1,2,3,4-tetrahydroisoquinoline-7-carboxamide (6.7 mg, 27%) as a white solid. 1H NMR (400 MHz, DMSO-d6) δ ppm 10.47 (s, 1H), 9.76 (d, J = 7.3 Hz, 1H), 8.21 (dd, J = 2.6, 6.5 Hz, 1H), 7.82 - 7.72 (m, 1H), 7.63 (s, 1H), 7.47 (t, J = 9.8 Hz, 1H), 6.90 (s, 1H), 4.67 (d, J = 9.5 Hz, 1H), 4.49 - 4.37 (m, 1H), 4.09 (q, J = 5.4 Hz, 1H), 3.94 (s, 3H), 3.57 (s, 2H), 3.38 (s, 3H), 3.20 - 3.10 (m, 2H), 3.06 (br t, J = 3.8 Hz, 1H), 2.99 (s, 3H), 2.90 - 2.79 (m, 4H), 2.74 - 2.66 (m, 3H), 1.91 - 1.83 (m, 1H), 1.81 - 1.74 (m, 1H), 1.55 - 1.33 (m, 3H), 0.80 - 0.65 (m, 2H), 0.34 (dd, J = 2.1, 4.8 Hz, 2H). LCMS: RT = 2.491 min, MS (ES):*m*/*z* = 664.3 [M+H] method B.

### Example 85:

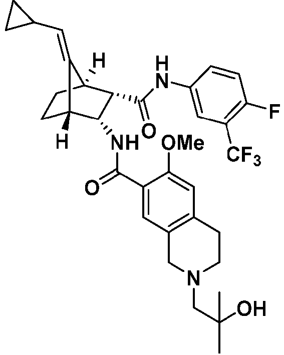

To a solution of **Example 83** and 1-chloro-2-methylpropan-2-ol (3.9 mg, 0.036 mmol) in DMF (1.0 mL) and THF (1.0 mL) was added TEA (0.015 mL, 0.11 mmol) at rt, then the mixture was heated at 80 °C for 14 h. The reaction mixture was allowed to cool, concentrated under reduced pressure and purified via preparative HPLC to afford N-((1R,2R,3 S,4R,Z)-7-(cyclopropylmethylene)-3-((4-fluoro-3-(trifluoromethyl)phenyl) carbamoyl)bicyclo[2.2.1]heptan-2-yl)-2-(2-hydroxy-2-methylpropyl)-6-methoxy-1,2,3,4-tetrahydroisoquinoline-7-carboxamide (1 mg, 5 % yield). 1H NMR (400 MHz, DMSO-d6) δ ppm 10.48 (s, 1H), 9.75 (d, J = 7.6 Hz, 1H), 8.22 (dd, J = 2.3, 6.5 Hz, 1H), 7.83 - 7.72 (m, 1H), 7.57 (s, 1H), 7.48 (t, J = 9.9 Hz, 1H), 6.88 (s, 1H), 4.67 (d, J = 9.8 Hz, 1H), 4.49 - 4.37 (m, 1H), 4.15 (s, 1H), 3.94 (s, 3H), 3.63 (s, 2H), 3.19 - 3.09 (m, 1H), 3.08 - 3.02 (m, 1H), 2.87 - 2.74 (m, 4H), 2.68 (br d, J = 8.6 Hz, 1H), 2.35 (s, 2H), 1.90 - 1.82 (m, 1H), 1.79 - 1.72 (m, 1H), 1.54 - 1.45 (m, 1H), 1.44 - 1.34 (m, 2H), 1.10 (s, 6H), 0.79 - 0.70 (m, 2H), 0.41 - 0.27 (m, 2H). LCMS: RT = 2.573 min, MS (ES):*m*/*z* = 630.4 [M+H] method B.

### Example 88:

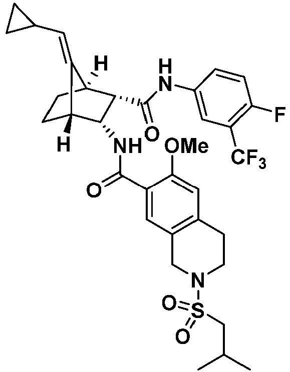

To a solution of **Example 83** (20 mg, 0.036 mmol), and 2-methylpropane-1-sulfonyl chloride (11 mg, 0.072 mmol) in DCM (2.0 mL) was added TEA (0.015 mL, 0.11 mmol) at 0 °C, then the reaction mixture was stirred at rt for 14 h. The reaction mixture concentrated under reduced pressure and purified via preparative HPLC to afford N-((1R,2R,3 S,4R,Z)-7-(cyclopropylmethylene)-3-((4-fluoro-3-(trifluoromethyl)phenyl) carbamoyl)bicyclo[2.2.1]heptan-2-yl)-2-(isobutylsulfonyl)-6-methoxy-1,2,3,4-tetrahydroisoquinoline-7-carboxamide (6.2 mg, 27% yield) as a white solid. 1H NMR (400 MHz, DMSO-d6) δ = 10.50 (s, 1H), 9.80 (d, *J* = 7.1 Hz, 1H), 8.22 (dd, *J=* 2.8, 6.7 Hz, 1H), 7.82 - 7.73 (m, 1H), 7.71 (s, 1H), 7.48 (t, *J=* 9.8 Hz, 1H), 6.99 (s, 1H), 4.68 (d, *J =* 9.8 Hz, 1H), 4.47 - 4.38 (m, 1H), 4.34 (s, 2H), 3.96 (s, 3H), 3.51 - 3.40 (m, 2H), 3.14 (dd, *J=* 4.5, 10.9 Hz, 1H), 3.07 (t, *J =* 3.9 Hz, 1H), 2.98 (d, *J =* 6.6 Hz, 2H), 2.92 (br t, *J* = 6.0 Hz, 2H), 2.70 (t, *J* = 3.7 Hz, 1H), 2.12 (td, *J =* 6.6, 13.4 Hz, 1H), 1.89 - 1.66 (m, 2H), 1.56 - 1.45 (m, 1H), 1.45 - 1.29 (m, 2H), 1.03 (d, *J =* 6.8 Hz, 6H), 0.81 - 0.68 (m, 2H), 0.34 (dd, *J =* 2.0, 4.6 Hz, 2H). LCMS: RT = 2.465 min, MS (ES):*m*/*z* = 658.3 [M+H⁺] method B.

### Example 90:

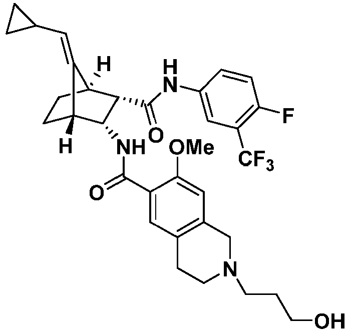

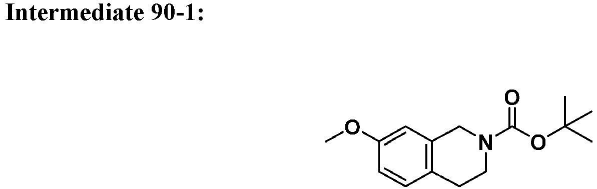

To tert-butyl 7-bromo-3,4-dihydroisoquinoline-2(1H)-carboxylate (4.0 g, 13 mmol), Pd₂(dba)₃ (1.2 g, 1.3 mmol), 2-di-tert-butylphosphino-2',4',6'-triisopropylbiphenyl (0.82 g, 1.9 mmol), methanol (5.2 mL, 130 mmol) and Cs₂CO₃ (21 g, 64 mmol) in dioxane (80.0 mL) was degassed for 5 min, then the reaction mixture was stirred at 85 °C for 12h. The reaction mixture was allowed to cool to rt and diluted with EtOAc The solid was filtered and the filtrate was concentrated under reduced pressure, then purified with silica gel chromatography eluted with 20-25 % EtOAc in to afford tert-butyl 7-methoxy-3,4-dihydroisoquinoline-2(1H)-carboxylate (3.0 g, 11 mmol, 89 % yield. LCMS: MS (ES):*m*/*z* = 164.1 [M+H-Boc].

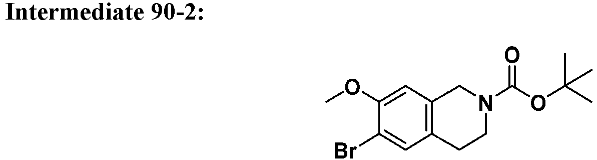

NBS (2.2 g, 12 mmol) was added to a solution of **90-1** (3.2 g, 12 mmol) in acetonitrile (60 mL) at rt and the reaction mixture was stirred at rt for 5h. The reaction mixture was concentrated under reduced pressure, the residue was dissolved in EtOAc, washed with water, dried over Na₂SO₄ and concentrated under reduced pressure, then purified with silica gel chromatography eluted with 25-35 % EtOAc in petroleum ether to afford tert-butyl 6-bromo-7-methoxy-3,4-dihydroisoquinoline-2(1H)-carboxylate (3.0 g, 8.8 mmol, 72 % yield) LCMS: MS (ES):*m*/*z* = 244.0 [M+2H-Boc].

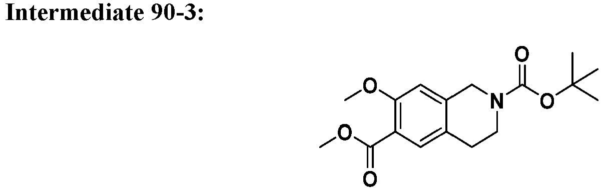

To a solution of 90-2 (2.0 g, 5.8 mmol) dissolved in methanol (70 mL) and DMF (70 mL) and degassed with nitrogen was added TEA (4.1 mL, 29 mmol), Pd(OAc)₂ (0.13 g, 0.58 mmol) and dppf (0.49 g, 0.88 mmol) at rt. The reaction mixture was stirred at under CO (g) at 10 kg pressure and 100 °C in an autoclave for 16 h. The reaction mixture was concentrated under reduced pressure, the residue was diluted with EtOAc and the solid was filtered through celite. The filter cake was washed with EtOAc, and the filtrate concentrated under reduced pressure, then purified with silica gel chromatography eluted with 30-40 % EtOAc in petroleum ether to afford 2-(tert-butyl) 6-methyl 7-methoxy-3,4-dihydroisoquinoline-2,6(1H)-dicarboxylate (1.2 g, 3.7 mmol, 64 % yield). LCMS: MS (ES):*m*/*z* = 322.2 [M+H].

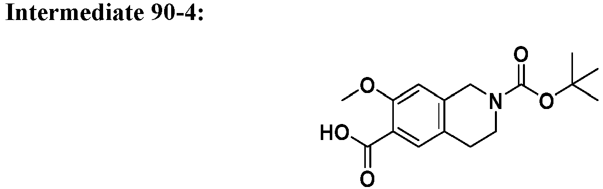

LiOH (0.75 g, 31 mmol) dissolved in water (5.0 mL) was added to a solution of 90-3 (1.0 g, 3.1 mmol) dissolved in methanol (10. mL) and tetrahydrofuran (10. mL). The reaction mixture was stirred for 14 h thenconcentrated under reduced pressure. The residue was diluted with water (10 mL), washed with 20 % EtOAc in petroleum ether (20 mL), the aqueous layer was acidified with 0.1N HCl and the resultant precipitate was filtered, washed with water and dried under reduced pressure to afford 2-(tert-butoxycarbonyl)-7-methoxy-1,2,3,4-tetrahydroisoquinoline-6-carboxylic acid (760 mg, 2.5 mmol, 79 % yield) which was used without further purification. LCMS: MS *(ES):m*/*z* = 306.2 [M-H].

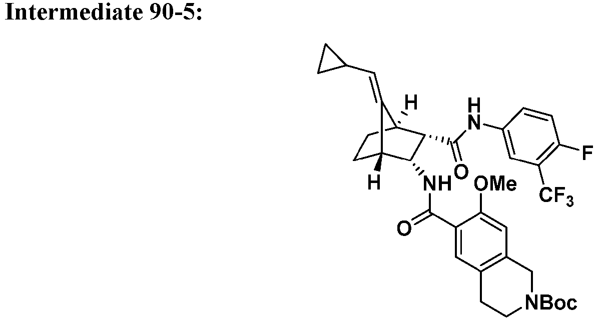

Intermediate 34-5 was prepared from intermediate **90-4** and **IV-4** according to the general procedure described for **Example 1** to afford tert-butyl 6-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-7-methoxy-3,4-dihydroisoquinoline-2(1H)-carboxylate (150 mg, 56% yield) as a white solid. 1H NMR (400 MHz, DMSO-d6) δ ppm 10.48 (s, 1H), 9.77 (d, *J* = 7.3 Hz, 1H), 8.21 (dd, *J* = 2.6, 6.5 Hz, 1H), 7.82 - 7.74 (m, 1H), 7.70 (s, 1H), 7.48 (t, *J* = 9.7 Hz, 1H), 7.01 (s, 1H), 4.67 (d, *J =* 9.5 Hz, 1H), 4.53 (br s, 2H), 4.43 (br s, 1H), 3.95 (s, 3H), 3.53 (br t, *J =* 6.0 Hz, 2H), 3.17 - 3.10 (m, 1H), 3.06 (br s, 1H), 2.76 - 2.67 (m, 3H), 1.90 - 1.82 (m, 1H), 1.79 - 1.71 (m, 1H), 1.53 - 1.46 (m, 1H), 1.45 - 1.29 (m, 11H), 0.79 - 0.64 (m, 2H), 0.34 (dd, *J* = 2.0, 4.6 Hz, 2H). LCMS: RT = 2.788 min, MS (ES):*m*/*z* = 658.3 [M+H] method B.

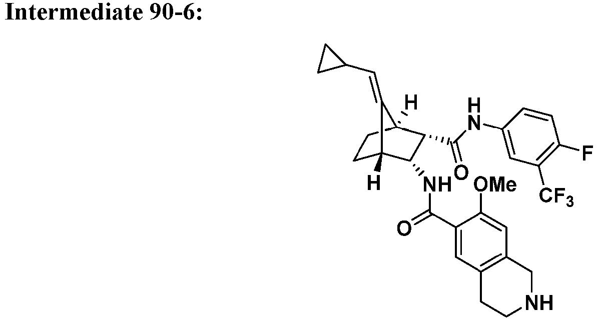

Intermediate 34-6 was prepared from **90-5** according to general procedure described for **Example 82.** N-((1R,2R,3S,4R,Z)-7-(cyclopropylmethylene)-3-((4-fluoro-3-(trifluoromethyl)phenyl)carbamoyl) bicyclo[2.2.1]heptan-2-yl)-7-methoxy-1,2,3,4-tetrahydroisoquinoline-6-carboxamide (150 mg, 0.27 mmol, 98 % yield). LCMS: MS (ES):*m*/*z* = 558.3 [M+H].

### Example 90:

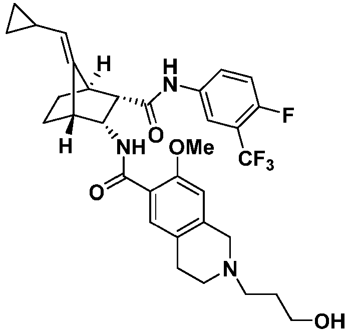

To Intermediate **90-6** (20 mg, 0.036 mmol) and 3-bromopropan-1-ol (5.0 mg, 0.036 mmol) dissolved in THF (1.0 mL) and DMF (1.0 mL) was added TEA (0.015 mL, 0.11 mmol) at 0 °C. The reaction mixture was allowed to warm to rt and stirred at rt for 14 h. The reaction mixture was purified with preparative HPLC to afford N-((1R,2R,3S,4R,Z)-7-(cyclopropylmethylene)-3-((4-fluoro-3-(trifluoromethyl)phenyl)carbamoyl)bicyclo[2.2.1] heptan-2-yl)-2-(3-hydroxypropyl)-7-methoxy-1,2,3,4-tetrahydroisoquinoline-6-carboxamide (10 mg, 40% yield). 1H NMR (400 MHz, DMSO-d6) δ ppm 10.49 (s, 1H), 9.76 (d, J = 7.3 Hz, 1H), 8.22 (dd, J = 2.7, 6.6 Hz, 1H), 7.81 - 7.74 (m, 1H), 7.66 (s, 1H), 7.48 (t, J = 9.8 Hz, 1H), 6.87 (s, 1H), 4.67 (d, J = 9.5 Hz, 1H), 4.53 - 4.32 (m, 2H), 3.93 (s, 3H), 3.57 (br s, 2H), 3.47 (t, J = 6.2 Hz, 2H), 3.18 - 3.11 (m, 1H), 3.08 - 3.02 (m, 1H), 2.77 - 2.59 (m, 5H), 1.85 (br d, J = 9.8 Hz, 1H), 1.81 - 1.58 (m, 3H), 1.53 - 1.28 (m, 3H), 0.82 - 0.65 (m, 2H), 0.41 - 0.30 (m, 2H). LCMS: RT = 1.86 min, MS (ES):*m*/*z* = 616.4 [M+H] method B.

Compounds **2** -to **32, 57** and **58** in Table 2 were prepared by the general procedures described for Example **1, 20, 21, 26,** and or **33**
Compounds **36** -to **39** in Table 2 were prepared by the general procedures described for Example **34** to **35**.Compounds **41** -to **48** in Table 2 were prepared by the general procedures described for Example **40** or **42.**

Compounds **52** in Table 2 were prepared by the general procedures described for Example **51.**

Compounds **54** in Table 2 were prepared by the general procedures described for Example **53.**

Compounds **60** to **70** in Table 3 were prepared by the general procedures described for Example **61** and **65.**

Compounds **73** in Table 2 were prepared by the general procedures described for Example **59.**

Compounds **75** to **76** in Table 3 were prepared by the general procedures described for Example **74.**

Compounds **79** to **80** in Table 3 were prepared by the general procedures described for Example **77.**

Compounds **86** to **95** in Table 3 were prepared by the general procedures described for Examples **84, 85,** and **88**.

**Table 2**

| **Ex. No.** | **Structure** | **Name** | **MS (ESI) (M+H )** | **¹H NMR** | **LC RT^{a} (min) and chiral conditio ns when separat ed** |
|---|---|---|---|---|---|
| 2 | 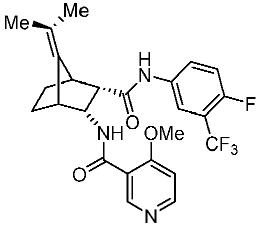 | N-[(2R,3S)-3-{[4-fluoro-3-(trifluoromethy l)phenyl]carba moyl}-7-(propan-2-ylidene)bicyclo [2.2.1]heptan-2-yl]-4-methoxypyridi ne-3-carboxamide | 492.0 | ¹H NMR (500 MHz, DMSO-d6) 10.59 (s, 1H), 9.86 (br d, J=6.7 Hz, 1H), 8.93 (br s, 1H), 8.62 (br s, 1H), 8.20 (br d, J=6.1 Hz, 1H), 7.85 - 7.76 (m, 1H), 7.48 (br t, J=9.6 Hz, 1H), 7.34 (br d, J=4.3 Hz, 1H), 4.37 - 4.29 (m, 1H), 4.09 (s, 3H), 3.15 - 3.08 (m, 1H), 3.07 - 3.01 (m, 1H), 2.98 - 2.90 (m, 1H), 1.83 - 1.62 (m, 8H), 1.43 - 1.27 (m, 2H) | 2.27 Method B |
| 3 | 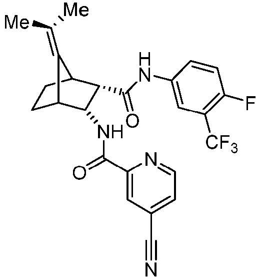 | 4-cyano-N-[(2R,3S)-3-{[4-fluoro-3-(trifluoromethy l)phenyl]carba moyl}-7-(propan-2-ylidene)bicyclo [2.2.1]heptan-2-yl]pyridine-2-carboxamide | 487.4 | ¹H NMR (500 MHz, DMSO-d6) 10.55 (br s, 1H), 10.22 (br d, J=7.0 Hz, 1H), 8.90 (br d, J=3.1 Hz, 1H), 8.28 (s, 1H), 8.02 (br s, 1H), 7.97 (br d, J=5.9 Hz, 1H), 7.84 - 7.72 (m, 1H), 7.45 (br t, J=9.6 Hz, 1H), 4.37 - 4.24 (m, 1H), 3.14 - 3.08 (m, 1H), 3.07 - 3.01 (m, 1H), 2.95 - 2.88 (m, 1H), 1.79 - 1.60 (m, 8H), 1.43 - 1.29 (m, 2H) | 2.42 |
| 4 | 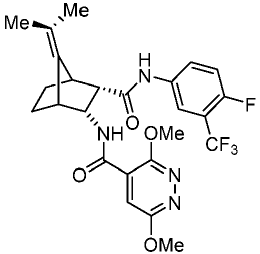 | N-[(2R,3S)-3-{[4-fluoro-3-(trifluoromethy l)phenyl]carba moyl}-7-(propan-2-ylidene)bicyclo [2.2.1]heptan-2-yl]-3,6-dimethoxypyri dazine-4-carboxamide | 523.1 | ¹H NMR (500 MHz, CDCl₃) 9.85 (br d, J=7.2 Hz, 1H), 7.93 (dd, J=6.1, 2.5 Hz, 1H), 7.68 (s, 1H), 7.65 - 7.56 (m, 2H), 7.15 (t, J=9.3 Hz, 1H), 4.61 - 4.52 (m, 1H), 4.27 (s, 3H), 4.08 (s, 3H), 3.09 - 3.03 (m, 1H), 3.01 - 2.94 (m, 2H), 2.04 - 1.96 (m, 1H), 1.82 - 1.75 (m, 1H), 1.73 (s, 3H), 1.72 (s, 3H), 1.57 - 1.48 (m, 2H) | 1.19 Method B |
| 5 | 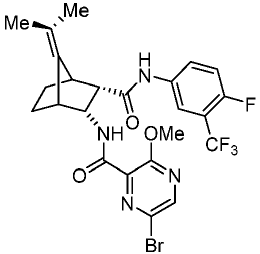 | 6-bromo-N-[(2R,3S)-3-{[4-fluoro-3-(trifluoromethy l)phenyl]carba moyl}-7-(propan-2-ylidene)bicyclo [2.2.1]heptan-2-yl]-3-methoxypyrazi ne-2-carboxamide | 570.9 | ¹H NMR (500 MHz, DMSO-d6) 10.53 (s, 1H), 9.65 (d, J=7.3 Hz, 1H), 8.54 (s, 1H), 8.14 (dd, J=6.4, 2.1 Hz, 1H), 7.81 - 7.72 (m, 1H), 7.45 (t, J=9.8 Hz, 1H), 4.33 - 4.22 (m, 1H), 3.94 (s, 3H), 3.08 (dd, J=10.4, 4.3 Hz, 1H), 3.05 - 2.99 (m, 1H), 2.94 - 2.86 (m, 1H), 1.79 - 1.73 (m, 1H), 1.73 - 1.68 (m, 7H), 1.39 - 1.29 (m, 2H) | 2.60 Method B |
| 6 | 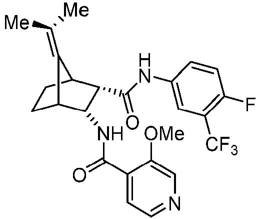 | N-[(2R,3S)-3-{[4-fluoro-3-(trifluoromethy l)phenyl]carba moyl}-7-(propan-2-ylidene)bicyclo [2.2.1]heptan-2-yl]-3-methoxypyridi ne-4-carboxamide | 492.4 | ¹H NMR (500 MHz, DMSO-d6) 10.56 (s, 1H), 9.94 (d, J=7.0 Hz, 1H), 8.58 (s, 1H), 8.32 (d, J=4.6 Hz, 1H), 8.19 (d, J=6.1 Hz, 1H), 7.82 - 7.78 (m, 1H), 7.76 (d, J=4.6 Hz, 1H), 7.48 (t, J=9.6 Hz, 1H), 4.37 - 4.28 (m, 1H), 4.10 (s, 3H), 3.10 (dd, J=10.4, 4.0 Hz, 1H), 3.06 - 3.01 (m, 1H), 2.96 - 2.90 (m, 1H), 1.81 - 1.75 (m, 1H), 1.75 - 1.65 (m, 7H), 1.40 - 1.29 (m, 2H) | 2.02 |
| 7 | 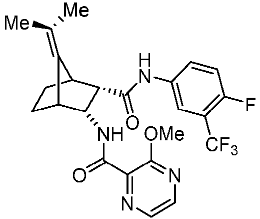 | N-[(2R,3S)-3-{[4-fluoro-3-(trifluoromethy l)phenyl]carba moyl}-7-(propan-2-ylidene)bicyclo [2.2.1]heptan-2-yl]-3-methoxypyrazi ne-2-carboxamide | 493.0 | ¹H NMR (500 MHz, DMSO-d6) 10.51 (s, 1H), 9.61 (br d, J=7.0 Hz, 1H), 8.38 (br d, J=1.5 Hz, 1H), 8.30 - 8.26 (m, 1H), 8.04 (d, J=4.6 Hz, 1H), 7.85 - 7.74 (m, 1H), 7.46 (t, J=9.8 Hz, 1H), 4.34 - 4.24 (m, 1H), 3.93 (s, 3H), 3.10 (dd, J=10.4, 3.7 Hz, 1H), 3.06 - 3.00 (m, 1H), 2.96 - 2.86 (m, 1H), 1.81 - 1.65 (m, 8H), 1.43 - 1.28 (m, 2H) | 2.36 Method B |
| 8 | 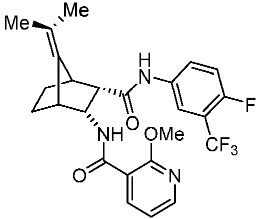 | N-[(2R,3S)-3-{[4-fluoro-3-(trifluoromethy l)phenyl]carba moyl}-7-(propan-2-ylidene)bicyclo [2.2.1]heptan-2-yl]-2-methoxypyridi ne-3-carboxamide | 492.1 | ¹H NMR (500 MHz, DMSO-d6) 10.53 (s, 1H), 9.96 (br d, J=6.7 Hz, 1H), 8.35 - 8.25 (m, 2H), 8.20 (d, J=4.3 Hz, 1H), 7.84 - 7.74 (m, 1H), 7.47 (t, J=9.8 Hz, 1H), 7.14 (dd, J=7.3, 5.2 Hz, 1H), 4.36 - 4.28 (m, 1H), 4.06 (s, 3H), 3.09 (dd, J=10.5, 3.8 Hz, 1H), 3.05 - 2.99 (m, 1H), 2.95 - 2.88 (m, 1H), 1.85 - 1.77 (m, 1H), 1.76 - 1.64 (m, 7H), 1.41 - 1.27 (m, 2H) | 2.60 Method B |
| 9 | 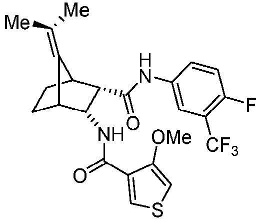 | N-[(2R,3S)-3-{[4-fluoro-3-(trifluoromethy l)phenyl]carba moyl}-7-(propan-2-ylidene)bicyclo [2.2.1]heptan-2-yl]-4-methoxythioph ene-3-carboxamide | 497.2 | ¹H NMR (500 MHz, DMSO-d6) 10.49 (s, 1H), 9.38 (d, J=7.0 Hz, 1H), 8.22 (d, J=4.6 Hz, 1H), 7.99 (d, J=3.4 Hz, 1H), 7.80 - 7.72 (m, 1H), 7.47 (t, J=9.8 Hz, 1H), 6.75 (d, J=3.4 Hz, 1H), 4.35 - 4.22 (m, 1H), 3.91 (s, 3H), 3.06 (dd, J=10.5, 3.8 Hz, 1H), 3.03 - 2.97 (m, 1H), 2.91 - 2.82 (m, 1H), 1.86 - 1.78 (m, 1H), 1.75 - 1.62 (m, 7H), 1.41 - 1.26 (m, 2H) | 2.50 Method B |
| 10 | 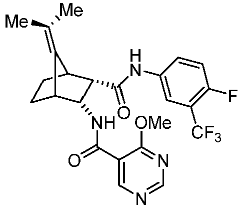 | N-[(2R,3S)-3-{[4-fluoro-3-(trifluoromethy l)phenyl]carba moyl}-7-(propan-2-ylidene)bicyclo [2.2.1]heptan-2-yl]-4-methoxypyrimi dine-5-carboxamide | 493.4 | ¹H NMR (500 MHz, DMSO-d6) 10.57 (s, 1H), 9.88 (br d, J=7.0 Hz, 1H), 8.98 (s, 1H), 8.90 (s, 1H), 8.20 (d, J=4.3 Hz, 1H), 7.85 - 7.78 (m, 1H), 7.48 (t, J=9.8 Hz, 1H), 4.35 - 4.27 (m, 1H), 4.12 (s, 3H), 3.10 (dd, J=10.7, 4.3 Hz, 1H), 3.06 - 3.02 (m, 1H), 2.96 - 2.91 (m, 1H), 1.79 - 1.69 (m, 8H), 1.42 - 1.28 (m, 2H) | 2.34 Method B |
| 11 | 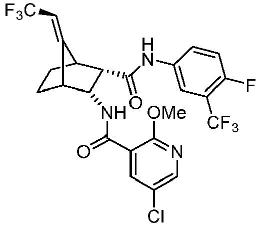 | 5-chloro-N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-2-methoxypyridi ne-3-carboxamide | 566.3 | ¹H NMR (500 MHz, DMSO-d6) δ 8.38 (d, J=2.4 Hz, 1H), 8.28 - 8.16 (m, 2H), 7.78 (br d, J=8.5 Hz, 1H), 7.49 (br t, J=9.6 Hz, 1H), 5.93 (q, J=7.7 Hz, 1H), 4.53 - 4.38 (m, 1H), 4.06 (s, 3H), 3.48 (br d, J=7.6 Hz, 1H), 3.33 - 3.14 (m, 2H), 2.99 (br s, 1H), 1.98 - 1.76 (m, 2H), 1.48 (br d, J=5.2 Hz, 2H) | 2.81 |
| 12 | 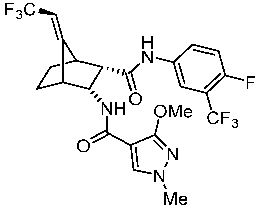 | N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-3-methoxy-1-methyl-1H-pyrazole-4-carboxamide | 535.2 | ¹H NMR (500 MHz, DMSO-d6) δ 10.60 (s, 1H), 8.75 (d, J=7.3 Hz, 1H), 8.24 (dd, J=6.4, 2.1 Hz, 1H), 7.91 (s, 1H), 7.82 - 7.70 (m, 1H), 7.50 (t, J=9.8 Hz, 1H), 5.92 (q, J=7.8 Hz, 1H), 4.50 - 4.33 (m, 1H), 4.02 - 3.87 (m, 3H), 3.70 (s, 3H), 3.22 (br dd, J=10.7, 4.0 Hz, 1H), 3.13 (br s, 1H), 2.96 (br s, 1H), 2.06 - 1.93 (m, 1H), 1.89 - 1.79 (m, 1H), 1.58 - 1.41 (m, 2H) | 2.23 |
| 13 | 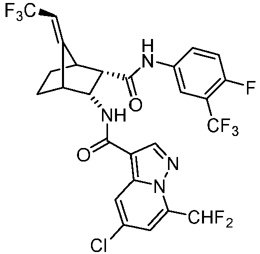 | 5-chloro-7-(difluoromethy l)-N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]pyrazolo[1,5 -a]pyridine-3-carboxamide | 625.3 | ¹H NMR (500 MHz, DMSO-d6) δ 10.73 (s, 1H), 9.79 (br d, J=6.7 Hz, 1H), 8.34 (br d, J=4.9 Hz, 1H), 8.17 (s, 1H), 7.70 (br s, 1H), 7.53 - 7.45 (m, 2H), 7.51 (br t, J=52.9 Hz, 1H), 7.12 (s, 1H), 5.95 (q, J=7.6 Hz, 1H), 4.47 (br s, 1H), 3.23 (br s, 1H), 3.04 - 2.98 (m, 1H), 1.88 (br d, J=7.9 Hz, 2H), 1.52 (br d, J=8.2 Hz, 2H) | 2.65 |
| 14 | 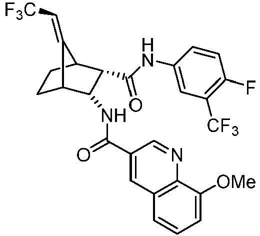 | N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-8-methoxyquinol ine-3-carboxamide | 582.3 | ¹H NMR (500 MHz, DMSO-d6) δ 10.66 (s, 1H), 9.42 (br d, J=7.2 Hz, 1H), 9.11 (d, J=1.9 Hz, 1H), 8.67 (d, J=2.0 Hz, 1H), 8.09 (br d, J=4.0 Hz, 1H), 7.89 - 7.75 (m, 1H), 7.66 - 7.58 (m, 2H), 7.50 (t, J=9.8 Hz, 1H), 7.33 (d, J=7.1 Hz, 1H), 5.97 (q, J=7.9 Hz, 1H), 4.72 - 4.53 (m, 1H), 4.00 (s, 3H), 3.33 (br dd, J=10.6, 4.6 Hz, 1H), 3.04 (br s, 1H), 2.14 (br t, J=8.9 Hz, 1H), 2.02 (br t, J=9.0 Hz, 1H), 1.55 (br d, J=7.3 Hz, 2H) | 2.03 |
| 15 | 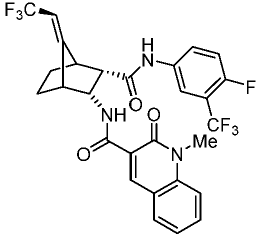 | N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-1-methyl-2-oxo-1,2-dihydroquinoli ne-3-carboxamide | 582.2 | ¹H NMR (500 MHz, DMSO-d6) δ 10.73 (d, J=7.9 Hz, 1H), 10.40 (s, 1H), 8.76 (s, 1H), 8.04 (dd, J=6.4, 2.1 Hz, 1H), 7.96 (d, J=7.0 Hz, 1H), 7.78 (t, J=7.9 Hz, 1H), 7.74 (d, J=8.1 Hz, 1H), 7.65 (d, J=8.5 Hz, 1H), 7.44 (t, J=9.2 Hz, 1H), 7.37 (t, J=7.5 Hz, 1H), 5.94 (q, J=7.7 Hz, 1H), 4.81 - 4.63 (m, 1H), 3.73 (s, 3H), 3.26 - 3.14 (m, 1H), 2.98 (br s, 1H), 2.32 (br t, J=9.2 Hz, 1H), 1.99 (br t, J=8.9 Hz, 1H), 1.63 - 1.46 (m, 2H) | 2.44 |
| 16 | 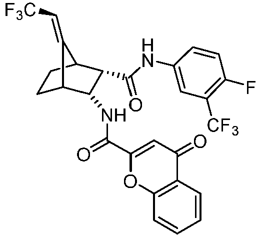 | N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-4-oxo-4H-chromene-2-carboxamide | 569.3 | 1H NMR (500 MHz, DMSO-d6) δ 8.21 (br d, J=4.8 Hz, 1H), 8.08 (d, J=7.9 Hz, 1H), 7.91 (t, J=7.9 Hz, 1H), 7.88 - 7.85 (m, 1H), 7.62 - 7.50 (m, 3H), 6.82 (s, 1H), 6.05 - 5.92 (m, 1H), 4.42 (br s, 1H), 3.51 (br s, 1H), 3.26 (br s, 1H), 3.06 (br s, 1H), 1.95 (br d, J=10.9 Hz, 1H), 1.90 - 1.82 (m, 1H), 1.54 (br s, 2H) | 2.48 Method B |
| 17 | 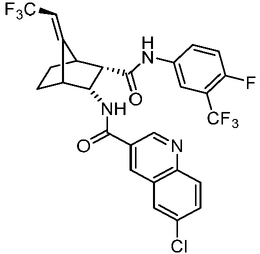 | 6-chloro-N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]quinoline-3-carboxamide | 586.1 | ¹H NMR (500 MHz, DMSO-d6) d 10.61 (s, 1H), 9.40 (br d, J=6.7 Hz, 1H), 9.16 (s, 1H), 8.69 (s, 1H), 8.22 (s, 1H), 8.11 (d, J=8.7 Hz, 1H), 8.07 - 8.04 (m, 1H), 7.90 - 7.80 (m, 2H), 7.48 (br t, J=9.8 Hz, 1H), 5.96 (q, J=7.7 Hz, 1H), 3.39 (br s, 1H), 3.36 - 3.27 (m, 1H), 3.23 (br s, 1H), 2.18 - 2.09 (m, 1H), 2.07 - 1.96 (m, 1H), 1.54 (br d, J=7.6 Hz, 2H) | 2.61 |
| 18 | 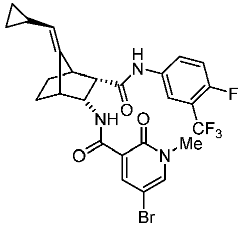 | 5-bromo-N-[(2R,3S,7Z)-7-(cyclopropylm ethylidene)-3-{[4-fluoro-3-(trifluoromethy l)phenyl]carba moyl}bicyclo[ 2.2.1]heptan-2-yl]-1-methyl-2-oxo-1,2-dihydropyridin e-3-carboxamide | 581.9 | ¹H NMR (500 MHz, DMSO-d6) δ 10.52 (d, J=8.2 Hz, 1H), 10.25 (s, 1H), 8.38 (d, J=2.7 Hz, 1H), 8.20 (d, J=2.7 Hz, 1H), 7.99 (d, J=6.7 Hz, 1H), 7.80 - 7.64 (m, 1H), 7.44 (t, J=9.8 Hz, 1H), 4.66 (d, J=9.5 Hz, 1H), 4.58 - 4.44 (m, 1H), 3.56 (s, 3H), 3.08 (dd, J=10.5, 3.8 Hz, 1H), 3.03 - 2.93 (m, 1H), 2.67 (br s, 1H), 2.12 (br t, J=9.0 Hz, 1H), 1.88 - 1.68 (m, 1H), 1.60 - 1.44 (m, 2H), 1.25 (m, 1H), 0.79 - 0.66 (m, 2H), 0.34 (br d, J=2.4 Hz, 2H) | 2.35 |
| 19 | 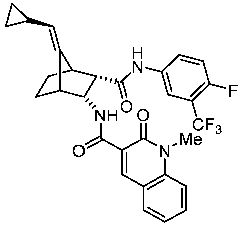 | N-[(2R,3S,7Z)-7-(cyclopropylm ethylidene)-3-{[4-fluoro-3-(trifluoromethy l)phenyl]carba moyl}bicyclo[ 2.2.1]heptan-2-yl]-1-methyl-2-oxo-1,2-dihydroquinoli ne-3-carboxamide | 554.0 | 1H NMR (500 MHz, DMSO-d6) d 10.66 (d, J=8.5 Hz, 1H), 10.29 (s, 1H), 8.76 (s, 1H), 8.02 (d, J=6.6 Hz, 1H), 7.97 (d, J=7.8 Hz, 1H), 7.85 - 7.70 (m, 2H), 7.65 (d, J=8.5 Hz, 1H), 7.43 (t, J=9.7 Hz, 1H), 7.37 (t, J=7.6 Hz, 1H), 4.68 (d, J=9.8 Hz, 1H), 4.64 - 4.43 (m, 1H), 3.74 (s, 3H), 3.12 (br dd, J=11.1, 3.8 Hz, 1H), 3.05 (t, J=4.1 Hz, 1H), 2.79 - 2.63 (m, 1H), 2.16 (br t, J=8.7 Hz, 1H), 2.03 - 1.83 (m, 1H), 1.56 - 1.47 (m, 2H), 1.43 (br d, J=13.1 Hz, 1H), 0.84 - 0.64 (m, 2H), 0.41 - 0.31 (m, 2H) | 2.52 |
| 22 | 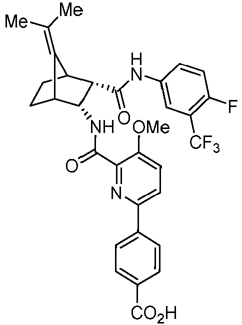 | 4-(6-{[(2R,3S)-3-{[4-fluoro-3-(trifluoromethy l)phenyl]carba moyl}-7-(propan-2-ylidene)bicyclo [2.2.1]heptan-2-yl]carbamoyl }-5-methoxypyridi n-2-yl)benzoic acid | 612.2 | ¹H NMR (500 MHz, DMSO-d6) δ 10.59 (s, 1H), 9.98 (br d, J=7.0 Hz, 1H), 8.35 - 8.26 (m, 2H), 8.20 (d, J=8.9 Hz, 1H), 8.05 (d, J=4.0 Hz, 1H), 7.99 - 7.88 (m, 3H), 7.73 (d, J=8.5 Hz, 1H), 7.52 - 7.40 (m, 1H), 4.32 - 4.21 (m, 1H), 3.88 (s, 3H), 3.19 - 3.11 (m, 1H), 3.08 - 3.02 (m, 1H), 2.98 - 2.91 (m, 1H), 1.83 - 1.66 (m, 8H), 1.44 - 1.28 (m, 2H) | 2.15 |
| 23 | 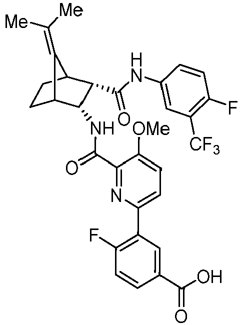 | 4-Fluoro-3-(6-{[(2R,3 S)-3-{[4-fluoro-3-(trifluoromethy l)phenyl]carba moyl}-7-(propan-2-ylidene)bicyclo [2.2.1]heptan-2-yl]carbamoyl }-5-methoxypyridi n-2-yl)benzoic acid | 630.1 | ¹H NMR (500 MHz, DMSO-d6) δ 10.49 (br s, 1H), 9.66 (br d, J=6.7 Hz, 1H), 8.64 - 8.48 (m, 1H), 8.06 - 7.87 (m, 3H), 7.82 - 7.64 (m, 2H), 7.48 - 7.29 (m, 2H), 4.34 - 4.23 (m, 1H), 3.79 (s, 2H), 3.12 - 3.05 (m, 1H), 3.04 - 2.98 (m, 1H), 2.97 - 2.91 (m, 1H), 1.85 - 1.64 (m, 8H), 1.43 - 1.26 (m, 2H) | 2.00 Method B |
| 24 | 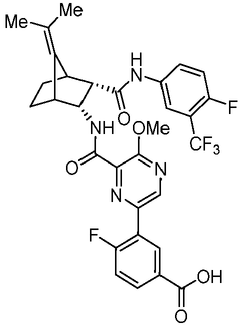 | 4-Fluoro-3-(6-{[(2R,3 S)-3-{[4-fluoro-3-(trifluoromethy l)phenyl]carba moyl}-7-(propan-2-ylidene)bicyclo [2.2.1]heptan-2-yl]carbamoyl }-5-methoxypyrazi n-2-yl)benzoic acid | 631.5 | 1H NMR (500 MHz, DMSO-d6) 10.49 (s, 1H), 9.82 (d, J=7.3 Hz, 1H), 8.80 (d, J=2.1 Hz, 1H), 8.57 (dd, J=7.5, 2.0 Hz, 1H), 8.10 - 8.01 (m, 2H), 7.85 (br dd, J=8.2, 4.0 Hz, 1H), 7.50 (dd, J=10.7, 8.9 Hz, 1H), 7.40 (t, J=9.8 Hz, 1H), 4.38 - 4.32 (m, 1H), 4.01 (s, 3H), 3.12 (dd, J=10.7, 4.3 Hz, 1H), 3.08 - 3.04 (m, 1H), 2.99 - 2.95 (m, 1H), 1.83 - 1.77 (m, 2H), 1.75 (s, 3H), 1.73 (s, 3H), 1.44 - 1.28 (m, 2H) | 2.12 Method B |
| 25 | 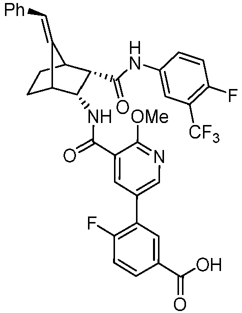 | 4-Fluoro-3-(5-{[(2R,3S,7Z)-3-{[4-fluoro-3-(trifluoromethy l)phenyl]carba moyl}-7-(phenylmethyli dene)bicyclo[2 .2.1]heptan-2-yl]carbamoyl }-6-methoxypyridi n-3-yl)benzoic acid | 678.2 | ¹H NMR (500 MHz, DMSO-d6) δ 13.19 (br s, 1H), 10.63 (s, 1H), 10.16 (d, J=6.9 Hz, 1H), 8.58 (dd, J=2.2, 1.4 Hz, 1H), 8.48 (dd, J=2.3, 1.2 Hz, 1H), 8.27 (dd, J=6.5, 2.6 Hz, 1H), 8.09 (dd, J=7.7, 2.2 Hz, 1H), 8.02 (ddd, J=8.5, 4.7, 2.2 Hz, 1H), 7.84 (dt, J=8.4, 3.6 Hz, 1H), 7.55 - 7.45 (m, 2H), 7.39 (d, J=4.4 Hz, 4H), 7.26 (quin, J=4.3 Hz, 1H), 6.40 (s, 1H), 4.64 - 4.46 (m, 2H), 4.16 (s, 3H), 3.45 - 3.40 (m, 1H), 2.99 - 2.93 (m, 1H), 1.96 - 1.85 (m, 2H), 1.62 - 1.50 (m, 2H) | 2.70 |
| 27 | 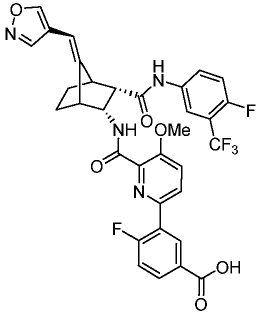 | 4-Fluoro-3-(6-{[(2R,3S,7Z)-3-{ [4-fluoro-3-(trifluoromethy l)phenyl]carba moyl }-7-[(1,2-oxazol-4-yl)methylidene ]bicyclo[2.2.1] heptan-2-yl]carbamoyl }-5-methoxypyridi n-2-yl)benzoic acid | 669.2 | ¹H NMR (500 MHz, DMSO-d6) δ 10.57 (s, 1H), 9.73 (br d, J=7.6 Hz, 1H), 9.05 (s, 1H), 8.78 (s, 1H), 8.65 - 8.61 (m, 1H), 8.06 - 8.01 (m, J=4.6 Hz, 2H), 7.99 (br d, J=7.9 Hz, 1H), 7.85 - 7.79 (m, 1H), 7.77 (d, J=8.9 Hz, 1H), 7.49 - 7.37 (m, 2H), 6.15 (s, 1H), 4.54 - 4.41 (m, 1H), 3.91 (s, 3H), 3.32 - 3.24 (m, 2H), 2.97 - 2.91 (m, 1H), 2.03 - 1.87 (m, 2H) | 2.09 |
| 28 | 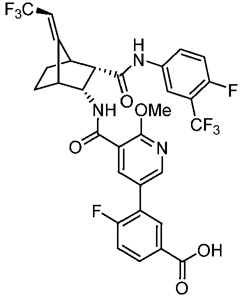 | 4-Fluoro-3-(5-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl }-6-methoxypyridi n-3-yl)benzoic acid | 670.0 | ¹H NMR (500 MHz, DMSO-d6) δ 10.66 (s, 1H), 10.08 (br d, J=6.7 Hz, 1H), 8.57 (s, 1H), 8.46 (s, 1H), 8.24 (br d, J=4.3 Hz, 1H), 8.08 (br d, J=7.0 Hz, 1H), 8.01 (br d, J=4.9 Hz, 1H), 7.85 - 7.77 (m, 1H), 7.56 - 7.43 (m, 2H), 5.95 (q, J=7.7 Hz, 1H), 4.52 (br d, J=4.0 Hz, 1H), 4.14 (s, 3H), 3.00 (br d, J=5.8 Hz, 1H), 2.02 - 1.83 (m, 2H), 1.50 (br d, J=3.7 Hz, 2H) | 2.53 |
| 29 | 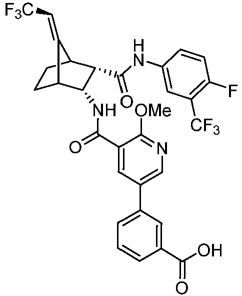 | 3-(5-{[(2R,3S,7Z)-3-{ [4-fluoro-3-(trifluoromethy l)phenyl]carba moyl}-7-(2,2,2-trifluoroethylid ene)bicyclo[2. 2.1]heptan-2-yl]carbamoyl }-6-methoxypyridi n-3-yl)benzoic acid | 652.2 | ¹H NMR (500 MHz, DMSO-d6) δ 10.67 (s, 1H), 10.09 (br d, J=6.7 Hz, 1H), 8.70 (d, J=2.1 Hz, 1H), 8.55 (d, J=2.1 Hz, 1H), 8.24 (br d, J=4.3 Hz, 1H), 8.17 (s, 1H), 7.96 (br t, J=6.7 Hz, 2H), 7.81 (br d, J=8.5 Hz, 1H), 7.62 (t, J=7.6 Hz, 1H), 7.50 (br t, J=9.8 Hz, 1H), 5.95 (q, J=7.9 Hz, 1H), 4.54 (br s, 1H), 4.14 (s, 3H), 3.00 (br d, J=8.2 Hz, 1H) | 2.48 |
| 30 | 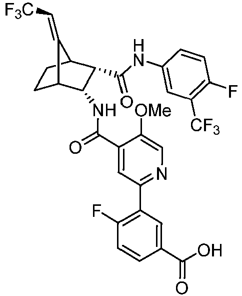 | 4-Fluoro-3-(4-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl }-5-methoxypyridin-2-yl)benzoic acid | 670.0 | ¹H NMR (500 MHz, DMSO-d6) δ 10.69 (s, 1H), 10.03 (br d, J=6.7 Hz, 1H), 8.77 (s, 1H), 8.55 (br d, J=7.3 Hz, 1H), 8.31 - 8.15 (m, 2H), 8.00 (br s, 1H), 7.83 - 7.71 (m, 1H), 7.55 - 7.33 (m, 2H), 5.93 (q, J=7.5 Hz, 1H), 4.51 (br s, 1H), 4.18 (s, 3H), 3.35 - 3.20 (m, 2H), 3.00 (br s, 1H), 2.03 - 1.82 (m, 2H), 1.50 (br d, J=5.2 Hz, 2H) | 2.33 |
| 31 | 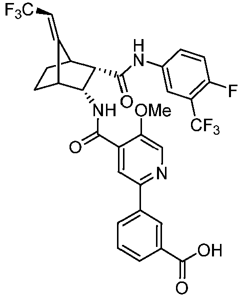 | 3-(4-{[(2R,3S,7Z)-3-{ [4-fluoro-3-(trifluoromethy l)phenyl]carba moyl}-7-(2,2,2-trifluoroethylid ene)bicyclo[2. 2.1]heptan-2-yl]carbamoyl }-5-methoxypyridi n-2-yl)benzoic acid | 652.0 | ¹H NMR (500 MHz, DMSO-d6) δ 10.70 (s, 1H), 10.05 (br d, J=6.7 Hz, 1H), 8.72 (s, 1H), 8.57 (s, 1H), 8.30 (s, 1H), 8.27 - 8.16 (m, 2H), 8.03 - 7.91 (m, 1H), 7.80 (br d, J=8.2 Hz, 1H), 7.60 (t, J=7.6 Hz, 1H), 7.50 (br t, J=9.8 Hz, 1H), 5.95 (q, J=7.8 Hz, 1H), 4.54 (br s, 1H), 4.17 (s, 3H), 3.35 - 3.25 (m, 1H), 3.01 (br s, 1H), 2.00 - 1.83 (m, 2H), 1.50 (br d, J=5.5 Hz, 2H) | 2.38 |
| 32 | 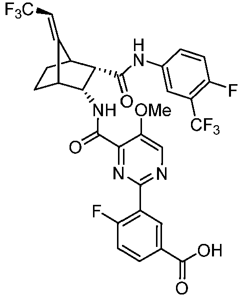 | 4-Fluoro-3-(4-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl }-5-methoxypyrimi din-2-yl)benzoic acid | 671.1 | ¹H NMR (500 MHz, DMSO-d6) δ 10.62 (s, 1H), 9.79 (d, J=7.2 Hz, 1H), 8.98 (s, 1H), 8.63 (dd, J=7.4, 2.0 Hz, 1H), 8.17 - 8.02 (m, 2H), 7.85 - 7.72 (m, 1H), 7.51 - 7.35 (m, 2H), 5.95 (q, J=7.8 Hz, 1H), 4.51 (dt, J=10.7, 5.3 Hz, 1H), 4.01 (s, 3H), 3.28 (dd, J=10.6, 4.4 Hz, 1H), 3.23 (br s, 1H), 3.00 (s, 1H), 2.03 - 1.86 (m, 2H), 1.51 (br d, J=8.1 Hz, 2H) | 2.23 |
| 36 | 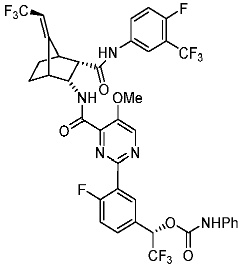 | (1S)-2,2,2-trifluoro-1-[4-fluoro-3-(4-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl}-5-methoxypyrimi din-2-yl)phenyl]ethyl N-phenylcarbama te | 844.3 | ¹H NMR (500 MHz, DMSO-d6) δ 10.59 (s, 1H), 10.30 (br s, 1H), 9.69 (br d, J=7.3 Hz, 1H), 8.99 (s, 1H), 8.25 (br d, J=5.5 Hz, 1H), 8.08 (dd, J=6.3, 2.3 Hz, 1H), 7.77 (br dd, J=4.6, 2.7 Hz, 2H), 7.54 - 7.38 (m, 4H), 7.30 (t, J=7.9 Hz, 2H), 7.04 (br t, J=7.3 Hz, 1H), 6.62 - 6.48 (m, 1H), 5.95 (q, J=7.8 Hz, 1H), 4.61 - 4.46 (m, 1H), 3.29 (br dd, J=10.7, 4.0 Hz, 1H), 3.23 (br s, 1H), 3.03 - 2.92 (m, 1H), 2.04 - 1.88 (m, 2H), 1.49 (br d, J=3.7 Hz, 2H) | 2.62 |
| 37 | 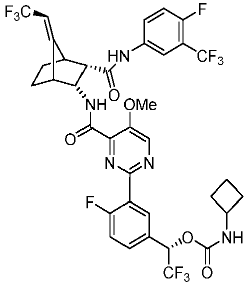 | (1S)-2,2,2-trifluoro-1-[4-fluoro-3-(4-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl }-5-methoxypyrimi din-2-yl)phenyl]ethyl N-cyclobutylcarb amate | 822.3 | ¹H NMR (500 MHz, DMSO-d6) δ 10.60 (s, 1H), 9.69 (br d, J=7.6 Hz, 1H), 8.99 (s, 1H), 8.16 (br d, J=7.6 Hz, 2H), 8.09 (br d, J=4.6 Hz, 1H), 7.84 - 7.75 (m, 1H), 7.73 - 7.64 (m, 1H), 7.48 - 7.36 (m, 2H), 6.40 - 6.30 (m, 1H), 5.96 (q, J=7.8 Hz, 1H), 4.59 - 4.45 (m, 1H), 4.02 (s, 3H), 3.97 - 3.88 (m, 1H), 3.34 - 3.27 (m, 1H), 3.26 - 3.17 (m, 1H), 3.01 (s, 1H), 2.23 - 2.03 (m, 2H), 2.02 - 1.83 (m, 4H), 1.63 - 1.44 (m, 4H) | 2.60 |
| 38 | 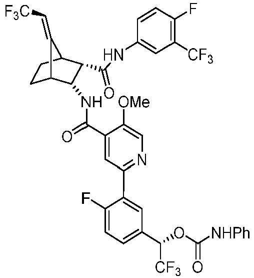 | (1S)-2,2,2-trifluoro-1-[4-fluoro-3-(4-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl }-5-methoxypyridi n-2-yl)phenyl]ethyl N-phenylcarbama te | 842.9 | ¹H NMR (500 MHz, DMSO-d6) δ 10.69 (s, 1H), 10.31 (br s, 1H), 10.06 (d, J=6.7 Hz, 1H), 8.78 (s, 1H), 8.30 - 8.19 (m, 3H), 7.80 (br dd, J=7.6, 4.0 Hz, 1H), 7.70 (br d, J=3.4 Hz, 1H), 7.56 - 7.42 (m, 4H), 7.30 (t, J=7.9 Hz, 2H), 7.04 (t, J=7.3 Hz, 1H), 6.57 (q, J=6.8 Hz, 1H), 5.96 (q, J=7.9 Hz, 1H), 4.58 - 4.48 (m, 1H), 4.20 (s, 3H), 3.35 - 3.24 (m, 1H), 3.01 (br d, J=4.0 Hz, 1H), 2.02 - 1.85 (m, 2H), 1.51 (br d, J=6.1 Hz, 2H) | 2.83 |
| 39 | 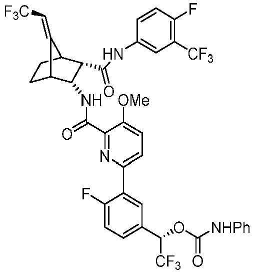 | (1S)-2,2,2-trifluoro-1-[4-fluoro-3-(6-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl }-5-methoxypyridi n-2-yl)phenyl]ethyl N-phenylcarbama te | 843.1 | ¹H NMR (500 MHz, DMSO-d6) δ 10.59 (s, 1H), 10.23 (br s, 1H), 9.61 (br d, J=7.6 Hz, 1H), 8.24 (br d, J=5.5 Hz, 1H), 8.02 - 7.90 (m, 2H), 7.81 - 7.63 (m, 3H), 7.54 - 7.44 (m, 1H), 7.41 (br d, J=8.2 Hz, 2H), 7.35 (br t, J=9.6 Hz, 1H), 7.27 (br t, J=7.9 Hz, 2H), 7.03 (br t, J=7.3 Hz, 1H), 6.46 - 6.33 (m, 1H), 5.92 (q, J=7.8 Hz, 1H), 4.50 (br t, J=6.7 Hz, 1H), 3.89 (s, 3H), 3.34 - 3.17 (m, 2H), 2.98 (br s, 1H), 1.99 (br d, J=10.1 Hz, 2H), 1.60 - 1.37 (m, 2H) | 2.74 |
| 41 | 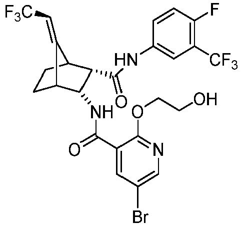 | 5-bromo-N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-2-(2-hydroxyethoxy )pyridine-3-carboxamide | 640.1 | ¹H NMR (500 MHz, DMSO-d6) δ 10.62 (s, 1H), 9.79 (br d, J=7.0 Hz, 1H), 8.44 (d, J=2.4 Hz, 1H), 8.31 (d, J=2.4 Hz, 1H), 8.17 - 8.04 (m, 1H), 7.86 - 7.73 (m, 1H), 7.48 (br t, J=9.6 Hz, 1H), 5.95 (q, J=8.0 Hz, 1H), 4.78 (t, J=5.5 Hz, 1H), 4.67 - 4.39 (m, 3H), 3.99 - 3.76 (m, 2H), 3.27 (br dd, J=10.7, 3.7 Hz, 1H), 3.19 (br s, 1H), 3.00 (br s, 1H), 2.01 (br d, J=8.2 Hz, 2H), 1.52 (br d, J=8.2 Hz, 2H) | 2.48 |
| 43 | 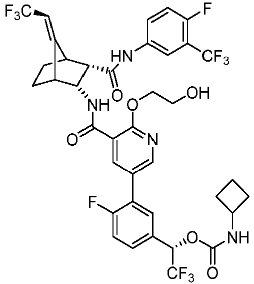 | (1S)-2,2,2-trifluoro-1-[4-fluoro-3-(5-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl }-6-(2-hydroxyethoxy )pyridin-3-yl)phenyl]ethyl N-cyclobutylcarb amate | 851.1 | ¹H NMR (500 MHz, DMSO-d6) δ 10.63 (s, 1H), 9.85 (br d, J=7.3 Hz, 1H), 8.53 (s, 1H), 8.44 (s, 1H), 8.19 - 8.07 (m, 2H), 7.84 - 7.72 (m, 2H), 7.60 (br s, 1H), 7.48 (br t, J=9.6 Hz, 2H), 6.45 - 6.32 (m, 1H), 6.01 - 5.88 (m, 1H), 4.81 (t, J=5.6 Hz, 1H), 4.74 - 4.65 (m, 1H), 4.61 (dt, J=10.7, 5.3 Hz, 2H), 4.00 - 3.82 (m, 3H), 3.22 (br s, 1H), 3.02 (br s, 1H), 2.27 - 2.00 (m, 4H), 1.98 - 1.81 (m, 2H), 1.69 - 1.45 (m, 4H) | 2.66 |
| 44 | 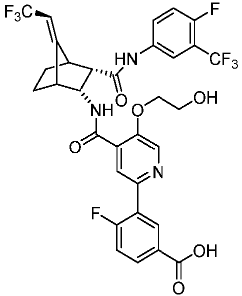 | 4-Fluoro-3-(4-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl}-5-(2-hydroxyethoxy )pyridin-2-yl)benzoic acid | 700.3 | ¹H NMR (500 MHz, DMSO-d6) δ 10.62 (s, 1H), 9.76 (br d, J=7.3 Hz, 1H), 8.83 (s, 1H), 8.57 (dd, J=7.9, 2.1 Hz, 1H), 8.21 (s, 1H), 8.12 - 8.07 (m, 1H), 8.06 - 7.95 (m, 1H), 7.81 - 7.68 (m, 1H), 7.55 - 7.39 (m, 2H), 5.95 (q, J=8.0 Hz, 1H), 4.67 - 4.55 (m, 1H), 4.55 - 4.44 (m, 2H), 4.04 - 3.84 (m, 2H), 3.29 (br dd, J=10.7, 3.7 Hz, 1H), 3.23 (br s, 1H), 3.05 - 2.95 (m, 1H), 2.14 - 1.96 (m, 2H), 1.59 - 1.41 (m, 2H) | 2.08 |
| 45 | 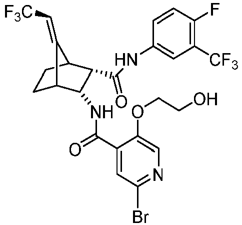 | 2-Bromo-N-[(2R,3S,7Z)-3-{[4-fluoro-3-(trifluoromethy l)phenyl]carba moyl}-7-(2,2,2-trifluoroethylid ene)bicyclo[2.2.1]heptan-2-yl]-5-(2-hydroxyethoxy )pyridine-4-carboxamide | 640.3 | ¹H NMR (500 MHz, DMSO-d6) δ 10.62 (s, 1H), 9.70 (br d, J=7.3 Hz, 1H), 8.46 (s, 1H), 8.17 - 8.00 (m, 1H), 7.82 - 7.69 (m, 2H), 7.47 (br t, J=9.8 Hz, 1H), 5.93 (q, J=7.8 Hz, 1H), 4.93 (t, J=5.5 Hz, 1H), 4.60 - 4.46 (m, 1H), 4.46 - 4.31 (m, 2H), 4.00 - 3.75 (m, 2H), 3.26 (br dd, J=10.7, 4.0 Hz, 1H), 3.21 - 3.14 (m, 1H), 2.99 (br s, 1H), 2.05 - 1.90 (m, 2H), 1.51 (br d, J=7.9 Hz, 2H) | 2.23 |
| 46 | 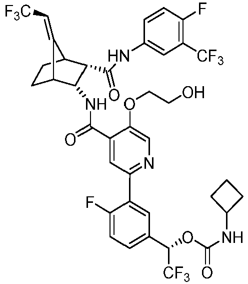 | (1S)-2,2,2-trifluoro-1-[4-fluoro-3-(4-{[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]carbamoyl }-5-(2-hydroxyethoxy )pyridin-2-yl)phenyl]ethyl N-cyclobutylcarb amate | 851.3 | ¹H NMR (500 MHz, DMSO-d6) δ 10.63 (s, 1H), 9.77 (br d, J=7.3 Hz, 1H), 8.82 (s, 1H), 8.22 - 8.04 (m, 3H), 7.76 (br dd, J=8.5, 3.7 Hz, 1H), 7.65 - 7.56 (m, 1H), 7.52 - 7.36 (m, 2H), 6.36 (q, J=6.7 Hz, 1H), 5.96 (q, J=7.5 Hz, 1H), 4.95 (br t, J=5.2 Hz, 1H), 4.67 - 4.43 (m, 3H), 4.03 - 3.84 (m, 2H), 3.24 (br s, 1H), 3.01 (br s, 1H), 2.19 - 1.83 (m, 5H), 1.63 - 1.45 (m, 3H) | 2.52 |
| 47 | 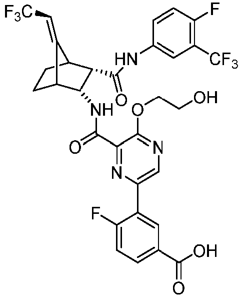 | 4-Fluoro-3-(6-{[(2R,3S,7Z)-3-{ [4-fluoro-3-(trifluoromethy l)phenyl]carba moyl}-7-(2,2,2-trifluoroethylid ene)bicyclo[2. 2.1]heptan-2-yl]carbamoyl }-5-(2-hydroxyethoxy )pyrazin-2-yl)benzoic acid | 701.2 | ¹H NMR (500 MHz, DMSO-d6) δ 9.85 (d, J=7.3 Hz, 1H), 8.57 (dd, J=7.4, 1.4 Hz, 1H), 8.08 (br dd, J=6.0, 2.7 Hz, 1H), 8.03 (br d, J=4.2 Hz, 1H), 7.89 - 7.78 (m, 1H), 7.55 - 7.47 (m, 1H), 7.41 (t, J=9.8 Hz, 1H), 6.03 - 5.90 (m, 1H), 4.52 (qd, J=11.7, 5.9 Hz, 3H), 3.86 - 3.70 (m, 2H), 3.26 (br s, 1H), 3.01 (br s, 1H), 2.11 - 2.01 (m, 1H), 1.99 - 1.90 (m, 1H), 1.52 (br d, J=6.3 Hz, 2H) | 2.10 |
| 48 | 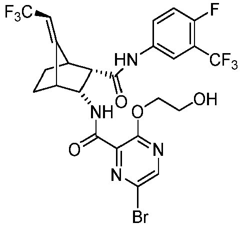 | 6-Bromo-N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-3-(2-hydroxyethoxy )pyrazine-2-carboxamide | 640.9 | ¹H NMR (500 MHz, DMSO-d6) δ 10.64 (s, 1H), 9.63 (d, J=7.4 Hz, 1H), 8.56 (s, 1H), 8.13 (br d, J=5.7 Hz, 1H), 7.82 - 7.73 (m, 1H), 7.49 (t, J=9.8 Hz, 1H), 5.94 (q, J=7.6 Hz, 1H), 4.96 - 4.80 (m, 1H), 4.57 - 4.37 (m, 3H), 3.75 (quin, J=5.2 Hz, 2H), 3.58 - 3.46 (m, 1H), 3.27 (br dd, J=10.6, 4.2 Hz, 1H), 3.20 (br s, 1H), 3.00 (br s, 1H), 2.05 - 1.88 (m, 2H), 1.51 (br d, J=7.7 Hz, 2H) | 2.25 |
| 52 | 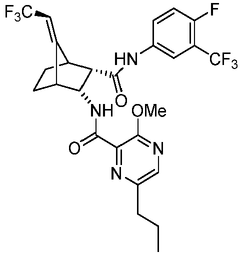 | N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-3-methoxy-6-propylpyrazine -2-carboxamide | 575.1 | ¹H NMR (500 MHz, DMSO-d6) δ 10.65 (s, 1H), 9.84 (d, J=7.0 Hz, 1H), 8.29 (s, 1H), 8.25 (dd, J=6.4, 2.1 Hz, 1H), 7.80 - 7.67 (m, 1H), 7.49 (t, J=9.6 Hz, 1H), 6.02 - 5.88 (m, 1H), 4.52 - 4.40 (m, 1H), 3.92 (s, 3H), 3.46 (s, 1H), 3.28 (dd, J=11.0, 4.3 Hz, 1H), 3.21 (br s, 1H), 3.00 (br s, 1H), 2.71 (td, J=7.3, 2.4 Hz, 2H), 2.00 - 1.83 (m, 2H), 1.74 (sxt, J=7.2 Hz, 2H), 1.57 - 1.43 (m, 2H), 0.88 (t, J=7.3 Hz, 3H) | 2.51 |
| 54 | 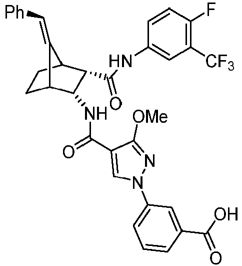 | 3-(4-{[(2R,3S,7Z)-3-{ [4-fluoro-3-(trifluoromethy l)phenyl]carba moyl}-7-(phenylmethyli dene)bicyclo[2 .2.1]heptan-2-yl]carbamoyl }-3-methoxy-1H-pyrazol-1-yl)benzoic acid | 641.2 | ¹H NMR (500MHz, DMSO-d6) δ 10.48 (s, 1H), 8.96 (d, J=7.3 Hz, 1H), 8.72 (s, 1H), 8.31 (s, 1H), 8.22 (d, J=4.4 Hz, 1H), 8.02 (d, J=8.0 Hz, 1H), 7.88 - 7.80 (m, 2H), 7.57 (t, J=7.9 Hz, 1H), 7.47 (t, J=9.8 Hz, 1H), 7.37 (d, J=4.3 Hz, 4H), 7.29 - 7.21 (m, 1H), 6.38 (s, 1H), 4.50 (br. s., 1H), 4.10 (s, 3H), 2.93 (br. s., 1H), 2.05 - 1.85 (m, 2H), 1.61 - 1.48 (m, 2H) | 2.13 Method B |
| 57 | 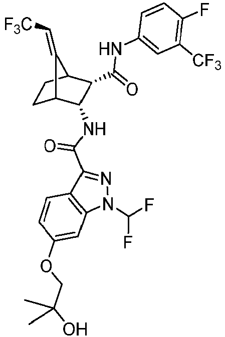 | 1-(difluoromethy l)-N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-6-(2-hydroxy-2-methylpropoxy )-1H-indazole-3-carboxamide | 679.3 | ¹H NMR (500 MHz, DMSO-d6) δ 10.67 (s, 1H), 9.64 - 9.57 (m, 1H), 8.10 - 8.04 (m, 2H), 7.83 - 7.78 (m, 1H), 7.50 (br t, J=9.5 Hz, 1H), 7.37 - 7.34 (m, 1H), 7.10 - 7.06 (m, 1H), 5.96 (q, J=8.0 Hz, 1H), 4.76 - 4.73 (m, 1H), 4.56 - 4.50 (m, 1H), 3.84 - 3.81 (m, 2H), 3.44 - 3.40 (m, 2H), 3.05 - 3.00 (m, 1H), 1.93 - 1.85 (m, 2H), 1.53 (br d, J=7.6 Hz, 2H), 1.23 (s, 6H) | 2.55 |
| 58 | 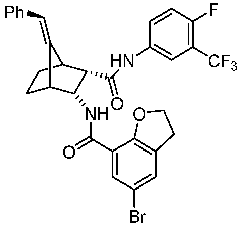 | 5-bromo-N-[(2R,3S,7Z)-3-{[4-fluoro-3-(trifluoromethy l)phenyl]carba moyl}-7-(2,2,2-trifluoroethylid ene)bicyclo[2. 2.1]heptan-2-yl]-2,3-dihydro-1-benzofuran-7-carboxamide | 622.0 | ¹H NMR (500 MHz, DMSO-d₆) δ 10.46 (s, 1H), 9.28 (br d, *J*=7.1 Hz, 1H), 8.12 (br d, *J*=4.8 Hz, 1H), 7.79 (br d, *J*=8.6 Hz, 1H), 7.68 (s, 1H), 7.56 (s, 1H), 7.46 (t, *J*=9.8 Hz, 1H), 5.90 (q, *J*=7.8 Hz, 1H), 4.82 (q, *J*=8.8 Hz, 1H), 4.65 (q, *J*=8.7 Hz, 1H), 4.53 (br s, 1H), 3.46 (br s, 1H), 3.36 (br s, 1H), 3.26 - 3.12 (m, 1H), 2.98 (br d, *J*=3.9 Hz, 1H), 2.57 - 2.53 (m, 4H), 2.13 - 1.99 (m, 1H), 1.97 - 1.84 (m, 1H), 1.51 (br s, 2H) | 2.75 |

| | | | | | |
|---|---|---|---|---|---|
| (a) All LC retention times are based on Method C unless otherwise specified. (b) 1-3 protons were not accounted for in these samples due to overlap with solvent residue and/or artifacts of water suppression. | | | | | |

**Table 3**

| **Ex. No.** | **Structure** | **Name** | **MS (ESI) (M+H)** | **¹H NMR^{b}** | **LC RTa (min) and chiral conditions when separated** |
|---|---|---|---|---|---|
| 60 | 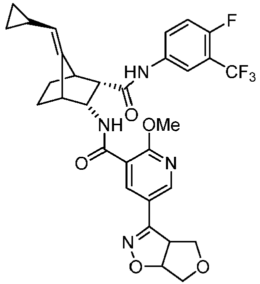 | 5-{3aH,4H,6H,6a H-furo[3,4-d][1,2]oxazol-3-yl }-N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-2-methoxypyridine -3-carboxamide | 615.3 | 5-{3aH,4H,6H,6aH-furo[3,4-d][1,2]oxazol-3-yl }-N-[(1R,2R,3S,4R,7Z) -7-(cyclopropylmethy lidene)-3-{[4-fluoro-3-(trifluoromethyl)p henyl] carbamoyl } bicyclo[2.2.1]hept an-2-yl]-2-methoxypyridine-3-carboxamide | 2.54/B |
| | | | | | From **61-6** |
| 62 | 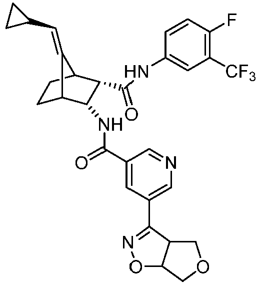 | 5-{3aH,4H,6H,6a H-furo[3,4-d][1,2]oxazol-3-yl }-N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]pyridine-3-carboxamide | 585.1 | 1H NMR (500 MHz, DMSO-d6) δ 10.55 (s, 1H), 9.43 (br d, J=7.3 Hz, 1H), 9.02 (s, 1H), 8.97 (s, 1H), 8.34 (s, 1H), 8.08 (br d, J=4.3 Hz, 1H), 7.90 - 7.70 (m, 1H), 7.49 (br t, J=9.8 Hz, 1H), 5.47 (dd, J=8.9, 3.4 Hz, 1H), 4.71 (br d, J=9.8 Hz, 1H), 4.63 (br t, J=7.6 Hz, 1H), 4.50 - 4.40 (m, 1H), 4.15 (br d, J=11.0 Hz, 1H), 4.00 (br d, J=9.5 Hz, 1H), 3.75 (br dd, J=9.3, 6.9 Hz, 1H), 3.69 (br dd, J=10.8, 3.5 Hz, 1H), 3.19 (br dd, J=10.2, 4.1 Hz, 1H), 3.10 (br s, 1H), 2.77 (br s, 1H), 1.99 - 1.92 (m, 1H), 1.85 - 1.74 (m, 1H), 1.60 - 1.50 (m, 1H), 1.46 - 1.36 (m, 1H), 0.82 - 0.67 (m, 2H), 0.37 (br s, 2H) | 2.27/C Peak 1 5-(3a,4,6,6a-tetrahydrofur o[3,4-d]isoxazol-3-yl)nicotinic acid-RT=1.54 min., >99% de)- SFC Column: Chiralpak AD-H, 21 x 250 mm, 5 micron, Mobile Phase: 30% IPA-0.1% DEA / 70% CO2, Flow Conditions: 45 mL/min, 150 Bar, 40°C,; Analytical method:Colu mn Chiralpak AD-H, 4.6 x 250 mm, 3 micron, Mobile Phase: 20% IPA-0.1% DEA / 80% CO2, Flow Conditions: 2 mL/min, 150 Bar, 40°C, to afford chiral (Peak-1, RT=1.54 min., >99% de) and chiral (Peak-2, RT=3.07 min., >99% de). |
| 63 | 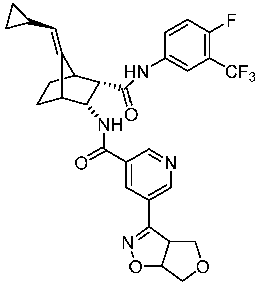 | 5-{3aH,4H,6H,6a H-furo[3,4-d][1,2]oxazol-3-yl }-N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]pyridine-3-carboxamide | 585.1 | 1H NMR (500 MHz, DMSO-d6) δ 10.53 (s, 1H), 9.39 (br d, J=7.0 Hz, 1H), 9.02 (br s, 1H), 8.96 (br s, 1H), 8.34 (br s, 1H), 8.14 - 8.04 (m, 1H), 7.89 - 7.75 (m, 1H), 7.49 (br t, J=9.6 Hz, 1H), 5.46 (br dd, J=9.2, 3.1 Hz, 1H), 4.71 (br d, J=9.5 Hz, 1H), 4.61 (br t, J=7.6 Hz, 1H), 4.49 - 4.38 (m, 1H), 4.15 (br d, J=10.7 Hz, 1H), 4.03 (br d, J=9.5 Hz, 1H), 3.84 - 3.74 (m, 1H), 3.69 (br dd, J=10.7, 3.4 Hz, 1H), 3.19 (br dd, J=9.9, 4.1 Hz, 1H), 3.11 (br s, 1H), 2.77 (br s, 1H), 2.02 - 1.89 (m, 1H), 1.85 - 1.75 (m, 1H), 1.65 - 1.50 (m, 1H), 1.48 - 1.34 (m, 2H), 0.84 - 0.68 (m, 2H), 0.37 (br s, 2H) | 2.27/C Peak-2 5-(3a,4,6,6a-tetrahydrofur o[3,4-d]isoxazol-3-yl)nicotinic acid, RT=3.07 min., >99% de). Column: Chiralpak AD-H, 21 x 250 mm, 5 micron, Mobile Phase: 30% IPA-0.1% DEA / 70% CO2, Flow Conditions: 45 mL/min, 150 Bar, 40°C; Analytical method: Colu mn Chiralpak AD-H, 4.6 x 250 mm, 3 micron, Mobile Phase: 20% IPA-0.1% DEA / 80% CO2, Flow Conditions: 2 mL/min, 150 Bar, 40°C, to afford chiral (Peak-1, RT=1.54 min., >99% de) and chiral (Peak-2, RT=3.07 min., >99% de). |
| 66 | 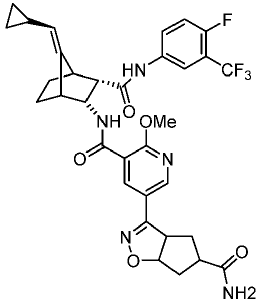 | 5-{5-carbamoyl-3aH,4H,5H,6H,6 aH-cyclopenta[d][1, 2]oxazol-3-yl}-N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-2-methoxypyridine -3-carboxamide | 656.2 | 1H NMR: (500 MHz, DMSO-d6) δ 10.54 (s, 1H), 10.04 (d, J=6.9 Hz, 1H), 8.61 - 8.58 (m, 2H), 8.23 (dd, J=6.4, 2.6 Hz, 1H), 7.82 - 7.78 (m, 1H), 7.50 (t, J=9.8 Hz, 1H), 7.34 (s, 1H), 6.82 (s, 1H), 5.19 (dd, J=8.5, 5.0 Hz, 1H), 4.70 (d, J=9.6 Hz, 1H), 4.43 (ddd, J=10.2, 6.4, 4.3 Hz, 1H), 4.34 (t, J=9.0 Hz, 1H), 4.14 (s, 3H), 3.17 (dd, J=10.8, 4.3 Hz, 1H), 3.12 (t, J=3.7 Hz, 1H), 2.74 (t, J=3.6 Hz, 1H), 2.58 - 2.52 (m, 1H), 2.14 - 1.88 (m, 4H), 1.85 - 1.75 (m, 2H), 1.54 - 1.46 (m, 1H), 1.42 (br s, 1H), 0.79 - 0.69 (m, 2H), 0.39 - 0.33 (m, 2H) | 2.29/C |
| | | | | | From **65-3** |
| 67 | 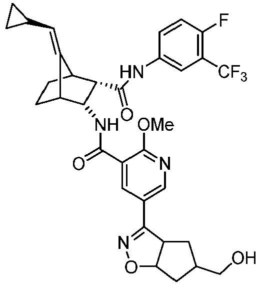 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-[5-(hydroxymethyl) 3aH,4H,5H,6H,6 aH-cyclopenta[d][1, 2]oxazol-3-yl]-2-methoxypyridine -3-carboxamide | 643.1 | 1H NMR: (500 MHz, DMSO-d6) δ 10.54 (s, 1H), 10.06 - 10.01 (m, 1H), 8.58 (dd, J=15.6, 2.3 Hz, 2H), 8.26 - 8.20 (m, 1H), 7.84 - 7.76 (m, 1H), 7.49 (t, J=9.7 Hz, 1H), 5.15 (dd, J=8.7, 5.2 Hz, 1H), 4.70 (d, J=9.6 Hz, 1H), 4.47 - 4.40 (m, 1H), 4.26 (br t, J=8.9 Hz, 1H), 4.13 (s, 3H), 3.36 (qd, J=10.7, 6.0 Hz, 2H), 3.17 (br dd, J=10.7, 4.1 Hz, 1H), 3.12 (br s, 1H), 2.73 (br s, 1H), 2.02 - 1.75 (m, 5H), 1.70 - 1.46 (m, 3H), 1.42 (br s, 2H), 0.78 - 0.69 (m, 2H), 0.39 - 0.31 (m, 2H) | 2.41/C Prepared from 5-(5-(hydroxymet hyl)-3a,5,6,6a-tetrahydro-4H-cyclopenta[d ]isoxazol-3-yl)-2-methoxynico tinic acid (Peak 1; >99%de; chiral analytical RT = 1.14 min); Preparative Chiral SFC conditions:C olumn: Chiralpak AD-H, 21 x 250 mm, 5 micron Mobile Phase: 30% MeOH / 70% CO2; Flow Conditions: 45 mL/min, 150 Bar, 40°C; Injection Details: 0.5 mL of ~22.5mg/mL in MeOH |
| 68 | 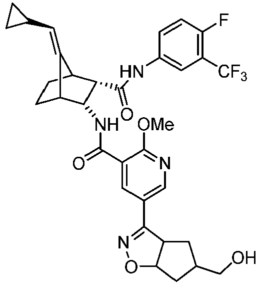 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-[5-(hydroxymethyl) 3aH,4H,5H,6H,6 aH-cyclopenta[d][1, 2]oxazol-3-yl]-2-methoxypyridine -3-carboxamide | 643.3 | 1H NMR: (500 MHz, DMSO-d6) δ 10.54 (s, 1H), 10.05 (d, J=7.0 Hz, 1H), 8.58 (s, 2H), 8.23 (dd, J=6.4, 2.4 Hz, 1H), 7.83 - 7.78 (m, 1H), 7.52 - 7.46 (m, 1H), 5.22 - 5.15 (m, 1H), 4.73 - 4.68 (m, 1H), 4.47 - 4.41 (m, 1H), 4.20 - 4.11 (m, 4H), 3.32 - 3.14 (m, 3H), 3.14 - 3.10 (m, 1H), 2.76 - 2.72 (m, 1H), 2.26 - 2.07 (m, 3H), 1.85 - 1.75 (m, 2H), 1.66 - 1.61 (m, 1H), 1.54 - 1.39 (m, 4H), 0.79 - 0.70 (m, 2H), 0.31 (s, 2H) | 2.42/C Prepared from 5-(5-(hydroxymet hyl)-3a,5,6,6a-tetrahydro-4H-cyclopenta[d ]isoxazol-3-yl)-2-methoxynico tinic acid (Peak 2; >99%de; chiral analytical RT = 2.352 min); Preparative Chiral SFC conditions: Column: Chiralpak AD-H, 21 x 250 mm, 5 micron Mobile Phase: 30% MeOH / 70% CO2; Flow Conditions: 45 mL/min, 150 Bar, 40°C; Injection Details: 0.5 mL of ~22.5mg/mL in MeOH |
| 69 | 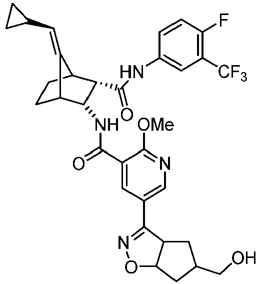 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-[5-(hydroxymethyl) 3aH,4H,5H,6H,6 aH-cyclopenta[d][1, 2]oxazol-3-yl]-2-methoxypyridine -3-carboxamide | 643.1 | 1H NMR: (500 MHz, DMSO-d6) δ 10.55 (s, 1H), 10.04 (d, J=7.0 Hz, 1H), 8.60 (s, 1H), 8.57 (s, 1H), 8.23 (dd, J=6.4, 2.4 Hz, 1H), 7.82 - 7.78 (m, 1H), 7.49 (t, J=9.8 Hz, 1H), 5.16 (dd, J=8.9, 5.2 Hz, 1H), 4.70 (d, J=9.6 Hz, 1H), 4.46 - 4.33 (m, 1H), 4.26 (br t, J=8.9 Hz, 1H), 4.13 (s, 3H), 3.37 (qd, J=10.8, 5.9 Hz, 2H), 3.17 (dd, J=10.7, 4.3 Hz, 1H), 3.11 (br s, 1H), 2.74 (br s, 1H), 2.00 (br dd, J=13.6, 5.6 Hz, 1H), 1.96 - 1.73 (m, 4H), 1.67 (td, J=12.2, 9.3 Hz, 1H), 1.61 - 1.44 (m, 2H), 1.41 (br s, 2H), 0.79 - 0.68 (m, 2H), 0.38 - 0.33 (m, 2H) | 2.41/C Prepared from 5-(5-(hydroxymet hyl)-3a,5,6,6a-tetrahydro-4H-cyclopenta[d ]isoxazol-3-yl)-2-methoxynico tinic acid (Peak 3; >99%de; chiral analytical RT = 4.265 min); Preparative Chiral SFC conditions: Column: Chiralpak AD-H, 21 x 250 mm, 5 micron Mobile Phase: 30% MeOH / 70% CO2; Flow Conditions: 45 mL/min, 150 Bar, 40°C; Injection Details: 0.5 mL of ~22.5mg/mL in MeOH |
| 70 | 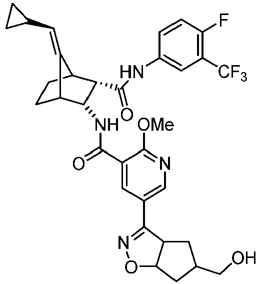 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-[5-(hydroxymethyl) 3aH,4H,5H,6H,6 aH-cyclopenta[d][1, 2]oxazol-3-yl]-2-methoxypyridine -3-carboxamide | 643.1 | 1H NMR: (500 MHz, DMSO-d6) δ 10.56 - 10.53 (m, 1H), 10.07 - 10.01 (m, 1H), 8.61 - 8.56 (m, 2H), 8.25 - 8.20 (m, 1H), 7.82 - 7.77 (m, 1H), 7.52 - 7.45 (m, 1H), 5.21 - 5.15 (m, 1H), 4.72 - 4.68 (m, 1H), 4.46 - 4.40 (m, 1H), 4.20 - 4.11 (m, 4H), 3.30 - 3.15 (m, 3H), 3.14 - 3.07 (m, 1H), 2.76 - 2.70 (m, 1H), 2.25 - 2.09 (m, 3H), 1.84 - 1.76 (m, 2H), 1.67 - 1.60 (m, 1H), 1.53 - 1.38 (m, 4H), 0.80 - 0.69 (m, 2H), 0.39 - 0.31 (m, 2H) | 2.40/C Prepared from 5-(5-(hydroxymet hyl)-3a,5,6,6a-tetrahydro-4H-cyclopenta[d ]isoxazol-3-yl)-2-methoxynico tinic acid (Peak 4; >99%de; chiral analytical RT = 6.240 min); Preparative Chiral SFC conditions: Column: Chiralpak AD-H, 21 x 250 mm, 5 micron Mobile Phase: 30% MeOH / 70% CO2; Flow Conditions: 45 mL/min, 150 Bar, 40°C; Injection Details: 0.5 mL of ~22.5mg/mL in MeOH |
| 73 | 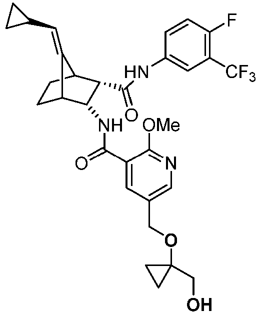 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-{[1-(hydroxymethyl) cyclopropoxy]m ethyl}-2-methoxypyridine -3-carboxamide | 604.4 | 1H NMR (500 MHz, DMSO-d6) δ 10.54 (s, 1H), 9.99 (br d, J=7.0 Hz, 1H), 8.26 (br d, J=4.6 Hz, 3H), 7.88 - 7.75 (m, 1H), 7.51 (br t, J=9.6 Hz, 1H), 4.79 (t, J=5.8 Hz, 1H), 4.71 (br d, J=9.8 Hz, 1H), 4.60 (s, 2H), 4.49 - 4.40 (m, 1H), 4.09 (s, 3H), 3.61 (br d, J=5.5 Hz, 2H), 3.18 (br dd, J=10.7, 4.0 Hz, 1H), 3.14 - 3.07 (m, 1H), 2.75 (br s, 1H), 1.89 - 1.73 (m, 2H), 1.57 - 1.47 (m, 1H), 1.47 - 1.38 (m, 2H), 0.82 - 0.69 (m, 4H), 0.63 - 0.55 (m, 2H), 0.43 - 0.29 (m, 2H) | 2.53/B |
| 75 | 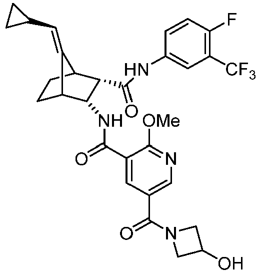 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-(3-hydroxyazetidine -1-carbonyl)-2-methoxypyridine -3-carboxamide | 603.2 | 1H NMR (400 MHz, DMSO-d6) δ 10.53 (s, 1H), 10.02 (d, J=7.3 Hz, 1H), 8.58 (d, J=2.4 Hz, 1H), 8.51 (d, J=2.4 Hz, 1H), 8.22 (dd, J=6.2, 2.3 Hz,1H), 7.80 (dt, J=8.7, 3.7 Hz, 1H), 7.49 (t, J=9.8 Hz, 1H), 5.77 (d, J=5.9 Hz, 1H), 4.70 (d, J=9.5 Hz, 1H), 4.56 - 4.46 (m, 2H), 4.46 - 4.38 (m, 1H),4.30 - 4.22 (m, 1H), 4.11 - 4.11 (m, 1H), 4.16 - 4.01 (m, 3H), 3.85 - 3.76 (m, 1H), 3.20 - 3.14 (m, 1H), 3.11 (br t, J=2.9 Hz, 1H), 2.73 (t, J=3.3 Hz,1H), 1.87 - 1.70 (m, 2H), 1.56 - 1.45 (m, 1H), 1.45 - 1.34 (m, 2H), 0.80 - 0.66 (m, 2H), 0.42 - 0.28 (m, 2H) | 2.15/A |
| 76 | 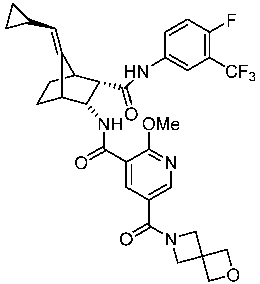 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-2-methoxy-5-{2-oxa-6-azaspiro[3.3]hept ane-6-carbonyl }pyridin e-3-carboxamide | 629.2 | 1H NMR (400 MHz, DMSO-d6) δ 10.53 (s, 1H), 10.02 (d, J=6.8 Hz, 1H), 8.57 (d, J=2.4 Hz, 1H), 8.50 (d, J=2.4 Hz, 1H), 8.22 (dd, J=6.6, 2.2 Hz,1H), 7.84 - 7.75 (m, 1H), 7.49 (t, J=9.7 Hz, 1H), 4.73 - 4.63 (m, H), 4.52 (br s, 2H), 4.42 (br s, 1H), 4.22 (br d, J=0.7 Hz, 2H), 4.14 (s, 3H), 3.20 -3.13 (m, 1H), 3.11 (br t, J=2.4 Hz, 1H), 2.73 (br d, J=3.2 Hz, 1H), 1.86 - 1.73 (m, 2H), 1.54 - 1.35 (m, 3H), 0.80 - 0.68 (m, 2H), 0.35 (dd, J=4.6, 1.7Hz, 2H) | 2.26/A |
| 79 | 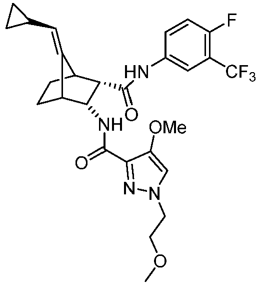 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-4-methoxy-1-(2-methoxyethyl)-1H-pyrazole-3-carboxamide | 551.2 | 1H NMR (400 MHz, DMSO-d6) δ ppm 10.48 (s, 1 H) 9.13 (d, J=7.53 Hz, 1 H) 8.21 (dd, J=6.53, 2.51 Hz, 1 H) 7.73 - 7.79 (m, 1 H) 7.46 - 7.51 (m, 2 H) 4.53 - 4.72 (m, 3 H) 4.40 (br t, J=10.54 Hz, 1 H) 3.90 (s, 3 H) 3.60 (t, J=5.77 Hz, 2 H) 3.11 - 3.17 (m, 4 H) 3.05 (t, J=3.76 Hz, 1 H) 2.69 - 2.86 (m, 1 H) 2.52 - 2.61 (m, 2 H) 1.67 - 1.91 (m, 2 H) 1.34 - 1.55 (m, 3 H) 0.68 - 0.79 (m, 2 H) | 3.30/C |
| 80 | 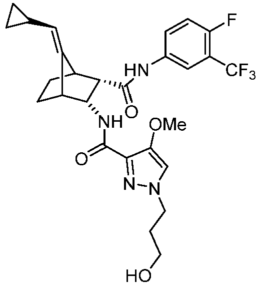 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-1-(3-hydroxypropyl)-4-methoxy-1H-pyrazole-3-carboxamide | 551.3 | 1H NMR (400 MHz, DMSO-d6) δ ppm 10.53 (s, 1 H) 9.15 (d, J=7.53 Hz, 1 H) 8.22 (dd, J=6.53, 2.51 Hz, 1 H) 7.77 (br d, J=9.04 Hz, 1 H) 7.45 - 7.51 (m, 2 H) 4.68 (d, J=9.54 Hz, 1 H) 4.35 - 4.55 (m, 1 H) 3.90 (s, 3 H) 3.41 (br d, J=19.58 Hz, 1 H) 3.10 - 3.27 (m, 2 H) 2.91 - 3.08 (m, 1 H) 2.67 - 2.81 (m, 2 H) 2.53 (br s, 1 H) 2.33 - 2.44 (m, 2 H) 1.68 - 1.91 (m, 2 H) 1.58 (br s, 1 H) 1.33 - 1.55 (m, 2 H) 1.24 (s, 1 H) 0.73 (br t, J=8.78 Hz, 2 H) 0.35 (br d, J=3.51 Hz, 2 H) | 3.27/C |
| 86 | 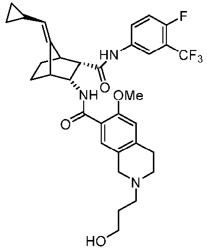 | N-[(1R,2R,3S,4R)-7-(cyclopropylmet hylidene)-3-{[4-fluoro-3-(trifluoromethyl) phenyl]carbamo yl}bicyclo[2.2.1] heptan-2-yl]-2-(3-hydroxypropyl)-6-methoxy-1,2,3,4-tetrahydroisoqui noline-7-carboxamide | 616.3 | 1H NMR (400MHz, DMSO-d6) δ ppm 10.51 (s, 1H), 9.76 (d, J=7.6 Hz, 1H), 8.26 - 8.20 (m, 1H), 7.81 - 7.74 (m, 1H), 7.61 (s, 1H), 7.48 (t, J=9.5 Hz, 1H), 6.89 (s, 1H), 4.67 (d, J=9.8 Hz, 1H), 4.46 - 4.40 (m, 1H), 3.94 (s, 3H), 3.47 - 3.46 (m, 3H), 3.15 (br d, J=4.2 Hz, 2H), 3.06 (br d, J=3.9 Hz, 2H), 2.84 - 2.79 (m, 2H), 2.71 - 2.68 (m, 1H), 2.63 - 2.60 (m, 1H), 1.85 (s, 6H), 1.75 - 1.63 (m, 3H), 1.53 - 1.33 (m, 3H), 0.91 (t, J=7.5 Hz, 1H), 0.80 - 0.69 (m, 2H), 0.35 (td, J=1.3, 2.1 Hz, 2H). | 2.25/B |
| 87 | 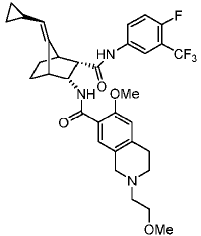 | N-[(1R,2R,3S,4R)-7-(cyclopropylmet hylidene)-3-{[4-fluoro-3-(trifluoromethyl) phenyl]carbamo yl}bicyclo[2.2.1] heptan-2-yl]-6-methoxy-2-(2-methoxyethyl)-1,2,3,4-tetrahydroisoqui noline-7-carboxamide | 616.4 | 1H NMR (400 MHz, DMSO-d6) δ ppm 10.48 (s, 1H), 9.76 (d, J = 7.1 Hz, 1H), 8.22 (dd, J = 2.7, 6.6 Hz, 1H), 7.82 - 7.69 (m, 1H), 7.60 (s, 1H), 7.48 (t, J = 9.5 Hz, 1H), 6.88 (s, 1H), 4.67 (d, J = 9.5 Hz, 1H), 4.43 (br s, 1H), 3.94 (s, 3H), 3.57 - 3.45 (m, 4H), 3.25 (s, 3H), 3.14 (dt, J = 4.9, 10.0 Hz, 1H), 3.06 (br s, 1H), 2.85 - 2.77 (m, 2H), 2.74 - 2.66 (m, 3H), 2.62 (t, J = 5.9 Hz, 2H), 1.91 - 1.81 (m, 1H), 1.81 - 1.72 (m, 1H), 1.54 - 1.45 (m, 1H), 1.45 - 1.29 (m, 2H), 0.79 - 0.67 (m, 2H), 0.34 (dd, J = 2.1, 4.8 Hz, 2H). | 2.48/B |
| 89 | 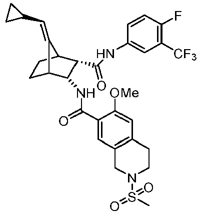 | N-[(1R,2R,3S,4R)-7-(cyclopropylmet hylidene)-3-{[4-fluoro-3-(trifluoromethyl) phenyl]carbamo yl}bicyclo[2.2.1] heptan-2-yl]-2-methanesulfonyl -6-methoxy-1,2,3,4-tetrahydroisoqui noline-7-carboxamide | 636.3 | 1H NMR (400MHz, DMSO-d6) δ ppm 10.52 (s, 1H), 9.81 (d, J=7.3 Hz, 1H), 8.23 (dd, J=2.1, 6.7 Hz, 1H), 7.84 - 7.75 (m, 1H), 7.73 (s, 1H), 7.48 (t, J=9.8 Hz, 1H), 7.00 (s, 1H), 4.68 (d, J=9.5 Hz, 1H), 4.48 - 4.39 (m, 1H), 4.31 (s, 2H), 3.97 (s, 4H), 3.45 - 3.38 (m, 3H), 3.14 (dd, J=4.2, 10.5 Hz, 1H), 3.07 (br s, 1H), 2.70 (br s, 1H), 2.46 - 2.37 (m, 1H), 1.89 - 1.81 (m, 1H), 1.80 - 1.73 (m, 1H), 1.56 - 1.46 (m, 1H), 1.44 - 1.31 (m, 2H), 0.73 (br t, J=8.9 Hz, 2H), 0.41 - 0.26 (m, 2H). | 2.40/B |
| 91 | 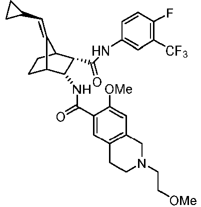 | N-[(1R,2R,3S,4R)-7-(cyclopropylmet hylidene)-3-{[4-fluoro-3-(trifluoromethyl) phenyl]carbamo yl}bicyclo[2.2.1] heptan-2-yl]-2-(2-hydroxy-2-methylpropyl)-7-methoxy-1,2,3,4-tetrahydroisoqui noline-6-carboxamide | 616.4 | 1H NMR (400 MHz, DMSO-d6) δ = 10.49 (s, 1H), 9.76 (d, J = 7.1 Hz, 1H), 8.22 (dd, J = 2.6, 6.5 Hz, 1H), 7.81 - 7.72 (m, 1H), 7.65 (s, 1H), 7.47 (t, J = 9.8 Hz, 1H), 6.84 (s, 1H), 4.67 (d, J = 9.8 Hz, 1H), 4.42 (dt, J = 3.4, 7.1 Hz, 1H), 3.93 (s, 3H), 3.62 (s, 2H), 3.51 (t, J = 5.7 Hz, 2H), 3.26 (s, 3H), 3.13 (br dd, J = 4.4, 10.8 Hz, 1H), 3.06 (br t, J = 3.5 Hz, 1H), 2.77 - 2.58 (m, 7H), 1.90 - 1.81 (m, 1H), 1.81 - 1.71 (m, 1H), 1.54 - 1.43 (m, 1H), 1.43 - 1.30 (m, 2H), 0.79 0.67 (m, 2H), 0.41 - 0.27 (m, 2H) | 1.95/F |
| 92 | 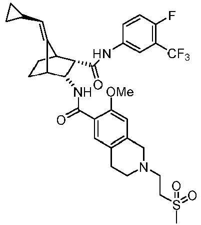 | (1R,2S,3R,4R,7 Z)-N-[4-fluoro-3-(trifluoromethyl) phenyl]-3-{5-[(1Z)-3-(4-hydroxypiperidin -1-yl)-3-oxoprop-1-en-1-yl]-2-methoxybenzami do}-7-(2,2,2-trifluoroethylide ne)bicyclo[2.2.1] heptane-2-carboxamide | 664.4 | 1H NMR (400 MHz, DMSO-d6) δ = 10.49 (s, 1H), 9.76 (d, J = 7.3 Hz, 1H), 8.21 (dd, J = 2.6, 6.5 Hz, 1H), 7.82 - 7.74 (m, 1H), 7.67 (s, 1H), 7.48 (t, J = 9.8 Hz, 1H), 6.85 (s, 1H), 4.67 (d, J = 9.5 Hz, 1H), 4.49 - 4.35 (m, 1H), 3.94 (s, 3H), 3.65 (s, 2H), 3.38 (s, 2H), 3.19 - 3.11 (m, 1H), 3.09 - 3.04 (m, 1H), 3.01 (s, 3H), 2.88 (t, J = 6.7 Hz, 2H), 2.79 - 2.65 (m, 5H), 1.86 (s, 1H), 1.80 - 1.69 (m, 1H), 1.56 - 1.45 (m, 1H), 1.45 - 1.29 (m, 2H), 0.81 - 0.62 (m, 2H), 0.34 (dd, J = 1.7, 4.6 Hz, 2H) | 1.88/F |
| 93 | 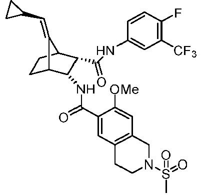 | (1R,2S,3R,4R,7 Z)-N-[4-fluoro-3-(trifluoromethyl) phenyl]-3-{5-[(1Z)-3-(4-hydroxypiperidin -1-yl)-2-methyl-3-oxoprop-1-en-1-yl]-2-methoxybenzami do}-7-(2,2,2-trifluoroethylide ne)bicyclo[2.2.1] heptane-2-carboxamide | 636.4 | 1H NMR (400 MHz, DMSO-d6) δ = 10.50 (s, 1H), 9.80 (d, J = 7.3 Hz, 1H), 8.22 (dd, J = 2.7, 6.6 Hz, 1H), 7.77 (td, J = 4.3, 7.5 Hz, 1H), 7.74 (s, 1H), 7.48 (t, J = 9.8 Hz, 1H), 7.04 (s, 1H), 4.67 (d, J = 9.5 Hz, 1H), 4.40 (s, 3H), 3.95 (s, 3H), 3.42 (t, J = 6.0 Hz, 2H), 3.14 (dd, J = 4.5, 11.1 Hz, 1H), 3.10 - 3.04 (m, 1H), 2.94 (s, 3H), 2.86 (br t, J = 5.9 Hz, 2H), 2.70 (br s, 1H), 1.85 (s, 1H), 1.76 (br d, J = 2.9 Hz, 1H), 1.54 - 1.44 (m, 1H), 1.44 - 1.27 (m, 2H), 0.81 - 0.64 (m, 2H), 0.34 (dd, J = 2.1, 4.8 Hz, 2H) | 2.40/E |
| 94 | 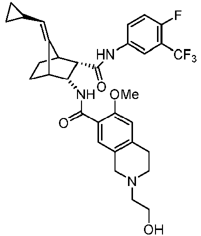 | (1R,2S,3R,4R,7 Z)-N-[4-fluoro-3-(trifluoromethyl) phenyl]-3-{5-[(1S,2R)-2-(4-hydroxypiperidin e-1-carbonyl)cyclopr opyl]-2-methoxybenzami do}-7-(2,2,2-trifluoroethylide ne)bicyclo[2.2.1] heptane-2-carboxamide | 602.4 | 1H NMR (400 MHz, DMSO-d6) δ = 10.48 (s, 1H), 9.76 (d, J = 7.3 Hz, 1H), 8.22 (dd, J = 2.8, 6.5 Hz, 1H), 7.84 - 7.72 (m, 1H), 7.60 (s, 1H), 7.48 (t, J = 9.7 Hz, 1H), 6.88 (s, 1H), 4.67 (d, J = 9.5 Hz, 1H), 4.51 - 4.34 (m, 2H), 3.94 (s, 3H), 3.63 - 3.51 (m, 4H), 3.18 - 3.09 (m, 1H), 3.06 (t, J = 3.5 Hz, 1H), 2.87 - 2.78 (m, 2H), 2.72 - 2.64 (m, 3H), 2.54 (t, J = 6.2 Hz, 2H), 1.85 (br d, J = 10.0 Hz, 1H), 1.80 - 1.72 (m, 1H), 1.54 - 1.44 (m, 1H), 1.44 - 1.29 (m, 2H), 0.81 - 0.63 (m, 2H), 0.34 (dd, J = 2.0, 4.6 Hz, 2H) | 2.25/E |
| 95 | 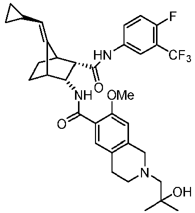 | methyl 4-(3-{[(1R,2R,3S,4R, 7Z)-3-{[4-fluoro-3-(trifluoromethyl) phenyl]carbamo yl}-7-(2,2,2-trifluoroethylide ne)bicyclo[2.2.1] heptan-2-yl]carbamoyl}-4-methoxyphenyl)-2-oxabicyclo[2.1.1 ]hexane-1-carboxylate | 630.4 | 1H NMR (400 MHz, DMSO-d6) δ = 10.49 (s, 1H), 9.75 (d, J = 7.1 Hz, 1H), 8.22 (dd, J = 2.7, 6.6 Hz, 1H), 7.82 - 7.72 (m, 1H), 7.65 (s, 1H), 7.48 (t, J = 10.0 Hz, 1H), 6.82 (s, 1H), 4.67 (d, J = 9.8 Hz, 1H), 4.49 - 4.37 (m, 1H), 4.18 (s, 1H), 3.93 (s, 3H), 3.72 (s, 2H), 3.17 - 3.10 (m, 1H), 3.06 (br d, J = 2.9 Hz, 1H), 2.75 (br dd, J = 4.5, 13.8 Hz, 5H), 2.36 (s, 2H), 1.85 (br d, J = 9.3 Hz, 1H), 1.81 - 1.72 (m, 1H), 1.55 - 1.46 (m, 1H), 1.46 - 1.31 (m, 2H), 1.11 (s, 6H), 0.80 - 0.64 (m, 2H), 0.40 - 0.25 (m, 2H) | 2.55/E |

| | | | | | |
|---|---|---|---|---|---|
| (a) All LC retention times are based on Method C unless otherwise specified. (b) 1-3 protons were not accounted for in these samples due to overlap with solvent residue and/or artifacts of water suppression. | | | | | |

**Table 4**

| **Ex. No.** | **Structure** | **Name** | **MS (ESI) (M+ H)** | **1H NMR** | **LC RT^{a} (min) and chiral conditions when separated** |
|---|---|---|---|---|---|
| 96 | 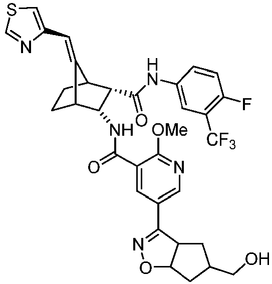 | N-((1R,2R,3S,4R,Z) -3-((4-fluoro-3-(trifluoromethyl)p henyl)carbamoyl) -7-(thiazol-4-ylmethylene)bicy clo[2.2.1]heptan-2-yl)-5-(5-(hydroxymethyl)-3a,5,6,6a-tetrahydro-4H-cyclopenta[d]isox azol-3-yl)-2-methoxynicotina mide | 686.2 | 1H NMR (400 MHz, DMSO-d6) δ = 10.62 (s, 1H), 10.04 (d, J = 7.3 Hz, 1H), 9.12 (d, J = 2.0 Hz, 1H), 8.62 - 8.51 (m, 2H), 8.25 (dd, J = 2.5, 6.5 Hz, 1H), 7.89 - 7.76 (m, 1H), 7.57 (d, J = 1.8 Hz, 1H), 7.50 (t, J = 9.6 Hz, 1H), 6.43 (s, 1H), 5.21 - 5.07 (m, 1H), 4.57 - 4.46 (m, 2H), 4.22 - 4.08 (m, 4H), 4.00 - 3.92 (m, 1H), 3.90 (s, 1H), 3.30 - 3.17 (m, 3H), 2.93 (br s, 1H), 2.27 - 2.04 (m, 3H), 1.99 - 1.78 (m, 2H), 1.65 (br dd, J = 6.4, 10.9 Hz, 1H), 1.58 - 1.42 (m, 3H) | 2.19, A |
| 97 | 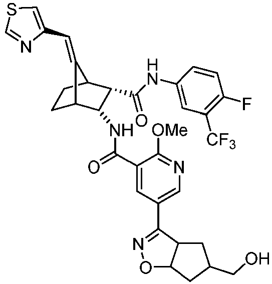 | N-((1R,2R,3S,4R,Z) -3-((4-fluoro-3-(trifluoromethyl)p henyl)carbamoyl) -7-(thiazol-4-ylmethylene)bicy clo[2.2.1]heptan-2-yl)-5-(5-(hydroxymethyl)-3a,5,6,6a-tetrahydro-4H-cyclopenta[d]isox azol-3-yl)-2-methoxynicotina mide | 686.2 | 1H NMR (400 MHz, DMSO-d6) δ = 10.61 (s, 1H), 10.03 (d, J = 7.3 Hz, 1H), 9.12 (d, J = 1.5 Hz, 1H), 8.60 (d, J = 2.5 Hz, 1H), 8.56 (d, J = 2.5 Hz, 1H), 8.25 (dd, J = 2.5, 6.5 Hz, 1H), 7.86 - 7.79 (m, 1H), 7.57 (d, J = 2.0 Hz, 1H), 7.50 (t, J = 9.8 Hz, 1H), 6.43 (s, 1H), 5.15 (dd, J = 5.1, 8.9 Hz, 1H), 4.50 (q, J = 5.1 Hz, 2H), 4.30 - 4.23 (m, 1H), 4.13 (s, 3H), 3.95 (br s, 1H), 3.36 (br d, J = 8.8 Hz, 2H), 3.29 (br d, J = 6.3 Hz, 1H), 2.96 - 2.89 (m, 1H), 2.03 - 1.73 (m, 5H), 1.72 - 1.43 (m, 4H) | 2.19, A |
| 99 | 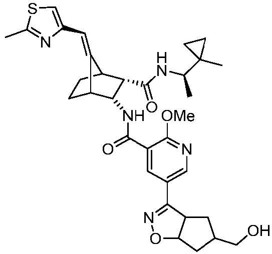 | 5-(5-(hydroxymethyl)-3a,5,6,6a-tetrahydro-4H-cyclopenta[d]isox azol-3-yl)-2-methoxy-N-((1R,2R,3S,4R,Z) -3-(((R)-1-(1-methylcyclopropy l)ethyl)carbamoyl )-7-((2-methylthiazol-4-yl)methylene)bicy clo[2.2.1]heptan-2-yl)nicotinamide | 620.3 | 1H NMR (400 MHz, DMSO-d6) δ = 10.25 (d, J = 6.8 Hz, 1H), 8.59 (d, J = 2.5 Hz, 1H), 8.51 (d, J = 2.3 Hz, 1H), 8.03 (d, J = 8.5 Hz, 1H), 7.29 (s, 1H), 6.27 (s, 1H), 5.16 (dd, J = 5.1, 8.9 Hz, 1H), 4.50 (t, J = 5.4 Hz, 1H), 4.38 - 4.31 (m, 1H), 4.27 (t, J = 8.8 Hz, 1H), 4.10 (s, 3H), 3.83 (t, J = 3.3 Hz, 1H), 3.53 - 3.46 (m, 1H), 3.41 - 3.36 (m, 3H), 3.06 (dd, J = 4.4, 10.9 Hz, 1H), 2.72 (br s, 1H), 2.66 (s, 3H), 2.05 - 1.87 (m, 3H), 1.84 - 1.74 (m, 2H), 1.72 - 1.54 (m, 2H), 1.48 - 1.39 (m, 2H), 1.05 (d, J = 7.0 Hz, 3H), 1.01 (s, 3H), 0.62 - 0.53 (m, 1H), 0.42 - 0.32 (m, 1H), 0.25 - 0.15 (m, 2H) | 2.00, A |
| 100 | 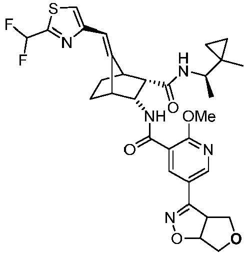 | N-((1R,2R,3S,4R,Z) -7-((2-(difluoromethyl)t hiazol-4-yl)methylene)-3-(((R)-1-(1-methylcyclopropy l)ethyl)carbamoyl )bicyclo[2.2.1]he ptan-2-yl)-2-methoxy-5-(3a,4,6,6a-tetrahydrofuro[3, 4-d]isoxazol-3-yl)nicotinamide | 628.2 | 1H NMR (400 MHz, DMSO-d6) δ ppm 10.27 (d, J=6.75 Hz, 1 H) 8.60 (d, J=2.25 Hz, 1 H) 8.52 (d, J=2.50 Hz, 1 H) 8.06 (d, J=8.51 Hz, 1 H) 7.81 (s, 1 H) 7.19 - 7.52 (m, 1 H) 6.40 (s, 1 H) 5.39 (dd, J=9.51, 3.50 Hz, 1 H) 4.52 - 4.61 (m, 1 H) 4.37 (ddd, J=10.76, 6.63, 4.63 Hz, 1 H) 4.11 (s, 4 H) 3.96 (d, J=9.26 Hz, 1 H) 3.72 - 3.82 (m, 2 H) 3.67 (dd, J=10.76, 3.75 Hz, 1 H) 3.46 - 3.54 (m, 1 H) 3.09 (dd, J=11.01, 4.50 Hz, 1 H) 2.78 (br s, 1 H) 1.98 (br t, J=8.76 Hz, 1 H) 1.80 - 1.88 (m, 1 H) 1.47 (br d, J=6.75 Hz, 2 H) 1.05 (d, J=6.75 Hz, 3 H) 0.55 - 0.60 (m, 1 H) 1.01 (s, 3 H) 0.32 - 0.40 (m, 1 H) 0.17 - 0.26 (m, 2 H) | 2.18, A |
| 101 | 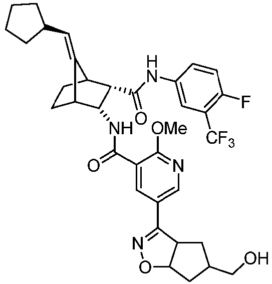 | N-((1R,2R,3S,4R,Z) -7-(cyclopentylmeth ylene)-3-((4-fluoro-3-(trifluoromethyl)p henyl)carbamoyl) bicyclo[2.2.1]hept an-2-yl)-5-(5-(hydroxymethyl)-3a,5,6,6a-tetrahydro-4H-cyclopenta[d]isox azol-3-yl)-2-methoxynicotina mide | 671.3 | 1H NMR (400 MHz, DMSO-d6) δ = 10.56 (s, 1H), 10.00 (d, J = 6.9 Hz, 1H), 8.58 (dd, J = 2.4, 13.7 Hz, 2H), 8.24 (dd, J = 2.4, 6.6 Hz, 1H), 7.82 -7.71 (m, 1H), 7.49 (t, J = 9.8 Hz, 1H), 5.24 - 5.07 (m, 2H), 4.50 (br t, J = 4.8 Hz, 1H), 4.42 - 4.32 (m, 1H), 4.26 (t, J = 8.7 Hz, 1H), 4.13 (s, 3H),3.17 (dd, J = 4.2, 10.7 Hz, 1H), 3.01 (br s, 1H), 2.73 (br d, J = 3.4 Hz, 1H), 2.65 - 2.56 (m, 1H), 2.01 (br dd, J = 5.6, 13.6 Hz, 1H), 1.95 - 1.70 (m,7H), 1.69 - 1.52 (m, 6H), 1.39 (br s, 2H), 1.33 - 1.22 (m, 2H) | 2.53, B |
| 102 | 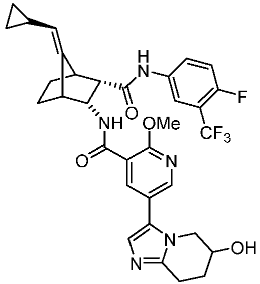 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-5-(6-hydroxy-5,6,7,8-tetrahydroimidazo [1,2-a]pyridin-3-yl)-2-methoxynicotina mide | 640.2 | 1H NMR (400 MHz, DMSO-d6) δ = 10.57 (s, 1H), 10.06 (br d, J=6.7 Hz, 1H), 8.41 (d, J=2.1 Hz, 1H), 8.32 (d, J=2.1 Hz, 1H), 8.23 (br d, J=4.3 Hz, 1H), 7.86 - 7.75 (m, 1H), 7.49 (br t, J=9.5 Hz, 1H), 7.04 (s, 1H), 4.71 (d, J=9.5 Hz, 1H), 4.50 - 4.39 (m, 1H), 4.18 (br s, 1H), 4.12 (s, 3H), 4.05 (br dd, J=11.9, 3.1 Hz, 1H), 3.71 (br dd, J=12.4, 3.8 Hz, 1H), 3.17 (br dd, J=10.7, 4.3 Hz, 1H), 3.12 (br s, 1H), 2.96 - 2.85 (m, 1H), 2.84 - 2.72 (m, 2H), 1.94 (br d, J=5.8 Hz, 2H), 1.86 - 1.74 (m, 2H), 1.57 - 1.35 (m, 3H), 0.74 (quin, J=9.8 Hz, 2H), 0.35 (br s, 2H) | 2.06 |
| 103 | 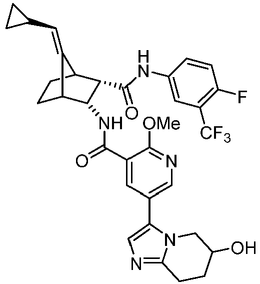 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-5-(6-hydroxy-5,6,7,8-tetrahydroimidazo [1,2-a]pyridin-3-yl)-2-methoxynicotina mide | 640.2 | 1H NMR (500 MHz, DMSO-d6) δ 10.65 - 10.59 (m, 1H), 10.06 (br d, J=5.8 Hz, 1H), 8.42 (s, 1H), 8.36 - 8.31 (m, 1H), 8.29 - 8.22 (m, 1H), 7.85 - 7.80 (m, 1H), 7.50 (br t, J=9.6 Hz, 1H), 7.09 - 7.02 (m, 1H), 4.75 - 4.68 (m, 1H), 4.49 - 4.42 (m, 1H), 4.17 (br d, J=5.2 Hz, 1H), 4.14 (s, 3H), 4.08 - 4.01 (m, 1H), 3.78 - 3.69 (m, 1H), 3.22 - 3.17 (m, 1H), 3.15 - 3.11 (m, 1H), 2.97 - 2.87 (m, 1H), 2.84 - 2.77 (m, 1H), 2.78 - 2.72 (m, 1H), 2.00 - 1.90 (m, 2H), 1.88 - 1.79 (m, 2H), 1.78 - 1.74 (m, 1H), 1.56 - 1.47 (m, 1H), 1.46 - 1.37 (m, 2H), 0.81 - 0.69 (m, 2H), 0.41 - 0.32 (m, 2H) | 2.05 |
| 104 | 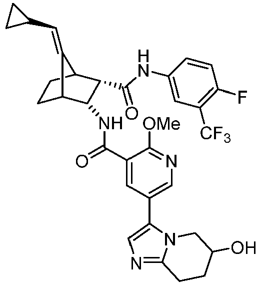 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-5-(6-hydroxy-5,6,7,8-tetrahydroimidazo [1,2-a]pyridin-3-yl)-2-methoxynicotina mide | 640.2 | 1H NMR (500 MHz, DMSO-d6) δ 10.58 (s, 1H), 10.07 (br d, J=7.0 Hz, 1H), 8.45 (d, J=1.8 Hz, 1H), 8.36 (d, J=1.8 Hz, 1H), 8.24 (br d, J=4.3 Hz, 1H), 7.87 - 7.77 (m, 1H), 7.50 (br t, J=9.8 Hz, 1H), 7.29 (s, 1H), 4.71 (d, J=9.8 Hz, 1H), 4.51 - 4.39 (m, 1H), 4.21 (br s, 1H), 4.14 (s, 3H), 4.07 (br dd, J=12.5, 3.1 Hz, 1H), 3.77 (br dd, J=12.1, 4.1 Hz, 1H), 3.18 (br dd, J=10.4, 4.0 Hz, 1H), 3.14 - 3.07 (m, 1H), 3.03 - 2.93 (m, 1H), 2.93 - 2.84 (m, 1H), 2.75 (br s, 1H), 1.97 (br d, J=4.3 Hz, 2H), 1.88 - 1.76 (m, 2H), 1.56 - 1.35 (m, 3H), 0.82 - 0.68 (m, 2H), 0.36 (br s, 2H) | 2.06 |
| 105 | 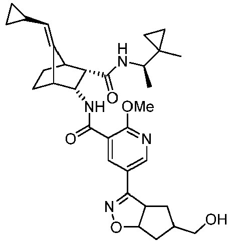 | N-((1R,2R,3S,4R,Z) -7-(cyclopropylmeth ylene)-3-(((R)-1-(1-methylcyclopropy l)ethyl)carbamoyl )bicyclo[2.2.1]he ptan-2-yl)-5-(5-(hydroxymethyl)-3a,5,6,6a-tetrahydro-4H-cyclopenta[d]isox azol-3-yl)-2-methoxynicotina mide | 563.0 | 1H NMR (500 MHz, DMSO-d6) δ 10.21 (br d, J=6.7 Hz, 1H), 8.51 (d, J=2.4 Hz, 1H), 8.45 (d, J=2.4 Hz, 1H), 7.92 (br d, J=8.5 Hz, 1H), 5.10 (br dd, J=8.7, 5.0 Hz, 1H), 4.58 (d, J=9.5 Hz, 1H), 4.20 (br t, J=8.4 Hz, 2H), 4.02 (s, 3H), 3.58 - 3.46 (m, 1H), 3.44 - 3.24 (m, 2H), 3.00 (br s, 1H), 2.90 (br dd, J=10.5, 3.8 Hz, 1H), 1.99 - 1.82 (m, 2H), 1.80 - 1.56 (m, 4H), 1.55 - 1.46 (m, 1H), 1.45 - 1.36 (m, 1H), 1.34 - 1.23 (m, 2H), 0.97 (d, J=6.7 Hz, 3H), 0.92 (s, 3H), 0.71 - 0.57 (m, 2H), 0.47 (br dd, J=8.4, 3.5 Hz, 1H), 0.32 - 0.21 (m, 3H), 0.18 - 0.06 (m, 2H) | 2.27 |
| 106 | 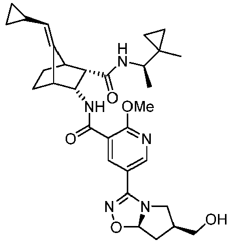 | N-((1R,2R,3S,4R,Z) -7-(cyclopropylmeth ylene)-3-(((R)-1-(1-methylcyclopropy l)ethyl)carbamoyl )bicyclo[2.2.1]he ptan-2-yl)-5-((6S,7aR)-6-(hydroxymethyl)-5,6,7,7a-tetrahydropyrrolo [1,2-d][1,2,4]oxadiazo l-3-yl)-2-methoxynicotina mide | 564.4 | 1H NMR (500 MHz, DMSO-d6) δ 10.34 - 10.25 (m, 1H), 8.61 - 8.56 (m, 1H), 8.49 - 8.44 (m, 1H), 7.95 (br d, J=8.5 Hz, 1H), 5.88 - 5.77 (m, 1H), 4.63 (d, J=9.5 Hz, 1H), 4.29 - 4.22 (m, 1H), 4.12 - 4.05 (m, 3H), 3.34 - 3.26 (m, 1H), 3.22 - 3.15 (m, 1H), 3.06 (br s, 1H), 2.98 - 2.87 (m, 1H), 2.35 - 1.93 (m, 3H), 1.86 - 1.67 (m, 2H), 1.51 - 1.42 (m, 1H), 1.35 (br s, 2H), 1.02 (br d, J=6.8 Hz, 3H), 0.97 (s, 3H), 0.75 - 0.65 (m, 2H), 0.57 - 0.48 (m, 1H), 0.36 - 0.27 (m, 3H), 0.23 - 0.12 (m, 2H) | 2.24 |
| 107 | 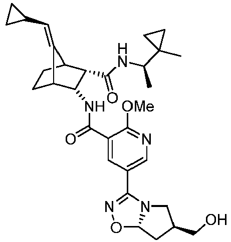 | N-((1R,2R,3S,4R,Z) -7-(cyclopropylmeth ylene)-3-(((R)-1-(1-methylcyclopropy l)ethyl)carbamoyl )bicyclo[2.2.1]he ptan-2-yl)-5-((6S,7aS)-6-(hydroxymethyl)-5,6,7,7a-tetrahydropyrrolo [1,2-d][1,2,4]oxadiazo l-3-yl)-2-methoxynicotina mide | 564.3 | 1H NMR (500 MHz, DMSO-d6) δ 10.36 - 10.24 (m, 1H), 8.65 - 8.56 (m, 1H), 8.53 - 8.41 (m, 1H), 7.95 (br d, J=7.9 Hz, 1H), 5.90 - 5.77 (m, 1H), 4.70 - 4.58 (m, 2H), 4.26 (br s, 1H), 4.16 - 4.04 (m, 3H), 3.52 - 3.42 (m, 1H), 3.41 - 3.31 (m, 1H), 3.06 (br s, 1H), 3.01 - 2.86 (m, 2H), 2.33 - 1.93 (m, 3H), 1.88 - 1.66 (m, 2H), 1.55 - 1.42 (m, 1H), 1.41 - 1.29 (m, 2H), 1.09 - 0.94 (m, 6H), 0.79 - 0.65 (m, 2H), 0.60 - 0.48 (m, 1H), 0.32 (br s, 3H), 0.18 (br s, 2H) | 2.24 |
| 108 | 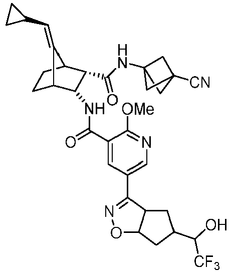 | N-((1R,2R,3S,4R,Z) -3-((3-cyanobicyclo[1.1. 1]pentan-1-yl)carbamoyl)-7-(cyclopropylmeth ylene)bicyclo[2.2. 1]heptan-2-yl)-2-methoxy-5-(5-(2,2,2-trifluoro-1-hydroxyethyl)-3a,5,6,6a-tetrahydro-4H-cyclopenta[d]isox azol-3-yl)nicotinamide | 640.2 | 1H NMR (500 MHz, DMSO-d6) δ 10.21 (br d, J=6.7 Hz, 1H), 8.92 (s, 1H), 8.61 (d, J=2.4 Hz, 1H), 8.56 (d, J=2.1 Hz, 1H), 5.19 (dd, J=8.5, 5.2 Hz, 1H), 4.62 (d, J=9.8 Hz, 1H), 4.31 (br t, J=9.3 Hz, 1H), 4.27 - 4.22 (m, 1H), 4.14 (s, 3H), 3.99 - 3.90 (m, 1H), 3.06 (br s, 1H), 2.83 (br dd, J=10.8, 4.1 Hz, 1H), 2.53 - 2.50 (m, 6H) (overlaps DMSO), 2.07 - 1.89 (m, 3H), 1.86 - 1.76 (m, 2H), 1.68 (br d, J=11.6 Hz, 2H), 1.50 - 1.40 (m, 1H), 1.34 (br d, J=8.5 Hz, 2H), 0.71 (quin, J=8.9 Hz, 2H), 0.32 (br d, J=4.0 Hz, 2H) | 2.13 |
| 109 | 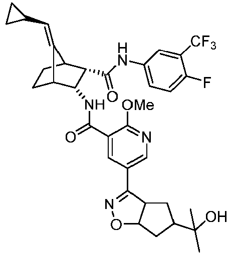 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-5-(5-(2-hydroxypropan-2-yl)-3a,5,6,6a-tetrahydro-4H-cyclopenta[d]isox azol-3-yl)-2-methoxynicotina mide | 671.3 | 1H NMR (500 MHz, DMSO-d6) δ 10.60 - 10.51 (m, 1H), 10.04 (br d, J=7.0 Hz, 1H), 8.59 (s, 1H), 8.58 (s, 1H), 8.24 (br d, J=4.3 Hz, 1H), 7.92 - 7.71 (m, 1H), 7.50 (t, J=9.8 Hz, 1H), 5.14 (br dd, J=8.7, 4.4 Hz, 2H), 4.71 (d, J=9.5 Hz, 1H), 4.54 - 4.45 (m, 1H), 4.34 - 4.20 (m, 2H), 4.17 - 4.07 (m, 3H), 3.21 - 3.10 (m, 2H), 2.74 (br s, 1H), 1.98 - 1.91 (m, 1H), 1.87 - 1.76 (m, 3H), 1.67 (br d, J=7.6 Hz, 1H), 1.56 - 1.49 (m, 1H), 1.47 - 1.38 (m, 2H), 1.06 (s, 3H), 1.03 (s, 3H), 0.82 - 0.63 (m, 3H), 0.50 - 0.23 (m, 2H) | 2.46, B |
| 110 | 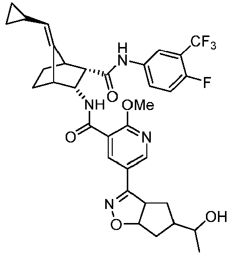 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-5-(5-(1-hydroxyethyl)-3a,5,6,6a-tetrahydro-4H-cyclopenta[d]isox azol-3-yl)-2-methoxynicotina mide | 657.4 | 1H NMR (400 MHz, CD3OD) δ 10.43 (br d, J=7.2 Hz, 1H), 10.23 - 10.10 (m, 1H), 8.71 (d, J=2.3 Hz, 1H), 8.62 (d, J=2.3 Hz, 1H), 8.19 (dd, J=6.3, 2.3 Hz, 1H), 7.78 (dt, J=8.8, 3.5 Hz, 1H), 7.31 (t, J=9.6 Hz, 1H), 5.23 (dd, J=8.9, 5.0 Hz, 1H), 4.76 (d, J=9.4 Hz, 1H), 4.63 - 4.45 (m, 1H), 4.26 (s, 3H), 3.61 (t, J=6.4 Hz, 1H), 3.28 - 3.23 (m, 1H), 3.18 (dd, J=10.9, 4.4 Hz, 1H), 2.75 (t, J=3.3 Hz, 1H), 2.31 (dd, J=13.7, 5.7 Hz, 1H), 2.06 - 1.98 (m, 1H), 1.94 - 1.86 (m, 2H), 1.85 - 1.81 (m, 2H), 1.76 - 1.66 (m, 1H), 1.60 - 1.50 (m, 3H), 1.15 (d, J=6.5 Hz, 3H), 0.78 (dd, J=8.1, 1.7 Hz, 2H), 0.38 (br dd, J=4.6, 2.6 Hz, 2H) | 2.47, A |
| 111 | 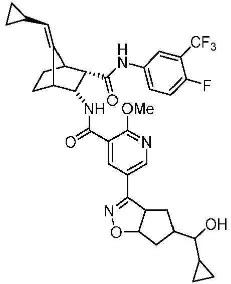 | 5-(5-(cyclopropyl(hydr oxy)methyl)-3a,5,6,6a-tetrahydro-4H-cyclopenta[d]isox azol-3-yl)-N-((1R,2R,3S,4R,Z) -7-(cyclopropylmeth ylene)-3-((4-fluoro-3-(trifluoromethyl)phenyl)carbamoyl) bicyclo[2.2.1]hept an-2-yl)-2-methoxynicotina mide | 683.2 | 1H NMR (500 MHz, DMSO-d6) δ 10.37 (s, 1H), 9.87 (br d, J=7.0 Hz, 1H), 8.41 (s, 1H), 8.40 (s, 1H), 8.12 - 8.01 (m, 1H), 7.70 - 7.57 (m, 1H), 7.31 (t, J=9.6 Hz, 1H), 5.08 - 4.89 (m, 1H), 4.52 (d, J=9.5 Hz, 1H), 4.38 - 4.23 (m, 2H), 4.00 - 3.90 (m, 4H), 2.99 (br dd, J=10.4, 4.0 Hz, 1H), 2.94 (br s, 1H), 2.56 (br s, 1H), 2.52 - 2.45 (m, 1H), 2.19 - 2.09 (m, 1H), 2.05 - 1.98 (m, 1H), 1.96 - 1.86 (m, 1H), 1.69 - 1.56 (m, 2H), 1.53 - 1.44 (m, 1H), 1.40 - 1.30 (m, 2H), 1.24 (br s, 2H), 0.65 - 0.47 (m, 3H), 0.26 - 0.16 (m, 3H), 0.15 - 0.08 (m, 1H), 0.01 (br d, J=3.7 Hz, 2H) | 2.60, B |
| 112 | 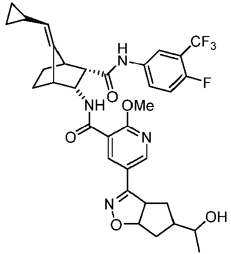 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-5-(5-(1-hydroxyethyl)-3a,5,6,6a-tetrahydro-4H-cyclopenta[d]isox azol-3-yl)-2-methoxynicotina mide | 657.4 | 1H NMR (500 MHz, DMSO-d6) δ 10.56 (s, 1H), 10.05 (br d, J=6.7 Hz, 1H), 8.60 (d, J=2.4 Hz, 1H), 8.57 (d, J=2.4 Hz, 1H), 8.30 - 8.15 (m, 1H), 7.87 - 7.73 (m, 1H), 7.49 (t, J=9.6 Hz, 1H), 5.15 (dd, J=8.5, 4.9 Hz, 1H), 4.71 (d, J=9.8 Hz, 1H), 4.49 - 4.39 (m, 1H), 4.25 (br t, J=8.4 Hz, 1H), 4.13 (s, 3H), 3.65 - 3.36 (m, 1H), 3.19 - 3.13 (m, 1H), 3.12 (br s, 1H), 2.74 (br s, 1H), 1.96 - 1.71 (m, 5H), 1.64 - 1.57 (m, 1H), 1.52 - 1.45 (m, 1H), 1.44 - 1.33 (m, 2H), 1.03 (d, J=6.1 Hz, 3H), 0.82 - 0.66 (m, 2H), 0.36 (br s, 2H). | 2.47, B |
| 113 | 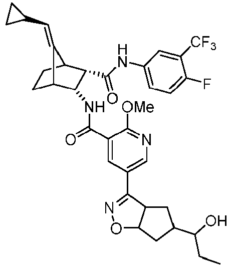 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-5-(5-(1-hydroxypropyl)-3a,5,6,6a-tetrahydro-4H-cyclopenta[d]isox azol-3-yl)-2-methoxynicotina mide | 671.2 | 1H NMR (500 MHz, DMSO-d6) δ 10.57 (s, 1H), 10.05 (br d, J=7.0 Hz, 1H), 8.59 (br s, 1H), 8.58 (br s, 1H), 8.24 (br d, J=4.9 Hz, 1H), 7.96 - 7.71 (m, 1H), 7.58 - 7.43 (m, 1H), 5.15 (br dd, J=8.7, 4.7 Hz, 1H), 4.72 (d, J=9.5 Hz, 1H), 4.48 (d, J=5.8 Hz, 1H), 4.46 - 4.41 (m, 1H), 4.29 - 4.23 (m, 1H), 4.14 (s, 3H), 3.28 - 3.17 (m, 1H), 3.13 (br s, 1H), 2.75 (br s, 1H), 2.16 - 2.02 (m, 1H), 1.90 - 1.73 (m, 4H), 1.65 (br dd, J=10.7, 6.1 Hz, 2H), 1.57 - 1.49 (m, 1H), 1.45 - 1.39 (m, 2H), 1.38 - 1.29 (m, 1H), 1.28 - 1.17 (m, 1H), 0.83 (br t, J=7.3 Hz, 3H), 0.78 - 0.64 (m, 2H), 0.50 - 0.30 (m, 2H). Some protons are obscured by water suppression | 2.59 |
| 114 | 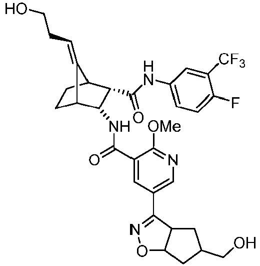 | N-((1R,2R,3S,4R,Z) -3-((4-fluoro-3-(trifluoromethyl)p henyl)carbamoyl) -7-(3-hydroxypropylide ne)bicyclo[2.2.1] heptan-2-yl)-5-(5-(hydroxymethyl)-3a,5,6,6a-tetrahydro-4H-cyclopenta[d]isox azol-3-yl)-2-methoxynicotina mide | 633.1 | 1H NMR (500 MHz, DMSO-d6) δ 10.53 (s, 1H), 9.94 (br d, J=6.8 Hz, 1H), 8.57 (s, 1H), 8.54 (br s, 1H), 8.22 (br d, J=4.3 Hz, 1H), 7.80 (br s, 1H), 7.48 (br t, J=9.6 Hz, 1H), 5.15 (dd, J=8.6, 5.5 Hz, 1H), 4.59 (br s, 1H), 4.25 (br t, J=8.8 Hz, 1H), 4.12 (s, 3H), 3.41 - 3.30 (m, 1H), 3.25 (br d, J=10.4 Hz, 1H), 2.99 (s, 1H), 2.49 - 2.33 (m, 1H), 2.29 (br s, 1H), 2.00 (br dd, J=13.7, 5.5 Hz, 1H), 1.93 - 1.74 (m, 3H), 1.72 - 1.53 (m, 4H), 1.51 - 1.33 (m, 6H), 0.97 (br t, J=6.7 Hz, 3H) | 2.55 |
| 115 | 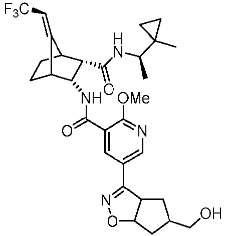 | 5-(5-(hydroxymethyl)-3a,5,6,6a-tetrahydro-4H-cyclopenta[d]isox azol-3-yl)-2-methoxy-N-((1R,2R,3S,4R,Z) -3-(((R)-1-(1-methylcyclopropy l)ethyl)carbamoyl )-7-(2,2,2-trifluoroethyliden e)bicyclo[2.2.1]he ptan-2-yl)nicotinamide | 591.2 | 1H NMR (500 MHz, DMSO-d6) δ 10.11 (br d, J=6.4 Hz, 1H), 8.39 (d, J=2.1 Hz, 1H), 8.31 (d, J=2.1 Hz, 1H), 7.91 (br d, J=8.9 Hz, 1H), 5.68 (q, J=7.9 Hz, 1H), 4.96 (br dd, J=8.4, 5.0 Hz, 1H), 4.45 - 4.28 (m, 1H), 4.13 (br s, 1H), 4.06 (br t, J=8.7 Hz, 1H), 3.89 (s, 3H), 3.43 - 3.25 (m, 1H), 3.25 - 3.09 (m, 1H), 3.00 (br s, 1H), 2.87 (br dd, J=10.5, 4.4 Hz, 1H), 2.63 (br s, 1H), 1.85 - 1.65 (m, 3H), 1.65 - 1.54 (m, 2H), 1.51 - 1.29 (m, 2H), 1.24 (br s, 2H), 1.05 (br d, J=6.7 Hz, 1H), 0.84 (d, J=7.0 Hz, 3H), 0.79 (s, 3H), 0.34 (br d, J=5.2 Hz, 1H), 0.24 - 0.08 (m, 1H), 0.04 - -0.12 (m, 2H) | 2.22 |
| 116 | 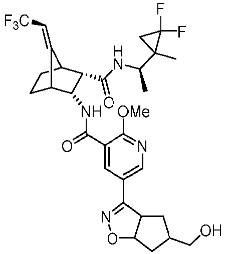 | N-((1R,2R,3S,4R,Z) -3-(((1R)-1-(2,2-difluoro-1-methylcyclopropy l)ethyl)carbamoyl )-7-(2,2,2-trifluoroethyliden e)bicyclo[2.2.1]he ptan-2-yl)-5-(5-(hydroxymethyl)-3a,5,6,6a-tetrahydro-4H-cyclopenta[d]isox azol-3-yl)-2-methoxynicotina mide | 627.2 | 1H NMR (500 MHz, DMSO-d6) δ 10.31 (br d, J=6.4 Hz, 1H), 8.61 (d, J=2.1 Hz, 1H), 8.57 - 8.49 (m, 1H), 8.23 (br d, J=7.9 Hz, 1H), 5.91 (q, J=8.0 Hz, 1H), 5.17 (br dd, J=8.5, 5.2 Hz, 1H), 4.44 - 4.32 (m, 1H), 4.28 (br t, J=8.9 Hz, 1H), 4.11 (s, 3H), 3.87 - 3.72 (m, 1H), 3.22 (br s, 1H), 3.11 (br dd, J=10.5, 4.1 Hz, 1H), 2.84 (br s, 1H), 2.01 (br dd, J=13.7, 5.8 Hz, 1H), 1.96 - 1.75 (m, 4H), 1.74 - 1.53 (m, 2H), 1.48 - 1.29 (m, 3H), 1.20 (br s, 1H), 1.15 (br d, J=7.3 Hz, 6H) | 2.14 |
| 117 | 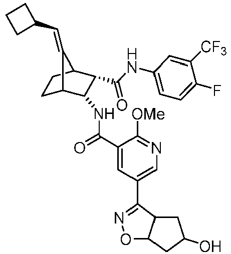 | N-((1R,2R,3S,4R,Z) -7-(cyclobutylmethyl ene)-3-((4-fluoro-3-(trifluoromethyl)p henyl)carbamoyl) bicyclo[2.2.1]hept an-2-yl)-5-(5-hydroxy-3a,5,6,6a-tetrahydro-4H-cyclopenta[d]isox azol-3-yl)-2-methoxynicotina mide | 643.2 | 1H NMR (500 MHz, DMSO-d6) δ 10.56 (s, 1H), 10.01 (br d, J=6.7 Hz, 1H), 8.56 (s, 2H), 8.23 (br d, J=4.6 Hz, 1H), 7.79 (br s, 1H), 7.49 (br t, J=9.8 Hz, 1H), 5.38 (d, J=8.5 Hz, 1H), 5.18 (br s, 1H), 4.34 (br s, 1H), 4.32 - 4.21 (m, 1H), 4.13 (s, 3H), 3.45 (br s, 1H), 3.30 - 3.13 (m, 1H), 3.10 (br d, J=7.6 Hz, 1H), 2.97 (br s, 1H), 2.72 (br s, 1H), 2.24 - 2.07 (m, 2H), 2.06 - 1.96 (m, 1H), 1.96 - 1.78 (m, 6H), 1.75 (br d, J=8.2 Hz, 2H), 1.37 (br s, 2H) | 2.47 |
| 118 | 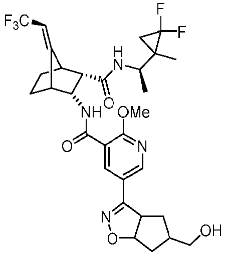 | N-((1R,2R,3S,4R,Z) -3-(((1R)-1-(2,2-difluoro-1-methylcyclopropy l)ethyl)carbamoyl )-7-(2,2,2-trifluoroethyliden e)bicyclo[2.2.1]he ptan-2-yl)-5-(5-(hydroxymethyl)-3a,5,6,6a-tetrahydro-4H-cyclopenta[d]isox azol-3-yl)-2-methoxynicotina mide | 627.2 | 1H NMR (500 MHz, DMSO-d6) δ 10.28 (br d, J=6.4 Hz, 1H), 8.58 (d, J=2.1 Hz, 1H), 8.52 (s, 1H), 8.47 (br d, J=7.9 Hz, 1H), 5.88 (q, J=8.0 Hz, 1H), 5.16 (br dd, J=8.7, 5.0 Hz, 1H), 4.63 (br t, J=5.2 Hz, 1H), 4.36 (br d, J=5.5 Hz, 1H), 4.25 (br t, J=8.9 Hz, 1H), 4.08 (s, 3H), 3.94 - 3.78 (m, 1H), 3.64 - 3.47 (m, 2H), 3.47 - 3.29 (m, 1H), 3.25 - 3.15 (m, 1H), 3.10 (br dd, J=10.7, 4.3 Hz, 1H), 2.85 (br s, 1H), 2.00 (br dd, J=13.7, 5.5 Hz, 1H), 1.95 - 1.74 (m, 4H), 1.70 - 1.49 (m, 3H), 1.44 (br s, 2H), 1.22 (br s, 1H), 1.14 - 1.07 (s, 6H) | 2.22 |
| 119 | 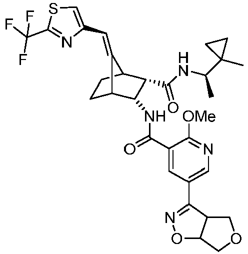 | 2-methoxy-N-((1R,2R,3S,4R,Z) -3-(((R)-1-(1-methylcyclopropy l)ethyl)carbamoyl )-7-((2-(trifluoromethyl)t hiazol-4-yl)methylene)bicy clo[2.2.1]heptan-2-yl)-5-(3a,4,6,6a-tetrahydrofuro[3, 4-d]isoxazol-3-yl)nicotinamide | 646.2 | 1H NMR (400 MHz, DMSO-d6) δ = 10.30 (d, J = 6.5 Hz, 1H), 8.60 (d, J = 2.5 Hz, 1H), 8.52 (d, J = 2.5 Hz, 1H), 8.07 (d, J = 8.3 Hz, 1H), 7.97 (s, 1H), 6.44 (s, 1H), 5.39 (dd, J = 3.4, 9.1 Hz, 1H), 4.62 - 4.53 (m, 1H), 4.43 - 4.31 (m, 1H), 4.14 - 4.09 (m, 4H), 3.96 (d, J = 9.8 Hz, 1H), 3.80 - 3.72 (m, 2H), 3.67 (dd, J = 3.5, 10.8 Hz, 1H), 3.53 - 3.47 (m, 1H), 3.10 (dd, J = 4.3, 11.0 Hz, 1H), 2.80 (br d, J = 1.0 Hz, 1H), 2.02 - 1.95 (m, 1H), 1.88 - 1.82 (m, 1H), 1.51 - 1.45 (m, 2H), 1.05 (d, J = 7.0 Hz, 3H), 1.02 (s, 3H), 0.63 - 0.52 (m, 1H), 0.42 - 0.31 (m, 1H), 0.27 - 0.16 (m, 2H) | 2.37, B |
| 120 | 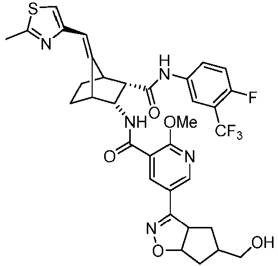 | N-((1R,2R,3S,4R,Z) -3-((4-fluoro-3-(trifluoromethyl)p henyl)carbamoyl) -7-((2-methylthiazol-4-yl)methylene)bicy clo[2.2.1]heptan-2-yl)-5-(5-(hydroxymethyl)-3a,5,6,6a-tetrahydro-4H-cyclopenta[d]isox azol-3-yl)-2-methoxynicotina mide | 700.2 | 1H NMR (400 MHz, DMSO-d6) δ = 10.61 (s, 1H), 10.00 (d, J = 7.1 Hz, 1H), 8.61 (d, J = 2.4 Hz, 1H), 8.56 (d, J = 2.4 Hz, 1H), 8.25 (dd, J = 2.6,6.5 Hz, 1H), 7.83 (td, J = 4.3, 7.5 Hz, 1H), 7.51 (t, J = 9.8 Hz, 1H), 7.34 (s, 1H), 6.32 (s, 1H), 5.16 (dd, J = 5.1, 8.8 Hz, 1H), 4.57 - 4.45 (m 2H), 4.32 - 4.21 (m, 1H), 4.14 (s, 3H), 3.96 - 3.87 (m, 1H), 3.42 - 3.35 (m, 2H), 3.31 - 3.26 (m, 1H), 2.91 (br s, 1H), 2.68 (s, 3H), 2.04 - 1.73 (m, 5H), 1.72 - 1.47 (m, 4H) | 2.11, B |
| 121 | 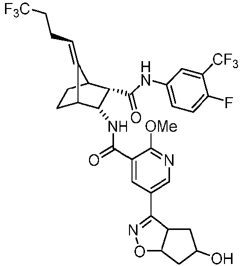 | N-((1R,2R,3S,4R,Z) -3-((4-fluoro-3-(trifluoromethyl)p henyl)carbamoyl) -7-(4,4,4-trifluorobutyliden e)bicyclo[2.2.1]he ptan-2-yl)-5-(5-hydroxy-3a,5,6,6a-tetrahydro-4H-cyclopenta[d]isox azol-3-yl)-2-methoxynicotina mide | 685.1 | 1H NMR (500 MHz, DMSO-d6) δ 10.58 (s, 1H), 10.02 (br d, J=6.7 Hz, 1H), 8.55 (br d, J=2.7 Hz, 2H), 8.27 - 8.17 (m, 1H), 7.80 (br dd, J=5.3, 3.5 Hz, 1H), 7.48 (br t, J=9.8 Hz, 1H), 5.28 (br t, J=6.4 Hz, 1H), 5.21 - 5.10 (m, 1H), 4.98 (br d, J=4.3 Hz, 1H), 4.47 - 4.36 (m, 1H), 4.32 - 4.20 (m, 1H), 4.15 - 4.00 (m, 4H), 3.62 - 3.43 (m, 1H), 3.20 - 3.12 (m, 2H), 3.00 (br s, 1H), 2.76 (br s, 1H), 2.45 - 2.28 (m, 4H), 2.04 - 1.88 (m, 3H), 1.85 - 1.72 (m, 3H) | 2.52 |
| 122 | 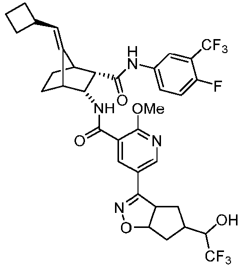 | N-((1R,2R,3S,4R,Z) -7-(cyclobutylmethyl ene)-3-((4-fluoro-3-(trifluoromethyl)p henyl)carbamoyl) bicyclo[2.2.1]hept an-2-yl)-2-methoxy-5-(5-(2,2,2-trifluoro-1-hydroxyethyl)-3a,5,6,6a-tetrahydro-4H-cyclopenta[d]isox azol-3-yl)nicotinamide | 725.1 | 1H NMR (500 MHz, DMSO-d6) δ 10.60 (s, 1H), 10.00 (br d, J=7.0 Hz, 1H), 8.57 (dd, J=13.3, 2.0 Hz, 2H), 8.22 (br d, J=4.0 Hz, 1H), 7.83 - 7.78 (m, 1H), 7.49 (br t, J=9.8 Hz, 1H), 6.34 (br d, J=7.0 Hz, 1H), 5.32 (br t, J=7.2 Hz, 1H), 5.14 (br dd, J=8.9, 4.3 Hz, 1H), 4.42 (br t, J=10.4 Hz, 1H), 4.23 (br t, J=9.0 Hz, 1H), 4.13 (s, 3H), 4.04 - 3.92 (m, 1H), 3.18 (br dd, J=10.4, 3.7 Hz, 1H), 2.98 (br s, 1H), 2.78 (br s, 1H), 2.44 - 2.26 (m, 2H), 1.98 - 1.63 (m, 6H), 1.47 - 1.32 (m, 2H), 1.04 (br d, J=15.6 Hz, 6H) | 2.45 |
| 123 | 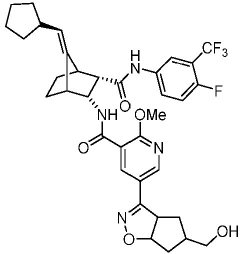 | N-((1R,2R,3S,4R,Z) -7-(cyclopentylmeth ylene)-3-((4-fluoro-3-(trifluoromethyl)p henyl)carbamoyl) bicyclo[2.2.1]hept an-2-yl)-5-(5-(hydroxymethyl)-3a,5,6,6a-tetrahydro-4H-cyclopenta[d]isox azol-3-yl)-2-methoxynicotina mide | 671.1 | 1H NMR (500 MHz, DMSO-d6) δ 10.56 (s, 1H), 10.01 (br d, J=6.7 Hz, 1H), 8.58 (dd, J=15.6, 2.1 Hz, 2H), 8.29 - 8.19 (m, 1H), 7.84 - 7.75 (m, 1H), 7.49 (t, J=9.8 Hz, 1H), 5.25 - 5.07 (m, 2H), 4.43 - 4.34 (m, 1H), 4.26 (br t, J=8.5 Hz, 1H), 4.13 (s, 3H), 3.17 (br dd, J=10.7, 4.3 Hz, 1H), 3.01 (br d, J=4.0 Hz, 1H), 2.73 (br s, 1H), 2.65 - 2.58 (m, 1H), 2.51 (br s, 3H), 2.01 (br dd, J=13.6, 5.3 Hz, 1H), 1.97 - 1.88 (m, 1H), 1.87 - 1.70 (m, 5H), 1.69 - 1.52 (m, 6H), 1.39 (br s, 2H), 1.32 - 1.20 (m, 2H) | 2.68 |
| 124 | 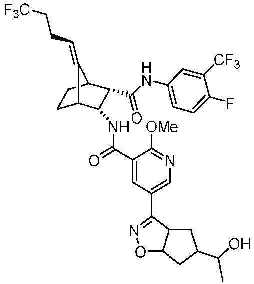 | N-((1R,2R,3S,4R,Z) -3-((4-fluoro-3-(trifluoromethyl)p henyl)carbamoyl) -7-(4,4,4-trifluorobutyliden e)bicyclo[2.2.1]he ptan-2-yl)-5-(5-(1-hydroxyethyl)-3a,5,6,6a-tetrahydro-4H-cyclopenta[d]isox azol-3-yl)-2-methoxynicotina mide | 713.2 | 1H NMR (500 MHz, DMSO-d6) δ 10.60 (s, 1H), 10.03 (br d, J=7.0 Hz, 1H), 8.66 - 8.50 (m, 2H), 8.25 (br d, J=4.3 Hz, 1H), 7.88 - 7.78 (m, 1H), 7.50 (br t, J=9.8 Hz, 1H), 5.30 (br t, J=6.7 Hz, 1H), 5.15 (br dd, J=8.5, 4.6 Hz, 1H), 4.50 (br d, J=5.2 Hz, 1H), 4.44 - 4.34 (m, 1H), 4.26 (br t, J=7.8 Hz, 1H), 4.14 (s, 3H), 3.47 - 3.35 (m, 1H), 3.19 (br dd, J=11.1, 3.8 Hz, 1H), 3.02 (br s, 1H), 2.78 (br s, 1H), 2.45 - 2.26 (m, 4H), 2.16 - 2.05 (m, 1H), 1.96 - 1.53 (m, 6H), 1.48 - 1.36 (m, 2H), 1.01 (br d, J=6.1 Hz, 3H) | 2.51 |
| 125 | 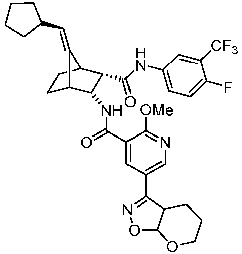 | N-((1R,2R,3S,4R,Z) -7-(cyclopentylmeth ylene)-3-((4-fluoro-3-(trifluoromethyl)p henyl)carbamoyl) bicyclo[2.2.1]hept an-2-yl)-2-methoxy-5-(3a,5,6,7a-tetrahydro-4H-pyrano[3,2-d]isoxazol-3-yl)nicotinamide | 657.2 | 1H NMR (500 MHz, DMSO-d6) δ 10.55 (s, 1H), 10.05 (br d, J=6.9 Hz, 1H), 8.86 (s, 1H), 8.64 (d, J=2.2 Hz, 1H), 8.56 (d, J=2.1 Hz, 1H), 8.29 - 8.19 (m, 1H), 7.86 - 7.76 (m, 1H), 7.49 (br t, J=9.8 Hz, 1H), 5.20 (d, J=8.8 Hz, 1H), 4.44 - 4.32 (m, 1H), 4.16 (s, 3H), 3.45 - 3.41 (m, 1H), 3.17 (br dd, J=11.1, 3.5 Hz, 2H), 3.02 (br s, 1H), 2.73 (br s, 1H), 2.59 (br d, J=7.3 Hz, 2H), 1.90 - 1.73 (m, 4H), 1.73 - 1.53 (m, 7H), 1.40 (br s, 2H), 1.32 - 1.15 (m, 3H) | 2.74 |
| 126 | 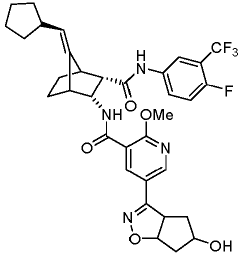 | N-((1R,2R,3S,4R,Z) -7-(cyclopentylmeth ylene)-3-((4-fluoro-3-(trifluoromethyl)p henyl)carbamoyl) bicyclo[2.2.1]hept an-2-yl)-5-(5-hydroxy-3a,5,6,6a-tetrahydro-4H-cyclopenta[d]isox azol-3-yl)-2-methoxynicotina mide | 657.1 | 1H NMR (500 MHz, DMSO-d6) δ 10.56 (s, 1H), 10.01 (br d, J=6.7 Hz, 1H), 8.55 (br d, J=2.7 Hz, 2H), 8.29 - 8.18 (m, 1H), 7.84 - 7.73 (m, 1H), 7.48 (br t, J=9.5 Hz, 1H), 5.24 - 5.13 (m, 2H), 4.37 (br s, 1H), 4.26 (td, J=9.5, 3.5 Hz, 1H), 4.16 - 4.05 (m, 3H), 3.90 (s, 1H), 3.23 - 3.12 (m, 3H), 3.00 (br s, 1H), 2.72 (br s, 1H), 2.08 - 1.88 (m, 3H), 1.85 - 1.71 (m, 5H), 1.68 - 1.49 (m, 4H), 1.39 (br s, 2H), 1.30 - 1.19 (m, 2H) | 2.72 |
| 127 | 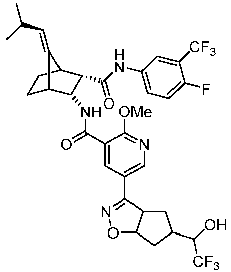 | N-((1R,2R,3S,4R,Z) -3-((4-fluoro-3-(trifluoromethyl)p henyl)carbamoyl) -7-(2-methylpropyliden e)bicyclo[2.2.1]he ptan-2-yl)-2-methoxy-5-(5-(2,2,2-trifluoro-1-hydroxyethyl)-3a,5,6,6a-tetrahydro-4H-cyclopenta[d]isox azol-3-yl)nicotinamide | 713.1 | 1H NMR (500 MHz, DMSO-d6) δ 10.62 - 10.52 (m, 1H), 10.02 (br d, J=7.0 Hz, 1H), 8.70 - 8.54 (m, 2H), 8.25 (br d, J=1.5 Hz, 1H), 7.87 - 7.76 (m, 1H), 7.61 - 7.45 (m, 1H), 6.25 (br d, J=6.7 Hz, 1H), 5.27 - 5.07 (m, 2H), 4.44 - 4.27 (m, 2H), 4.20 - 4.08 (m, 3H), 3.98 (br d, J=8.5 Hz, 1H), 3.41 (br s, 1H), 3.21 - 3.12 (m, 1H), 3.01 (br s, 1H), 2.72 (br s, 1H), 2.11 - 1.93 (m, 3H), 1.86 - 1.69 (m, 4H), 1.52 - 1.34 (m, 2H), 1.10 - 0.93 (m, 6H) | 2.75 |
| 128 | 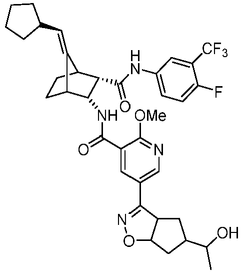 | N-((1R,2R,3S,4R,Z) -7-(cyclopentylmeth ylene)-3-((4-fluoro-3-(trifluoromethyl)p henyl)carbamoyl) bicyclo[2.2.1]hept an-2-yl)-5-(5-(1-hydroxyethyl)-3a,5,6,6a-tetrahydro-4H-cyclopenta[d]isox azol-3-yl)-2-methoxynicotina mide | 685.3 | 1H NMR (500 MHz, DMSO-d6) δ 10.57 (s, 1H), 10.01 (br d, J=6.7 Hz, 1H), 8.58 (br d, J=9.5 Hz, 2H), 8.24 (br d, J=5.5 Hz, 1H), 7.86 - 7.77 (m, 1H), 7.49 (br t, J=9.6 Hz, 1H), 5.20 (br d, J=8.9 Hz, 1H), 5.15 (br dd, J=8.4, 4.7 Hz, 1H), 4.37 (br d, J=6.7 Hz, 1H), 4.28 - 4.21 (m, 1H), 4.17 - 4.08 (m, 3H), 3.91 (s, 1H), 3.17 (br dd, J=10.2, 3.8 Hz, 1H), 3.01 (br s, 1H), 2.73 (br s, 1H), 2.64 - 2.57 (m, 1H), 2.16 - 2.07 (m, 1H), 1.89 - 1.53 (m, 12H), 1.46 - 1.35 (m, 2H), 1.33 - 1.18 (m, 3H), 1.00 (br d, J=5.8 Hz, 3H) | 2.72 |
| 129 | 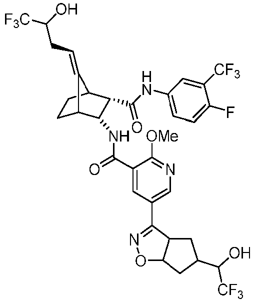 | N-((1R,2R,3S,4R,Z) -3-((4-fluoro-3-(trifluoromethyl)p henyl)carbamoyl) -7-(4,4,4-trifluoro-3-hydroxybutyliden e)bicyclo[2.2.1]he ptan-2-yl)-2-methoxy-5-(5-(2,2,2-trifluoro-1-hydroxyethyl)-3a,5,6,6a-tetrahydro-4H-cyclopenta[d]isox azol-3-yl)nicotinamide | 783.0 | 1H NMR (500 MHz, DMSO-d6) δ 10.59 (br s, 1H), 10.02 (br s, 1H), 8.66 - 8.52 (m, 2H), 8.21 (br s, 1H), 7.80 (br d, J=2.7 Hz, 1H), 7.54 - 7.42 (m, 1H), 5.32 (br d, J=1.8 Hz, 1H), 5.22 - 5.12 (m, 1H), 4.44 - 4.36 (m, 1H), 4.33 - 4.24 (m, 1H), 4.12 (br d, J=3.1 Hz, 3H), 4.04 - 3.88 (m, 2H), 3.21 - 3.13 (m, 1H), 2.99 (br s, 1H), 2.78 (br s, 1H), 2.48 - 2.36 (m, 2H), 2.36 - 2.24 (m, 1H), 2.10 - 1.90 (m, 4H), 1.85 - 1.71 (m, 4H), 1.41 (br s, 2H) | 2.46 |
| 130 | 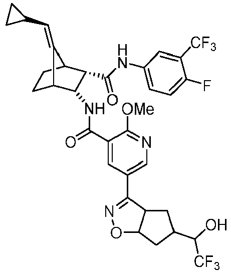 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-2-methoxy-5-(5-(2,2,2-trifluoro-1-hydroxyethyl)-3a,5,6,6a-tetrahydro-4H-cyclopenta[d]isox azol-3-yl)nicotinamide | 711.2 | 1H NMR (500 MHz, DMSO-d6) δ 10.53 (s, 1H), 10.04 (d, J=7.0 Hz, 1H), 8.63 - 8.60 (m, 1H), 8.59 - 8.55 (m, 1H), 8.25 - 8.21 (m, 1H), 7.80 (br dd, J=7.9, 3.6 Hz, 1H), 7.48 (t, J=9.7 Hz, 1H), 5.18 (dd, J=8.7, 4.9 Hz, 1H), 4.73 - 4.67 (m, 1H), 4.46 - 4.39 (m, 1H), 4.30 (t, J=8.9 Hz, 1H), 4.13 (s, 3H), 3.99 - 3.91 (m, 1H), 3.19 - 3.14 (m, 1H), 3.13 - 3.08 (m, 1H), 2.73 (br s, 1H), 2.05 - 1.88 (m, 3H), 1.86 - 1.75 (m, 4H), 1.54 - 1.45 (m, 1H), 1.44 - 1.34 (m, 2H), 0.80 - 0.69 (m, 2H), 0.39 - 0.30 (m, 2H) | 2.53, B |
| 131 | 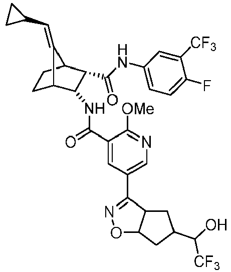 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-2-methoxy-5-(5-(2,2,2-trifluoro-1-hydroxyethyl)-3a,5,6,6a-tetrahydro-4H-cyclopenta[d]isox azol-3-yl)nicotinamide | 711.2 | 1H NMR (400 MHz, DMSO-d6) δ 10.54 (s, 1H), 10.05 (d, J=7.0 Hz, 1H), 8.66 - 8.61 (m, 1H), 8.58 (d, J=2.3 Hz, 1H), 8.24 (dd, J=6.5, 2.5 Hz, 1H), 7.81 (dt, J=8.4, 3.7 Hz, 1H), 7.50 (t, J=9.8 Hz, 1H), 5.19 (dd, J=8.8, 4.9 Hz, 1H), 4.71 (d, J=9.4 Hz, 1H), 4.44 (ddd, J=10.4, 6.6, 4.2 Hz, 1H), 4.32 (t, J=8.6 Hz, 1H), 4.14 (s, 3H), 4.01 - 3.90 (m, 1H), 3.18 (dd, J=10.8, 4.3 Hz, 1H), 3.14 - 3.09 (m, 1H), 2.74 (br s, 1H), 2.08 - 1.89 (m, 3H), 1.87 - 1.75 (m, 4H), 1.56 - 1.46 (m, 1H), 1.42 (br d, J=8.4 Hz, 2H), 0.80 - 0.69 (m, 2H), 0.40 - 0.30 (m, 2H) | 2.57, B |
| 132 | 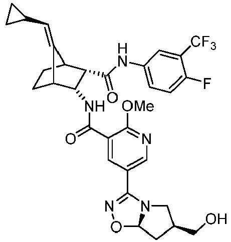 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-5-((6S,7aR)-6-(hydroxymethyl)-5,6,7,7a-tetrahydropyrrolo [1,2-d][1,2,4]oxadiazo l-3-yl)-2-methoxynicotina mide | 644.3 | 1H NMR (400 MHz, DMSO-d6) δ 10.58 - 10.50 (m, 1H), 10.10 - 10.02 (m, 1H), 8.64 - 8.60 (m, 1H), 8.55 - 8.49 (m, 1H), 8.26 - 8.18 (m, 1H), 7.84 - 7.75 (m, 1H), 7.49 (t, J=9.8 Hz, 1H), 5.89 - 5.77 (m, 1H), 4.70 (d, J=9.6 Hz, 1H), 4.47 - 4.39 (m, 1H), 4.15 (d, J=2.2 Hz, 3H), 3.38 - 3.27 (m, 3H), 3.21 - 3.07 (m, 4H), 2.78 - 2.71 (m, 1H), 2.33 - 2.25 (m, 1H), 2.11 - 1.93 (m, 1H), 1.85 - 1.73 (m, 3H), 1.55 - 1.37 (m, 3H), 0.82 - 0.67 (m, 2H), 0.41 - 0.27 (m, 2H) | 2.37, B |
| 133 | 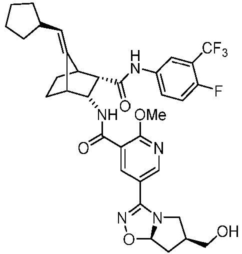 | N-((1R,2R,3S,4R,Z) -7-(cyclopentylmeth ylene)-3-((4-fluoro-3-(trifluoromethyl)p henyl)carbamoyl) bicyclo[2.2.1]hept an-2-yl)-5-((6S,7aR)-6-(hydroxymethyl)-5,6,7,7a-tetrahydropyrrolo [1,2-d][1,2,4]oxadiazo l-3-yl)-2-methoxynicotina mide | 672.3 | 1H NMR (500 MHz, DMSO-d6) δ 10.57 (s, 1H), 10.04 - 9.98 (m, 1H), 8.62 - 8.57 (m, 1H), 8.51 (d, J=1.5 Hz, 1H), 8.22 (br d, J=2.7 Hz, 1H), 7.81 - 7.76 (m, 1H), 7.48 (br t, J=9.8 Hz, 1H), 5.89 - 5.77 (m, 1H), 5.19 (br d, J=8.9 Hz, 1H), 4.41 - 4.33 (m, 1H), 4.14 (d, J=2.7 Hz, 3H), 3.19 - 3.13 (m, 1H), 3.02 - 2.97 (m, 1H), 2.95 - 2.88 (m, 1H), 2.74 - 2.70 (m, 1H), 2.62 - 2.56 (m, 1H), 2.32 - 2.14 (m, 2H), 2.08 - 1.94 (m, 2H), 1.86 - 1.70 (m, 5H), 1.69 - 1.51 (m, 5H), 1.44 - 1.34 (m, 2H), 1.30 - 1.23 (m, 2H) | 2.60, B |
| 134 | 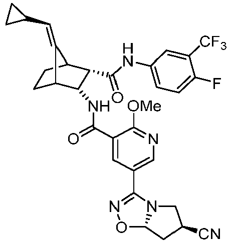 | 5-((6S,7aS)-6-cyano-5,6,7,7a-tetrahydropyrrolo [1,2-d][1,2,4]oxadiazo l-3-yl)-N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-2-methoxynicotina mide | 639.2 | 1H NMR (500 MHz, DMSO-d6) δ 10.55 (s, 1H), 10.08 - 10.03 (m, 1H), 8.60 - 8.56 (m, 1H), 8.52 - 8.48 (m, 1H), 8.22 (dd, J=6.1, 1.8 Hz, 1H), 7.82 - 7.76 (m, 1H), 7.52 - 7.45 (m, 1H), 5.91 - 5.87 (m, 1H), 4.70 (d, J=9.8 Hz, 1H), 4.46 - 4.39 (m, 1H), 4.15 (s, 3H), 3.75 (dd, J=11.4, 7.2 Hz, 1H), 3.38 - 3.25 (m, 1H), 3.19 - 3.09 (m, 2H), 2.73 (br s, 1H), 2.54 - 2.51 (m, 2H), 1.84 - 1.74 (m, 2H), 1.55 - 1.46 (m, 1H), 1.45 - 1.36 (m, 2H), 0.80 - 0.67 (m, 2H), 0.39 - 0.31 (m, 2H) | 2.50, B |
| 135 | 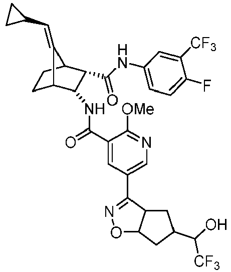 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-2-methoxy-5-(5-(2,2,2-trifluoro-1-hydroxyethyl)-3a,5,6,6a-tetrahydro-4H-cyclopenta[d]isox azol-3-yl)nicotinamide | 711.2 | 1H NMR (400 MHz, DMSO-d6) δ 10.54 (s, 1H), 10.06 - 9.99 (m, 1H), 8.63 - 8.55 (m, 2H), 8.23 (dd, J=6.5, 2.3 Hz, 1H), 7.83 - 7.77 (m, 1H), 7.48 (t, J=9.8 Hz, 1H), 5.19 - 5.14 (m, 1H), 4.69 (d, J=9.4 Hz, 1H), 4.46 - 4.39 (m, 1H), 4.31 (br t, J=8.8 Hz, 1H), 4.15 - 4.10 (m, 3H), 4.00 - 3.90 (m, 1H), 3.19 - 3.09 (m, 2H), 2.75 - 2.71 (m, 1H), 2.06 - 1.95 (m, 2H), 1.94 - 1.86 (m, 1H), 1.85 - 1.73 (m, 4H), 1.55 - 1.46 (m, 1H), 1.44 - 1.35 (m, 2H), 0.79 - 0.68 (m, 2H), 0.40 - 0.28 (m, 2H) | 2.57, B |
| 136 | 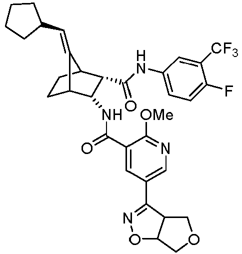 | N-((1R,2R,3S,4R,Z) -7-(cyclopentylmeth ylene)-3-((4-fluoro-3-(trifluoromethyl)p henyl)carbamoyl) bicyclo[2.2.1]hept an-2-yl)-2-methoxy-5-(3a,4,6,6a-tetrahydrofuro[3, 4-d]isoxazol-3-yl)nicotinamide | 643.2 | 1H NMR (400 MHz, DMSO-d6) δ = 10.55 (s, 1H), 10.00 (d, J = 6.9 Hz, 1H), 8.66 - 8.54 (m, 2H), 8.24 (dd, J = 2.4, 6.6 Hz, 1H), 7.86 - 7.71 (m, 1H), 7.49 (t, J = 9.8 Hz, 1H), 5.39 (dd, J = 3.5, 9.3 Hz, 1H), 5.20 (d, J = 8.9 Hz, 1H), 4.55 (br t, J = 7.4 Hz, 1H), 4.43 - 4.29 (m, 1H), 4.17 - 4.06 (m, 4H), 3.96 (d, J = 9.1 Hz, 1H), 3.76 (dd, J = 6.8, 9.6 Hz, 1H), 3.67 (dd, J = 3.6, 10.8 Hz, 1H), 3.20 - 3.12 (m, 1H), 3.01 (br s, 1H), 2.73 (br s, 1H), 2.63 - 2.56 (m, 1H), 1.88 - 1.72 (m, 4H), 1.70 - 1.53 (m, 4H), 1.39 (br s, 2H), 1.27 (dt, J = 4.3, 8.3 Hz, 2H) | 2.64, A |
| 137 | 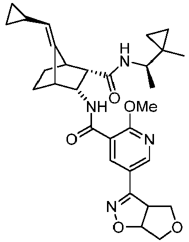 | N-((1R,2R,3S,4R,Z) -7-(cyclopropylmeth ylene)-3-(((R)-1-(1-methylcyclopropy l)ethyl)carbamoyl )bicyclo[2.2.1]he ptan-2-yl)-2-methoxy-5-(3a,4,6,6a-tetrahydrofuro[3, 4-d]isoxazol-3-yl)nicotinamide | 535.2 | 1H NMR (400 MHz, DMSO-d6) δ = 10.27 (d, J = 6.8 Hz, 1H), 8.72 - 8.45 (m, 2H), 7.95 (d, J = 8.5 Hz, 1H), 5.39 (dd, J = 3.4, 9.1 Hz, 1H), 4.65 (d, J = 9.5 Hz, 1H), 4.62 - 4.52 (m, 1H), 4.33 - 4.22 (m, 1H), 4.15 - 4.07 (m, 4H), 3.96 (br d, J = 9.3 Hz, 1H), 3.77 (dd, J = 6.8, 9.5 Hz, 1H), 3.70 - 3.64 (m, 1H), 3.54 - 3.43 (m, 1H), 3.09 - 3.05 (m, 1H), 3.04 - 3.01 (m, 1H), 2.96 (dd, J = 4.4, 11.1 Hz, 1H), 2.55 (br s, 2H), 1.93 - 1.70 (m, 2H), 1.53 - 1.44 (m, 1H), 1.42 - 1.32 (m, 2H), 1.06 - 1.01 (m, 4H), 0.99 (s, 2H), 0.78 - 0.67 (m, 2H), 0.61 - 0.51 (m, 1H), 0.39 - 0.30 (m, 3H), 0.25 - 0.13 (m, 2H) | 2.21, A |
| 138 | 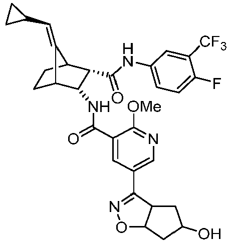 | N-((1R,2R,3S,4R,Z) -7-(cyclopropylmeth ylene)-3-((4-fluoro-3-(trifluoromethyl)p henyl)carbamoyl) bicyclo[2.2.1]hept an-2-yl)-5-(5-hydroxy-3a,5,6,6a-tetrahydro-4H-cyclopenta[d]isox azol-3-yl)-2-methoxynicotina mide | 629.2 | 1H NMR (400 MHz, DMSO-d6) δ = 10.54 (s, 1H), 10.04 (d, J = 6.8 Hz, 1H), 8.57 (s, 2H), 8.23 (dd, J = 2.3, 7.0 Hz, 1H), 7.84 - 7.75 (m, 1H), 7.50 (t, J = 9.6 Hz, 1H), 5.22 - 5.13 (m, 1H), 4.87 (d, J = 4.3 Hz, 1H), 4.71 (d, J = 9.5 Hz, 1H), 4.49 - 4.41 (m, 1H), 4.32 - 4.25 (m, 1H), 4.14 (s, 4H), 3.20 - 3.15 (m, 1H), 3.13 - 3.09 (m, 1H), 2.75 (br d, J = 4.3 Hz, 1H), 2.02 - 1.88 (m, 3H), 1.85 - 1.76 (m, 3H), 1.56 - 1.47 (m, 1H), 1.44 - 1.35 (m, 2H), 0.82 - 0.65 (m, 2H), 0.40 - 0.31 (m, 2H) | 2.37, A |
| 139 | 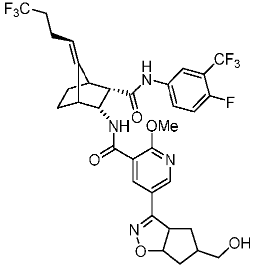 | N-((1R,2R,3S,4R,Z) -3-((4-fluoro-3-(trifluoromethyl)p henyl)carbamoyl) -7-(4,4,4-trifluorobutyliden e)bicyclo[2.2.1]he ptan-2-yl)-5-(5-(hydroxymethyl)-3a,5,6,6a-tetrahydro-4H-cyclopenta[d]isox azol-3-yl)-2-methoxynicotina mide | 699.2 | 1H NMR (500 MHz, DMSO-d6) δ 10.59 (s, 1H), 10.02 (d, J=6.9 Hz, 1H), 8.60 (d, J=2.3 Hz, 1H), 8.56 (d, J=2.4 Hz, 1H), 8.28 - 8.18 (m, 1H), 7.86 - 7.75 (m, 1H), 7.49 (t, J=9.7 Hz, 1H), 5.28 (t, J=6.9 Hz, 1H), 5.16 (dd, J=8.6, 5.0 Hz, 1H), 4.59 - 4.48 (m, 1H), 4.44 - 4.35 (m, 1H), 4.26 (br t, J=9.0 Hz, 1H), 4.13 (s, 3H), 3.18 (br dd, J=10.6, 4.5 Hz, 1H), 3.01 (br s, 1H), 2.77 (br s, 1H), 2.44 - 2.24 (m, 4H), 2.00 (br dd, J=13.5, 6.0 Hz, 1H), 1.95 - 1.72 (m, 4H), 1.72 - 1.62 (m, 1H), 1.62 - 1.52 (m, 1H), 1.40 (br d, J=6.0 Hz, 2H) | 2.45 |
| 140 | 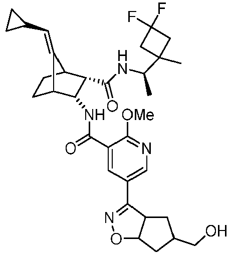 | N-((1R,2R,3S,4R,Z) -7-(cyclopropylmeth ylene)-3-(((R)-1-(3,3-difluoro-1-methylcyclobutyl) ethyl)carbamoyl) bicyclo[2.2.1]hept an-2-yl)-5-(5-(hydroxymethyl)-3a,5,6,6a-tetrahydro-4H-cyclopenta[d]isox azol-3-yl)-2-methoxynicotina mide | 613.3 | 1H NMR (500 MHz, DMSO-d6) δ 9.93 (br d, J=6.4 Hz, 1H), 8.33 (d, J=2.1 Hz, 1H), 8.27 (d, J=2.1 Hz, 1H), 7.86 (br d, J=9.2 Hz, 1H), 4.91 (br dd, J=8.4, 5.3 Hz, 1H), 4.40 (br d, J=9.5 Hz, 1H), 4.11 - 3.97 (m, 2H), 3.85 (s, 3H), 3.79 (quin, J=7.2 Hz, 1H), 3.20 - 3.04 (m, 1H), 2.82 (br s, 1H), 2.74 (br dd, J=10.5, 4.1 Hz, 1H), 2.46 - 2.33 (m, 1H), 2.24 - 2.07 (m, 1H), 2.03 - 1.81 (m, 2H), 1.76 (br dd, J=13.6, 6.0 Hz, 1H), 1.71 - 1.59 (m, 1H), 1.58 - 1.38 (m, 4H), 1.37 - 1.27 (m, 1H), 1.26 - 1.17 (m, 1H), 1.17 - 1.02 (m, 2H), 0.89 (s, 3H), 0.70 (br d, J=7.0 Hz, 3H), 0.53 - 0.41 (m, 2H), 0.07 (br d, J=3.1 Hz, 2H) | 2.26 |
| 141 | 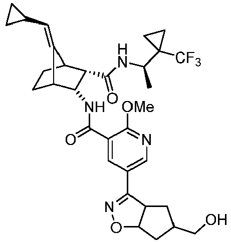 | N-((1R,2R,3S,4R,Z) -7-(cyclopropylmeth ylene)-3-(((R)-1-(1-(trifluoromethyl)c yclopropyl)ethyl) carbamoyl)bicycl o[2.2.1]heptan-2-yl)-5-(5-(hydroxymethyl)-3a,5,6,6a-tetrahydro-4H-cyclopenta[d]isox azol-3-yl)-2-methoxynicotina mide | 617.5 | 1H NMR (500 MHz, DMSO-d6) δ 9.91 (br d, J=6.7 Hz, 1H), 8.33 (d, J=2.4 Hz, 1H), 8.27 (d, J=2.1 Hz, 1H), 7.90 (br d, J=8.5 Hz, 1H), 4.91 (br dd, J=8.5, 5.5 Hz, 1H), 4.40 (d, J=9.5 Hz, 1H), 4.09 - 3.97 (m, 2H), 3.93 - 3.80 (m, 3H), 2.82 (br s, 1H), 2.76 - 2.69 (m, 1H), 1.76 (br dd, J=13.3, 5.3 Hz, 1H), 1.70 - 1.60 (m, 2H), 1.59 - 1.38 (m, 4H), 1.37 - 1.28 (m, 1H), 1.27 - 1.17 (m, 1H), 1.16 - 1.03 (m, 2H), 0.87 (br d, J=7.0 Hz, 3H), 0.74 (br d, J=11.0 Hz, 1H), 0.67 - 0.57 (m, 2H), 0.56 - 0.40 (m, 3H), 0.07 (br d, J=3.7 Hz, 2H) | 2.29 |
| 142 | 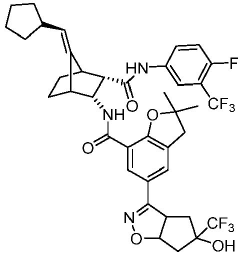 **Isomer 1** | N-[(1R,2R,3S,4R,7Z )-7-(cyclopentylmeth ylidene)-3-{[4-fluoro-3-(trifluoromethyl)p henyl]carbamoyl } bicyclo[2.2.1]hept an-2-yl]-5-[5-hydroxy-5-(trifluoromethyl)-3aH,4H,5H,6H,6a H-cyclopenta[d][1,2 ]oxazol-3-yl]-2,2-dimethyl-2,3-dihydro-1-benzofuran-7-carboxamide | 764.3 | (400 MHz, DMSO-d6) δ = 10.43 (br s, 1H), 8.12 - 8.09 (m, 1H), 8.03 (d, J = 7.0 Hz, 1H), 7.87 - 7.83 (m, 2H), 7.70 (s, 1H), 7.46 (t, J = 9.8Hz, 1H), 5.26 (dd, J = 6.1, 8.9 Hz, 1H), 5.02 (d, J = 8.9 Hz, 1H), 4.59 - 4.55 (m, 1H), 4.40 - 4.34 (m, 1H), 3.17 (br d, J = 3.3 Hz, 2H), 2.97 (t, J =3.4 Hz, 1H), 2.58 (br s, 2H), 2.31 - 2.22 (m, 5H), 2.00 - 1.94 (m, 2H), 1.81 - 1.70 (m, 6H), 1.63 - 1.49 (m, 19H), 1.26 - 1.22 (m, 3H) | 2.65, B |
| 143 | 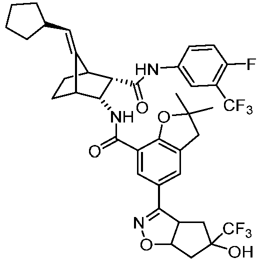 **Isomer 2** | (1R,2S,3R,4R,7Z) -7-(cyclopentylmeth ylidene)-N-[4-fluoro-3-(trifluoromethyl)p henyl]-3-{5-[5-hydroxy-5-(trifluoromethyl)-3aH,4H,5H,6H,6a H-cyclopenta[d][1,2 ]oxazol-3-yl]-2-methoxybenzami do}bicyclo[2.2.1] heptane-2-carboxamide | 724.2 | (400 MHz, DMSO-d6) δ = 10.53 (s, 1H), 10.31 (s, 1H), 9.89 (d, J = 7.1 Hz, 1H), 8.30 - 8.17 (m, 1H), 7.91 - 7.71 (m, 1H), 7.53 - 7.39 (m, 1H), 7.33 - 7.15 (m, 1H), 6.02 - 5.86 (m, 1H), 5.36 - 5.23 (m, 1H), 5.20 (d, J = 8.9 Hz, 1H), 4.43 - 4.37 (m, 1H), 4.04 (s, 1H), 3.51 - 3.36 (m, 9H), 2.56 - 2.53 (m, 2H), 2.41 (br d, J = 4.9 Hz, 1H), 2.34 - 2.24 (m, 2H), 1.82 - 1.72 (m, 1H), 1.68 - 1.52 (m, 2H), 1.25 (br d, J = 8.4 Hz, 1H) | 2.67, B |
| 144 | 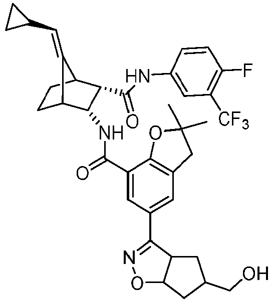 | N-[(1R,2R,3S,4R,7Z )-7-(cyclopropylmeth ylidene)-3-{[4-fluoro-3-(trifluoromethyl)p henyl]carbamoyl} bicyclo[2.2.1]hept an-2-yl]-5-[5-(hydroxymethyl)-3aH,4H,5H,6H,6a H-cyclopenta[d][1,2 ]oxazol-3-yl]-2,2-dimethyl-2,3-dihydro-1-benzofuran-7-carboxamide | 682.3 | 1H NMR (400 MHz, DMSO-d6) δ ppm 10.33 - 10.62 (m, 1 H) 9.48 - 9.69 (m, 1 H) 8.31 - 8.47 (m, 1 H) 7.81 - 7.98 (m, 1 H) 7.59 - 7.74 (m, 2 H) 7.39 - 7.54 (m, 1 H) 5.01 - 5.21 (m, 1 H) 4.65 - 4.77 (m, 1 H) 4.38 - 4.52 (m, 2 H) 4.01 - 4.24 (m, 1 H) 2.98 - 3.21 (m, 4 H) 2.61 - 2.83 (m, 2 H) 2.10 - 2.27 (m, 1 H) 1.70 - 2.02 (m, 5 H) 1.58 - 1.68 (m, 4 H) 1.31 - 1.56 (m, 11 H) 0.66 - 0.84 (m, 2 H) 0.20 - 0.43 (m, 2 H) | 3.93, C |
| 145 | 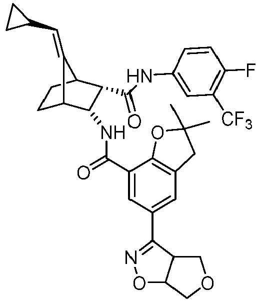 | 5-{3aH,4H,6H,6aH-furo[3,4-d][1,2]oxazol-3-yl }-N-[(1R,2R,3S,4R,7Z )-7-(cyclopropylmeth ylidene)-3-{[4-fluoro-3-(trifluoromethyl)p henyl]carbamoyl} bicyclo[2.2.1]hept an-2-yl]-2,2-dimethyl-2,3-dihydro-1-benzofuran-7-carboxamide | 654.6 | 1H NMR (400 MHz, DMSO-d6) δ ppm 10.24 - 10.78 (m, 1 H) 9.52 - 9.71 (m, 1 H) 8.30 - 8.45 (m, 1 H) 7.83 - 7.96 (m, 1 H) 7.60 - 7.74 (m, 2 H) 7.39 - 7.51 (m, 1 H) 5.32 (dd, J=9.54, 3.51 Hz, 1 H) 4.69 (d, J=9.54 Hz, 1 H) 4.42 - 4.54 (m, 2 H) 4.06 - 4.13 (m, 1 H) 3.85 - 3.94 (m, 1 H) 3.71 - 3.81 (m, 1 H) 3.58 - 3.68 (m, 1 H) 3.45 - 3.52 (m, 1 H) 3.12 - 3.20 (m, 3 H) 3.02 - 3.11 (m, 2 H) 2.64 - 2.75 (m, 2 H) 2.32 - 2.37 (m, 1 H) 2.12 - 2.20 (m, 1 H) 1.74 - 1.95 (m, 3 H) 1.59 - 1.68 (m, 3 H) 1.33 - 1.58 (m, 11 H) 0.66 - 0.82 (m, 2 H) 0.26 - 0.37 (m, 2 H) | 3.66, C |
| 146 | 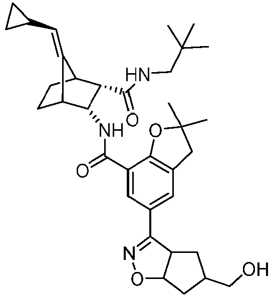 | N-[(1R,2R,3S,4R,7Z )-7-(cyclopropylmeth ylidene)-3-[(2,2-dimethylpropyl)c arbamoyl]bicyclo [2.2.1]heptan-2-yl]-5-[5-(hydroxymethyl)-3aH,4H,5H,6H,6a H-cyclopenta[d][1,2 ]oxazol-3-yl]-2,2-dimethyl-2,3-dihydro-1-benzofuran-7-carboxamide | 590.3 | (400 MHz, DMSO-d6) δ = 9.70 (d, J = 7.5 Hz, 1H), 7.90 - 7.81 (m, 2H), 7.67 (s, 1H), 5.09 (dd, J = 5.1, 8.9 Hz, 1H), 4.62 (d, J = 9.8 Hz,1H), 4.47 (t, J = 5.3 Hz, 1H), 4.38 - 4.29 (m, 1H), 4.16 (t, J = 9.0 Hz, 1H), 3.41 - 3.33 (m, 2H), 3.10 (br d, J = 4.0 Hz, 2H), 3.05 - 2.93 (m, 3H),2.77 (dd, J = 6.1, 13.1 Hz, 1H), 2.04 - 1.85 (m, 3H), 1.83 - 1.74 (m, 2H), 1.65 (br dd, J = 9.3, 12.0 Hz, 1H), 1.57 (d, J = 10.5 Hz, 7H), 1.50 - 1.42(m, 1H), 1.40 - 1.27 (m, 2H), 0.86 - 0.77 (m, 10H), 0.71 (br t, J = 8.9 Hz, 2H), 0.32 (br d, J = 3.0 Hz, 2H) | 2.32, A |
| 147 | 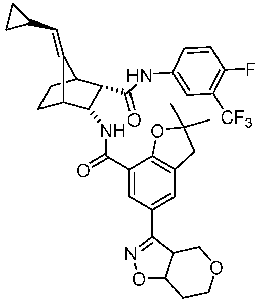 | 5-{3aH,4H,6H,7H,7 aH-pyrano[3,4-d][1,2]oxazol-3-yl }-N-[(1R,2R,3S,4R,7Z )-7-(cyclopropylmeth ylidene)-3-{[4-fluoro-3-(trifluoromethyl)p henyl]carbamoyl } bicyclo[2.2.1]hept an-2-yl]-2,2-dimethyl-2,3-dihydro-1-benzofuran-7-carboxamide | 668.3 | (400 MHz, DMSO-d6) δ = 10.46 (s, 1H), 9.60 (d, J = 7.5 Hz, 1H), 8.39 (dd, J = 2.5, 6.6 Hz, 1H), 7.93 (d, J = 1.9 Hz, 1H), 7.70 (d, J = 1.8Hz, 1H), 7.63 (td, J = 3.9, 8.0 Hz, 1H), 7.46 (t, J = 9.8 Hz, 1H), 4.69 (d, J = 9.6 Hz, 1H), 4.61 (td, J = 3.5, 7.2 Hz, 1H), 4.54 - 4.41 (m, 1H), 3.98(dd, J = 6.7, 11.6 Hz, 1H), 3.81 - 3.63 (m, 2H), 3.47 (dt, J = 3.3, 11.5 Hz, 1H), 3.21 - 3.10 (m, 4H), 3.09 - 3.02 (m, 1H), 2.75 - 2.69 (m, 1H), 2.10 -2.00 (m, 1H), 1.97 - 1.85 (m, 2H), 1.84 - 1.75 (m, 1H), 1.62 (s, 3H), 1.53 - 1.29 (m, 5H), 0.82 - 0.64 (m, 2H), 0.35 (dd, J = 2.2, 4.6 Hz, 2H) | 2.65, A |

| | | | | | |
|---|---|---|---|---|---|
| (a) All LC retention times are based on Method C unless otherwise specified. (b) 1-3 protons were not accounted for in these samples due to overlap with solvent residue and/or artifacts of water suppression. | | | | | |

**Table 5**

| **Ex. No.** | **Structure** | **Name** | **MS (ESI) (M+ H)** | **¹H NMR** | **LC RT^{a} (min) and chiral conditions when separated** |
|---|---|---|---|---|---|
| 148 | 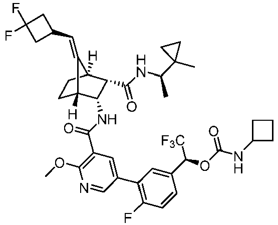 | (S)-1-(3-(5-(((1R,2R,3S,4R, Z)-7-((3,3-difluorocyclobut yl)methylene)-3-(((R)-1-(1-methylcycloprop yl)ethyl)carbamo yl)bicyclo[2.2.1] heptan-2-yl)carbamoyl)-6-methoxypyridin-3-yl)-4-fluorophenyl)-2,2,2-trifluoroethyl cyclobutylcarba mate | 763.4 | 1H NMR (500 MHz, DMSO-d6) δ 10.12 (br d, J=6.6 Hz, 1H), 8.34 (s, 1H), 8.24 (d, J=1.1 Hz, 1H), 7.96 (br d, J=7.8 Hz, 1H), 7.81 (br d, J=8.5 Hz, 1H), 7.58 (br d, J=7.1 Hz, 1H), 7.42 (br d, J=2.7 Hz, 1H), 7.35 - 7.23 (m, 1H), 6.18 (q, J=6.9 Hz, 1H), 5.16 (d, J=8.3 Hz, 1H), 4.12 - 3.98 (m, 1H), 3.92 (s, 3H), 3.81 - 3.70 (m, 1H), 2.84 - 2.56 (m, 5H), 2.41 (br s, 1H), 2.29 - 2.12 (m, 2H), 2.06 - 1.84 (m, 2H), 1.80 - 1.62 (m, 3H), 1.60 - 1.50 (m, 1H), 1.46 - 1.29 (m, 2H), 1.22 - 1.06 (m, 2H), 0.85 (d, J=6.9 Hz, 3H), 0.80 (s, 3H), 0.42 - 0.30 (m, 1H), 0.21 - 0.10 (m, 1H), 0.07 - -0.06 (m, 2H) | 2.89 |
| 149 | 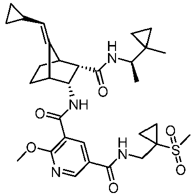 | N3-[(1R,2R,3S,4R,7 Z)-7-(cyclopropylmet hylidene)-3-{[(1R)-1-(1-methylcycloprop yl)ethyl]carbamo yl}bicyclo[2.2.1] heptan-2-yl]-N5-[(1-methanesulfonylcyclopropyl)met hyl]-2-methoxypyridine -3,5-dicarboxamide | 599.3 | 1H NMR (400 MHz, DMSO-d6) δ = 10.27 (d, J = 6.6 Hz, 1H), 8.87 (t, J = 6.1 Hz, 1H), 8.77 (d, J = 2.5 Hz, 1H), 8.71 (d, J = 2.5 Hz, 1H), 7.96 (d, J = 8.5 Hz, 1H), 4.65 (d, J = 9.5 Hz, 1H), 4.34 - 4.18 (m, 1H), 4.10 (s, 3H), 3.83 (d, J = 6.0 Hz, 2H), 3.47 (dd, J = 6.9, 8.4 Hz, 1H), 3.11 - 3.04 (m, 4H), 3.01 - 2.92 (m, 1H), 2.55 (br d, J = 3.6 Hz, 1H), 1.90 - 1.79 (m, 1H), 1.77 - 1.59 (m, 1H), 1.52 - 1.44 (m, 1H), 1.35 (br d, J = 7.8 Hz, 2H), 1.28 - 1.21 (m, 2H), 1.16 - 1.12 (m, 2H), 1.03 (d, J = 6.9 Hz, 3H), 0.99 (s, 3H), 0.78 - 0.69 (m, 2H), 0.59 - 0.48 (m, 1H), 0.39 - 0.29 (m, 3H), 0.25 - 0.11 (m, 2H) | 2.13, A |
| 150 | 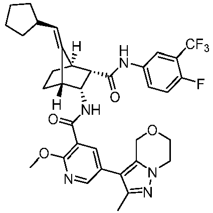 | N-[(1R,2R,3S,4R,7 Z)-7-(cyclopentylmet hylidene)-3-{[4-fluoro-3-(trifluoromethyl) phenyl]carbamo yl}bicyclo[2.2.1] heptan-2-yl]-2-methoxy-5-{2-methyl-4H,6H,7H-pyrazolo[3,2-c][1,4]oxazin-3-yl}pyridine-3-carboxamide | 668.3 | 1H NMR (400 MHz, DMSO-d6) δ = 10.52 (s, 1H), 9.97 (d, J = 7.0 Hz, 1H), 8.27 - 8.18 (m, 2H), 8.16 (d, J = 2.6 Hz, 1H), 7.84 - 7.72 (m, 1H), 7.49 (t, J = 9.8 Hz, 1H), 5.20 (d, J = 8.9 Hz, 1H), 4.83 (s, 2H), 4.39 (ddd, J = 3.7, 6.7, 10.5 Hz, 1H), 4.09 (d, J = 12.4 Hz, 7H), 3.20 - 3.12 (m, 1H), 3.01 (br s, 1H), 2.73 (br s, 1H), 2.64 - 2.56 (m, 1H), 2.21 (s, 3H), 1.91 - 1.72 (m, 4H), 1.70 - 1.53 (m, 4H), 1.48 - 1.20 (m, 5H) | 2.73, A |
| 151 | 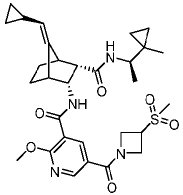 | N-[(1R,2R,4R,7Z)-7-(cyclopropylmet hylidene)-3-{[(1R)-1-(1-methylcycloprop yl)ethyl]carbamo yl}bicyclo[2.2.1] heptan-2-yl]-5-(3-methanesulfonyl azetidine-1-carbonyl)-2-methoxypyridine -3-carboxamide | 585.6 | 1H NMR (400 MHz, DMSO-d6) δ = 10.29 (d, J = 6.5 Hz, 1H), 8.61 (d, J = 2.4 Hz, 1H), 8.48 (d, J = 2.5 Hz, 1H), 7.96 (d, J = 8.5 Hz, 1H), 4.81 - 4.61 (m, 2H), 4.51 (br d, J = 5.5 Hz, 1H), 4.43 - 4.20 (m, 4H), 4.11 (s, 3H), 3.50 - 3.43 (m, 1H), 3.07 (s, 4H), 2.96 (dd, J = 4.3, 10.9 Hz, 1H), 2.55 (br d, J = 3.8 Hz, 1H), 1.92 - 1.63 (m, 2H), 1.48 (dt, J = 4.1, 8.4 Hz, 1H), 1.42 - 1.29 (m, 2H), 1.06 - 0.91 (m, 6H), 0.79 - 0.64 (m, 2H), 0.54 (dd, J = 3.9, 8.3 Hz, 1H), 0.43 - 0.25 (m, 3H), 0.24 - 0.13 (m, 2H) | 2.03, A |
| 152 | 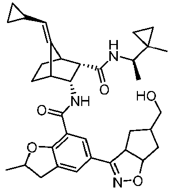 | N-[(1R,2R,3S,4R,7 Z)-7-(cyclopropylmet hylidene)-3-{[(1R)-1-(1-methylcycloprop yl)ethyl]carbamo yl}bicyclo[2.2.1] heptan-2-yl]-5-[5-(hydroxymethyl) - 3aH,4H,5H,6H,6 aH-cyclopenta[d][1, 2]oxazol-3-yl]-2-methyl-2,3-dihydro-1-benzofuran-7-carboxamide | 588.3 | 1H NMR (400 MHz, DMSO-d6) δ = 9.69 (d, J = 7.0 Hz, 1H), 7.91 - 7.77 (m, 2H), 7.68 (s, 1H), 5.17 - 4.99 (m, 2H), 4.63 (d, J = 9.5 Hz, 1H), 4.49 (t,J = 5.3 Hz, 1H), 4.38 - 4.25 (m, 1H), 4.17 (t, J = 8.9 Hz, 1H), 3.47 - 3.38 (m, 3H), 3.02 (br s, 1H), 2.97 - 2.83 (m, 2H), 2.04 - 1.85 (m, 3H), 1.84 -1.72 (m, 2H), 1.70 - 1.43 (m, 6H), 1.35 (br s, 2H), 1.00 (d, J = 6.9 Hz, 3H), 0.97 (s, 3H), 0.77 - 0.63 (m, 2H), 0.58 - 0.46 (m, 1H), 0.39 - 0.25 (m,3H), 0.22 - 0.09 (m, 2H). | 2.27, A |
| 153 | 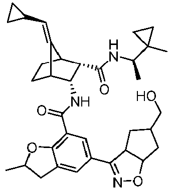 | N-[(1R,2R,3S,4R,7 Z)-7-(cyclopropylmet hylidene)-3-{[(1R)-1-(1-methylcycloprop yl)ethyl]carbamo yl}bicyclo[2.2.1] heptan-2-yl]-5-[5-(hydroxymethyl) - 3aH,4H,5H,6H,6 aH-cyclopenta[d][1, 2]oxazol-3-yl]-2-methyl-2,3-dihydro-1-benzofuran-7-carboxamide | 588.3 | 1H NMR (400 MHz, DMSO-d6) δ = 9.76 (d, J = 7.0 Hz, 1H), 7.91 - 7.79 (m, 2H), 7.68 (s, 1H), 5.26 - 5.17 (m, 1H), 5.10 (dd, J = 5.1, 8.6 Hz, 1H),4.63 (d, J = 9.5 Hz, 1H), 4.49 (t, J = 5.2 Hz, 1H), 4.38 - 4.26 (m, 1H), 4.17 (t, J = 9.1 Hz, 1H), 3.46 - 3.38 (m, 3H), 3.00 (br s, 1H), 2.96 - 2.80 (m,2H), 2.03 - 1.84 (m, 3H), 1.83 - 1.72 (m, 2H), 1.70 - 1.61 (m, 1H), 1.58 - 1.42 (m, 5H), 1.40 - 1.26 (m, 2H), 1.03 - 0.90 (m, 6H), 0.72 (quin, J = 8.7Hz, 2H), 0.60 - 0.48 (m, 1H), 0.41 - 0.26 (m, 3H), 0.24 - 0.10 (m, 2H). | 2.28, A |
| 154 | 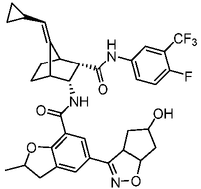 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-{5-hydroxy-3aH,4H,5H,6H,6 aH-cyclopenta[d][1, 2]oxazol-3-yl}-2-methyl-2,3-dihydro-1-benzofuran-7-carboxamide | 654.3 | 1H NMR (400 MHz, DMSO-d6) δ = 10.50 (s, 1H), 9.55 (d, J = 7.4 Hz, 1H), 8.35 (dd, J = 2.5, 6.5 Hz, 1H), 7.90 (d, J = 1.8 Hz, 1H), 7.72 - 7.56 (m,2H), 7.47 (t, J = 9.8 Hz, 1H), 5.34 - 5.19 (m, 1H), 5.08 (ddd, J = 2.4, 6.9, 9.7 Hz, 1H), 4.70 (d, J = 9.5 Hz, 1H), 4.55 - 4.45 (m, 1H), 4.43 (br s, 1H),4.14 (br s, 1H), 4.07 (dt, J = 3.8, 9.5 Hz, 1H), 3.47 (br dd, J = 8.9, 16.2 Hz, 1H), 3.16 (dd, J = 4.3, 10.8 Hz, 1H), 3.06 (br s, 1H), 2.88 (dd, J = 6.9,16.0 Hz, 1H), 2.77 - 2.69 (m, 1H), 2.15 - 2.00 (m, 2H), 1.94 - 1.67 (m, 4H), 1.57 - 1.32 (m, 6H), 0.83 - 0.63 (m, 2H), 0.36 (dd, J = 2.1, 4.4 Hz, 2H). | 2.38, A |
| 155 | 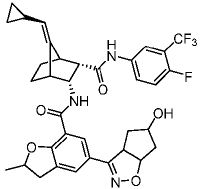 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-{5-hydroxy-3aH,4H,5H,6H,6 aH-cyclopenta[d][1, 2]oxazol-3-yl}-2-methyl-2,3-dihydro-1-benzofuran-7-carboxamide | 654.3 | H NMR (400 MHz, DMSO-d6) δ = 10.46 (s, 1H), 9.52 (d, J = 7.3 Hz, 1H), 8.27 (dd, J = 2.4, 6.6 Hz, 1H), 7.87 (d, J = 1.5 Hz, 1H), 7.77 - 7.69 (m,1H), 7.68 (s, 1H), 7.48 (t, J = 9.8 Hz, 1H), 5.20 - 4.99 (m, 2H), 4.84 (d, J = 4.3 Hz, 1H), 4.70 (d, J = 9.6 Hz, 1H), 4.51 - 4.37 (m, 1H), 4.18 (dt, J =4.3, 9.2 Hz, 1H), 4.13 - 4.00 (m, 1H), 3.41 (br dd, J = 8.6, 16.3 Hz, 1H), 3.15 (dd, J = 3.8, 10.5 Hz, 1H), 3.10 (br s, 1H), 2.93 (dd, J = 7.8, 15.8 Hz, 1H), 2.71 (br d, J = 3.4 Hz, 1H), 2.04 - 1.84 (m, 4H), 1.83 - 1.71 (m, 2H), 1.58 (d, J = 6.3 Hz, 3H), 1.54 - 1.28 (m, 3H), 0.82 - 0.64 (m, 2H), 0.44 -0.23 (m, 2H). | 2.41, A |
| 156 | 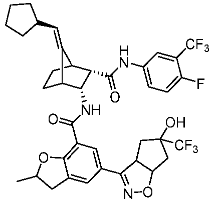 | N-[(1R,2R,3S,4R,7 Z)-7-(cyclopentylmet hylidene)-3-{[4-fluoro-3-(trifluoromethyl) phenyl]carbamo yl}bicyclo[2.2.1] heptan-2-yl]-5-[5-hydroxy-5-(trifluoromethyl) - 3aH,4H,5H,6H,6 aH-cyclopenta[d][1, 2]oxazol-3-yl]-2-methyl-2,3-dihydro-1-benzofuran-7-carboxamide | 750.2 | 1H NMR (400 MHz, DMSO-d6) δ = 10.47 (s, 1H), 9.53 (d, J = 7.4 Hz, 1H), 8.35 (dd, J = 2.6, 6.6 Hz, 1H), 7.89 (d, J = 1.8 Hz, 1H), 7.75 - 7.59 (m,2H), 7.46 (t, J = 9.8 Hz, 1H), 5.91 (s, 1H), 5.33 - 5.15 (m, 3H), 4.43 (ddd, J = 3.8, 7.4, 10.8 Hz, 1H), 4.36 (br t, J = 9.7 Hz, 1H), 3.51 - 3.44 (m,1H), 3.15 (dd, J = 4.1, 10.7 Hz, 1H), 2.96 (br s, 1H), 2.88 (dd, J = 6.8, 16.1 Hz, 1H), 2.71 (br s, 1H), 2.63 - 2.56 (m, 1H), 2.31 - 2.20 (m, 3H), 1.98(br d, J = 13.9 Hz, 1H), 1.91 - 1.74 (m, 4H), 1.70 - 1.53 (m, 5H), 1.47 (d, J = 6.3 Hz, 3H), 1.40 (br d, J = 6.0 Hz, 2H), 1.34 - 1.15 (m, 3H). | 2.80, A |
| 157 | 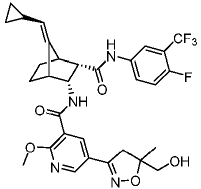 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-[5-(hydroxymethyl) -5-methyl-4,5-dihydro-1,2-oxazol-3-yl]-2-methoxypyridine -3-carboxamide | 617.3 | 1H NMR (400 MHz, DMSO-d6) δ = 10.54 (s, 1H), 10.03 (d, J = 6.9 Hz, 1H), 8.57 (d, J = 2.4 Hz, 1H), 8.52 (d, J = 2.4 Hz, 1H), 8.23 (dd, J = 2.4,6.6 Hz, 1H), 7.90 - 7.70 (m, 1H), 7.49 (t, J = 9.8 Hz, 1H), 5.11 (t, J = 5.7 Hz, 1H), 4.70 (d, J = 9.6 Hz, 1H), 4.49 - 4.35 (m, 1H), 4.13 (s, 3H), 3.47 -3.40 (m, 2H), 3.17 (dd, J = 4.6, 10.4 Hz, 1H), 3.14 - 2.95 (m, 2H), 2.74 (br s, 1H), 1.91 - 1.70 (m, 2H), 1.57 - 1.37 (m, 3H), 1.33 (s, 3H), 0.86 - 0.66(m, 2H), 0.43 - 0.24 (m, 2H). | 2.33, A |
| 158 | 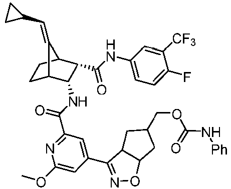 | [3-(2-{[(2R,3S,7Z)-7-(cyclopropylmet hylidene)-3-{[4-fluoro-3-(trifluoromethyl) phenyl]carbamo yl}bicyclo[2.2.1] heptan-2-yl]carbamoyl}-6-methoxypyridin-4-yl)-3aH,4H,5H,6H,6 aH-cyclopenta[d][1,2]oxazol-5-yl]methyl N-phenylcarbamate | 762.3 | 1H NMR (500 MHz, DMSO-d6) δ 10.60 (s, 1H), 10.49 - 10.40 (m, 1H), 9.59 - 9.47 (m, 1H), 8.45 - 8.21 (m, 1H), 7.89 (s, 1H), 7.70 (br dd, J=4.4, 3.5 Hz, 1H), 7.49 (br t, J=9.5 Hz, 1H), 7.42 (br d, J=7.9 Hz, 2H), 7.32 - 7.19 (m, 3H), 6.97 (br t, J=7.3 Hz, 1H), 5.29 (br dd, J=8.7, 5.0 Hz, 2H), 4.72 (br d, J=9.8 Hz, 1H), 4.49 - 4.26 (m, 2H), 4.10 - 3.93 (m, 2H), 3.57 - 3.29 (m, 2H), 3.26 - 3.14 (m, 1H), 2.89 - 2.67 (m, 1H), 2.27 - 2.02 (m, 2H), 1.97 - 1.85 (m, 1H), 1.85 - 1.62 (m, 4H), 1.60 - 1.49 (m, 1H), 1.48 - 1.35 (m, 2H), 0.92 - 0.67 (m, 2H), 0.37 (br s, 2H) One H is hidden under water/solvent peak | 2.88, B |
| 159 | 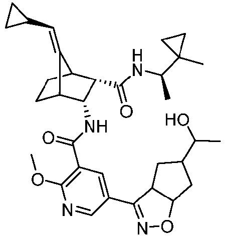 | N-[(1R,2R,3S,4R,7 Z)-7-(cyclopropylmet hylidene)-3-{[(1R)-1-(1-methylcycloprop yl)ethyl]carbamo yl}bicyclo[2.2.1] heptan-2-yl]-5-[5-(1-hydroxyethyl)-3aH,4H,5H,6H,6 aH-cyclopenta[d][1, 2]oxazol-3-yl]-2-methoxypyridine -3-carboxamide | 577.3 | 1H NMR (500 MHz, DMSO-d6) δ 10.08 (br d, J=7.0 Hz, 1H), 8.39 (br s, 1H), 8.34 (s, 1H), 7.85 - 7.72 (m, 1H), 7.09 - 6.74 (m, 2H), 5.11 - 4.91 (m, 2H), 4.47 (br d, J=10.1 Hz, 1H), 4.15 - 4.02 (m, 2H), 3.90 (s, 3H), 3.34 - 3.21 (m, 1H), 3.19 - 3.06 (m, 3H), 2.98 - 2.85 (m, 1H), 2.78 (br dd, J=13.0, 4.4 Hz, 1H), 1.99 - 1.86 (m, 1H), 1.73 - 1.64 (m, 1H), 1.61 - 1.54 (m, 2H), 1.50 - 1.41 (m, 2H), 1.33 - 1.25 (m, 1H), 1.22 - 1.14 (m, 2H), 0.84 (br dd, J=13.0, 6.6 Hz, 6H), 0.59 - 0.49 (m, 2H), 0.36 (br dd, J=9.9, 3.5 Hz, 1H), 0.23 - 0.10 (m, 3H), 0.07 - -0.10 (m, 3H) | 2.34 |
| 160 | 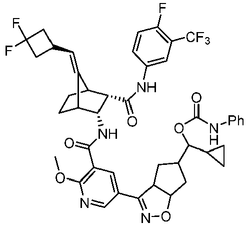 | cyclopropyl[3-(5-{[(2R,3S,7Z)-7-[(3,3-difluorocyclobut yl)methylidene]-3-{[4-fluoro-3-(trifluoromethyl) phenyl]carbamo yl}bicyclo[2.2.1] heptan-2-yl]carbamoyl}-6-methoxypyridin-3-yl)-3aH,4H,5H,6H,6 aH-cyclopenta[d][1, 2]oxazol-5-yl]methyl N-phenylcarbamate | 852.3 | 1H NMR (500 MHz, DMSO-d6) δ 10.57 (s, 1H), 10.02 (d, J=6.9 Hz, 1H), 9.53 - 9.39 (m, 1H), 8.60 (d, J=2.4 Hz, 1H), 8.56 (d, J=2.4 Hz, 1H), 8.21 (dd, J=6.5, 2.3 Hz, 1H), 7.78 (br dd, J=8.3, 3.3 Hz, 1H), 7.48 (t, J=9.8 Hz, 1H), 7.39 (br d, J=8.0 Hz, 2H), 7.28 - 7.19 (m, 2H), 6.95 (t, J=7.4 Hz, 1H), 5.39 (d, J=8.5 Hz, 1H), 5.19 (dd, J=8.7, 4.8 Hz, 1H), 4.38 - 4.33 (m, 1H), 4.32 - 4.26 (m, 1H), 4.14 - 4.07 (m, 4H), 3.52 - 3.43 (m, 1H), 3.20 - 3.11 (m, 1H), 2.99 (br s, 1H), 2.96 - 2.90 (m, 1H), 2.87 - 2.79 (m, 2H), 2.79 - 2.73 (m, 1H), 2.47 - 2.38 (m, 2H), 2.20 - 2.10 (m, 2H), 1.90 - 1.81 (m, 2H), 1.78 - 1.70 (m, 2H), 1.42 - 1.33 (m, 2H), 1.02 - 0.93 (m, 1H), 0.58 - 0.53 (m, 1H), 0.46 - 0.40 (m, 1H), 0.38 - 0.31 (m, 2H), | 2.81 |
| 161 | 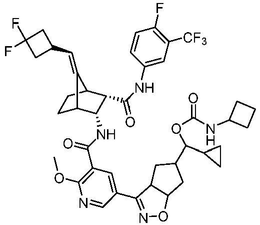 | cyclopropyl[3-(5-{[(2R,3S,7Z)-7-[(3,3-difluorocyclobut yl)methylidene]-3-{[4-fluoro-3-(trifluoromethyl) phenyl]carbamo yl}bicyclo[2.2.1] heptan-2-yl]carbamoyl }-6-methoxypyridin-3-yl)-3aH,4H,5H,6H,6 aH-cyclopenta[d][1, 2]oxazol-5-yl]methyl N-cyclobutylcarba mate | 830.1 | 1H NMR (500 MHz, DMSO-d6) δ 10.35 (s, 1H), 9.78 (d, J=6.9 Hz, 1H), 8.36 (d, J=2.4 Hz, 1H), 8.31 (d, J=2.4 Hz, 1H), 7.97 (dd, J=6.4, 2.5 Hz, 1H), 7.64 - 7.51 (m, 1H), 7.24 (t, J=9.8 Hz, 1H), 7.06 - 6.96 (m, 1H), 5.16 (d, J=8.5 Hz, 1H), 4.92 (dd, J=8.7, 4.9 Hz, 1H), 4.21 - 4.08 (m, 1H), 4.04 - 3.97 (m, 1H), 3.88 (s, 3H), 3.74 - 3.68 (m, 1H), 3.67 - 3.58 (m, 1H), 3.33 - 3.21 (m, 1H), 2.97 - 2.85 (m, 1H), 2.79 - 2.67 (m, 2H), 2.65 - 2.55 (m, 2H), 2.21 - 2.07 (m, 2H), 1.82 (br d, J=8.5 Hz, 4H), 1.65 - 1.43 (m, 7H), 1.32 - 1.25 (m, 2H), 1.20 - 1.11 (m, 2H), 0.71 - 0.57 (m, 1H), 0.29 - 0.22 (m, 1H), 0.17 - 0.10 (m, 1H), 0.06 - -0.07 (m, 2H) | 2.84 |
| 162 | 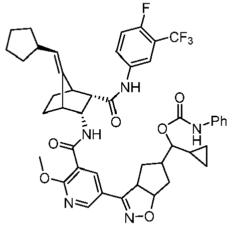 | [3-(5-{[(2R,3S,7Z)-7-(cyclopentylmet hylidene)-3-{[4-fluoro-3-(trifluoromethyl) phenyl]carbamo yl}bicyclo[2.2.1] heptan-2-yl]carbamoyl}-6-methoxypyridin-3-yl)-3aH,4H,5H,6H,6 aH-cyclopenta[d][1, 2]oxazol-5-yl](cyclopropyl) methyl N-phenylcarbamate | 830.3 | 1H NMR (500 MHz, DMSO-d6) δ 10.54 (s, 1H), 10.01 (d, J=6.8 Hz, 1H), 9.47 (s, 1H), 8.61 (d, J=2.4 Hz, 1H), 8.57 (d, J=2.4 Hz, 1H), 8.23 (dd, J=6.4, 2.5 Hz, 1H), 7.79 (br dd, J=8.4, 3.5 Hz, 1H), 7.57 - 7.45 (m, 1H), 7.40 (s, 1H), 7.32 - 7.18 (m, 2H), 6.96 (t, J=7.3 Hz, 1H), 5.24 - 5.10 (m, 2H), 4.50 - 4.33 (m, 1H), 4.33 - 4.23 (m, 1H), 3.50 - 3.36 (m, 1H), 3.23 - 3.10 (m, 1H), 3.00 (br s, 1H), 2.72 (br s, 1H), 2.62 - 2.55 (m, 5H), 2.21 - 2.11 (m, 2H), 1.91 - 1.70 (m, 7H), 1.69 - 1.60 (m, 2H), 1.58 - 1.49 (m, 1H), 1.48 - 1.35 (m, 2H), 1.28 - 1.19 (m, 2H), 1.02 - 0.89 (m, 1H), 0.61 - 0.52 (m, 1H), 0.48 - 0.41 (m, 1H), 0.36 (br s, 2H),One H is hidden under water/solvent peak | 3.11, B |
| 163 | 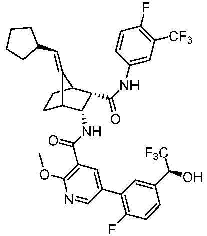 | N-[(1R,2R,3S,4R,7 Z)-7-(cyclopentylmet hylidene)-3-{[4-fluoro-3-(trifluoromethyl) phenyl]carbamo yl}bicyclo[2.2.1] heptan-2-yl]-5-{2-fluoro-5-[(1S)-2,2,2-trifluoro-1-hydroxyethyl]ph enyl}-2-methoxypyridine -3-carboxamide | 724.5 | 1H NMR (500 MHz, DMSO-d6) δ 10.56 (s, 1H), 10.03 (br d, J=6.9 Hz, 1H), 8.53 (br s, 1H), 8.46 (br s, 1H), 8.24 (br d, J=3.7 Hz, 1H), 7.84 - 7.76 (m, 1H), 7.69 (br d, J=6.6 Hz, 1H), 7.58 (br d, J=3.4 Hz, 1H), 7.53 - 7.34 (m, 2H), 5.28 (br d, J=6.3 Hz, 1H), 5.20 (br d, J=8.8 Hz, 1H), 4.39 (br s, 1H), 4.14 (s, 3H), 3.17 (br dd, J=10.3, 3.4 Hz, 1H), 3.02 (br s, 1H), 2.73 (br s, 1H), 2.64 - 2.58 (m, 1H), 1.92 - 1.74 (m, 4H), 1.70 - 1.50 (m, 4H), 1.40 (br d, J=1.5 Hz, 2H), 1.32 - 1.20 (m, 2H) proton missing due to water suppression | 2.93 |
| 164 | 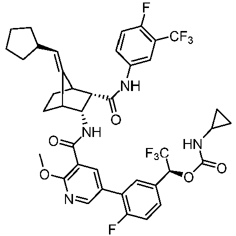 | (1S)-1-[3-(5-{[(1R,2R,3S,4R, 7Z)-7-(cyclopentylmet hylidene)-3-{[4-fluoro-3-(trifluoromethyl) phenyl]carbamo yl}bicyclo[2.2.1] heptan-2-yl]carbamoyl}-6-methoxypyridin-3-yl)-4-fluorophenyl]-2,2,2-trifluoroethyl N-cyclopropylcarba mate | 807.1 | 1H NMR (500 MHz, DMSO-d6) δ 10.56 (s, 1H), 10.03 (br d, J=6.6 Hz, 1H), 8.59 - 8.40 (m, 2H), 8.29 - 8.16 (m, 1H), 8.01 (br d, J=2.3 Hz, 1H), 7.85 - 7.71 (m, 2H), 7.67 - 7.56 (m, 1H), 7.54 - 7.39 (m, 2H), 6.38 (q, J=7.2 Hz, 1H), 5.19 (d, J=8.8 Hz, 1H), 4.46 - 4.34 (m, 1H), 4.14 (s, 3H), 3.49 (br d, J=2.0 Hz, 1H), 3.24 - 3.10 (m, 1H), 3.01 (br s, 1H), 2.73 (br s, 1H), 2.63 - 2.58 (m, 1H), 1.88 - 1.72 (m, 4H), 1.71 - 1.52 (m, 4H), 1.45 - 1.33 (m, 2H), 1.30 - 1.17 (m, 2H), 0.65 - 0.55 (m, 2H), 0.52 - 0.35 (m, 2H) | 3.14 |
| 165 | 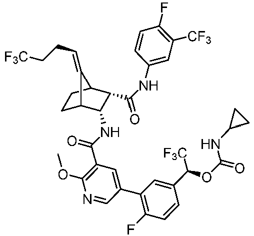 | (1S)-2,2,2-trifluoro-1-[4-fluoro-3-(5-{[(1R,2R,3S,4R, 7Z)-3-{[4-fluoro-3-(trifluoromethyl) phenyl]carbamo yl}-7-(4,4,4-trifluorobutylide ne)bicyclo[2.2.1] heptan-2-yl]carbamoyl}-6-methoxypyridin-3-yl)phenyl]ethyl N-cyclopropylcarba mate | 835.2 | 1H NMR (500 MHz, DMSO-d6) δ 10.36 (s, 1H), 9.83 (br d, J=6.7 Hz, 1H), 8.32 (s, 1H), 8.25 (s, 1H), 8.07 - 7.99 (m, 1H), 7.79 (br s, 1H), 7.64 - 7.51 (m, 2H), 7.38 (br d, J=2.4 Hz, 1H), 7.28 - 7.20 (m, 2H), 6.18 (q, J=5.9 Hz, 1H), 5.11 - 4.97 (m, 1H), 4.24 - 4.17 (m, 1H), 3.93 (s, 3H), 2.97 (br dd, J=11.1, 4.0 Hz, 1H), 2.80 (br s, 1H), 2.56 (br s, 1H), 2.23 - 2.04 (m, 5H), 1.66 - 1.53 (m, 2H), 1.26 - 1.15 (m, 2H), 0.37 (br s, 2H), 0.24 - 0.16 (m, 2H) | 2.89 |
| 166 | 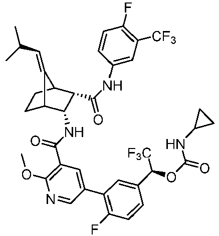 | (1S)-2,2,2-trifluoro-1-[4-fluoro-3-(5-{[(1R,2R,3S,4R, 7Z)-3-{[4-fluoro-3-(trifluoromethyl) phenyl]carbamo yl}-7-(2-methylpropylide ne)bicyclo[2.2.1] heptan-2-yl]carbamoyl}-6-methoxypyridin-3-yl)phenyl]ethyl N-cyclopropylcarba mate | 781.2 | 1H NMR (500 MHz, DMSO-d6) δ 10.32 (s, 1H), 9.78 (br d, J=6.9 Hz, 1H), 8.29 (s, 1H), 8.22 (d, J=1.4 Hz, 1H), 8.05 - 7.97 (m, 1H), 7.77 (br d, J=2.4 Hz, 1H), 7.62 - 7.47 (m, 2H), 7.44 - 7.30 (m, 1H), 7.29 - 7.15 (m, 2H), 6.26 - 6.08 (m, 1H), 4.87 (d, J=8.9 Hz, 1H), 4.22 - 4.10 (m, 1H), 3.90 (s, 3H), 3.19 - 3.10 (m, 1H), 2.93 (br dd, J=10.7, 4.3 Hz, 1H), 2.80 - 2.75 (m, 1H), 2.47 (br s, 1H), 2.24 - 2.18 (m, 1H), 1.66 - 1.44 (m, 2H), 1.25 - 1.08 (m, 2H), 0.86 - 0.68 (m, 6H), 0.41 - 0.29 (m, 2H), 0.26 - 0.11 (m, 2H) | 3.06 |
| 167 | 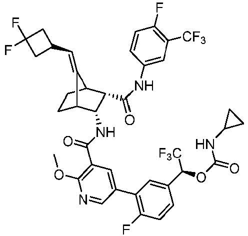 | (1S)-1-[3-(5-{[(1R,2R,3S,4R, 7Z)-7-[(3,3-difluorocyclobut yl)methylidene]-3-{[4-fluoro-3-(trifluoromethyl) phenyl]carbamo yl}bicyclo[2.2.1] heptan-2-yl]carbamoyl }-6-methoxypyridin-3-yl)-4-fluorophenyl]-2,2,2-trifluoroethyl N-cyclopropylcarba mate | 829.2 | 1H NMR (500 MHz, DMSO-d6) δ 10.36 (s, 1H), 9.83 (br d, J=6.7 Hz, 1H), 8.33 (s, 1H), 8.25 (d, J=1.0 Hz, 1H), 8.08 - 7.99 (m, 1H), 7.80 (br s, 1H), 7.63 - 7.53 (m, 2H), 7.39 (br s, 1H), 7.29 - 7.21 (m, 2H), 6.18 (br d, J=7.4 Hz, 1H), 5.19 (d, J=8.5 Hz, 1H), 4.24 - 4.15 (m, 1H), 3.93 (s, 3H), 3.20 - 3.12 (m, 1H), 2.97 (br dd, J=11.1, 4.0 Hz, 1H), 2.81 (br s, 1H), 2.77 - 2.59 (m, 2H), 2.57 (br s, 1H), 2.26 - 2.12 (m, 2H), 1.67 - 1.53 (m, 2H), 1.19 (br s, 2H), 0.83 (d, J=6.1 Hz, 1H), 0.37 (br d, J=1.9 Hz, 2H), 0.27 - 0.16 (m, 2H) | 2.86 |
| 168 | 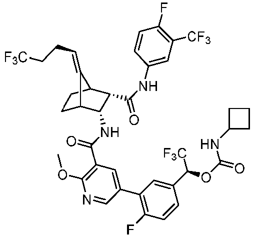 | (1S)-2,2,2-trifluoro-1-[4-fluoro-3-(5-{[(2R,3S,7Z)-3-{[4-fluoro-3-(trifluoromethyl) phenyl]carbamo yl}-7-(4,4,4-trifluorobutylide ne)bicyclo[2.2.1] heptan-2-yl]carbamoyl }-6-methoxypyridin-3-yl)phenyl]ethyl N-cyclobutylcarba mate | 849.2 | 1H NMR (500 MHz, DMSO-d6) δ 10.60 (s, 1H), 10.05 (br d, J=6.9 Hz, 1H), 8.55 (s, 1H), 8.48 (d, J=1.5 Hz, 1H), 8.25 (dd, J=6.3, 2.1 Hz, 1H), 8.15 (d, J=7.7 Hz, 1H), 7.88 - 7.76 (m, 2H), 7.61 (br s, 1H), 7.55 - 7.41 (m, 2H), 6.39 (q, J=7.2 Hz, 1H), 5.29 (t, J=6.9 Hz, 1H), 4.50 - 4.36 (m, 1H), 4.16 (s, 3H), 3.99 - 3.87 (m, 1H), 3.20 (dd, J=10.9, 4.3 Hz, 1H), 3.03 (br s, 1H), 2.78 (br s, 1H), 2.45 - 2.30 (m, 4H), 2.19 - 2.06 (m, 2H), 2.01 - 1.78 (m, 4H), 1.64 - 1.53 (m, 2H), 1.51 - 1.37 (m, 2H) | 2.97 |
| 169 | 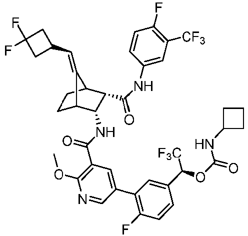 | (1S)-1-[3-(5-{[(2R,3S,7Z)-7-[(3,3-difluorocyclobut yl)methylidene]-3-{[4-fluoro-3-(trifluoromethyl) phenyl]carbamo yl}bicyclo[2.2.1] heptan-2-yl]carbamoyl}-6-methoxypyridin-3-yl)-4-fluorophenyl]-2,2,2-trifluoroethyl N-cyclobutylcarba mate | 843 | 1H NMR (500 MHz, DMSO-d6) δ 10.58 (s, 1H), 10.15 - 9.91 (m, 1H), 8.55 (s, 1H), 8.46 (d, J=1.3 Hz, 1H), 8.25 (dd, J=6.2, 2.0 Hz, 1H), 8.15 (d, J=7.8 Hz, 1H), 7.86 - 7.72 (m, 2H), 7.61 (br dd, J=5.9, 2.1 Hz, 1H), 7.52 - 7.44 (m, 2H), 7.22 (s, 1H), 7.12 (s, 1H), 7.02 (s, 1H), 6.38 (br d, J=7.1 Hz, 1H), 5.41 (d, J=8.6 Hz, 1H), 4.51 - 4.36 (m, 1H), 4.15 (s, 3H), 3.98 - 3.93 (m, 1H), 3.23 - 3.15 (m, 1H), 3.03 (br s, 1H), 2.91 - 2.85 (m, 1H), 2.81 - 2.75 (m, 1H), 2.45 - 2.37 (m, 1H), 2.18 - 2.10 (m, 1H), 1.97 - 1.80 (m, 4H), 1.66 - 1.52 (m, 2H), 1.48 - 1.37 (m, 2H) | 3.03 |
| 170 | 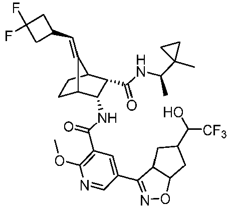 | N-[(1R,2R,3S,4R,7 Z)-7-[(3,3-difluorocyclobut yl)methylidene]-3-{[(1R)-1-(1-methylcycloprop yl)ethyl]carbamo yl}bicyclo[2.2.1] heptan-2-yl]-2-methoxy-5-[5-(2,2,2-trifluoro-1-hydroxyethyl)-3aH,4H,5H,6H,6 aH-cyclopenta[d][1, 2]oxazol-3-yl]pyridine-3-carboxamide | 681.1 | 1H NMR (500 MHz, DMSO-d6) Shift 10.25 (br d, J=6.4 Hz, 1H), 8.60 - 8.50 (m, 2H), 7.97 (br d, J=8.5 Hz, 1H), 6.27 (br d, J=6.7 Hz, 1H), 5.35 - 5.30 (m, 1H), 5.17 (br dd, J=8.4, 5.0 Hz, 1H), 4.29 (br t, J=8.9 Hz, 1H), 4.19 (br d, J=3.7 Hz, 1H), 4.10 - 4.03 (m, 3H), 3.98 - 3.89 (m, 1H), 2.86 - 2.73 (m, 2H), 2.42 - 2.31 (m, 2H), 2.03 - 1.89 (m, 3H), 1.87 - 1.77 (m, 3H), 1.73 - 1.67 (m, 1H), 1.38 - 1.25 (m, 4H), 1.05 - 0.92 (m, 7H), 0.56 - 0.50 (m, 1H), 0.35 - 0.29 (m, 1H), 0.22 - 0.14 (m, 2H) | 2.61 |
| 171 | 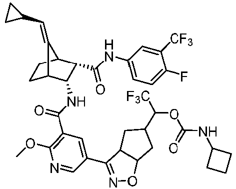 | 1-[3-(5-{[(2R,3S,7Z)-7-(cyclopropylmet hylidene)-3-{[4-fluoro-3-(trifluoromethyl) phenyl]carbamo yl}bicyclo[2.2.1] heptan-2-yl]carbamoyl}-6-methoxypyridin-3-yl)-3aH,4H,5H,6H,6 aH-cyclopenta[d][1, 2]oxazol-5-yl]-2,2,2-trifluoroethyl N-cyclobutylcarba mate | 808.2 | 1H NMR (500 MHz, DMSO-d6) d 10.54 (s, 1H), 10.03 (d, J=6.8 Hz, 1H), 8.62 (d, J=2.4 Hz, 1H), 8.57 (d, J=2.4 Hz, 1H), 8.22 (dd, J=6.4, 2.5 Hz, 1H), 7.86 (d, J=7.9 Hz, 1H), 7.81 - 7.77 (m, 1H), 7.48 (t, J=9.8 Hz, 1H), 5.29 - 5.22 (m, 1H), 5.19 (dd, J=8.7, 4.9 Hz, 1H), 4.70 (d, J=9.5 Hz, 1H), 4.42 (ddd, J=10.6, 6.8, 3.9 Hz, 1H), 4.34 (br t, J=9.1 Hz, 1H), 4.14 - 4.11 (m, 3H), 3.97 - 3.88 (m, 1H), 3.16 (dd, J=10.6, 4.0 Hz, 1H), 3.10 (br s, 1H), 2.76 - 2.71 (m, 1H), 2.17 - 2.04 (m, 4H), 1.91 - 1.76 (m, 7H), 1.59 - 1.52 (m, 2H), 1.51 - 1.47 (m, 1H), 1.41 (br d, J=2.5 Hz, 2H), 0.78 - 0.69 (m, 2H), 0.38 - 0.33 (m, 2H) | 2.97 |
| 172 | 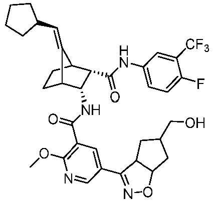 | N-[(1R,2R,3S,4R,7 Z)-7-(cyclopentylmet hylidene)-3-{[4-fluoro-3-(trifluoromethyl) phenyl]carbamo yl}bicyclo[2.2.1] heptan-2-yl]-5-[5-(hydroxymethyl) - 3aH,4H,5H,6H,6 aH-cyclopenta[d][1, 2]oxazol-3-yl]-2-methoxypyridine -3-carboxamide | 671.1 | 1H NMR (500 MHz, DMSO-d6) Shift 10.54 (s, 1H), 10.00 (d, J=7.0 Hz, 1H), 8.57 (dd, J=16.1, 2.4 Hz, 2H), 8.23 (dd, J=6.6, 2.6 Hz, 1H), 7.82 - 7.77 (m, 1H), 7.48 (t, J=9.7 Hz, 1H), 5.21 - 5.13 (m, 2H), 4.52 (t, J=5.2 Hz, 1H), 4.40 - 4.34 (m, 1H), 4.25 (br t, J=9.3 Hz, 1H), 4.12 (s, 3H), 3.16 (dd, J=10.4, 4.0 Hz, 1H), 3.00 (br d, J=3.3 Hz, 1H), 2.72 (br s, 1H), 2.61 - 2.56 (m, 1H), 2.00 (br dd, J=13.3, 5.7 Hz, 1H), 1.93 - 1.69 (m, 7H), 1.68 - 1.53 (m, 6H), 1.43 - 1.35 (m, 2H), 1.31 - 1.22 (m, 2H) | 2.67 |
| 173 | 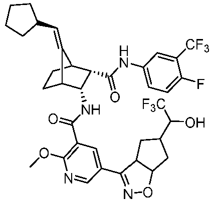 | N-[(1R,2R,3S,4R,7 Z)-7-(cyclopentylmet hylidene)-3-{[4-fluoro-3-(trifluoromethyl) phenyl]carbamo yl}bicyclo[2.2.1] heptan-2-yl]-2-methoxy-5-[5-(2,2,2-trifluoro-1-hydroxyethyl)-3aH,4H,5H,6H,6 aH-cyclopenta[d][1, 2]oxazol-3-yl]pyridine-3-carboxamide | 739.2 | 1H NMR (500 MHz, DMSO-d6) Shift 10.57 - 10.54 (m, 1H), 10.00 (br d, J=6.8 Hz, 1H), 8.62 - 8.56 (m, 2H), 8.23 (dd, J=6.4, 2.3 Hz, 1H), 7.81 - 7.77 (m, 1H), 7.48 (t, J=9.8 Hz, 1H), 5.21 - 5.16 (m, 2H), 4.40 - 4.34 (m, 1H), 4.32 - 4.27 (m, 1H), 4.12 (s, 3H), 4.00 - 3.91 (m, 1H), 3.16 (br dd, J=10.4, 4.3 Hz, 1H), 3.03 - 2.98 (m, 1H), 2.72 (br s, 1H), 2.64 - 2.56 (m, 1H), 2.04 - 1.90 (m, 3H), 1.86 - 1.69 (m, 7H), 1.68 - 1.54 (m, 4H), 1.42 - 1.35 (m, 2H), 1.31 - 1.22 (m, 2H) | 2.82 |
| 174 | 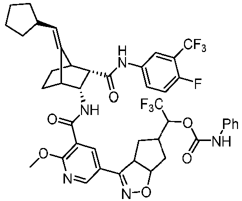 | 1-[3-(5-{[(1R,2R,3S,4R, 7Z)-7-(cyclopentylmet hylidene)-3-{[4-fluoro-3-(trifluoromethyl) phenyl]carbamo yl}bicyclo[2.2.1] heptan-2-yl]carbamoyl}-6-methoxypyridin-3-yl)-3aH,4H,5H,6H,6 aH-cyclopenta[d][1, 2]oxazol-5-yl]-2,2,2-trifluoroethyl N-phenylcarbamate | 858.2 | 1H NMR (500 MHz, DMSO-d6) δ 10.54 (s, 1H), 10.04 - 9.97 (m, 2H), 8.63 (d, J=2.4 Hz, 1H), 8.58 (d, J=2.4 Hz, 1H), 8.24 (dd, J=6.4, 2.4 Hz, 1H), 7.81 - 7.77 (m, 1H), 7.51 - 7.41 (m, 3H), 7.29 (t, J=7.9 Hz, 2H), 7.03 (t, J=7.3 Hz, 1H), 5.50 - 5.42 (m, 1H), 5.24 - 5.17 (m, 2H), 4.37 (br t, J=8.8 Hz, 2H), 4.12 (s, 3H), 3.18 - 3.13 (m, 1H), 3.03 - 2.97 (m, 1H), 2.75 - 2.71 (m, 1H), 2.63 - 2.56 (m, 1H), 2.28 - 2.19 (m, 1H), 2.16 - 2.07 (m, 1H), 2.03 - 1.72 (m, 7H), 1.69 - 1.61 (m, 2H), 1.60 - 1.52 (m, 2H), 1.44 - 1.33 (m, 2H), 1.32 - 1.23 (m, 2H) | 2.72 |
| 175 | 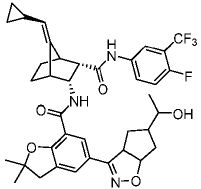 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-[5-(1-hydroxyethyl)-3aH,4H,5H,6H,6 aH-cyclopenta[d][1, 2]oxazol-3-yl]-2,2-dimethyl-2,3-dihydro-1-benzofuran-7-carboxamide | 696.4 | 1H NMR (400 MHz, DMSO-d6) δ = 10.46 (s, 1H), 9.59 (d, J = 7.5 Hz, 1H), 8.39 (dd, J = 2.3, 6.5 Hz, 1H), 7.90 (s, 1H), 7.69 (s, 1H), 7.66 - 7.58 (m,1H), 7.46 (t, J = 9.8 Hz, 1H), 5.09 (td, J = 6.5, 10.3 Hz, 1H), 4.69 (d, J = 9.5 Hz, 1H), 4.54 - 4.31 (m, 2H), 4.11 - 4.00 (m, 1H), 3.47 - 3.41 (m, 1H),3.19 - 3.02 (m, 4H), 2.71 (br s, 1H), 2.27 (td, J = 6.9, 13.3 Hz, 1H), 2.16 - 2.04 (m, 1H), 1.95 - 1.75 (m, 3H), 1.61 (s, 3H), 1.56 - 1.22 (m, 9H), 0.98(d, J = 6.1 Hz, 3H), 0.81 - 0.61 (m, 2H), 0.40 - 0.19 (m, 2H) | 2.59, A |
| 176 | 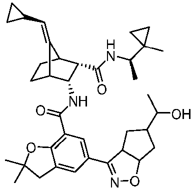 | N-[(1R,2R,3S,4R,7 Z)-7-(cyclopropylmet hylidene)-3-{[(1R)-1-(1-methylcycloprop yl)ethyl]carbamo yl}bicyclo[2.2.1] heptan-2-yl]-5-[5-(1-hydroxyethyl)-3aH,4H,5H,6H,6 aH-cyclopenta[d][1, 2]oxazol-3-yl]-2,2-dimethyl-2,3-dihydro-1-benzofuran-7-carboxamide | 616.5 | 1H NMR (400 MHz, DMSO-d6) δ = 9.81 (d, J = 7.1 Hz, 1H), 7.90 - 7.78 (m, 2H), 7.68 (d, J = 1.6 Hz, 1H), 5.09 (td, J = 6.7, 10.2 Hz, 1H), 4.63 (d, J= 9.6 Hz, 1H), 4.46 (d, J = 5.0 Hz, 1H), 4.36 - 4.21 (m, 1H), 4.12 - 3.98 (m, 1H), 3.46 - 3.41 (m, 2H), 3.15 - 3.05 (m, 2H), 3.03 - 2.98 (m, 1H), 2.93(dd, J = 4.3, 11.1 Hz, 1H), 2.30 - 2.23 (m, 1H), 2.18 - 2.08 (m, 1H), 2.00 - 1.84 (m, 2H), 1.81 - 1.73 (m, 1H), 1.55 (d, J = 18.9 Hz, 6H), 1.40 - 1.27(m, 3H), 1.02 - 0.98 (m, 6H), 0.97 (s, 3H), 0.78 - 0.65 (m, 2H), 0.61 - 0.55 (m, 1H), 0.40 - 0.26 (m, 3H), 0.22 - 0.12 (m, 2H) | 2.46, A |
| 177 | 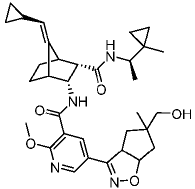 | N-[(1R,2R,3S,4R,7 Z)-7-(cyclopropylmet hylidene)-3-{[(1R)-1-(1-methylcycloprop yl)ethyl]carbamo yl}bicyclo[2.2.1] heptan-2-yl]-5-[5-(hydroxymethyl) -5-methyl-3aH,4H,5H,6H,6aH-cyclopenta[d][1, 2]oxazol-3-yl]-2-methoxypyridine -3-carboxamide | 577.3 | 1H NMR (400 MHz, DMSO-d6) d = 10.27 (d, J = 6.5 Hz, 1H), 8.54 (dd, J = 2.4, 14.4 Hz, 2H), 7.95 (d, J = 8.4 Hz, 1H), 5.20 (ddd, J = 3.2, 6.7, 9.8 Hz, 1H), 4.75 (t, J = 5.4 Hz, 1H), 4.65 (d, J = 9.5 Hz, 1H), 4.25 (dq, J = 5.4, 10.0 Hz, 2H), 4.09 (s, 3H), 3.52 - 3.45 (m, 1H), 3.16 (d, J = 5.4 Hz, 2H), 3.07 (br s, 1H), 2.96 (dd, J = 4.4, 10.8 Hz, 1H), 2.55 (br d, J = 3.4 Hz, 1H), 2.24 - 2.03 (m, 2H), 1.91 - 1.80 (m, 1H), 1.78 - 1.70 (m, 1H), 1.61 (dd, J = 2.9, 13.8 Hz, 1H), 1.47 (ddd, J = 4.3, 8.5, 12.9 Hz, 1H), 1.42 - 1.28 (m, 3H), 1.11 - 0.96 (m, 6H), 0.92 (s, 3H), 0.80 - 0.63 (m, 2H), 0.60 -0.48 (m, 1H), 0.41 - 0.28 (m, 3H), 0.25 - 0.05 (m, 2H) | 2.31, A |
| 178 | 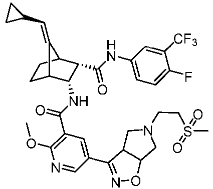 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-[5-(2-methanesulfonyl ethyl)-3aH,4H,5H,6H,6 aH-pyrrolo[3,4-d][1,2]oxazol-3-yl]-2-methoxypyridine -3-carboxamide | 674.2 | 1H NMR (400 MHz, DMSO-d6) d = 10.51 (s, 1H), 9.62 (d, J = 7.3 Hz, 1H), 8.12 (dd, J = 2.6, 6.4 Hz, 1H), 7.97 (s, 1H), 7.85 - 7.66 (m, 2H), 7.48 (t,J = 9.8 Hz, 1H), 5.20 (d, J = 8.9 Hz, 1H), 4.68 (td, J = 3.6, 7.3 Hz, 1H), 4.41 (ddd, J = 3.9, 6.9, 10.6 Hz, 1H), 4.08 (d, J = 2.8 Hz, 3H), 4.01 (dd, J =6.6, 11.6 Hz, 1H), 3.84 - 3.69 (m, 2H), 3.52 - 3.45 (m, 1H), 3.22 - 3.10 (m, 2H), 3.00 (br s, 1H), 2.73 (br s, 1H), 2.63 - 2.55 (m, 1H), 2.12 - 2.00 (m,1H), 1.97 - 1.89 (m, 1H), 1.88 - 1.73 (m, 4H), 1.69 - 1.50 (m, 4H), 1.48 - 1.33 (m, 2H), 1.32 - 1.15 (m, 2H) | 2.79, A |
| 179 | 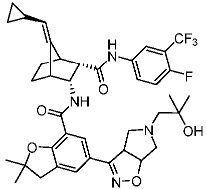 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-5-[5-(2-hydroxy-2-methylpropyl)-3aH,4H,5H,6H,6 aH-pyrrolo[3,4-d][1,2]oxazol-3-yl]-2,2-dimethyl-2,3-dihydro-1-benzofuran-7-carboxamide | 724.3 | 1H NMR (400 MHz, DMSO-d6) d = 10.46 (s, 1H), 9.56 (d, J = 7.6 Hz, 1H), 8.40 (dd, J = 2.4, 6.4 Hz, 1H), 7.89 (s, 1H), 7.69 (s, 1H), 7.65 - 7.56 (m,1H), 7.54 - 7.35 (m, 1H), 5.28 - 5.07 (m, 2H), 4.74 (t, J = 5.4 Hz, 1H), 4.51 - 4.30 (m, 1H), 4.13 (dt, J = 5.2, 9.8 Hz, 1H), 3.14 (br dd, J = 4.9, 11.4Hz, 5H), 2.96 (br s, 1H), 2.71 (br s, 1H), 2.62 - 2.56 (m, 1H), 2.19 - 1.99 (m, 2H), 1.95 - 1.72 (m, 4H), 1.70 - 1.53 (m, 8H), 1.50 (s, 3H), 1.43 - 1.16(m, 5H), 0.91 (s, 3H) | 2.85, A |
| 180 | 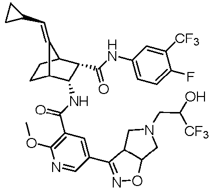 | N-[(1R,2R,3S,4R,7 Z)-7-(cyclopropylmet hylidene)-3-{[4-fluoro-3-(trifluoromethyl) phenyl]carbamo yl}bicyclo[2.2.1] heptan-2-yl]-2-methoxy-5-[5-(3,3,3-trifluoro-2-hydroxypropyl)-3aH,4H,5H,6H,6 aH-pyrrolo[3,4-d][1,2]oxazol-3-yl]pyridine-3-carboxamide | 725.3 | 1H NMR (400 MHz, DMSO-d6) d = 10.46 (s, 1H), 9.61 (d, J = 7.5 Hz, 1H), 8.40 (dd, J = 2.5, 6.6 Hz, 1H), 7.88 (d, J = 1.8 Hz, 1H), 7.68 (d, J = 1.6Hz, 1H), 7.66 - 7.55 (m, 1H), 7.47 (t, J = 9.8 Hz, 1H), 5.08 (dd, J = 4.3, 9.6 Hz, 1H), 4.69 (d, J = 9.6 Hz, 1H), 4.56 - 4.38 (m, 1H), 4.23 (t, J = 8.1Hz, 1H), 4.03 (s, 1H), 3.23 - 3.10 (m, 4H), 3.09 - 2.97 (m, 2H), 2.75 - 2.66 (m, 1H), 2.48 - 2.41 (m, 1H), 2.32 - 2.18 (m, 2H), 1.94 - 1.86 (m, 1H),1.85 - 1.76 (m, 1H), 1.61 (s, 3H), 1.56 - 1.34 (m, 6H), 1.02 (s, 3H), 0.96 (s, 3H), 0.81 - 0.66 (m, 2H), 0.35 (dd, J = 2.0, 4.5 Hz, 2H) | 2.61, A |
| 181 | 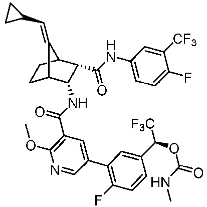 | (1S)-1-[3-(5-{[(2R,3S,7Z)-7-(cyclopropylmet hylidene)-3-{[4-fluoro-3-(trifluoromethyl) phenyl]carbamo yl}bicyclo[2.2.1] heptan-2-yl]carbamoyl }-6-methoxypyridin-3-yl)-4-fluorophenyl]-2,2,2-trifluoroethyl N-methylcarbamate | 775.1 (M+ Na)+ | 1H NMR (500 MHz, DMSO-d6) δ 10.56 (s, 1H), 10.07 (d, J=6.9 Hz, 1H), 8.60 - 8.45 (m, 2H), 8.24 (dd, J=6.4, 2.3 Hz, 1H), 7.84 - 7.75 (m, 2H), 7.74 - 7.68 (m, 1H), 7.64 - 7.58 (m, 1H), 7.53 - 7.44 (m, 2H), 6.43 - 6.37 (m, 1H), 4.71 (d, J=9.5 Hz, 1H), 4.52 - 4.42 (m, 1H), 4.15 (s, 3H), 3.21 - 3.17 (m, 1H), 3.13 (br d, J=3.1 Hz, 1H), 2.75 (br s, 1H), 2.61 (d, J=4.6 Hz, 3H), 1.91 - 1.76 (m, 2H), 1.57 - 1.35 (m, 3H), 0.82 - 0.67 (m, 2H), 0.41 - 0.31 (m, 2H) | 2.87, B |
| 182 | 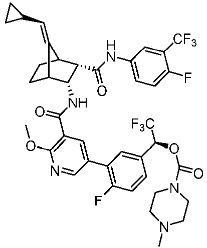 | (1S)-1-[3-(5-{[(2R,3S,7Z)-7-(cyclopropylmet hylidene)-3-{[4-fluoro-3-(trifluoromethyl) phenyl]carbamo yl}bicyclo[2.2.1] heptan-2-yl]carbamoyl }-6-methoxypyridin-3-yl)-4-fluorophenyl]-2,2,2-trifluoroethyl 4-methylpiperazine -1-carboxylate | 822.2 | 1H NMR (500 MHz, DMSO-d6) δ 10.31 (s, 1H), 9.79 (d, J=6.9 Hz, 1H), 8.31 - 8.17 (m, 2H), 7.98 (dd, J=6.5, 2.4 Hz, 1H), 7.59 - 7.51 (m, 2H), 7.43 - 7.35 (m, 1H), 7.29 - 7.15 (m, 2H), 6.19 (q, J=6.7 Hz, 1H), 4.45 (d, J=9.6 Hz, 1H), 4.25 - 4.15 (m, 1H), 3.90 (s, 3H), 2.93 (dd, J=10.5, 4.3 Hz, 1H), 2.87 (br s, 1H), 2.49 (br s, 1H), 2.30 (s, 4H), 2.10 - 1.98 (m, 4H), 1.92 (s, 3H), 1.64 - 1.50 (m, 2H), 1.32 - 1.11 (m, 3H), 0.49 (br t, J=9.3 Hz, 2H), 0.15 - 0.06 (m, 2H) | 2.93, B |
| 183 | 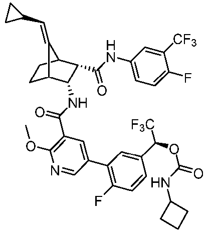 | (1S)-1-[3-(5-{[(2R,3S,7Z)-7-(cyclopropylmet hylidene)-3-{[4-fluoro-3-(trifluoromethyl) phenyl]carbamo yl}bicyclo[2.2.1] heptan-2-yl]carbamoyl }-6-methoxypyridin-3-yl)-4-fluorophenyl]-2,2,2-trifluoroethyl N-cyclobutylcarba mate | 815.1 (M+ Na)+ | 1H NMR (500 MHz, DMSO-d6) δ 10.55 (s, 1H), 10.07 (br d, J=7.0 Hz, 1H), 8.57 - 8.41 (m, 2H), 8.24 (dd, J=6.4, 2.3 Hz, 1H), 8.14 (d, J=7.9 Hz, 1H), 7.84 - 7.74 (m, 2H), 7.65 - 7.57 (m, 1H), 7.48 (td, J=9.6, 6.4 Hz, 2H), 6.43 - 6.34 (m, 1H), 4.71 (d, J=9.5 Hz, 1H), 4.48 - 4.42 (m, 1H), 4.15 (s, 3H), 3.98 - 3.89 (m, 1H), 3.18 (dd, J=10.7, 4.3 Hz, 1H), 3.13 (br s, 1H), 2.75 (br s, 1H), 2.21 - 2.04 (m, 2H), 1.97 - 1.77 (m, 4H), 1.65 - 1.37 (m, 5H), 0.81 - 0.66 (m, 2H), 0.40 - 0.30 (m, 2H) | 3.02, B |
| 184 | 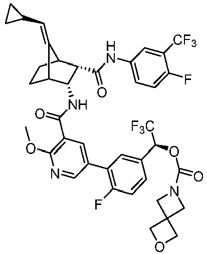 | (1S)-1-[3-(5-{[(2R,3S,7Z)-7-(cyclopropylmet hylidene)-3-{[4-fluoro-3-(trifluoromethyl) phenyl]carbamo yl}bicyclo[2.2.1] heptan-2-yl]carbamoyl }-6-methoxypyridin-3-yl)-4-fluorophenyl]-2,2,2-trifluoroethyl 2-oxa-6-azaspiro[3.3]hept ane-6-carboxylate | 843.1 (M+ Na)+ | 1H NMR (500 MHz, DMSO-d6) δ 10.55 (s, 1H), 10.06 (d, J=7.1 Hz, 1H), 8.58 - 8.45 (m, 2H), 8.24 (dd, J=6.3, 2.3 Hz, 1H), 7.86 - 7.73 (m, 2H), 7.66 - 7.58 (m, 1H), 7.53 - 7.43 (m, 2H), 6.43 - 6.34 (m, 1H), 4.75 - 4.59 (m, 5H), 4.50 - 4.41 (m, 1H), 4.36 - 4.24 (m, 2H), 4.15 (s, 3H), 4.11 (br d, J=11.4 Hz, 1H), 3.18 (dd, J=10.6, 4.5 Hz, 1H), 3.15 - 3.11 (m, 1H), 2.75 (br s, 1H), 1.89 - 1.77 (m, 2H), 1.55 - 1.39 (m, 3H), 0.81 - 0.67 (m, 2H), 0.36 (br d, J=1.8 Hz, 2H) | 2.88, B |
| 185 | 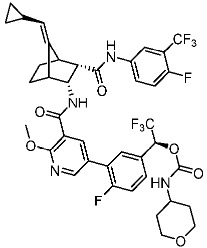 | (1S)-1-[3-(5-{[(2R,3S,7Z)-7-(cyclopropylmet hylidene)-3-{[4-fluoro-3-(trifluoromethyl) phenyl]carbamo yl}bicyclo[2.2.1] heptan-2-yl]carbamoyl }-6-methoxypyridin-3-yl)-4-fluorophenyl]-2,2,2-trifluoroethyl N-(oxan-4-yl)carbamate | 845.1 (M+ Na)+ | 1H NMR (500 MHz, DMSO-d6) δ 10.31 (s, 1H), 9.83 (br d, J=7.0 Hz, 1H), 8.36 - 8.18 (m, 2H), 8.00 (dd, J=6.4, 2.4 Hz, 1H), 7.68 (d, J=7.6 Hz, 1H), 7.61 - 7.52 (m, 2H), 7.42 - 7.35 (m, 1H), 7.29 - 7.19 (m, 2H), 6.17 (br d, J=6.8 Hz, 1H), 4.47 (d, J=9.5 Hz, 1H), 4.26 - 4.16 (m, 1H), 3.92 (s, 3H), 3.61 - 3.50 (m, 2H), 3.13 (br s, 1H), 3.05 (br d, J=2.3 Hz, 1H), 2.99 - 2.85 (m, 2H), 2.51 (br s, 1H), 1.64 - 1.46 (m, 3H), 1.42 (br d, J=11.7 Hz, 1H), 1.29 - 1.08 (m, 5H), 0.57 - 0.43 (m, 2H), 0.17 - 0.07 (m, 2H) | 2.79, B |
| 186 | 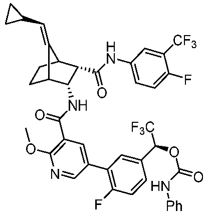 | (1S)-1-[3-(5-{[(2R,3S,7Z)-7-(cyclopropylmet hylidene)-3-{[4-fluoro-3-(trifluoromethyl) phenyl]carbamo yl}bicyclo[2.2.1] heptan-2-yl]carbamoyl }-6-methoxypyridin-3-yl)-4-fluorophenyl]-2,2,2-trifluoroethyl N-phenylcarbamate | 837.1 (M+ Na)+ | 1H NMR (500 MHz, DMSO-d6) δ 10.31 (s, 1H), 10.04 (br s, 1H), 9.83 (d, J=6.9 Hz, 1H), 8.35 - 8.19 (m, 2H), 8.00 (dd, J=6.4, 2.4 Hz, 1H), 7.67 - 7.53 (m, 2H), 7.45 (br dd, J=3.6, 2.9 Hz, 1H), 7.31 - 7.18 (m, 4H), 7.06 (t, J=7.9 Hz, 2H), 6.80 (t, J=7.3 Hz, 1H), 6.33 (br d, J=6.9 Hz, 1H), 4.47 (d, J=9.5 Hz, 1H), 4.25 - 4.16 (m, 1H), 3.91 (s, 3H), 2.96 - 2.86 (m, 2H), 2.50 (br d, J=3.2 Hz, 1H), 1.62 - 1.53 (m, 2H), 1.31 - 1.13 (m, 3H), 0.56 - 0.44 (m, 2H), 0.16 - 0.06 (m, 2H) | 3.02, B |
| 187 | 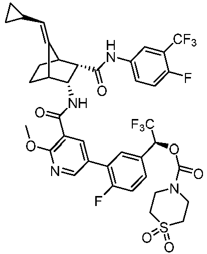 | (1S)-1-[3-(5-{[(2R,3S,7Z)-7-(cyclopropylmet hylidene)-3-{[4-fluoro-3-(trifluoromethyl) phenyl]carbamo yl}bicyclo[2.2.1] heptan-2-yl]carbamoyl }-6-methoxypyridin-3-yl)-4-fluorophenyl]-2,2,2-trifluoroethyl 1,1-dioxo-1λ⁶-thiomorpholine-4-carboxylate | 879.1 (M+ Na)+ | 1H NMR (500 MHz, DMSO-d6) δ 10.57 (s, 1H), 10.06 (d, J=7.0 Hz, 1H), 8.58 - 8.45 (m, 2H), 8.25 (dd, J=6.5, 2.6 Hz, 1H), 7.90 - 7.85 (m, 1H), 7.84 - 7.76 (m, 1H), 7.72 - 7.66 (m, 1H), 7.56 - 7.42 (m, 2H), 6.48 (br d, J=6.6 Hz, 1H), 4.71 (d, J=9.5 Hz, 1H), 4.50 - 4.42 (m, 1H), 4.16 (s, 3H), 4.05 (br s, 1H), 3.74 - 3.74 (m, 1H), 3.91 (s, 2H), 3.18 (d, J=4.8 Hz, 5H), 3.13 (br s, 1H), 2.79 - 2.72 (m, 1H), 1.87 - 1.76 (m, 2H), 1.56 - 1.37 (m, 3H), 0.82 - 0.67 (m, 2H), 0.41 - 0.34 (m, 2H) | 2.80, B |
| 188 | 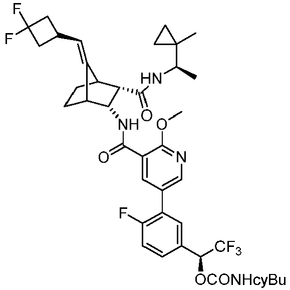 | (1S)-1-[3-(5-{[(1R,2R,3S,4R,7Z) -7-[(3,3-difluorocyclobutyl) methylidene]-3-{[(1R)-1-(1-methylcyclopropyl)e thyl]carbamoyl}bicy clo[2.2.1]heptan-2-yl]carbamoyl }-6-methoxypyridin-3-yl)-4-fluorophenyl]-2,2,2-trifluoroethyl N-cyclobutylcarbamate | 763.4 | (500 MHz, DMSO-d6) δ 10.12 (br d, J=6.6 Hz, 1H), 8.34 (s, 1H), 8.24 (d, J=1.1 Hz, 1H), 7.96 (br d, J=7.8 Hz, 1H), 7.81 (br d, J=8.5 Hz, 1H), 7.58 (br d, J=7.1 Hz, 1H), 7.42 (br d, J=2.7 Hz, 1H), 7.35 - 7.23 (m, 1H), 6.18 (q, J=6.9 Hz, 1H), 5.16 (d, J=8.3 Hz, 1H), 4.12 - 3.98 (m, 1H), 3.92 (s, 3H), 3.81 - 3.70 (m, 1H), 2.84 - 2.56 (m, 5H), 2.41 (br s, 1H), 2.29 - 2.12 (m, 2H), 2.06 - 1.84 (m, 2H), 1.80 - 1.62 (m, 3H), 1.60 - 1.50 (m, 1H), 1.46 - 1.29 (m, 2H), 1.22 - 1.06 (m, 2H), 0.85 (d, J=6.9 Hz, 3H), 0.80 (s, 3H), 0.42 - 0.30 (m, 1H), 0.21 - 0.10 (m, 1H), 0.07 - - 0.06 (m, 2H) | 2.89, C |

| | | | | | |
|---|---|---|---|---|---|
| (a) All LC retention times are based on Method C unless otherwise specified. (b) 1-3 protons were not accounted for in these samples due to overlap with solvent residue and/or artifacts of water suppression. | | | | | |

It will be evident to one skilled in the art that the present disclosure is not limited to the foregoing illustrative examples. It is therefore desired that the examples be considered in all respects as illustrative and not restrictive, reference being made to the appended claims, rather than to the foregoing examples.

## Claims

1. A compound of Formula (I): 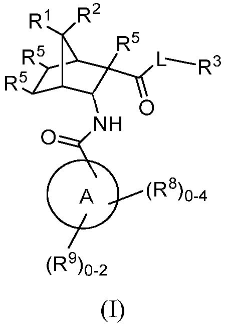 or a pharmaceutically acceptable salt thereof, wherein:
L is -O- or -NH-;
Ring A is 5- to 15-membered heterocyclyl comprising 1-5 heteroatoms selected from O, S(=O)ₚ, N, and NR¹⁶;
R¹ is C₁₋₃ alkyl substituted with 1 aryl or C₃₋₆ cycloalkyl substituent;
R² is H; or R¹ and R² are taken together to be =CR⁶R⁷, wherein "=" is a double bond;
or R¹ and R² together with the carbon atom to which they are both attached form a dioxolanyl substituted with 0-1 aryl substituent;
R³ is C₁₋₈ alkyl substituted with 0-5 R⁴, -(CR^{d}R^{d})ₙ-C₃₋₁₀-carbocyclyl substituted with 0-5 R⁴, or -(CR^{d}R^{d})ₙ-3- to 12-membered heterocyclyl comprising 1-4 heteroatoms selected from O, S(=O)ₚ, N, NR^{4a}, and substituted with 0-5 R⁴;
R⁴ is halo, CN, -OH, SF₅, S(=O)ₚR^{c}, C₁₋₄ alkyl substituted with 0-5 halo, -OH, or -OC₁₋₄ alkyl substituents, -OC₁₋₄ alkyl substituted with 0-5 halo substituents, -(CR^{d}R^{d})ₙ-C₃₋₁₀ carbocyclyl substituted with 0-5 R^{e}, or -(CR^{d}R^{d})ₙ4- to 9-membered heterocyclyl comprising 1-4 heteroatoms selected from O, S(=O)ₚ, N, and NR^{4a};
R^{4a} is H, C₁₋₄ alkyl, or -S(=O)₂CF₃;
R⁵ is H, halo, -OH, C₁₋₄ alkyl substituted with 0-5 halo substituents, or -OC₁₋₄ alkyl substituted with 0-5 halo substituents;
R⁶ is H, halo, CN, C₁₋₇ alkyl substituted with 0-3 R^{6a}, C₂₋₇ alkenyl substituted with 0-3 R^{6a}, C₂₋₇ alkynyl substituted with 0-3 R^{6a}, -C(=O)OR^{6b}, -CONR^{6b}R^{6b}, -(CH₂)ₙ-C₃₋₁₀ carbocyclyl substituted with 0-5 R¹⁴, or 3- to 12-membered heterocyclyl comprising 1-4 heteroatoms selected from O, S(=O)ₚ, N, or NR^{14a}, and substituted with 0-5 R¹⁴;
R^{6a} is halo, -OH, -OC₁₋₄ alkyl, C₁₋₄ alkyl, aryl, or C₃₋₆ cycloalkyl substituted with 0-4 halo substituents;
R^{6b} is H, C₁₋₄ alkyl substituted with 0-1 aryl, or C₃₋₆ cycloalkyl substituted with 0-4 halo substituents;
R⁷ is H or C₁₋₄ alkyl;
R⁸ is =O, C₁₋₄ alkyl, or -OC₁₋₆ alkyl substituted with 0-5 halo, -OH, -OC₁₋₄ alkyl, C₃₋₆ cycloalkyl, aryl, or 3- to 6-membered heterocyclyl substituents;
R⁹ is halo, CN, -C(=O)OR^{b}, -C(=O)NR¹⁷R¹⁷, C₁₋₈ alkyl substituted with 0-4 R¹⁰ and 0-2 R¹¹, C₂₋₈ alkenyl substituted with 0-2 R¹⁰ and 0-2 R¹¹, C₂₋₈ alkynyl substituted with 0-2 R¹⁰ and 0-2 R¹¹, -(A)₀₋₁-C₃₋₆ carbocyclyl substituted with 0-3 R¹⁰ and 0-2 R¹¹, -(A)₀₋₁-C₆₋₉ spirocycloalkyl substituted with 0-2 R¹⁰ and 0-2 R¹¹, or -(A)₀₋₁-3- to 12-membered heterocyclyl comprising 1-5 heteroatoms selected from O, S(=O)ₚ, N, and NR^{11a}, and substituted with 0-3 R¹⁰ and 0-2 R¹¹;
A is -O-, -S-, -CH₂O-, or -OCH₂-;
R¹⁰ is halo, CN, C₁₋₄ alkyl, =O, -OH, or -OC₁₋₄ alkyl;
R¹¹ is C₁₋₄ alkyl substituted with 0-5 R¹² and 0-2 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₉ carbocyclyl substituted with 0-5 R^{e}, aryl substituted with 0-5 R^{e}, 3- to 12-membered heterocyclyl comprising 1-4 heteroatoms selected from O, S(=O)ₚ, N and NR¹⁵, and substituted with 0-5 R^{e};
R^{11a} is H, C₁₋₄ alkyl substituted with 0-4 R^{11b}, -C(=O)R^{b}, -C(=O)OR^{b}, -C(=O)NR^{a}R^{a}, C₃₋₆ cycloalkyl substituted with 0-5 R^{e}, aryl substituted with 0-5 R^{e}, 4- to 6-membered heterocyclyl comprising 1-4 heteroatoms selected from O, S(=O)ₚ, N and NR¹⁵, and substituted with 0-5 R^{e};
R^{11b} is halo, -OH, -C(=O)OH, -C(=O)OC₁₋₄ alkyl, or aryl;
R¹² is halo, -C(=O)OR^{b}, -C(=O)NR^{a}R^{a}, -C(=O)NR^{a}OR^{b}, C₁₋₄ alkyl substituted with 0-3 halo or -OH substituents, or C₃₋₆ cycloalkyl;
R¹³ is -OR^{b}, -NR^{a}R^{a}, -NR^{a}C(=O)R^{b}, -NR^{a}C(=O)OR^{b}, -NR^{a}C(=O)NR^{a}R^{a}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, - S(=O)ₚR^{c}, -(CH₂)ₙ-C₃₋₁₀ carbocyclyl substituted with 0-3 R^{e}, or -(CH₂)ₙ-3- to 12-membered heterocyclyl comprising 1-4 heteroatoms selected from O, S(=O)ₚ, and N, and substituted with 0-3 R^{e};
R¹⁴ is halo, CN, C₁₋₄ alkyl substituted with 0-3 halo substituents, -OC₁₋₄ alkyl substituted with 0-3 halo substituents, -(CH₂)ₙ-NR^{a}R^{a}, -(CH₂)ₙ-aryl substituted with 0-3 R^{e}, -O-aryl substituted with 0-3 R^{e}, or -(CH₂)ₙ- 3- to 12-membered heterocyclyl comprising 1-4 heteroatoms selected from O, S(=O)ₚ, and N, and substituted with 0-3 R^{e};R^{14a} is H, -C(=O)C₁₋₄ alkyl, C₁₋₃ alkyl substituted with 0-3 -Si(C₁₋₃ alkyl)₃, or aryl substituted with 0-2 halo substituents;
R¹⁵ is H, C₁₋₄ alkyl, or aryl;
R¹⁶ is H, -C(=O)R^{b}, -C(=O)OR^{b}, -C(=O)NR^{a}R^{a}, -S(=O)ₚR^{c}, C₁₋₄ alkyl substituted with 0-4 R^{16a}, or aryl substituted with 0-4 R^{16a};
R^{16a} is halo, C₁₋₄ alkyl, OR^{b}, -C(=O)OR^{b}, or -S(=O)ₚR^{c};
R¹⁷ is H or C₁₋₄ alkyl substituted with 0-3 R¹⁰ and 0-2 R¹¹; or R¹⁷ and R¹⁷ together with the nitrogen atom to which they are both attached form a 3- to 12-membered heterocyclyl comprising 1-5 heteroatoms selected from O, S(=O)ₚ, N, and NR^{11a}, and substituted with 0-3 R¹⁰ and 0-2 R¹¹;
R^{a} is H, C₁₋₆ alkyl substituted with 0-5 R^{e}, C₂₋₆ alkenyl substituted with 0-5 R^{e}, C₂₋₆ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-5 heteroatoms selected from O, S(=O)ₚ, and N, and substituted with 0-5 R^{e}; or R^{a} and R^{a} together with the nitrogen atom to which they are both attached form a 3- to 12-membered heterocyclyl comprising 1-5 heteroatoms selected from O, S(=O)ₚ, and N, and substituted with 0-5 R^{e};
R^{b} is H, C₁₋₆ alkyl substituted with 0-5 R^{e}, C₂₋₆ alkenyl substituted with 0-5 R^{e}, C₂₋₆ alkynyl substituted with 0-5 R^{e}, -(CH₂)ₙ-C₃₋₁₀ carbocyclyl substituted with 0-5 R^{e}, or -(CH₂)ₙ- 3- to 12-membered heterocyclyl comprising 1-5 heteroatoms selected from O, S(=O)ₚ, and N, and substituted with 0-5 R^{e};
R^{c} is C₁₋₆ alkyl substituted with 0-5 R^{e}, C₂₋₆ alkenyl substituted with 0-5 R^{e}, C₂₋₆ alkynyl substituted with 0-5 R^{e}, C₃₋₆ carbocyclyl, or 3- to 12-membered heterocyclyl comprising 1-5 heteroatoms selected from O, S(=O)p, and N;
R^{d} is H,C₁₋₄ alkyl, or C₃₋₆ cycloalkyl;
R^{e} is halo, CN, NO₂, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₁₀ carbocyclyl substituted with 0-5 R^{g}, -(CH₂)ₙ- 3- to 12-membered heterocyclyl comprising 1-5 heteroatoms selected from O, S(=O)ₚ, and N, and substituted with 0-5 R^{g}, -(CH₂)ₙOR^{f}, - C(=O)OR^{f}, -C(=O)NR^{f}R^{f}, -NR^{f}C(=O)R^{f}, -S(=O)ₚR^{f}, -S(=O)ₚNR^{f}R^{f}, - NR^{f}S(=O)ₚR^{f}, -NR^{f}C(=O)OR^{f}, -OC(=O)NR^{f}R^{f}, or -(CH₂)ₙNR^{f}R^{f};
R^{f} is H, C₁₋₆ alkyl, C₃₋₆ cycloalkyl, aryl, or heterocyclyl; or R^{f} and R^{f} together with the nitrogen atom to which they are both attached form a heterocyclyl;
R^{g} is halo, CN, -OH, C₁₋₆ alkyl, C₃₋₆ cycloalkyl, or aryl;
n is zero, 1, 2, or 3; and
p is zero, 1, or 2.

2. The compound of claim 1, having Formula (II): 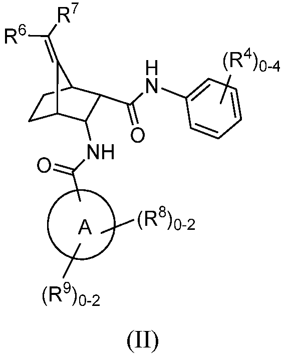 or a pharmaceutically acceptable salt thereof, wherein:
Ring A is 5- to 12-membered heterocyclyl comprising 1-4 heteroatoms selected from O, S(=O)ₚ, N, and NR¹⁶;
R⁴ is halo, C₁₋₄ alkyl substituted with 0-4 halo substituents, -OC₁₋₄ alkyl substituted with 0-4 halo substituents, or -S(=O)ₚC₁₋₄ alkyl substituted with 0-4 halo substituents;
R⁶ is halo, C₁₋₇ alkyl substituted with 0-3 R^{6a}, C₃₋₆ cycloalkyl substituted with 0-3 R¹⁴, C₃₋₆ cycloalkenyl substituted with 0-3 R¹⁴, C₆₋₁₀ aryl substituted with 0-3 R¹⁴, or 4- to 6-membered heterocyclyl comprising 1-3 heteroatoms selected from O, S(=O)ₚ, N, and NR^{14a} and substituted with 0-3 R¹⁴;
R^{6a} is halo, -OH, C₃₋₆ cycloalkyl, or aryl;
R⁷ is H or C₁₋₃ alkyl;
R⁸ is =O or -OC₁₋₄ alkyl substituted with 0-5 halo, -OH, -OC₁₋₄ alkyl, or aryl substituents;
R⁹ is halo, CN, -C(=O)OR^{b}, -C(=O)NR¹⁷R¹⁷, C₁₋₇ alkyl substituted with 0-3 R¹⁰ and 0-2 R¹¹, C₂₋₇ alkenyl substituted with 0-2 R¹⁰ and 0-2 R¹¹, C₂₋₇ alkynyl substituted with 0-2 R¹⁰ and 0-2 R¹¹, phenyl substituted with 0-3 R¹⁰ and 0-2 R¹¹, or 3- to 12-membered heterocyclyl comprising 1-5 heteroatoms selected from O, S(=O)ₚ, and substituted with 0-3 R¹⁰ and 0-2 R¹¹;
R¹⁰ is halo, CN, C₁₋₄ alkyl, -OH, or -OC₁₋₄ alkyl;
R¹¹ is C₁₋₃ alkyl substituted with 0-1 R¹² and 0-1 R¹³, -OR^{b}, -NR^{a}R^{a}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl substituted with 0-5 R^{e}, 4- to 6-membered heterocyclyl comprising 1-4 heteroatoms selected from O, S(=O)ₚ, N and NR¹⁵, and substituted with 0-5 R^{e};
R¹² is halo, -C(=O)OR^{b}, -C(=O)NHR^{a}, or C₁₋₄ alkyl substituted with 0-3 halo or OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, or -S(=O)ₚR^{c};
R¹⁴ is halo, CN, C₁₋₄ alkyl substituted with 0-3 halo substituents, -OC₁₋₄ alkyl substituted with 0-3 halo substituents;
R^{14a} is H, C(=O)C₁₋₄ alkyl, or C₁₋₃ alkyl substituted with 0-3 aryl substituted with 0-2 halo substituents;
R¹⁶ is H, C₁₋₃ alkyl substituted with 0-4 R^{16a}, or aryl substituted with 0-4 R^{16a};
R^{16a} is halo, C₁₋₃ alkyl, OR^{b}, C(=O)OR^{b}, or -S(=O)ₚR^{c};
R¹⁷ is H or C₁₋₃ alkyl substituted with 0-3 R¹⁰ and 0-2 R¹¹; or R¹⁷ and R¹⁷ together with the nitrogen atom to which they are both attached form a 3- to 10-membered heterocyclyl comprising 1-5 heteroatoms selected from O, S(=O)ₚ, N, and NR^{11a}, and substituted with 0-3 R¹⁰ and 0-2 R¹¹;
R^{a} is H, C₁₋₅ alkyl substituted with 0-5 R^{e}, C₂₋₅ alkenyl substituted with 0-5 R^{e}, C₂₋₅ alkynyl substituted with 0-5 R^{e}, -(CH₂)ₙ-C₃₋₁₀ carbocyclyl substituted with 0-5 R^{e}, or -(CH₂)ₙ-3- to 10-membered heterocyclyl comprising 1-5 heteroatoms selected from O, S(=O)ₚ, and N, and substituted with 0-5 R^{e}; or R^{a} and R^{a} together with the nitrogen atom to which they are both attached form a 3- to 10-membered heterocyclyl comprising 1-5 heteroatoms selected from O, S(=O)ₚ, and N, and substituted with 0-5 R^{e};
R^{b} is H, C₁₋₅ alkyl substituted with 0-5 R^{e}, C₂₋₅ alkenyl substituted with 0-5 R^{e}, C₂₋₅ alkynyl substituted with 0-5 R^{e}, -(CH₂)ₙ-C₃₋₁₀ carbocyclyl substituted with 0-5 R^{e}, or -(CH₂)ₙ-3- to 10-membered heterocyclyl comprising 1-5 heteroatoms selected from O, S(=O)ₚ, and N, and substituted with 0-5 R^{e};
R^{c} is C₁₋₅ alkyl substituted with 0-5 R^{e}, C₂₋₅ alkenyl substituted with 0-5 R^{e}, C₂₋₅ alkynyl substituted with 0-5 R^{e}, C₃₋₆ carbocyclyl, or 3- to 10-membered heterocyclylcomprising 1-5 heteroatoms selected from O, S(=O)p, and N;
R^{e} is halo, CN, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₆ carbocyclyl substituted with 0 5 R^{g}, -(CH₂)ₙ-4- to 6-membered heterocyclyl comprising 1-4 heteroatoms selected from O, S(=O)p, and N, and substituted with 0-5 R^{g}, -(CH₂)ₙOR^{f}, - C(=O)OR^{f}, -C(=O)NR^{f}R^{f}, -NR^{f}C(=O)R^{f}, -S(=O)ₚR^{f}, -NR^{f}C(=O)OR^{f}, - OC(=O)NR^{f}R^{f}, or -(CH₂)ₙNR^{f}R^{f};
R^{f} is H, C₁₋₅ alkyl, C₃₋₆ cycloalkyl, or aryl; or R^{f} and R^{f} together with the nitrogen atom to which they are both attached form a heterocyclyl;
R^{g} is halo, CN, -OH, C₁₋₆ alkyl, C₃₋₆ cycloalkyl, or aryl;
n is zero, 1, 2, or 3; and
p is zero, 1, or 2.

3. The compound of claim 2, having Formula (III): 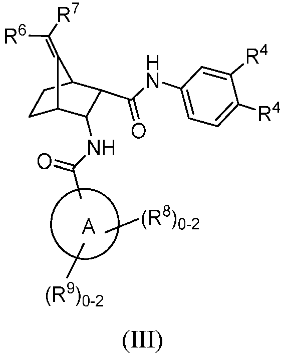 or a pharmaceutically acceptable salt thereof, wherein: 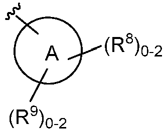 is 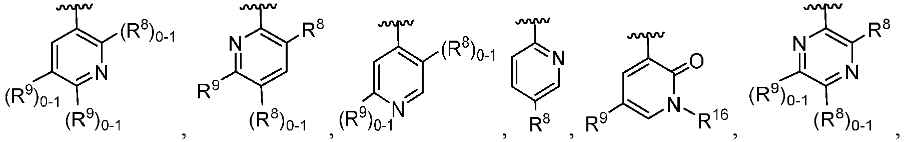 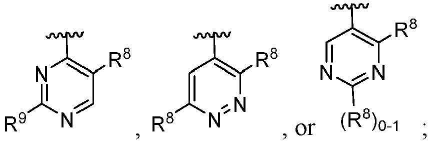
R⁴ is halo or C₁₋₃ alkyl substituted with 0-4 halo;
R⁶ is halo, C₁₋₄ alkyl substituted with 0-3 R^{6a}, C₃₋₆ cycloalkyl, phenyl substituted with 0-3 R¹⁴, naphthyl, or 5- to 6-membered heterocyclyl comprising 1-3 heteroatoms selected from O, S, and N;
R^{6a} is halo, -OH, or C₃₋₆ cycloalkyl;
R⁷ is H;
R⁸ is -OC₁₋₄ alkyl substituted with 0-5 halo or OH substituents;
R⁹ is halo, CN, -C(=O)OR^{b}, -C(=O)NR¹⁷R¹⁷, C₁₋₅ alkyl substituted with 0-3 R¹⁰ and 0-2 R¹¹, C₂₋₄ alkynyl substituted with 0-2 R¹⁰ and 0-2 R¹¹, phenyl substituted with 0-3 R¹⁰ and 0-2 R¹¹, or 3- to 9-membered heterocyclyl comprising 1-5 heteroatoms selected from O, S(=O)ₚ, and substituted with 0-3 R¹⁰ and 0-2 R¹¹;
R¹⁰ is halo, CN, C₁₋₄ alkyl, or -OH;
R¹¹ is -OR^{b}, -C(=O)OR^{b}, -C(=O)NR^{a}R^{a}, C₁₋₃ alkyl substituted with 0-1 R¹² and 0-1 R¹³, or C₃₋₆ cycloalkyl substituted with 0-5 R^{e};
R¹² is halo, -C(=O)OR^{b}, or C₁₋₃ alkyl substituted with 0-3 halo or OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -OC(=O)NR^{a}R^{a}, - OC(=O)NR^{a}OR^{b}, or -S(=O)ₚR^{c};
R¹⁶ is H or C₁₋₃ alkyl;
R¹⁷ is H or C₁₋₂ alkyl substituted with 0-3 R¹⁰ and 0-2 R¹¹, or R¹⁷ and R¹⁷ together with the nitrogen atom to which they are both attached form a 3- to 10-membered heterocycle comprising 1-5 heteroatoms selected from O, S(=O)ₚ, N, and NR^{11a}, and substituted with 0-3 R¹⁰ and 0-2 R¹¹;
R^{a} is H, C₁₋₆ alkyl substituted with 0-5 R^{e}, -(CH₂)ₙ-phenyl substituted with 0-5 R^{e}, or -(CH₂)ₙ-heterocyclyl substituted with 0-5 R^{e}; or R^{a} and R^{a} together with the nitrogen atom to which they are both attached form a heterocyclyl substituted with 0-5 R^{e};
R^{b} is H, C₁₋₆ alkyl substituted with 0-5 R^{e}, -(CH₂)₀₋₁-C₃₋₆ cycloalkyl substituted with 0-5 R^{e}, -(CH₂)₀₋₁-phenyl substituted with 0-5 R^{e}, or -(CH₂)ₙ-heterocyclyl substituted with 0-5 R^{e};
R^{e} is halo, CN, =O, C(=O)OH, C₁₋₆ alkyl, (CH₂)ₙOR^{f} or -S(=O)ₚR^{f};
R^{f} is H or C₁₋₅ alkyl; and
n is zero, 1, 2, or 3.

4. The compound of claim 3, or a pharmaceutically acceptable salt thereof, wherein:
R⁴ is halo or C₁₋₄ alkyl substituted with 0-4 F;
R⁶ is C₁₋₃ alkyl substituted with 0-3 R^{6a}, C₃₋₆ cycloalkyl, phenyl, or 5- to 6-membered heteroaryl comprising 1-3 heteroatoms selected from O, S, and N;
R^{6a} is halo;
R⁷ is H;
R⁸ is -OC₁₋₃ alkyl substituted with 0-4 halo or OH substituents;
R⁹ is 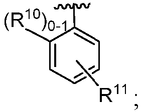
R¹⁰ is halo;
R¹¹ is -C(=O)OR^{b} or C₁₋₂ alkyl substituted with 0-1 R¹² and 0-1 R¹³;
R¹² is halo, -C(=O)OR^{b}, or C₁₋₂ alkyl substituted with 0-3 halo or OH substituents;
R¹³ is -OR^{b}, -NR^{a}R^{a}, -OC(=O)NR^{a}R^{a}, or -S(=O)ₚR^{c};
R^{a} is H, C₁₋₅ alkyl substituted with 0-4 R^{e}, -(CH₂)ₙ-phenyl substituted with 0-4 R^{e}, or -(CH₂)ₙ-heterocyclyl substituted with 0-4 R^{e}; or R^{a} and R^{a} together with the nitrogen atom to which they are both attached form a heterocyclyl substituted with 0-4 R^{e};
R^{b} is H, C₁₋₅ alkyl substituted with 0-4 R^{e}, -(CH₂)ₙ-phenyl substituted with 0-4 R^{e}, or -(CH₂)ₙ-heterocyclyl substituted with 0-4 R^{e};
R^{c} is C₁₋₅ alkyl substituted with 0-4 R^{e};
R^{e} is halo, CN, =O, C₁₋₅ alkyl; and
n is zero, 1, or 2.

5. The compound of claim 4, or a pharmaceutically acceptable salt thereof, wherein:
R⁴ is F or CF₃;
R⁶ is CF₃, cyclopropyl, phenyl, or 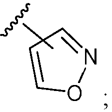
R⁸ is -OC₁₋₄alkyl substituted with 0-2 OH substituents;
R⁹ is 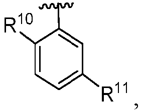
R¹⁰ is F;
R¹¹ is -C(=O)OR^{b} or 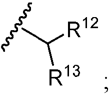
R¹² is halo, -C(=O)OR^{b}, or CF₃;
R¹³ is -OH or -OC(=O)NR^{a}R^{a};
R^{a} is H, C₁₋₄ alkyl, C₃₋₆ cycloalkyl, or phenyl; and
R^{b} is H or C₁₋₃ alkyl.

6. The compound of claim 2, or a pharmaceutically acceptable salt thereof, wherein: 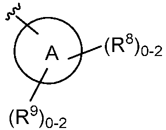 is 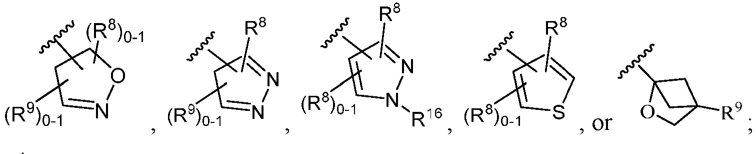
R⁴ is halo or C₁₋₄ alkyl substituted with 0-4 halo;
R⁶ is C₁₋₄ alkyl substituted with 0-3 R^{6a}, C₃₋₆ cycloalkyl, phenyl substituted with 0-3 R¹⁴, or 5- to 6-membered heterocyclyl comprising 1-3 heteroatoms selected from O, S, and N;
R^{6a} is halo;
R⁷ is H;
R⁸ is -OC₁₋₄ alkyl substituted with 0-5 halo or OH substituents;
R⁹ is halo, CN, C₁₋₄ alkyl substituted with 0-3 R¹⁰, or phenyl substituted with 0-3 R¹⁰ and 0-2 R¹¹;
R¹⁰ is halo, CN, or C₁₋₄ alkyl;
R¹¹ is -C(=O)OR^{b};
R¹⁶ is H, C₁₋₄ alkyl substituted with 0-4 R^{16a}, or phenyl substituted with 0-4 R^{16a};
R^{16a} is halo, -OR^{b}, -C(=O)OR^{b} or -S(=O)ₚR^{c};
R^{b} is H or C₁₋₄ alkyl; and
R^{c} is C₁₋₃ alkyl.

7. The compound of claim 2, or a pharmaceutically acceptable salt thereof, wherein: 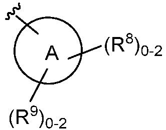 is 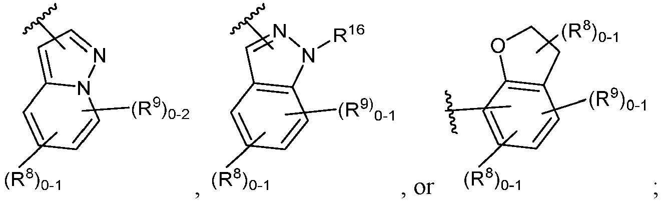
R⁴ is halo or C₁₋₄ alkyl substituted with 0-4 halo substituents;
R⁶ is C₁₋₄ alkyl substituted with 0-3 R^{6a}, C₃₋₆ cycloalkyl, phenyl substituted with 0-3 R¹⁴, or 5- to 6-membered heterocyclyl comprising 1-3 heteroatoms selected from O, S, and N;
R⁷ is H;
R⁸ is -OC₁₋₄ alkyl substituted with 0-5 halo or OH substituents;
R⁹ is halo, CN, or C₁₋₄ alkyl substituted with 0-3 R¹⁰;
R¹⁰ is halo;
R¹⁶ is H, C₁₋₄ alkyl substituted with 0-4 R^{16a}, or aryl substituted with 0-4 R^{16a}; and
R^{16a} is halo, C₁₋₄ alkyl, or C(=O)OR^{b}.

8. The compound of claim 2, or a pharmaceutically acceptable salt thereof, wherein: 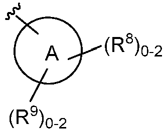 is 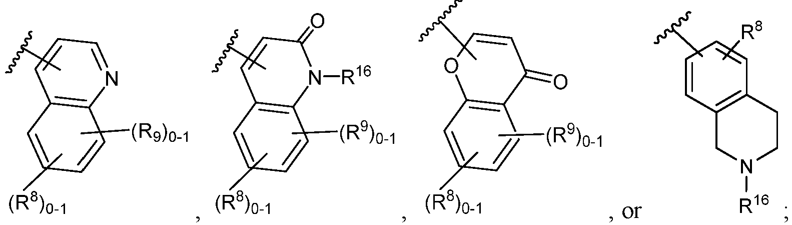
R⁴ is halo or C₁₋₄ alkyl substituted with 0-4 halo substituents;
R⁶ is C₁₋₄ alkyl substituted with 0-3 R^{6a}, C₃₋₆ cycloalkyl, phenyl substituted with 0-3 R¹⁴, or 5- to 6-membered heterocyclyl comprising 1-3 heteroatoms selected from O, S, and N;
R⁷ is H;
R⁸ is -OC₁₋₄ alkyl substituted with 0-5 halo or OH substituents;
R⁹ is halo or CN;
R¹⁶ is H, C₁₋₄ alkyl, -S(=O)ₚR^{c}, or C₁₋₄ alkyl substituted with 0-4 R^{16a}; and
R^{16a} is halo, C₁₋₄ alkyl, -OH, OC₁₋₃ alkyl, or -S(=O)ₚC₁₋₃ alkyl.

9. The compound of claim 2, or a pharmaceutically acceptable salt thereof, wherein: 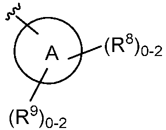 is 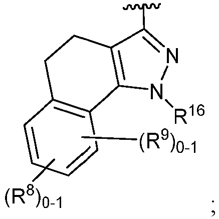
R⁴ is halo or C₁₋₄ alkyl substituted with 0-4 halo substituents;
R⁶ is C₁₋₄ alkyl substituted with 0-3 R^{6a}, C₃₋₆ cycloalkyl, phenyl substituted with 0-3 R¹⁴, or 5- to 6-membered heterocyclyl comprising 1-3 heteroatoms selected from O, S, and N;
R⁷ is H;
R⁸ is -OC₁₋₄ alkyl substituted with 0-5 halo or OH substituents;
R⁹ is halo or CN;
R¹⁶ is H or C₁₋₄ alkyl substituted with 0-4 R^{16a}; and
R^{16a} is halo.

10. The compound of claim 3, having Formula (IV): 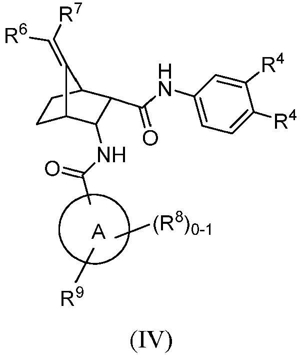 or a pharmaceutically acceptable salt thereof, wherein: 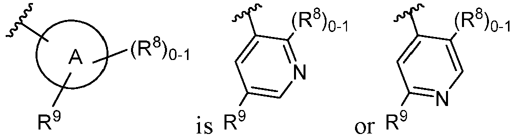
R⁴ is F or CF₃;
R⁶ is C₃₋₆ cycloalkyl;
R⁷ is H;
R⁸ is -OC₁₋₃ alkyl;
R⁹ is -C(=O)OR^{b}, -C(=O)NR¹⁷R¹⁷, C₁₋₃ alkyl substituted with 0-1 R¹¹, 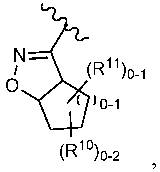
or 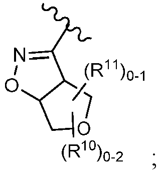
R¹⁰ is halo, CN, C₁₋₄ alkyl, or -OH;
R¹¹ is -OR^{b}, -C(=O)OR^{b}, -C(=O)NR^{a}R^{a}, C₁₋₃ alkyl substituted with 0-1 R¹³, or C₃₋₆ cycloalkyl substituted with 0-5 R^{e};
R¹³ is -OH;
R¹⁷ is H or C₁₋₂ alkyl substituted with 0-3 R¹⁰ and 0-2 R¹¹; or R¹⁷ and R¹⁷ together with the nitrogen atom to which they are both attached form 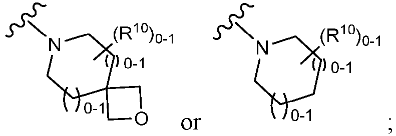
R^{a} is H or C₁₋₃ alkyl;
R^{b} is H, C₁₋₃ alkyl substituted with 0-5 R^{e}, or -(CH₂)₀₋₁-C₃₋₆ cycloalkyl substituted with 0-5 R^{e}; and
R^{e} is halo, CN, =O, C(=O)OH, C₁₋₆ alkyl, CH₂OH, or -S(=O)₂C₁₋₃ alkyl.

11. The compound of claim 1, having Formula (V): 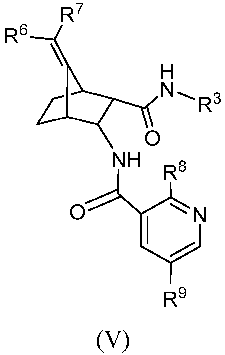 or a pharmaceutically acceptable salt thereof, wherein:
R³ is C₁₋₄ alkyl substituted with 0-3 R⁴, -(CHR^{d})ₙ-C₃₋₆-carbocyclyl substituted with 0-3 R⁴;
R⁴ is halo CN, or C₁₋₄ alkyl substituted with 0-5 halo substituents;
R⁶ is halo, C₁₋₄ alkyl substituted with 0-3 R^{6a}, C₃₋₆ cycloalkyl, or 5- to 6-membered heterocyclyl comprising 1-3 heteroatoms selected from O, S, and N, and substituted with 0-5 R¹⁴;
R^{6a} is halo;
R⁷ is H;
R⁸ is -OC₁₋₃ alkyl; 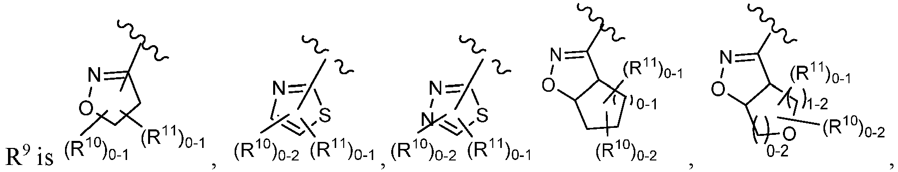 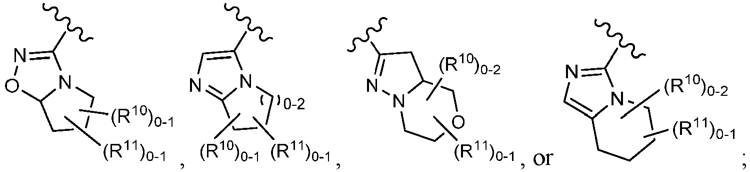
R¹⁰ is halo, CN, C₁₋₄ alkyl, or -OH;
R¹¹ is C₁₋₃ alkyl substituted with 0-3 R¹² and 0-2 R¹³, CN, or OR^{b};
R¹² is halo;
R¹³ is -OR^{b} or C₃₋₆ carbocyclyl;
R¹⁴ is halo, CN, or C₁₋₄ alkyl substituted with 0-3 halo;
R^{b} is H or C₁₋₃ alkyl substituted with 0-5 R^{e};
R^{d} is H or C₁₋₄ alkyl;
R^{e} is halo or OH; and
n is zero or 1.

12. The compound of claim 1, having Formula (VI): 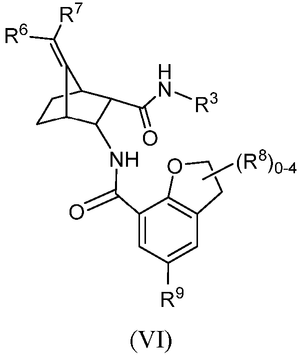 or a pharmaceutically acceptable salt thereof, wherein:
R³ is C₁₋₄ alkyl substituted with 0-3 R⁴, -(CHR^{d})ₙ-C₃₋₆-carbocyclyl substituted with 0-3 R⁴;
R⁴ is halo CN, or C₁₋₄ alkyl substituted with 0-5 halo substituents;
R⁶ is halo, C₁₋₄ alkyl substituted with 0-3 R^{6a}, C₃₋₆ cycloalkyl, or 5- to 6-membered heterocyclyl comprising 1-3 heteroatoms selected from O, S, and N, and substituted with 0-5 R¹⁴;
R^{6a} is halo or -OH;
R⁷ is H;
R⁸ is -OC₁₋₃ alkyl; 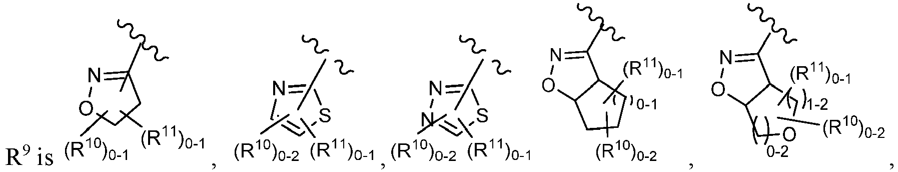 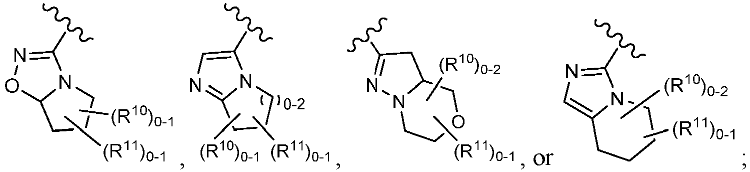
R¹⁰ is halo, CN, C₁₋₄ alkyl, or -OH;
R¹¹ is C₁₋₃ alkyl substituted with 0-3 R¹² and 0-2 R¹³, CN, or OR^{b};
R¹² is halo;
R¹³ is -OR^{b} or C₃₋₆ carbocyclyl;
R¹⁴ is halo, CN, or C₁₋₄ alkyl substituted with 0-3 halo substituents;
R^{b} is H or C₁₋₃ alkyl substituted with 0-5 R^{e};
R^{d} is H or C₁₋₄ alkyl;
R^{e} is halo or OH; and
n is zero or 1.

13. The compound of claim 1, having Formula (VII): 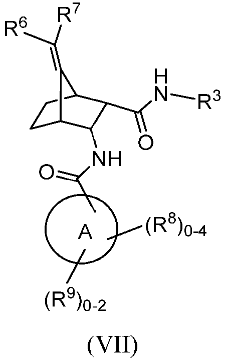 or a pharmaceutically acceptable salt thereof, wherein: 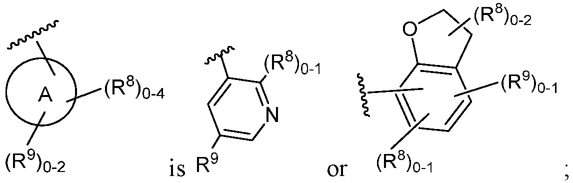
R³ is -(CHR^{d})ₙ-C₃₋₁₀-carbocyclyl substituted with 0-5 R⁴;
R⁴ is halo, CN, C₁₋₄ alkyl substituted with 0-5 halo or -OH, -OC₁₋₄ alkyl substituted with 0-5 halo substituents, or -S(=O)ₚR^{c};
R⁶ is C₁₋₄ alkyl substituted with 0-3 R^{6a} or C₃₋₆ cycloalkyl substituted with 0-5 R¹⁴;
R^{6a} is halo;
R⁷ is H;
R⁸ is C₁₋₃ alkyl or -OC₁₋₃ alkyl;
R⁹ is -C(=O)NR¹⁷R¹⁷, C₃₋₆ carbocyclyl substituted with 0-3 R¹⁰ and 0-2 R¹¹, or 5- to 12-membered heterocyclyl comprising 1-5 heteroatoms selected from O, S(=O)ₚ, N, and NR^{11a}, and substituted with 0-3 R¹⁰ and 0-2 R¹¹;
R¹⁰ is halo, CN, C₁₋₄ alkyl, =O, -OH, or -OC₁₋₄ alkyl;
R¹¹ is C₁₋₄ alkyl substituted with 0-5 R¹² and 0-2 R¹³, -S(=O)ₚR^{c}, C₃₋₆ cycloalkyl substituted with 0-5 R^{e};
R^{11a} is H, C₁₋₄ alkyl substituted with 0-4 R^{11b}, -C(=O)R^{b}, -C(=O)OR^{b}, -C(=O)NR^{a}R^{a}, C₃₋₆ cycloalkyl substituted with 0-5 R^{e}, aryl substituted with 0-5 R^{e}, 4- to 6-membered heterocyclyl comprising 1-4 heteroatoms selected from O, S(=O)ₚ, N and NR¹⁵, and substituted with 0-5 R^{e};
R^{11b} is halo, -OH, -C(=O)OH, -C(=O)OC₁₋₄ alkyl, or aryl;
R¹² is halo, -C(=O)OR^{b}, C₁₋₄ alkyl substituted with 0-3 halo or -OH, or C₃₋₆ cycloalkyl;
R¹³ is -OR^{b}, -NR^{a}R^{a}, -OC(=O)NR^{a}R^{a},;
R¹⁴ is halo;
R¹⁷ is H or C₁₋₄ alkyl substituted with 0-3 R¹⁰ and 0-2 R¹¹; or R¹⁷ and R¹⁷ together with the nitrogen atom to which they are both attached form a 3- to 9-membered heterocyclyl comprising 1-5 heteroatoms selected from O, S(=O)ₚ, N, and NR^{11a}, and substituted with 0-3 R¹⁰ and 0-2 R¹¹;
R^{a} is H, C₁₋₆ alkyl substituted with 0-5 R^{e}, C₃₋₁₀ carbocyclyl substituted with 0-5 R^{e}; or R^{a} and R^{a} together with the nitrogen atom to which they are both attached form a heterocyclyl substituted with 0-5 R^{e};
R^{b} is H or C₁₋₄ alkyl substituted with 0-5 R^{e};
R^{c} is C₁₋₄ alkyl;
R^{d} is H or C₁₋₃ alkyl;
R^{e} is halo, CN, NO₂, =O, C₁₋₆ alkyl substituted with 0-5 R^{g}, C₃₋₆ cycloalkyl, or -S(=O)ₚR^{f};
R^{f} is H, C₁₋₆ alkyl,
R^{g} is halo, CN, -OH, or C₁₋₆ alkyl;
n is zero or 1; and
p is zero, 1, or 2.

14. The compound of claim 13, or a pharmaceutically acceptable salt thereof, wherein:
R³ is -CHR^{d}-C₃₋₆ cycloalkyl substituted with 0-2 R⁴ or phenyl substituted with 0-2 R⁴;
R⁴ is F, CH₃, or CF₃;
R⁶ is C₁₋₄ alkyl substituted with 0-3 halo or C₃₋₆ cycloalkyl substituted with 0-3 halo substituents;
R⁷ is H;
R⁸ is -OCH₃;
R⁹ is -C(=O)NR¹⁷R¹⁷, 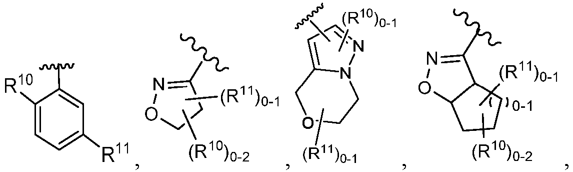 or 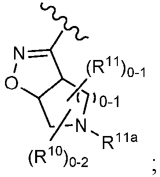
R¹⁰ is halo;
R¹¹ is C₁₋₄ alkyl substituted with 0-3 R¹² and 0-2 R¹³, -S(=O)ₚR^{c}, or C₃₋₆ cycloalkyl substituted with 0-3 R^{e};
R^{11a} is H or C₁₋₄ alkyl substituted with 0-3 R^{11b};
R^{11b} is -OH;
R¹² is C₁₋₄ alkyl substituted with 0-3 halo substituents;
R¹³ is -OC(=O)NR^{a}R^{a};
R¹⁴ is halo;
R¹⁷ is H or C₁₋₄ alkyl substituted with 0-2 R¹⁰ and 0-2 R¹¹; or R¹⁷ and R¹⁷ together with the nitrogen atom to which they are both attached form a 3- to 9-membered heterocyclyl comprising 1-4 heteroatoms selected from O, S(=O)ₚ, N, and NR^{11a}, and substituted with 0-2 R¹⁰ and 0-2 R¹¹;
R^{a} is H, C₁₋₄ alkyl substituted with 0-4 R^{e}, C₃₋₁₀ carbocyclyl substituted with 0- R^{e}; or R^{a} and R^{a} together with the nitrogen atom to which they are both attached form a heterocyclyl substituted with 0-4 R^{e};
R^{d} is H or C₁₋₃ alkyl;
R^{c} is C₁₋₃ alkyl;
R^{d} is H or C₁₋₃ alkyl;
R^{e} is C₁₋₄ alkyl, C₃₋₆ cycloalkyl, or -S(=O)ₚR^{f};
R^{f} is C₁₋₄ alkyl;
n is zero or 1; and
p is zero, 1, or 2.

15. A pharmaceutical composition comprising a compound of any of claims 1 to 14, or a pharmaceutically acceptable salt thereof, and a pharmaceutically acceptable carrier.

16. A compound of any of claims 1 to 14, or a pharmaceutical composition of claim 15, for use in therapy.

17. A compound of any of claims 1 to 14, or a pharmaceutical composition of claim 15, for use in treating a disease selected from the group consisting of angina pectoris, unstable angina, myocardial infarction, heart failure, acute coronary disease, acute heart failure, chronic heart failure, and cardiac iatrogenic damage, .

18. The compound or composition for use of claim 17, wherein the disease is heart failure.

19. A compound of any of claims 1 to 14, or a pharmaceutical composition of claim 15, for use in treating fibrosis.

## Patentansprüche

1. Verbindung der Formel (I): 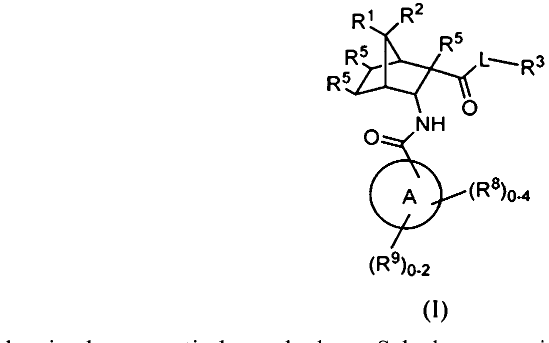 oder ein pharmazeutisch annehmbares Salz davon, worin:
L -O- oder -NH- ist;
Ring A 5- bis 15-gliedriges Heterocyclyl, umfassend 1-5 Heteroatome, ausgewählt aus O, S(=O)ₚ, N, und NR¹⁶, ist;
R¹ C₁₋₃-Alkyl, substituiert mit 1 Aryl- oder C₃₋₆-Cycloalkylsubstituenten, ist;
R² H ist; oder R¹ und R² zusammengenommen =CR⁶R⁷ sind, wobei "=" eine Doppelbindung ist;
oder R¹ und R² zusammen mit dem Kohlenstoffatom, an das sie beide gebunden sind, ein mit 0-1 Arylsubstituenten substituiertes Dioxolanyl bilden;
R³ C₁₋₈-Alkyl, substituiert mit 0-5 R⁴, -(CR^{d}R^{d})ₙ-C₃₋₁₀-Carbocyclyl, substituiert mit 0-5 R⁴, oder -(CR^{d}R^{d})ₙ-3- bis 12-gliedriges Heterocyclyl, umfassend 1-4 Heteroatome, ausgewählt aus O, S(=O)ₚ, N, NR^{4a} und substituiert mit 0-5 R⁴, ist;
R⁴ Halogen, CN, -OH, SF₅, S(=O)ₚR^{c}, C₁₋₄-Alkyl, substituiert mit 0-5 Halogen, -OH oder -OC₁₋₄-Alkylsubstituenten, -OC₁₋₄-Alkyl, substituiert mit 0-5 Halogensubstituenten, - (CR^{d}R^{d})ₙ-C₃₋₁₀-Carbocyclyl, substituiert mit 0-5 R^{e}, oder -(CR^{d}R^{d})ₙ4- bis 9-gliedriges Heterocyclyl, umfassend 1-4 Heteroatome, ausgewählt aus O, S(=O)ₚ, N, und NR^{4a}, ist;
R^{4a} H, C₁₋₄-Alkyl oder -S(=O)₂CF₃ ist;
R⁵ H, Halogen, -OH, C₁₋₄-Alkyl, substituiert mit 0-5 Halogensubstituenten, oder -OC₁₋₄-Alkyl, substituiert mit 0-5 Halogensubstituenten, ist;
R⁶ H, Halogen, CN, C₁₋₇-Alkyl, substituiert mit 0-3 R^{6a}, C₂₋₇-Alkenyl, substituiert mit 0-3 R^{6a}, C₂₋₇-Alkinyl, substituiert mit 0-3 R^{6a}, -C(=O)OR^{6b}, -CONR^{6b}R^{6b}, -(CH₂)ₙ-C₃₋₁₀-Carbocyclyl, substituiert mit 0-5 R¹⁴, oder 3- bis 12-gliedriges Heterocyclyl, umfassend 1-4 Heteroatome, ausgewählt aus O, S(=O)ₚ, N oder NR ^{14a}, und substituiert mit 0-5 R¹⁴, ist;
R^{6a} Halogen, -OH, -OC₁₋₄-Alkyl, C₁₋₄-Alkyl, Aryl oder C₃₋₆-Cycloalkyl, substituiert mit 0-4 Halogensubstituenten, ist;
R^{6b} H, C₁₋₄-Alkyl, substituiert mit 0-1 Aryl, oder C₃₋₆-Cycloalkyl, substituiert mit 0-4 Halogensubstituenten, ist;
R⁷ H oder C₁₋₄-Alkyl ist;
R⁸ =O, C₁₋₄-Alkyl oder -OC₁₋₆-Alkyl, substituiert mit 0-5 Halogen, -OH, -OC₁₋₄-Alkyl, C₃₋₆-Cycloalkyl, Aryl oder 3- bis 6-gliedrigen Heterocyclylsubstituenten, ist;
R⁹ Halogen, CN, -C(=O)OR^{b}, -C(=O)NR¹⁷R¹⁷, C₁₋₈-Alkyl, substituiert mit 0-4 R¹⁰ und 0-2 R¹¹, C₂₋₈-Alkenyl, substituiert mit 0-2 R¹⁰ und 0-2 R¹¹, C₂₋₈-Alkinyl, substituiert mit 0-2 R¹⁰ und 0-2 R¹¹, -(A)₀₋₁-C₃₋₆-Carbocyclyl, substituiert mit 0-3 R¹⁰ und 0-2 R¹¹, -(A)₀₋₁-C₆₋₉-Spirocycloalkyl, substituiert mit 0-2 R¹⁰ und 0-2 R¹¹, oder -(A)₀₋₁-3- bis 12-gliedriges Heterocyclyl, umfassend 1-5 Heteroatome, ausgewählt aus O, S(=O)ₚ, N und NR^{11a}, und substituiert mit 0-3 R¹⁰ und 0-2 R¹¹, ist;
A -O-, -S-, -CH₂O- oder -OCH₂- ist;
R¹⁰ Halogen, CN, C₁₋₄-Alkyl, =O, -OH oder -OC₁₋₄-Alkyl ist;
R¹¹ C₁₋₄-Alkyl, substituiert mit 0-5 R¹² und 0-2 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₉-Carbocyclyl, substituiert mit 0-5 R^{e}, Aryl, substituiert mit 0-5 R^{e}, 3- bis 12-gliedriges Heterocyclyl, umfassend 1-4 Heteroatome, ausgewählt aus O, S(=O)ₚ, N und NR¹⁵, und substituiert mit 0-5 R^{e}, ist;
R^{11a} H, C₁₋₄-Alkyl, substituiert mit 0-4 R^{11b}, -C(=O)R^{b}, -C(=O)OR^{b}, -C(=O)NR^{a}R^{a}, C₃₋₆-Cycloalkyl, substituiert mit 0-5 R^{e}, Aryl, substituiert mit 0-5 R^{e}, 4- bis 6-gliedriges Heterocyclyl, umfassend 1-4 Heteroatome, ausgewählt aus O, S(=O)ₚ, N und NR¹⁵, und substituiert mit 0-5 R^{e}, ist;
R^{11b} Halogen, -OH, -C(=O)OH, -C(=O)OC₁₋₄-Alkyl oder Aryl ist;
R¹² Halogen, -C(=O)OR^{b}, -C(=O)NR^{a}R^{a}, -C(=O)NR^{a}OR^{b}, C₁₋₄-Alkyl, substituiert mit 0-3 Halogen oder -OH-Substituenten, oder C₃₋₆-Cycloalkyl ist;
R¹³ -OR^{b}, - NR^{a}R^{a}, -NR^{a}C(=O)R^{b}, -NR^{a}C(=O)OR^{b}, -NR^{a}C(=O)NR^{a}R^{a}, -NR^{a}S(=O)ₚR^{c}, - NR^{a}S(=O)ₚNR^{a}R^{a}, -OC(=O)NR^{a}R^{a}, -OC(=O)NR^{a}OR^{b}, -S(=O)ₚNR^{a}R^{a}, -S(=O)ₚR^{c}, -(CH₂)ₙ-C₃₋₁₀-Carbocyclyl, substituiert mit 0-3 R^{e}, oder -(CH₂)ₙ-3- bis 12-gliedriges Heterocyclyl umfassend 1-4 Heteroatome ausgewählt aus O, S(=O)ₚ und N und substituiert mit 0-3 R^{e} ist;
R¹⁴ Halogen, CN, C₁₋₄-Alkyl, substituiert mit 0-3 Halogensubstituenten, -OC₁₋₄-Alkyl, substituiert mit 0-3 Halogensubstituenten, -(CH₂)ₙ-NR^{a}R^{a}, -(CH₂)ₙ-Aryl, substituiert mit 0-3 R^{e}, -O-Aryl, substituiert mit 0-3 R^{e}, oder -(CH₂)ₙ-3- bis 12-gliedriges Heterocyclyl, umfassend 1-4 Heteroatome, ausgewählt aus O, S(=O)ₚ und N, und substituiert mit 0-3 R^{e}, ist;
R^{14a} H, -C(=O)C₁₋₄-Alkyl, C₁₋₃-Alkyl, substituiert mit 0-3 -Si(C₁₋₃-Alkyl)₃ oder Aryl, substituiert mit 0-2 Halogensubstituenten, ist;
R¹⁵ H, C₁₋₄-Alkyl, oder Aryl ist;
R¹⁶ H, -C(=O)R^{b}, -C(=O)OR^{b}, -C(=O)NR^{a}R^{a}, -S(=O)ₚR^{c}, C₁₋₄-Alkyl, substituiert mit 0-4 R^{16a} oder Aryl, substituiert mit 0-4 R^{16a}, ist;
R^{16a} Halogen, C₁₋₄-Alkyl, OR^{b}, -C(=O)OR^{b} oder -S(=O)ₚR^{c}, ist;
R¹⁷ H oder C₁₋₄-Alkyl, substituiert mit 0-3 R¹⁰ und 0-2 R¹¹ ist oder R¹⁷ und R¹⁷ zusammen mit dem Stickstoffatom, an das sie beide gebunden sind, ein 3- bis 12-gliedriges Heterocyclyl, umfassend 1-5 Heteroatome, ausgewählt aus O, S(=O)ₚ, N, und NR^{11a}, und substituiert mit 0-3 R¹⁰ und 0-2 R¹¹, bilden;
R^{a} H, C₁₋₆-Alkyl, substituiert mit 0-5 R^{e}, C₂₋₆-Alkenyl, substituiert mit 0-5 R^{e}, C₂₋₆-Alkinyl, substituiert mit 0-5 R^{e}, -(CH₂)ₙ-C₃₋₁₀-Carbocyclyl, substituiert mit 0-5 R^{e}, oder -(CH₂)ₙ-3-bis 12-gliedriges Heterocyclyl, umfassend 1-5 Heteroatome, ausgewählt aus O, S(=O)ₚ und N, und substituiert mit 0-5 R^{e}, ist oder R^{a} und R^{a} zusammen mit dem Stickstoffatom, an das sie beide gebunden sind, ein 3- bis 12-gliedriges Heterocyclyl, umfassend 1-5 Heteroatome, ausgewählt aus O, S(=O)ₚ und N, und substituiert mit 0-5 R^{e}, bilden;
R^{b} H, C₁₋₆-Alkyl, substituiert mit 0-5 R^{e}, C₂₋₆-Alkenyl, substituiert mit 0-5 R^{e}, C₂₋₆-Alkinyl, substituiert mit 0-5 R^{e}, -(CH₂)ₙ-C₃₋₁₀-Carbocyclyl, substituiert mit 0-5 R^{e}, oder-(CH₂)ₙ- 3-bis 12-gliedriges Heterocyclyl, umfassend 1-5 Heteroatome, ausgewählt aus O, S(=O)ₚ und N, und substituiert mit 0-5 R^{e}, ist;
R^{c} C₁₋₆-Alkyl, substituiert mit 0-5 R^{e}, C₂₋₆-Alkenyl, substituiert mit 0-5 R^{e}, C₂₋₆-Alkinyl, substituiert mit 0-5 R^{e}, C₃₋₆-Carbocyclyl oder 3- bis 12-gliedriges Heterocyclyl, umfassend 1-5 Heteroatome, ausgewählt aus O, S(=O)p, und N, ist;
R^{d} H, C₁₋₄-Alkyl oder C₃₋₆-Cycloalkyl ist;
R^{e} Halogen, CN, NO₂, =O, C₁₋₆-Alkyl, substituiert mit 0-5 R^{g}, C₂₋₆ Alkenyl, substituiert mit 0-5 R^{g}, C₂₋₆-Alkinyl, substituiert mit 0-5 R^{g}, -(CH₂)ₙ-C₃₋₁₀-Carbocyclyl, substituiert mit 0-5 R^{g}, -(CH₂)ₙ- 3- bis 12-gliedriges Heterocyclyl, umfassend 1-5 Heteroatome, ausgewählt aus O, S(=O)ₚ und N, und substituiert mit 0-5 R^{g}, -(CH₂)ₙOR^{f}, -C(=O)OR^{f}, -C(=O)NR^{f}R^{f}, -NR^{f}C(=O)R^{f}, -S(=O)ₚR^{f}, -S(=O)ₚNR^{f}R^{f}, -NR^{f}S(=O)ₚR^{f}, -NR^{f}C(=O)OR^{f}, -OC(=O)NR^{f}R^{f}, oder -(CH₂)ₙNR^{f}R^{f}, ist;
R^{f} H, C₁₋₆-Alkyl, C₃₋₆-Cycloalkyl, Aryl oder Heterocyclyl ist oder R^{f} und R^{f} zusammen mit dem Stickstoffatom, an das sie beide gebunden sind, ein Heterocyclyl bilden;
R^{g} Halogen, CN, -OH, C₁₋₆-Alkyl, C₃₋₆-Cycloalkyl oder Aryl ist;
n null, 1, 2 oder 3 ist und
p null, 1 oder 2 ist.

2. Verbindung nach Anspruch 1 mit der Formel (II): 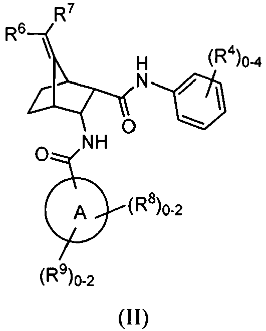 oder ein pharmazeutisch annehmbares Salz davon, worin:
Ring A 5- bis 12-gliedriges Heterocyclyl, umfassend 1-4 Heteroatome, ausgewählt aus O, S(=O)ₚ, N und NR¹⁶, ist;
R⁴ Halogen, C₁₋₄-Alkyl, substituiert mit 0-4 Halogensubstituenten, -OC₁₋₄-Alkyl, substituiert mit 0-4 Halogensubstituenten, oder -S(=O)ₚC₁₋₄-Alkyl, substituiert mit 0-4 Halogensubstituenten, ist;
R⁶ Halogen, C₁₋₇-Alkyl, substituiert mit 0-3 R^{6a}, C₃₋₆-Cycloalkyl, substituiert mit 0-3 R¹⁴, C₃₋₆-Cycloalkenyl, substituiert mit 0-3 R¹⁴, C₆₋₁₀-Aryl, substituiert mit 0-3 R¹⁴, oder 4- bis 6-gliedriges Heterocyclyl, umfassend 1-3 Heteroatome, ausgewählt aus O, S(=O)ₚ, N und NR^{14a} und substituiert mit 0-3 R¹⁴ ist;
R^{6a} Halogen, -OH, C₃₋₆-Cycloalkyl oder Aryl ist;
R⁷ H oder C₁₋₃-Alkyl ist;
R⁸ =O oder -OC₁₋₄-Alkyl, substituiert mit 0-5 Halogen, -OH, -OC₁₋₄-Alkyl oder Arylsubstituenten, ist;
R⁹ Halogen, CN, -C(=O)OR^{b}, -C(=O)NR¹⁷R¹⁷, C₁₋₇-Alkyl, substituiert mit 0-3 R¹⁰ und 0-2 R¹¹, C₂₋₇-Alkenyl, substituiert mit 0-2 R¹⁰ und 0-2 R¹¹, C₂₋₇ Alkinyl, substituiert mit 0-2 R¹⁰ und 0-2 R¹¹, Phenyl, substituiert mit 0-3 R¹⁰ und 0-2 R¹¹, oder 3- bis 12-gliedriges Heterocyclyl, umfassend 1-5 Heteroatome, ausgewählt aus O, S(=O)ₚ, und substituiert mit 0-3 R¹⁰ und 0-2 R¹¹, ist;
R¹⁰ Halogen, CN, C₁₋₄-Alkyl, -OH oder -OC₁₋₄-Alkyl ist;
R¹¹ C₁₋₃-Alkyl, substituiert mit 0-1 R¹² und 0-1 R¹³, -OR^{b}, -NR^{a}R^{a}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆-Cycloalkyl, substituiert mit 0-5 R^{e}, 4- bis 6-gliedriges Heterocyclyl, umfassend 1-4 Heteroatome, ausgewählt aus O, S(=O)ₚ, N und NR¹⁵, und substituiert mit 0-5 R^{e}, ist;
R¹² Halogen, -C(=O)OR^{b}, -C(=O)NHR^{a} oder C₁₋₄-Alkyl, substituiert mit 0-3 Halogen oder OH-Substituenten, ist;
R¹³ --OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, - OC(=O)NR^{a}R^{a}, -OC(=O)NR^{a}OR^{b}, -S(=O)ₚNR^{a}R^{a} oder -S(=O)ₚR^{c} ist;
R¹⁴ Halogen, CN, C₁₋₄-Alkyl, substituiert mit 0-3 Halogensubstituenten, -OC₁₋₄-Alkyl, substituiert mit 0-3 Halogensubstituenten, ist;
R^{14a} H, C(=O)C₁₋₄-Alkyl oder C₁₋₃-Alkyl, substituiert mit 0-3 Aryl, substituiert mit 0-2 Halogensubstituenten, ist;
R¹⁶ H, C₁₋₃-Alkyl, substituiert mit 0-4 R^{16a}, oder Aryl, substituiert mit 0-4 R^{16a}, ist;
R^{16a} Halogen, C₁₋₃-Alkyl, OR^{b}, C(=O)OR^{b} oder -S(=O)ₚR^{c} ist;
R¹⁷ H oder C₁₋₃-Alkyl, substituiert mit 0-3 R¹⁰ und 0-2 R¹¹ ist oder R¹⁷ und R¹⁷ zusammen mit dem Stickstoffatom, an das sie beide gebunden sind, ein 3- bis 10-gliedriges Heterocyclyl, umfassend 1-5 Heteroatome, ausgewählt aus O, S(=O)ₚ, N, und NR^{11a}, und substituiert mit 0-3 R¹⁰ und 0-2 R¹¹, bilden;
R^{a} H, C₁₋₅-Alkyl, substituiert mit 0-5 R^{e}, C₂₋₃-Alkenyl, substituiert mit 0-5 R^{e}, C₂₋₅-Alkinyl, substituiert mit 0-5 R^{e}, -(CH₂)ₙ-C₃₋₁₀-Carbocyclyl, substituiert mit 0-5 R^{e}, oder -(CH₂)ₙ-3-bis 10-gliedriges Heterocyclyl, umfassend 1-5 Heteroatome, ausgewählt aus O, S(=O)ₚ und N, und substituiert mit 0-5 R^{e} ist oder R^{a} und R^{a} zusammen mit dem Stickstoffatom, an das sie beide gebunden sind, ein 3- bis 10-gliedriges Heterocyclyl, umfassend 1-5 Heteroatome, ausgewählt aus O, S(=O)ₚ und N, und substituiert mit 0-5 R^{e}, bilden;
R^{b} H, C₁₋₅-Alkyl, substituiert mit 0-5 R^{e}, C₂₋₃-Alkenyl, substituiert mit 0-5 R^{e}, C₂₋₅-Alkinyl, substituiert mit 0-5 R^{e}, -(CH₂)ₙ-C₃₋₁₀-Carbocyclyl, substituiert mit 0-5 R^{e}, oder -(CH₂)ₙ-3-bis 10-gliedriges Heterocyclyl, umfassend 1-5 Heteroatome, ausgewählt aus O, S(=O)ₚ und N, und substituiert mit 0-5 R^{e}, ist;
R^{c} C₁₋₅-Alkyl, substituiert mit 0-5 R^{e}, C₂₋₃-Alkenyl, substituiert mit 0-5 R^{e}, C₂₋₅-Alkinyl, substituiert mit 0-5 R^{e}, C₃₋₆-Carbocyclyl oder 3- bis 10-gliedriges Heterocyclyl, umfassend 1-5 Heteroatome, ausgewählt aus O, S(=O)p und N, ist;
R^{e} Halogen, CN, =O, C₁₋₆-Alkyl, substituiert mit 0-5 R^{g}, C₂₋₆-Alkenyl, substituiert mit 0-5 R^{g}, C₂₋₆-Alkinyl, substituiert mit 0-5 R^{g}, -(CH₂)ₙ-C₃₋₆-Carbocyclyl, substituiert mit 0 5 R^{g}, -(CH₂)ₙ-4- bis 6-gliedriges Heterocyclyl, umfassend 1-4 Heteroatome, ausgewählt aus O, S(=O)p und N, und substituiert mit 0-5 R^{g}, -(CH₂)ₙOR^{f}, -C(=O)OR^{f}, -C(=O)NR^{f}R^{f}, - NR^{f}C(=O)R^{f}, -S(=O)ₚR^{f}, -NR^{f}C(=O)OR^{f}, -OC(=O)NR^{f}R^{f}, oder -(CH₂)ₙNR^{f}R^{f}, ist;
R^{f} H, C₁₋₅-Alkyl, C₃₋₆-Cycloalkyl oder Aryl ist oder R^{f} und R^{f} zusammen mit dem Stickstoffatom, an das sie beide gebunden sind, ein Heterocyclyl bilden;
R^{g} Halogen, CN, -OH, C₁₋₆-Alkyl, C₃₋₆-Cycloalkyl oder Aryl ist;
n null, 1, 2 oder 3 ist und
p null, 1 oder 2 ist.

3. Verbindung nach Anspruch 2 mit der Formel (III): 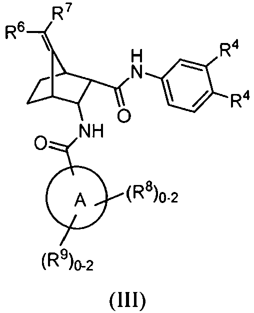 oder ein pharmazeutisch annehmbares Salz davon, worin: 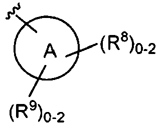 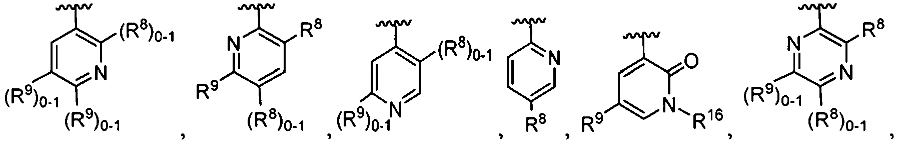 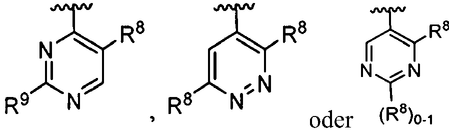 ist
R⁴ Halogen oder C₁₋₃-Alkyl, substituiert mit 0-4 Halogen, ist;
R⁶ Halogen, C₁₋₄-Alkyl, substituiert mit 0-3 R^{6a}, C₃₋₆-Cycloalkyl, Phenyl, substituiert mit 0-3 R¹⁴, Naphthyl oder 5- bis 6-gliedriges Heterocyclyl, umfassend 1-3 Heteroatome, ausgewählt aus O, S und N, ist;
R^{6a} Halogen, -OH oder C₃₋₆-Cycloalkyl ist;
R⁷ H ist;
R⁸ -OC₁₋₄-Alkyl, substituiert mit 0-5 Halogen oder OH-Substituenten, ist;
R⁹ Halogen, CN, -C(=O)OR^{b}, -C(=O)NR¹⁷R¹⁷, C₁₋₅-Alkyl, substituiert mit 0-3 R¹⁰ und 0-2 R¹¹, C₂₋₄-Alkinyl, substituiert mit 0-2 R¹⁰ und 0-2 R¹¹, Phenyl, substituiert mit 0-3 R¹⁰ und 0-2 R¹¹ oder 3- bis 9-gliedriges Heterocyclyl, umfassend 1-5 Heteroatome, ausgewählt aus O, S(=O)ₚ und substituiert mit 0-3 R¹⁰ und 0-2 R¹¹, ist;
R¹⁰ Halogen, CN, C₁₋₄-Alkyl, oder -OH ist;
R¹¹ -OR^{b}, -C(=O)OR^{b}, -C(=O)NR^{a}R^{a}, C₁₋₃ -Alkyl, substituiert mit 0-1 R¹² und 0-1 R¹³, oder C₃₋₆-Cycloalkyl, substituiert mit 0-5 R^{e} ist;
R¹² Halogen, -C(=O)OR^{b} oder C₁₋₃-Alkyl, substituiert mit 0-3 Halogen oder OH-Substituenten, ist;
R¹³ -OR^{b}, -NR^{a}R^{a}, -NR^{a}C(=O)R^{b}, -NR^{a}C(=O)OR^{b}, -NR^{a}S(=O)ₚR^{c}, -OC(=O)NR^{a}R^{a}, - OC(=O)NR^{a}OR^{b} oder -S(=O)ₚR^{c} ist;
R¹⁶ H oder C₁₋₃-Alkyl ist;
R¹⁷ H oder C₁₋₂-Alkyl, substituiert mit 0-3 R¹⁰ und 0-2 R¹¹ ist oder R¹⁷ und R¹⁷ zusammen mit dem Stickstoffatom, an das sie beide gebunden sind, ein 3- bis 10-gliedriges Heterocyclyl, umfassend 1-5 Heteroatome, ausgewählt aus O, S(=O)ₚ, N und NR^{11a}, und substituiert mit 0-3 R¹⁰ und 0-2 R¹¹, bilden;
R^{a} H, C₁₋₆ -Alkyl, substituiert mit 0-5 R^{e}, -(CH₂)ₙ-Phenyl, substituiert mit 0-5 R^{e}, oder - (CH₂)ₙ-Heterocyclyl, substituiert mit 0-5 R^{e} ist oder R^{a} und R^{a} zusammen mit dem Stickstoffatom, an das sie beide gebunden sind, ein Heterocyclyl, substituiert mit 0-5 R^{e}, bilden;
R^{b} H, C₁₋₆-Alkyl, substituiert mit 0-5 R^{e}, -(CH₂)₀₋₁-C₃₋₆ -Cycloalkyl, substituiert mit 0-5 R^{e}, -(CH₂)₀₋₁-Phenyl, substituiert mit 0-5 R^{e}, oder -(CH₂)ₙ-Heterocyclyl, substituiert mit 0-5 R^{e}, ist;
R^{e} Halogen, CN, =O, C(=O)OH, C₁₋₆-Alkyl, (CH₂)ₙOR^{f} oder -S(=O)ₚR^{f} ist;
R^{f} H oder C₁₋₅-Alkyl ist und
n null, 1, 2, oder 3 ist.

4. Verbindung nach Anspruch 3 oder ein pharmazeutisch annehmbares Salz davon, worin:
R⁴ Halogen oder C₁₋₄-Alkyl, substituiert mit 0-4 F, ist;
R⁶ C₁₋₃-Alkyl, substituiert mit 0-3 R^{6a}, C₃₋₆-Cycloalkyl, Phenyl oder 5- bis 6-gliedriges Heteroaryl, umfassend 1-3 Heteroatome, ausgewählt aus O, S und N, ist;
R^{6a} Halogen ist;
R⁷ H ist;
R⁸ -OC₁₋₃-Alkyl, substituiert mit 0-4 Halogen oder OH-Substituenten, ist;
R⁹ 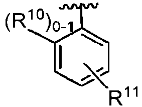 ist;
R¹⁰ Halogen ist;
R¹¹ -C(=O)OR^{b} oder C₁₋₂-Alkyl, substituiert mit 0-1 R¹² und 0-1 R¹³ ist;
R¹² ist Halogen, -C(=O)OR^{b} oder C₁₋₂-Alkyl, substituiert mit 0-3 Halogen oder OH-Substituenten;
R¹³ -OR^{b}, -NR^{a}R^{a}, -OC(=O)NR^{a}R^{a}, oder -S(=O)ₚR^{c};
R^{a} H, C₁₋₅ -Alkyl, substituiert mit 0-4 R^{e} , -(CH₂)ₙ-Phenyl, substituiert mit 0-4 R^{e} oder - (CH₂)ₙ-Heterocyclyl, substituiert mit 0-4 R^{e} , oder R^{a} und R^{a} zusammen mit dem Stickstoffatom, an das sie beide gebunden sind, ein Heterocyclyl, substituiert mit 0-4 R^{e}, bilden;
R^{b} H, C₁₋₅-Alkyl, substituiert mit 0-4 R^{e}, -(CH₂)ₙ-Phenyl, substituiert mit 0-4 R^{e}, oder - (CH₂)ₙ-Heterocyclyl, substituiert mit 0-4 R^{e}, ist;
R^{c} C₁₋₅ Alkyl, substituiert mit 0-4 R^{e}, ist;
R^{e} Halogen, CN, =O, C₁₋₅-Alkyl ist und
n null, 1 oder 2 ist.

5. Verbindung nach Anspruch 4 oder ein pharmazeutisch annehmbares Salz davon, worin:
R⁴ F oder CF₃ ist;
R⁶ CF₃, Cyclopropyl, Phenyl oder 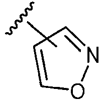 ist;
R⁸ -OC₁₋₄-Alkyl, substituiert mit 0-2 OH-Substituenten, ist;
R⁹ 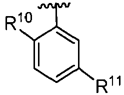 ist,
R¹⁰ F ist;
R¹¹ -C(=O)OR^{b} oder 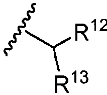 ist;
R¹² Halogen, -C(=O)OR^{b} oder CF₃ ist;
R¹³ -OH oder -OC(=O)NR^{a}R^{a} ist;
R^{a} H, C₁₋₄-Alkyl, C₃₋₆-Cycloalkyl oder Phenyl ist; und
R^{b} H oder C₁₋₃-Alkyl ist.

6. Verbindung nach Anspruch 2 oder ein pharmazeutisch annehmbares Salz davon, worin: 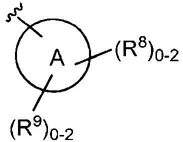 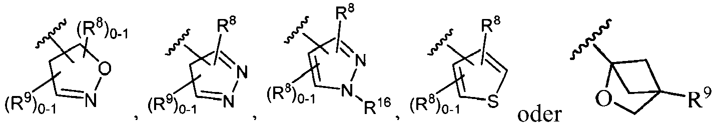 ist;
R⁴ Halogen oder C₁₋₄-Alkyl, substituiert mit 0-4 Halogen, ist;
R⁶ C₁₋₄-Alkyl, substituiert mit 0-3 R^{6a}, C₃₋₆-Cycloalkyl, Phenyl, substituiert mit 0-3 R¹⁴, oder 5- bis 6-gliedriges Heterocyclyl, umfassend 1-3 Heteroatome, ausgewählt aus O, S und N, ist;
R^{6a} Halogen ist;
R⁷ H ist;
R⁸ -OC₁₋₄-Alkyl, substituiert mit 0-5 Halogen oder OH-Substituenten, ist;
R⁹ Halogen, CN, C₁₋₄-Alkyl, substituiert mit 0-3 R¹⁰, oder Phenyl, substituiert mit 0-3 R¹⁰ und 0-2 R¹¹, ist;
R¹⁰ Halogen, CN oder C₁₋₄-Alkyl ist;
R¹¹ -C(=O)OR^{b} ist;
R¹⁶ H, C₁₋₄-Alkyl, substituiert mit 0-4 R^{16a}, oder Phenyl, substituiert mit 0-4 R^{16a}, ist; R^{16a} Halogen, -OR^{b}, -C(=O)OR^{b} oder -S(=O)ₚR^{c}, ist;
R^{b} H oder C₁₋₄-Alkyl ist und
R^{c} C₁₋₃-Alkyl ist.

7. Verbindung nach Anspruch 2 oder ein pharmazeutisch annehmbares Salz davon, worin: 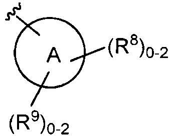 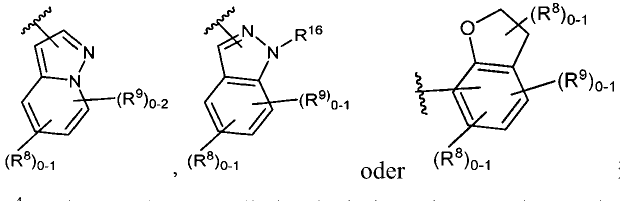 ist;
R⁴ Halogen oder C₁₋₄-Alkyl, substituiert mit 0-4 Halogensubstituenten, ist;
R⁶ C₁₋₄-Alkyl, substituiert mit 0-3 R^{6a}, C₃₋₆-Cycloalkyl, Phenyl, substituiert mit 0-3 R¹⁴, oder 5- bis 6-gliedriges Heterocyclyl, umfassend 1-3 Heteroatome, ausgewählt aus O, S und N, ist;
R⁷ H ist;
R⁸ -OC₁₋₄-Alkyl, substituiert mit 0-5 Halogen oder OH-Substituenten, ist;
R⁹ Halogen, CN oder C₁₋₄-Alkyl, substituiert mit 0-3 R¹⁰, ist;
R¹⁰ Halogen ist;
R¹⁶ H, C₁₋₄-Alkyl, substituiert mit 0-4 R^{16a} oder Aryl, substituiert mit 0-4 R^{16a} ist; und
R^{16a} Halogen, C₁₋₄-Alkyl oder C(=O)OR^{1b} ist.

8. Verbindung nach Anspruch 2 oder ein pharmazeutisch annehmbares Salz davon, worin: 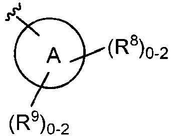 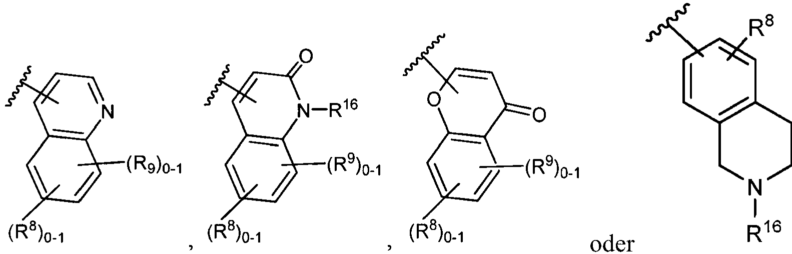 ist;
R⁴ Halogen oder C₁₋₄-Alkyl, substituiert mit 0-4 Halogensubstituenten, ist;
R⁶ C₁₋₄-Alkyl, substituiert mit 0-3 R^{6a}, C₃₋₆-Cycloalkyl, Phenyl, substituiert mit 0-3 R¹⁴, oder 5- bis 6-gliedriges Heterocyclyl, umfassend 1-3 Heteroatome, ausgewählt aus O, S und N, ist;
R⁷ H ist;
R⁸ -OC₁₋₄-Alkyl, substituiert mit 0-5 Halogen oder OH-Substituenten, ist;
R⁹ Halogen oder CN ist;
R¹⁶ H, C₁₋₄-Alkyl, -S(=O)ₚR^{c} oder C₁₋₄-Alkyl, substituiert mit 0-4 R^{16a}, ist; und
R^{16a} Halogen, C₁₋₄-Alkyl, -OH, OC₁₋₃-Alkyl oder -S(=O)ₚC₁₋₃-Alkyl ist.

9. Verbindung nach Anspruch 2 oder ein pharmazeutisch annehmbares Salz davon, worin: 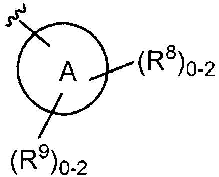 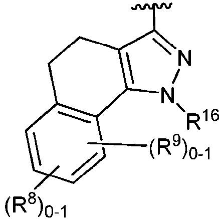 ist;
R⁴ Halogen oder C₁₋₄-Alkyl, substituiert mit 0-4 Halogensubstituenten, ist;
R⁶ C₁₋₄-Alkyl, substituiert mit 0-3 R^{6a}, C₃₋₆-Cycloalkyl, Phenyl, substituiert mit 0-3 R¹⁴, oder 5- bis 6-gliedriges Heterocyclyl, umfassend 1-3 Heteroatome, ausgewählt aus O, S und N, ist;
R⁷ H ist;
R⁸ -OC₁₋₄-Alkyl, substituiert mit 0-5 Halogen oder OH-Substituenten, ist;
R⁹ Halogen oder CN ist;
R¹⁶ H oder C₁₋₄-Alkyl, substituiert mit 0-4 R^{16a}, ist; und
R^{16a} Halogen ist.

10. Verbindung nach Anspruch 3 mit der Formel (IV): 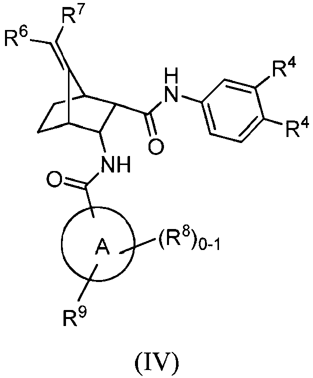 oder ein pharmazeutisch annehmbares Salz davon, worin: 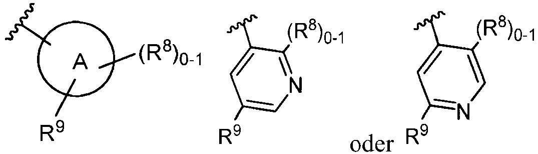 ist;
R⁴ F oder CF₃ ist;
R⁶ C₃₋₆-Cycloalkyl ist;
R⁷ H ist;
R⁸ -OC₁₋₃-Alkyl ist;
R⁹ -C(=O)OR^{b}, -C(=O)NR¹⁷R¹⁷, C₁₋₃-Alkyl substituiert mit 0-1 R¹¹, 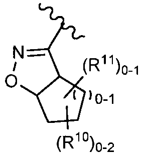 oder 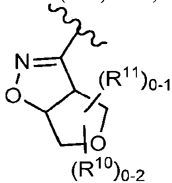 ist;
R¹⁰ Halogen, CN, C₁₋₄-Alkyl, oder -OH ist;
R¹¹ -OR^{b}, -C(=O)OR^{b}, -C(=O)NR^{a}R^{a}, C₁₋₃-Alkyl, substituiert mit 0-1 R¹³, oder C₃₋₆ - Cycloalkyl, substituiert mit 0-5 R^{e}, ist;
R¹³ -OH ist;
R¹⁷ H oder C₁₋₂-Alkyl, substituiert mit 0-3 R¹⁰ und 0-2 R¹¹, ist oder R¹⁷ und R¹⁷ zusammen mit dem Stickstoffatom, an das sie beide gebunden sind, 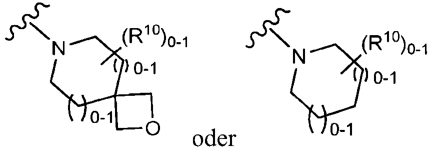 bilden;
R^{a} H oder C₁₋₃-Alkyl ist;
R^{b} H, C₁₋₃ Alkyl, substituiert mit 0-5 R^{e} oder -(CH₂)₀₋₁-C₃₋₆-Cycloalkyl, substituiert mit 0-5 R^{e}, ist und
R^{e} Halogen, CN, =O, C(=O)OH, C₁₋₆-Alkyl, CH₂OH oder -S(=O)₂C₁₋₃-Alkyl ist.

11. Verbindung nach Anspruch 1 mit der Formel (V): 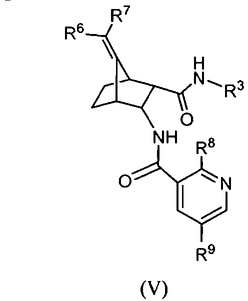 oder ein pharmazeutisch annehmbares Salz davon, worin:
R³ C₁₋₄-Alkyl, substituiert mit 0-3 R⁴, -(CHR^{d})ₙ-C₃₋₆-Carbocyclyl, substituiert mit 0-3 R⁴, ist;
R⁴ Halogen CN oder C₁₋₄-Alkyl, substituiert mit 0-5 Halogensubstituenten, ist;
R⁶ Halogen, C₁₋₄-Alkyl, substituiert mit 0-3 R^{6a}, C₃₋₆-Cycloalkyl, oder 5- bis 6-gliedriges Heterocyclyl, umfassend 1-3 Heteroatome, ausgewählt aus O, S und N, und substituiert mit 0-5 R¹⁴, ist;
R^{6a} Halogen ist;
R⁷ H ist;
R⁸ -OC₁₋₃-Alkyl ist; 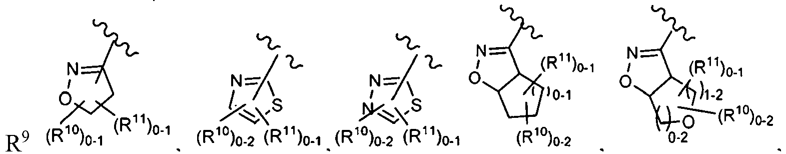 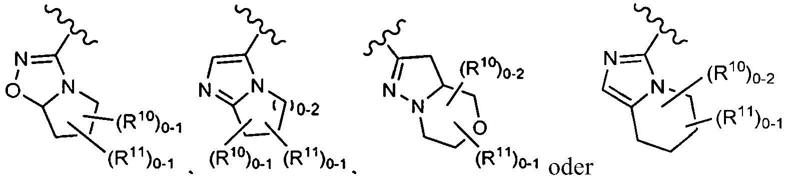 ist;
R¹⁰ Halogen, CN, C₁₋₄-Alkyl, oder -OH ist;
R¹¹ C₁₋₃-Alkyl, substituiert mit 0-3 R¹² und 0-2 R¹³, CN oder OR^{b}, ist;
R¹² Halogen ist;
R¹³ -OR^{b} oder C₃₋₆-Carbocyclyl ist;
R¹⁴ Halogen, CN oder C₁₋₄-Alkyl, substituiert mit 0-3 Halogen, ist;
R^{b} H oder C₁₋₃-Alkyl, substituiert mit 0-5 R^{e}, ist;
R^{d} H oder C₁₋₄-Alkyl ist;
R^{e} Halogen oder OH ist und
n null oder 1 ist.

12. Verbindung nach Anspruch 1 mit der Formel (VI): 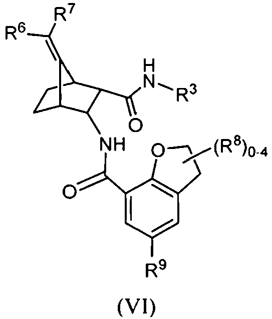 oder ein pharmazeutisch annehmbares Salz davon, worin:
R³ C₁₋₄-Alkyl, substituiert mit 0-3 R⁴, -(CHR^{d})ₙ-C₃₋₆-Carbocyclyl, substituiert mit 0-3 R⁴, ist;
R⁴ Halogen CN oder C₁₋₄-Alkyl, substituiert mit 0-5 Halogensubstituenten, ist;
R⁶ Halogen, C₁₋₄-Alkyl, substituiert mit 0-3 R^{6a}, C₃₋₆-Cycloalkyl, oder 5- bis 6-gliedriges Heterocyclyl, umfassend 1-3 Heteroatome, ausgewählt aus O, S und N, und substituiert mit 0-5 R¹⁴, ist;
R^{6a} Halogen oder -OH ist;
R⁷ H ist;
R⁸ -OC₁₋₃-Alkyl ist;
R⁹ 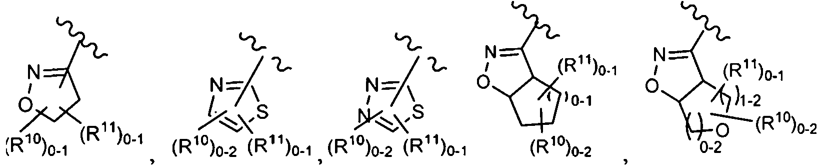 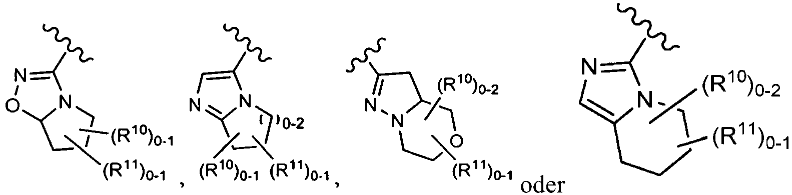 ist;
_{R}¹⁰ Halogen, CN, C₁₋₄-Alkyl, oder -OH;
R¹¹ C₁₋₃-Alkyl, substituiert mit 0-3 R¹² und 0-2 R¹³, CN oder OR^{b} ist;
R¹² Halogen ist;
R¹³ -OR^{b} oder C₃₋₆-Carbocyclyl ist;
R¹⁴ Halogen, CN oder C₁₋₄-Alkyl, substituiert mit 0-3 Halogensubstituenten, ist;
R^{b} H oder C₁₋₃-Alkyl, substituiert mit 0-5 R^{e} ist;
R^{d} H oder C₁₋₄-Alkyl ist;
R^{e} Halogen oder OH ist und
n null oder 1 ist.

13. Verbindung nach Anspruch 1 mit der Formel (VII): 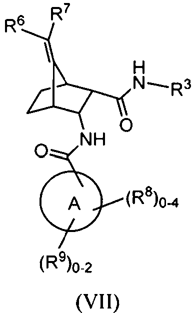 oder ein pharmazeutisch annehmbares Salz davon, worin: 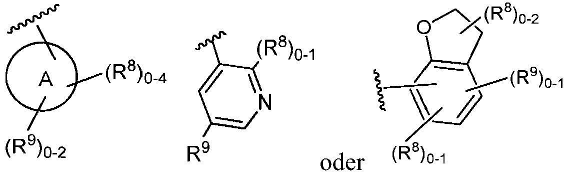 ist;
R³ -(CHR^{d})ₙ-C₃₋₁₀-Carbocyclyl, substituiert mit 0-5 R⁴, ist;
R⁴ Halogen, CN, C₁₋₄-Alkyl, substituiert mit 0-5 Halogen oder -OH, -OC₁₋₄-Alkyl, substituiert mit 0-5 Halogensubstituenten, oder -S(=O)ₚR^{c}, ist;
R⁶ C₁₋₄-Alkyl, substituiert mit 0-3 R^{6a}, oder C₃₋₆-Cycloalkyl substituiert mit 0-5 R¹⁴, ist; R^{6a} Halogen ist;
R⁷ H ist;
R⁸ C₁₋₃-Alkyl oder -OC₁₋₃-Alkyl ist;
R⁹ -C(=O)NR¹⁷R¹⁷, C₃₋₆-Carbocyclyl, substituiert mit 0-3 R¹⁰ und 0-2 R¹¹, oder 5- bis 12-gliedriges Heterocyclyl, umfassend 1-5 Heteroatome, ausgewählt aus O, S(=O)ₚ, N und NR^{11a} und substituiert mit 0-3 R¹⁰ und 0-2 R¹¹, ist;
R¹⁰ Halogen, CN, C₁₋₄-Alkyl, =O, -OH oder -OC₁₋₄-Alkyl ist;
R¹¹ C₁₋₄-Alkyl, substituiert mit 0-5 R¹² und 0-2 R¹³, -S(=O)ₚR^{c}, C₃₋₆-Cycloalkyl, substituiert mit 0-5 R^{e}, ist;
R^{11a} H, C₁₋₄-Alkyl, substituiert mit 0-4 R^{11b}, -C(=O)R^{b}, -C(=O)OR^{b}, -C(=O)NR^{a}R^{a}, C₃₋₆-Cycloalkyl, substituiert mit 0-5 R^{e}, Aryl, substituiert mit 0-5 R^{e}, 4- bis 6-gliedriges Heterocyclyl, umfassend 1-4 Heteroatome, ausgewählt aus O, S(=O)ₚ, N und NR¹⁵, und substituiert mit 0-5 R^{e}, ist;
R^{11b} Halogen, -OH, -C(=O)OH, -C(=O)OC₁₋₄-Alkyl oder Aryl ist;
R¹² Halogen, -C(=O)OR^{b}, C₁₋₄-Alkyl, substituiert mit 0-3 Halogen oder -OH oder C₃₋₆-Cycloalkyl, ist;
R¹³ -OR^{b}, -NR^{a}R^{a}, -OC(=O)NR^{a}R^{a} ist;
R¹⁴ Halogen ist;
R¹⁷ H oder C₁₋₄-Alkyl, substituiert mit 0-3 R¹⁰ und 0-2 R¹¹ ist oder R¹⁷ und R¹⁷ zusammen mit dem Stickstoffatom, an das sie beide gebunden sind, ein 3- bis 9-gliedriges Heterocyclyl, umfassend 1-5 Heteroatome, ausgewählt aus O, S(=O)ₚ, N, und NR^{11a}, und substituiert mit 0-3 R¹⁰ und 0-2 R¹¹, bilden;
R^{a} H, C₁₋₆-Alkyl, substituiert mit 0-5 R^{e}, C₃₋₁₀-Carbocyclyl, substituiert mit 0-5 R^{e}, ist oder R^{a} und R^{a} zusammen mit dem Stickstoffatom, an das sie beide gebunden sind, ein Heterocyclyl, substituiert mit 0-5 R^{e}, bilden;
R^{b} H oder C₁₋₄-Alkyl, substituiert mit 0-5 R^{e}, ist;
R^{c} C₁₋₄-Alkyl ist;
R^{d} H oder C₁₋₃-Alkyl ist;
R^{e} Halogen, CN, NO₂, =O, C₁₋₆-Alkyl, substituiert mit 0-5 R^{g}, C₃₋₆-Cycloalkyl oder - S(=O)ₚR^{f}, ist;
R^{f} H, C₁₋₆-Alkyl ist,
R^{g} Halogen, CN, -OH oder C₁₋₆-Alkyl ist;
n null oder 1 ist und
p null, 1 oder 2 ist.

14. Verbindung nach Anspruch 13 oder ein pharmazeutisch annehmbares Salz davon, worin:
R³ -CHR^{d}-C₃₋₆ -Cycloalkyl, substituiert mit 0-2 R⁴, oder Phenyl, substituiert mit 0-2 R⁴, ist;
R⁴ F, CH₃ oder CF₃ ist;
R⁶ C₁₋₄-Alkyl, substituiert mit 0-3 Halogen, oder C₃₋₆-Cycloalkyl, substituiert mit 0-3 Halogensubstituenten, ist;
R⁷ H ist;
R⁸ -OCH₃ ist;
R⁹ -C(=O)NR¹⁷R¹⁷, 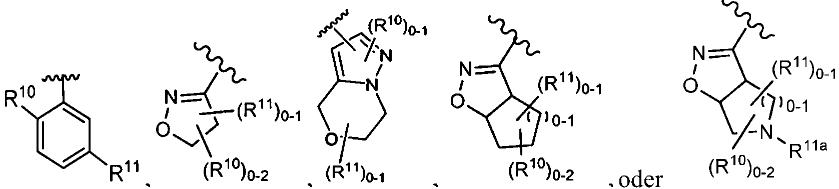 ist;
R¹⁰ Halogen ist;
R¹¹ C₁₋₄-Alkyl, substituiert mit 0-3 R¹² und 0-2 R¹³, -S(=O)ₚR^{c} oder C₃₋₆-Cycloalkyl, substituiert mit 0-3 R^{e}, ist;
R^{11a} H oder C₁₋₄-Alkyl, substituiert mit 0-3 R^{11b}, ist;
R^{11b} -OH ist;
R¹² C₁₋₄-Alkyl, substituiert mit 0-3 Halogensubstituenten, ist;
R¹³ -OC(=O)NR^{a}R^{a} ist;
R¹⁴ Halogen ist;
R¹⁷ H oder C₁₋₄-Alkyl, substituiert mit 0-2 R¹⁰ und 0-2 R¹¹, ist oder R¹⁷ und R¹⁷ zusammen mit dem Stickstoffatom, an das sie beide gebunden sind, ein 3- bis 9-gliedriges Heterocyclyl, umfassend 1-4 Heteroatome, ausgewählt aus O, S(=O)ₚ, N und NR^{11a}, und substituiert mit 0-2 R¹⁰ und 0-2 R¹¹, bilden;
R^{a} H, C₁₋₄-Alkyl, substituiert mit 0-4 R^{e}, C₃₋₁₀-Carbocyclyl, substituiert mit 0- R^{e}, ist oder R^{a} und R^{a} zusammen mit dem Stickstoffatom, an das sie beide gebunden sind, ein Heterocyclyl, substituiert mit 0-4 R^{e}, bilden;
R^{b} H oder C₁₋₃-Alkyl ist;
R^{c} C₁₋₃-Alkyl ist;
R^{d} H oder C₁₋₃-Alkyl ist;
R^{e} C₁₋₄-Alkyl, C₃₋₆-Cycloalkyl oder -S(=O)ₚR^{f} ist;
R^{f} C₁₋₄-Alkyl ist;
n null oder 1 ist und
p null, 1 oder 2 ist.

15. Pharmazeutische Zusammensetzung, umfassend eine Verbindung nach einem der Ansprüche 1 bis 14 oder ein pharmazeutisch annehmbares Salz davon und einen pharmazeutisch annehmbaren Träger.

16. Verbindung nach einem der Ansprüche 1 bis 14 oder eine pharmazeutische Zusammensetzung nach Anspruch 15 zur Verwendung in Therapie.

17. Verbindung nach einem der Ansprüche 1 bis 14 oder eine pharmazeutische Zusammensetzung nach Anspruch 15 zur Verwendung bei der Behandlung einer Krankheit, ausgewählt aus der Gruppe, bestehend aus Angina pectoris, instabiler Angina, Myokardinfarkt, Herzinsuffizienz, akuter Koronarerkrankung, akuter Herzinsuffizienz, chronischer Herzinsuffizienz und kardialer iatrogener Schädigung.

18. Verbindung oder Zusammensetzung zur Verwendung nach Anspruch 17, wobei die Krankheit Herzinsuffizienz ist.

19. Verbindung nach einem der Ansprüche 1 bis 14 oder eine pharmazeutische Zusammensetzung nach Anspruch 15 zur Verwendung bei der Behandlung von Fibrose.

## Revendications

1. Composé de formule (1) : 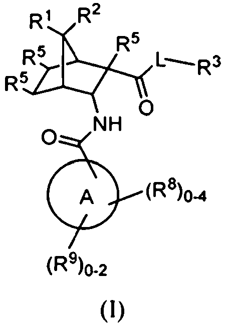 ou sel pharmaceutiquement acceptable de celui-ci, où :
L est -O- ou -NH- ;
le cycle A est un hétérocyclyle de 5 à 15 chaînons comprenant 1 à 5 hétéroatomes choisis parmi O, S(=O)ₚ, N et NR¹⁶ ;
R¹ est un C₁₋₃ alkyle substitué par 1 substituant aryle ou C₃₋₆ cycloalkyle ;
R² est H ; ou R¹ et R² pris ensemble représentent =CR⁶R⁷, où « = » est une liaison double ; ou R¹ et R² conjointement avec l'atome de carbone auquel ils sont tous les deux liés forment un dioxolanyle substitué par 0 ou 1 substituant aryle ;
R³ est un C₁₋₈ alkyle substitué par 0 à 5 R⁴, un -(CR^{d}R^{d})ₙ-C₃₋₁₀ carbocyclyle substitué par 0 à 5 R⁴ ou un -(CR^{d}R^{d})ₙ-hétérocyclyle de 3 à 12 chaînons comprenant 1 à 4 hétéroatomes choisis parmi O, S(=O)ₚ, N, NR^{4a} et substitué par 0 à 5 R⁴ ;
R⁴ est un halo, CN, -OH, SF₅, S(=O)ₚR^{c}, un C₁₋₄ alkyle substitué par 0 à 5 substituants halo, -OH ou -OC₁₋₄ alkyle, un -OC₁₋₄ alkyle substitué par 0 à 5 substituants halo, un -(CR^{d}R^{d})ₙ-C₃₋₁₀ carbocyclyle substitué par 0 à 5 R^{e} ou un -(CR^{d}R^{d})ₙ-hétérocyclyle de 4 à 9 chaînons comprenant 1 à 4 hétéroatomes choisis parmi O, S(=O)ₚ, N et NR^{4a} ;
R^{4a} est H, un C₁₋₄ alkyle ou -S(=O)₂CF₃ ;
R⁵ est H, un halo, -OH, un C₁₋₄ alkyle substitué par 0 à 5 substituants halo ou un - OC₁₋₄ alkyle substitué par 0 à 5 substituants halo ;
R⁶ est H, un halo, CN, un C₁₋₇ alkyle substitué par 0 à 3 R^{6a}, un C₂₋₇ alcényle substitué par 0 à 3 R^{6a}, un C₂₋₇ alcynyle substitué par 0 à 3 R^{6a}, -C(=O)OR^{6b}, -CONR^{6b}R^{6b}, un -(CH₂)ₙ-C₃₋₁₀ carbocyclyle substitué par 0 à 5 R¹⁴ ou un hétérocyclyle de 3 à 12 chaînons comprenant 1 à 4 hétéroatomes choisis parmi O, S(=O)ₚ, N ou NR^{4a} et substitué par 0 à 5 R¹⁴ ;
R^{6a} est un halo, -OH, un -OC₁₋₄ alkyle, un C₁₋₄ alkyle, un aryle ou un C₃₋₆ cycloalkyle substitué par 0 à 4 substituants halo ;
R^{6b} est H, un C₁₋₄ alkyle substitué par 0 ou 1 aryle ou un C₃₋₆ cycloalkyle substitué par 0 à 4 substituants halo ;
R⁷ est H ou un C₁₋₄ alkyle ;
R⁸ est =O, un C₁₋₄ alkyle ou un -OC₁₋₆ alkyle substitué par 0 à 5 substituants halo, - OH, -OC₁₋₄ alkyle, C₃₋₆ cycloalkyle, aryle ou hétérocyclyle de 3 à 6 chaînons ;
R⁹ est un halo, CN, -C(=O)OR^{b}, -C(=O)NR¹⁷R¹⁷, un C₁₋₈ alkyle substitué par 0 à 4 R¹⁰ et 0 à 2 R¹¹, un C₂₋₈ alcényle substitué par 0 à 2 R¹⁰ et 0 à 2 R¹¹, un C₂₋₈ alcynyle substitué par 0 à 2 R¹⁰ et 0 à 2 R¹¹, un -(A)₀₋₁-C₃₋₆ carbocyclyle substitué par 0 à 3 R¹⁰ et 0 à 2 R¹¹, un -(A)₀₋₁-C₆₋₉ spirocycloalkyle substitué par 0 à 2 R¹⁰ et 0 à 2 R¹¹ ou un -(A)₀₋₁-hétérocyclyle de 3 à 12 chaînons comprenant 1 à 5 hétéroatomes choisis parmi O, S(=O)p, N et NR^{11a} et substitué par 0 à 3 R¹⁰ et 0 à 2 R¹¹ ;
A est -O-, -S-, -CH₂O- ou -OCH₂- ;
R¹⁰ est un halo, CN, un C₁₋₄ alkyle, =O, -OH ou un -OC₁₋₄ alkyle ;
R¹¹ est un C₁₋₄ alkyle substitué par 0 à 5 R¹² et 0 à 2 R¹³, -OR^{b}, -NR^{a}R^{a}, - NR^{a}C(=O)R^{b}, -NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, un C₃₋₉ carbocyclyle substitué par 0 à 5 R^{e}, un aryle substitué par 0 à 5 R^{e}, un hétérocyclyle de 3 à 12 chaînons comprenant 1 à 4 hétéroatomes choisis parmi O, S(=O)ₚ, N et NR¹⁵ et substitué par 0 à 5 R^{e} ;
R^{11a} est H, un C₁₋₄ alkyle substitué par 0 à 4 R^{11b}, C(=O)R^{b}, -C(=O)OR^{b}, - C(=O)NR^{a}R^{a}, un C₃₋₆ cycloalkyle substitué par 0 à 5 R^{e}, un aryle substitué par 0 à 5 R^{e}, un hétérocyclyle de 4 à 6 chaînons comprenant 1 à 4 hétéroatomes choisis parmi O, S(=O)ₚ, N et NR¹⁵ et substitué par 0 à 5 R^{e} ;
R^{11b} est un halo, -OH, -C(=O)OH, un -C(=O)OC₁₋₄ alkyle ou un aryle ;
R¹² est un halo, -C(=O)OR^{b}, -C(=O)NR^{a}R^{a}, -C(=O)NR^{a}OR^{b}, un C₁₋₄ alkyle substitué par 0 à 3 substituants halo ou -OH ou un C₃₋₆ cycloalkyle ;
R¹³ est -OR^{b}, -NR^{a}R^{a}, -NR^{a}C(=O)R^{b}, -NR^{a}C(=O)OR^{b}, -NR^{a}C(=O)NR^{a}R^{a}, - NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, - S(=O)ₚR^{c}, un -(CH₂)ₙ-C₃₋₁₀ carbocyclyle substitué par 0 à 3 R^{e} ou un -(CH₂)ₙ-hétérocyclyle de 3 à 12 chaînons comprenant 1 à 4 hétéroatomes choisis parmi O, S(=O)ₚ et N et substitué par 0 à 3 R^{e};
R¹⁴ est un halo, CN, un C₁₋₄ alkyle substitué par 0 à 3 substituants halo, un -OC₁₋₄ alkyle substitué par 0 à 3 substituants halo, -(CH₂)ₙ-NR^{a}R^{a}, un -(CH₂)ₙ-aryle substitué par 0 à 3 R^{e}, un -O-aryle substitué par 0 à 3 R^{e} ou un -(CH₂)ₙ-hétérocyclyle de 3 à 12 chaînons comprenant 1 à 4 hétéroatomes choisis parmi O, S(=O)ₚ et N et substitué par 0 à 3 R^{e} ;
R^{14a} est H, un -C(=O)C₁₋₄ alkyle ou un C₁₋₃ alkyle substitué par 0 à 3 substituants -Si(C₁₋₃ alkyle)₃ ou aryles substitués par 0 à 2 substituants halo ;
R¹⁵ est H, un C₁₋₄ alkyle ou un aryle ;
R¹⁶ est H, -C(=O)R^{b}, -C(=O)OR^{b}, -C(=O)NR^{a}R^{a}, -S(=O)ₚR^{c}, un C₁₋₄ alkyle substitué par 0 à 4 R^{16a} ou un aryle substitué par 0 à 4 R^{16a} ;
R^{16a} est un halo, un C₁₋₄ alkyle, OR^{b}, -C(=O)OR^{b} ou -S(=O)ₚR^{c} ;
R¹⁷ est H ou un C₁₋₄ alkyle substitué par 0 à 3 R¹⁰ et 0 à 2 R¹¹ ; ou R¹⁷ et R¹⁷ conjointement avec l'atome d'azote auquel ils sont tous les deux liés forment un hétérocyclyle de 3 à 12 chaînons comprenant 1 à 5 hétéroatomes choisis parmi O, S(=O)ₚ, N et NR^{11a} et substitué par 0 à 3 R¹⁰ et 0 à 2 R¹¹ ;
R^{a} est H, un C₁₋₆ alkyle substitué par 0 à 5 R^{e}, un C₂₋₆ alcényle substitué par 0 à 5 R^{e}, un C₂₋₆ alcynyle substitué par 0 à 5 R^{e}, un -(CH₂)ₙ-C₃₋₁₀ carbocyclyle substitué par 0 à 5 R^{e} ou un -(CH₂)ₙ-hétérocyclyle de 3 à 12 chaînons comprenant 1 à 5 hétéroatomes choisis parmi O, S(=O)ₚ et N et substitué par 0 à 5 R^{e}; ou R^{a} et R^{a} conjointement avec l'atome d'azote auquel ils sont tous les deux liés forment un hétérocyclyle de 3 à 12 chaînons comprenant 1 à 5 hétéroatomes choisis parmi O, S(=O)ₚ et N et substitué par 0 à 5 R^{e} ;
R^{b} est H, un C₁₋₆ alkyle substitué par 0 à 5 R^{e}, un C₂₋₆ alcényle substitué par 0 à 5 R^{e}, un C₂₋₆ alcynyle substitué par 0 à 5 R^{e}, un -(CH₂)ₙ-C₃₋₁₀ carbocyclyle substitué par 0 à 5 R^{e} ou un -(CH₂)ₙ-hétérocyclyle de 3 à 12 chaînons comprenant 1 à 5 hétéroatomes choisis parmi O, S(=O)ₚ et N et substitué par 0 à 5 R^{e} ;
R^{c} est un C₁₋₆ alkyle substitué par 0 à 5 R^{e}, un C₂₋₆ alcényle substitué par 0 à 5 R^{e}, un C₂₋₆ alcynyle substitué par 0 à 5 R^{e}, un C₃₋₆ carbocyclyle ou un hétérocyclyle de 3 à 12 chaînons comprenant 1 à 5 hétéroatomes choisis parmi O, S(=O)ₚ et N ;
R^{d} est H, un C₁₋₄ alkyle ou un C₃₋₆ cycloalkyle ;
R^{e} est un halo, CN, NO₂, =O, un C₁₋₆ alkyle substitué par 0 à 5 R^{g}, un C₂₋₆ alcényle substitué par 0 à 5 R^{g}, un C₂₋₆ alcynyle substitué par 0 à 5 R^{g}, un -(CH₂)ₙ-C₃₋₁₀ carbocyclyle substitué par 0 à 5 R^{g}, un -(CH₂)ₙ-hétérocyclyle de 3 à 12 chaînons comprenant 1 à 5 hétéroatomes choisis parmi O, S(=O)ₚ et N et substitué par 0 à 5 R^{g}, -(CH₂)ₙOR^{f}, - C(=O)OR^{f}, -C(=O)NR^{f}R^{f}, -NR^{f}C(=O)R^{f}, -S(=O)ₚR^{f}, -S(=O)ₚNR^{f}R^{f}, -NR^{f}S(=O)ₚR^{f}, - NR^{f}C(=O)OR^{f}, -OC(=O)NR^{f}R^{f} ou -(CH₂)ₙNR^{f}R^{f} ;
R^{f} est H, un C₁₋₆ alkyle, un C₃₋₆ cycloalkyle, un aryle ou un hétérocyclyle ; ou R^{f} et R^{f} conjointement avec l'atome d'azote auquel ils sont tous les deux liés forment un hétérocyclyle ;
R^{g} est un halo, CN, -OH, un C₁₋₆ alkyle, un C₃₋₆ cycloalkyle ou un aryle ;
n vaut zéro, 1, 2 ou 3 ; et
p vaut zéro, 1 ou 2.

2. Composé selon la revendication 1, présentant la formule (II) : 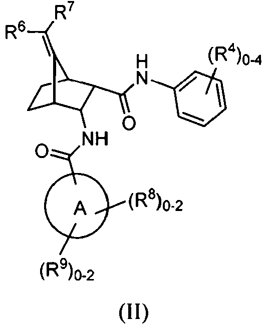 ou sel pharmaceutiquement acceptable de celui-ci, où :
le cycle A est un hétérocyclyle de 5 à 12 chaînons comprenant 1 à 4 hétéroatomes choisis parmi O, S(=O)ₚ, N et NR¹⁶ ;
R⁴ est un halo, un C₁₋₄ alkyle substitué par 0 à 4 substituants halo, un -OC₁₋₄ alkyle substitué par 0 à 4 substituants halo ou un -S(O)ₚC₁₋₄ alkyle substitué par 0 à 4 substituants halo ;
R⁶ est un halo, un C₁₋₇ alkyle substitué par 0 à 3 R^{6a}, un C₃₋₆ cycloalkyle substitué par 0 à 3 R¹⁴, un C₃₋₆ cycloalcényle substitué par 0 à 3 R¹⁴, un C₆₋₁₀ aryle substitué par 0 à 3 R¹⁴ ou un hétérocyclyle de 4 à 6 chaînons comprenant 1 à 3 hétéroatomes choisis parmi O, S(=O)ₚ, N ou NR^{14a} et substitué par 0 à 3 R¹⁴ ;
R^{6a} est un halo, -OH, un C₃₋₆ cycloalkyle ou un aryle ;
R⁷ est H ou un C₁₋₃ alkyle ;
R⁸ est =O ou un -OC₁₋₄ alkyle substitué par 0 à 5 substituants halo, -OH, -OC₁₋₄ alkyle ou aryle ;
R⁹ est un halo, CN, -C(=O)OR^{b}, -C(=O)NR¹⁷R¹⁷, un C₁₋₇ alkyle substitué par 0 à 3 R¹⁰ et 0 à 2 R¹¹, un C₂₋₇ alcényle substitué par 0 à 2 R¹⁰ et 0 à 2 R¹¹, un C₂₋₇ alcynyle substitué par 0 à 2 R¹⁰ et 0 à 2 R¹¹, un phényle substitué par 0 à 3 R¹⁰ et 0 à 2 R¹¹ ou un hétérocyclyle de 3 à 12 chaînons comprenant 1 à 5 hétéroatomes choisis parmi O, S(=O)ₚ et substitué par 0 à 3 R¹⁰ et 0 à 2 R¹¹ ;
R¹⁰ est un halo, CN, un C₁₋₄ alkyle, -OH ou un -OC₁₋₄ alkyle ;
R¹¹ est un C₁₋₃ alkyle substitué par 0 ou 1 R¹² et 0 ou 1 R¹³, -OR^{b}, -NR^{a}R^{a}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, un C₃₋₆ cycloalkyle substitué par 0 à 5 R^{e}, un hétérocyclyle de 4 à 6 chaînons comprenant 1 à 4 hétéroatomes choisis parmi O, S(=O)ₚ, N et NR¹⁵ et substitué par 0 à 5 R^{e} ;
R¹² est un halo, -C(=O)OR^{b}, -C(=O)NHR^{a} ou un C₁₋₄ alkyle substitué par 0 à 3 substituants halo ou OH ;
R¹³ est -OR^{b}, -NR^{a}R^{a}, -NR^{a}C(=O)R^{b}, -NR^{a}C(=O)OR^{b}, -NR^{a}S(=O)ₚR^{c}, - NR^{a}S(=O)ₚNR^{a}R^{a}, -OC(=O)NR^{a}R^{a}, -OC(=O)NR^{a}OR^{b}, -S(=O)ₚNR^{a}R^{a} ou -S(=O)pR^{c} ;
R¹⁴ est un halo, CN, un C₁₋₄ alkyle substitué par 0 à 3 substituants halo, un -OC₁₋₄ alkyle substitué par 0 à 3 substituants halo ;
R^{14a} est H, un C(=O)C₁₋₄ alkyle ou un C₁₋₃ alkyle substitué par 0 à 3 aryles substitués par 0 à 2 substituants halo ;
R¹⁶ est H, un C₁₋₃ alkyle substitué par 0 à 4 R^{16a} ou un aryle substitué par 0 à 4 R^{16a} ;
R^{16a} est un halo, un C₁₋₃ alkyle, OR^{b}, C(=O)OR^{b} ou -S(=O)ₚR^{c} ;
R¹⁷ est H ou un C₁₋₃ alkyle substitué par 0 à 3 R¹⁰ et 0 à 2 R¹¹ ; ou R¹⁷ et R¹⁷ conjointement avec l'atome d'azote auquel ils sont tous les deux liés forment un hétérocyclyle de 3 à 10 chaînons comprenant 1 à 5 hétéroatomes choisis parmi O, S(=O)ₚ, N et NR^{11a} et substitué par 0 à 3 R¹⁰ et 0 à 2 R¹¹ ;
R^{a} est H, un C₁₋₅ alkyle substitué par 0 à 5 R^{e}, un C₂₋₅ alcényle substitué par 0 à 5 R^{e}, un C₂₋₅ alcynyle substitué par 0 à 5 R^{e}, un -(CH₂)ₙ-C₃₋₁₀ carbocyclyle substitué par 0 à 5 R^{e} ou un -(CH₂)ₙ-hétérocyclyle de 3 à 10 chaînons comprenant 1 à 5 hétéroatomes choisis parmi O, S(=O)ₚ et N et substitué par 0 à 5 R^{e}; ou R^{a} et R^{a} conjointement avec l'atome d'azote auquel ils sont tous les deux liés forment un hétérocyclyle de 3 à 10 chaînons comprenant 1 à 5 hétéroatomes choisis parmi O, S(=O)ₚ et N et substitué par 0 à 5 R^{e} ;
R^{b} est H, un C₁₋₅ alkyle substitué par 0 à 5 R^{e}, un C₂₋₅ alcényle substitué par 0 à 5 R^{e}, un C₂₋₅ alcynyle substitué par 0 à 5 R^{e}, un -(CH₂)ₙ-C₃₋₁₀ carbocyclyle substitué par 0 à 5 R^{e} ou un -(CH₂)ₙ-hétérocyclyle de 3 à 10 chaînons comprenant 1 à 5 hétéroatomes choisis parmi O, S(=O)ₚ et N et substitué par 0 à 5 R^{e} ;
R^{c} est un C₁₋₅ alkyle substitué par 0 à 5 R^{e}, un C₂₋₅ alcényle substitué par 0 à 5 R^{e}, un C₂₋₅ alcynyle substitué par 0 à 5 R^{e}, un C₃₋₆ carbocyclyle ou un hétérocyclyle de 3 à 10 chaînons comprenant 1 à 5 hétéroatomes choisis parmi O, S(=O)ₚ et N ;
R^{e} est un halo, CN, =O, un C₁₋₆ alkyle substitué par 0 à 5 R^{g}, un C₂₋₆ alcényle substitué par 0 à 5 R^{g}, un C₂₋₆ alcynyle substitué par 0 à 5 R^{g}, un -(CH₂)ₙ-C₃₋₆ carbocyclyle substitué par 0 à 5 R^{g}, un -(CH₂)ₙ-hétérocyclyle de 4 à 6 chaînons comprenant 1 à 4 hétéroatomes choisis parmi O, S(=O)ₚ et N et substitué par 0 à 5 R^{g}, -(CH₂)ₙOR^{f}, - C(=O)OR^{f}, -C(=O)NR^{f}R^{f}, -NR^{f}C(=O)R^{f}, -S(=O)ₚR^{f}, -NR^{f}C(=O)OR^{f}, -OC(=O)NR^{f}R^{f} ou - (CH₂)ₙNR^{f}R^{f} ;
R^{f} est H, un C₁₋₅ alkyle, un C₃₋₆ cycloalkyle ou un aryle ; ou R^{f} et R^{f} conjointement avec l'atome d'azote auquel ils sont tous les deux liés forment un hétérocyclyle ;
R^{g} est un halo, CN, -OH, un C₁₋₆ alkyle, un C₃₋₆ cycloalkyle ou un aryle ;
n vaut zéro, 1, 2 ou 3 ; et
p vaut zéro, 1 ou 2.

3. Composé selon la revendication 2, présentant la formule (III) : 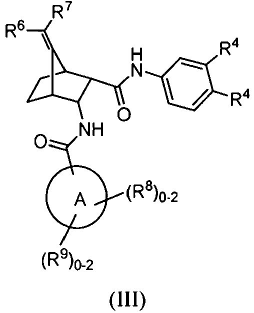 ou sel pharmaceutiquement acceptable de celui-ci, où : 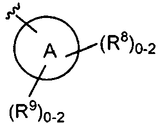 est 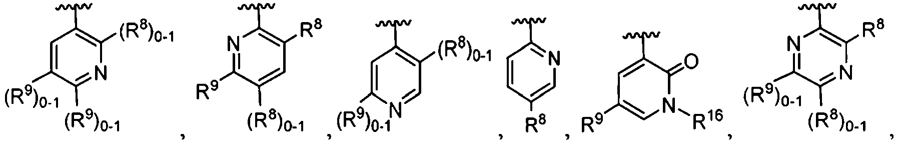 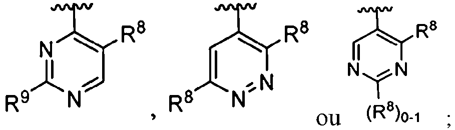
R⁴ est un halo ou un C₁₋₃ alkyle substitué par 0 à 4 halo ;
R⁶ est un halo, un C₁₋₄ alkyle substitué par 0 à 3 R^{6a}, un C₃₋₆ cycloalkyle, un phényle substitué par 0 à 3 R¹⁴, un naphtyle ou un hétérocyclyle de 5 ou 6 chaînons comprenant 1 à 3 hétéroatomes choisis parmi O, S et N ;
R^{6a} est un halo, -OH ou un C₃₋₆ cycloalkyle ;
R⁷ est H ;
R⁸ est un -OC₁₋₄ alkyle substitué par 0 à 5 substituants halo ou OH ;
R⁹ est un halo, CN, -C(=O)OR^{b}, -C(=O)NR¹⁷R¹⁷, un C₁₋₅ alkyle substitué par 0 à 3 R¹⁰ et 0 à 2 R¹¹, un C₂₋₄ alcynyle substitué par 0 à 2 R¹⁰ et 0 à 2 R¹¹, un phényle substitué par 0 à 3 R¹⁰ et 0 à 2 R¹¹ ou un hétérocyclyle de 3 à 9 chaînons comprenant 1 à 5 hétéroatomes choisis parmi O, S(=O)ₚ et substitué par 0 à 3 R¹⁰ et 0 à 2 R¹¹ ;
R¹⁰ est un halo, CN, un C₁₋₄ alkyle ou -OH ;
R¹¹ est -OR^{b}, -C(=O)OR^{b}, -C(=O)NR^{a}R^{a}, un C₁₋₃ alkyle substitué par 0 ou 1 R¹² et 0 ou 1 R¹³ ou un C₃₋₆ cycloalkyle substitué par 0 à 5 R^{e} ;
R¹² est un halo, -C(=O)OR^{b} ou un C₁₋₃ alkyle substitué par 0 à 3 substituants halo ou OH ;
R¹³ est -OR^{b}, -NR^{a}R^{a}, -NR^{a}C(=O)R^{b}, -NR^{a}C(=O)OR^{b}, -NR^{a}S(=O)ₚR^{c}, - OC(=O)NR^{a}R^{a}, -OC(=O)NR^{a}OR^{b} ou -S(=O)ₚR^{c} ;
R¹⁶ est H ou un C₁₋₃ alkyle ;
R¹⁷ est H ou un C₁₋₂ alkyle substitué par 0 à 3 R¹⁰ et 0 à 2 R¹¹ ; ou R¹⁷ et R¹⁷ conjointement avec l'atome d'azote auquel ils sont tous les deux liés forment un hétérocyclyle de 3 à 10 chaînons comprenant 1 à 5 hétéroatomes choisis parmi O, S(=O)ₚ, N et NR^{11a} et substitué par 0 à 3 R¹⁰ et 0 à 2 R¹¹ ;
R^{a} est H, un C₁₋₆ alkyle substitué par 0 à 5 R^{e}, un -(CH₂)ₙ-phényle substitué par 0 à 5 R^{e} ou un -(CH₂)ₙ-hétérocyclyle substitué par 0 à 5 R^{e} ; ou R^{a} et R^{a} conjointement avec l'atome d'azote auquel ils sont tous les deux liés forment un hétérocyclyle substitué par 0 à 5 R^{e} ;
R^{b} est H, un C₁₋₆ alkyle substitué par 0 à 5 R^{e}, un -(CH₂)₀₋₁-C₃₋₆ cycloalkyle substitué par 0 à 5 R^{e}, un -(CH₂)₀₋₁-phényle substitué par 0 à 5 R^{e} ou un -(CH₂)ₙ-hétérocyclyle substitué par 0 à 5 R^{e} ;
R^{e} est un halo, CN, =O, C(=O)OH, un C₁₋₆ alkyle, -(CH₂)ₙOR^{f} ou -S(=O)ₚR^{f} ;
R^{f} est H ou un C₁₋₅ alkyle ; et
n vaut zéro, 1, 2 ou 3.

4. Composé selon la revendication 3, ou sel pharmaceutiquement acceptable de celui-ci, où :
R⁴ est un halo ou un C₁₋₄ alkyle substitué par 0 à 4 F ;
R⁶ est un C₁₋₃ alkyle substitué par 0 à 3 R^{6a}, un C₃₋₆ cycloalkyle, un phényle ou un hétéroaryle de 5 ou 6 chaînons comprenant 1 à 3 hétéroatomes choisis parmi O, S et N ;
R^{6a} est un halo ;
R⁷ est H ;
R⁸ est un -OC₁₋₃ alkyle substitué par 0 à 4 substituants halo ou OH ;
R⁹ est 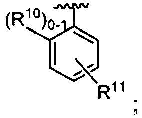
R¹⁰ est un halo ;
R¹¹ est -C(=O)OR^{b} ou un C₁₋₂ alkyle substitué par 0 ou 1 R¹² et 0 ou 1 R¹³ ;
R¹² est un halo, -C(=O)OR^{b} ou un C₁₋₂ alkyle substitué par 0 à 3 substituants halo ou OH ;
R¹³ est -OR^{b}, -NR^{a}R^{a}, -OC(=O)NR^{a}R^{a} ou -S(=O)ₚR^{c} ;
R^{a} est H, un C₁₋₅ alkyle substitué par 0 à 4 R^{e}, un -(CH₂)ₙ-phényle substitué par 0 à 4 R^{e} ou un -(CH₂)ₙ-hétérocyclyle substitué par 0 à 4 R^{c} ; ou R^{a} et R^{a} conjointement avec l'atome d'azote auquel ils sont tous les deux liés forment un hétérocyclyle substitué par 0 à4 R^{e} ;
R^{b} est H, un C₁₋₅ alkyle substitué par 0 à 4 R^{e}, un -(CH₂)ₙ-phényle substitué par 0 à 4 R^{e} ou un -(CH₂)ₙ-hétérocyclyle substitué par 0 à 4 R^{e} ;
R^{c} est un C₁₋₅ alkyle substitué par 0 à 4 R^{e} ;
R^{e} est un halo, CN, =O, un C₁₋₅ alkyle ; et
n vaut zéro, 1 ou 2.

5. Composé selon la revendication 4, ou sel pharmaceutiquement acceptable de celui-ci, où :
R⁴ est F ou CF₃ ;
R⁶ est CF₃, un cyclopropyle, un phényle ou 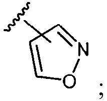
R⁸ est un -OC₁₋₄ alkyle substitué par 0 à 2 substituants OH ;
R⁹ est 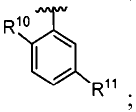
R¹⁰ est F ;
R¹¹ est -C(=O)OR^{b} ou 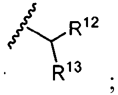
R¹² est un halo, -C(=O)OR^{b} ou CF₃ ;
R¹³ est -OH ou -OC(=O)NR^{a}R^{a} ;
R^{a} est H, un C₁₋₄ alkyle, un C₃₋₆ cycloalkyle ou un phényle ; et
R^{b} est H ou un C₁₋₃ alkyle.

6. Composé selon la revendication 2, ou sel pharmaceutiquement acceptable de celui-ci, où : 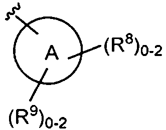 est 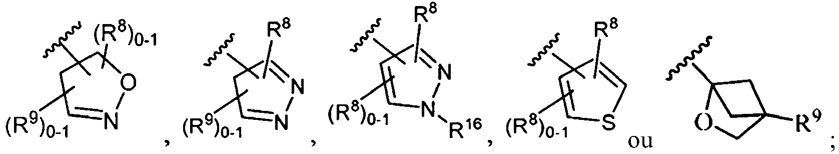
R⁴ est un halo ou un C₁₋₄ alkyle substitué par 0 à 4 halo ;
R⁶ est un C₁₋₄ alkyle substitué par 0 à 3 R^{6a}, un C₃₋₆ cycloalkyle, un phényle substitué par 0 à 3 R¹⁴ ou un hétérocyclyle de 5 ou 6 chaînons comprenant 1 à 3 hétéroatomes choisis parmi O, S et N ;
R^{6a} est un halo ;
R⁷ est H ;
R⁸ est un -OC₁₋₄ alkyle substitué par 0 à 5 substituants halo ou OH ;
R⁹ est un halo, CN, un C₁₋₄ alkyle substitué par 0 à 3 R¹⁰ ou un phényle substitué par 0 à 3 R¹⁰ et 0 à 2 R¹¹ ;
R¹⁰ est un halo, CN ou un C₁₋₄ alkyle ;
R¹¹ est -C(=O)OR^{b} ;
R¹⁶ est H, un C₁₋₄ alkyle substitué par 0 à 4 R^{16a} ou un phényle substitué par 0 à 4 R^{16a} ;
R^{16a} est un halo, -OR^{b}, -C(=O)OR^{b} ou -S(=O)ₚR^{c} ;
R^{b} est H ou un C₁₋₄ alkyle ; et
R^{c} est un C₁₋₃ alkyle.

7. Composé selon la revendication 2, ou sel pharmaceutiquement acceptable de celui-ci, où : 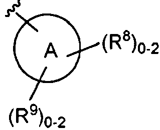 est : 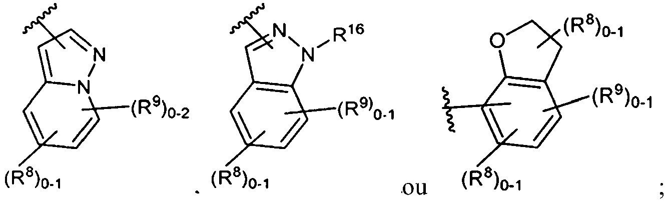
R⁴ est un halo ou un C₁₋₄ alkyle substitué par 0 à 4 substituants halo ;
R⁶ est un C₁₋₄ alkyle substitué par 0 à 3 R^{6a}, un C₃₋₆ cycloalkyle, un phényle substitué par 0 à 3 R¹⁴ ou un hétérocyclyle de 5 ou 6 chaînons comprenant 1 à 3 hétéroatomes choisis parmi O, S et N ;
R⁷ est H ;
R⁸ est un -OC₁₋₄ alkyle substitué par 0 à 5 substituants halo ou OH ;
R⁹ est un halo, CN ou un C₁₋₄ alkyle substitué par 0 à 3 R¹⁰ ;
R¹⁰ est un halo ;
R¹⁶ est H, un C₁₋₄ alkyle substitué par 0 à 4 R^{16a} ou un aryle substitué par 0 à 4 R^{16a} ; et
R^{16a} est un halo, un C₁₋₄ alkyle ou C(=O)OR^{b}.

8. Composé selon la revendication 2, ou sel pharmaceutiquement acceptable de celui-ci, où : 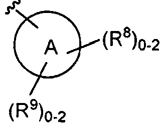 est 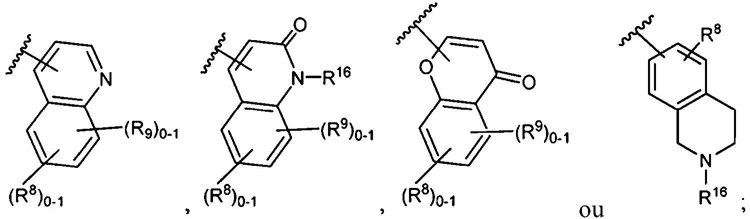
R⁴ est un halo ou un C₁₋₄ alkyle substitué par 0 à 4 substituants halo ;
R⁶ est un C₁₋₄ alkyle substitué par 0 à 3 R^{6a}, un C₃₋₆ cycloalkyle, un phényle substitué par 0 à 3 R¹⁴ ou un hétérocyclyle de 5 ou 6 chaînons comprenant 1 à 3 hétéroatomes choisis parmi O, S et N ;
R⁷ est H ;
R⁸ est un -OC₁₋₄ alkyle substitué par 0 à 5 substituants halo ou OH ;
R⁹ est un halo ou CN ;
R¹⁶ est H, un C₁₋₄ alkyle, -S(=O)ₚR^{c} ou un C₁₋₄ alkyle substitué par 0 à 4 R^{16a} ; et
R^{16a} est un halo, un C₁₋₄ alkyle, -OH, un OC₁₋₃ alkyle ou -S(=O)ₚC₁₋₃ alkyle.

9. Composé selon la revendication 2, ou sel pharmaceutiquement acceptable de celui-ci, où : 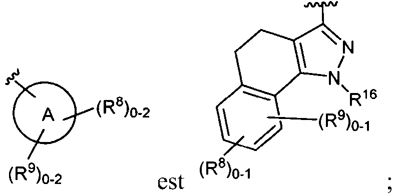
R⁴ est un halo ou un C₁₋₄ alkyle substitué par 0 à 4 substituants halo ;
R⁶ est un C₁₋₄ alkyle substitué par 0 à 3 R^{6a}, un C₃₋₆ cycloalkyle, un phényle substitué par 0 à 3 R¹⁴ ou un hétérocyclyle de 5 ou 6 chaînons comprenant 1 à 3 hétéroatomes choisis parmi O, S et N ;
R⁷ est H ;
R⁸ est un -OC₁₋₄ alkyle substitué par 0 à 5 substituants halo ou OH ;
R⁹ est un halo ou CN ;
R¹⁶ est H ou un C₁₋₄ alkyle substitué par 0 à 4 R^{16a} ; et
R^{16a} est un halo.

10. Composé selon la revendication 3, présentant la formule (IV) : 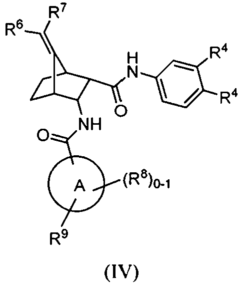 ou sel pharmaceutiquement acceptable de celui-ci, où : 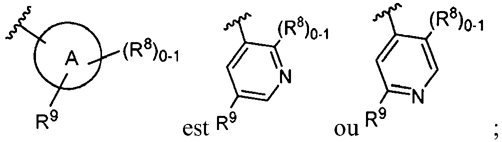
R⁴ est F ou CF₃ ;
R⁶ est un C₃₋₆ cycloalkyle ;
R⁷ est H ;
R⁸ est un -OC₁₋₃ alkyle ;
R⁹ est -C(=O)OR^{b}, -C(=O)NR¹⁷R¹⁷, un C₁₋₃ alkyle substitué par 0 ou 1 R¹¹, 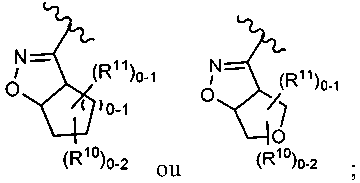
R¹⁰ est un halo, CN, un C₁₋₄ alkyle ou -OH ;
R¹¹ est -OR^{b}, -C(=O)OR^{b}, -C(=O)NR^{a}R^{a}, un C₁₋₃ alkyle substitué par 0 ou 1 R¹³ ou un C₃₋₆ cycloalkyle substitué par 0 à 5 R^{e} ;
R¹³ est -OH ;
R¹⁷ est H ou un C₁₋₂ alkyle substitué par 0 à 3 R¹⁰ et 0 à 2 R¹¹ ; ou R¹⁷ et R¹⁷ conjointement avec l'atome d'azote auquel ils sont tous les deux liés forment 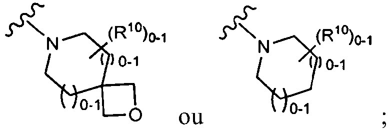
R^{a} est H ou un C₁₋₃ alkyle ;
R^{b} est H, un C₁₋₃ alkyle substitué par 0 à 5 R^{e} ou un -(CH₂)₀₋₁-C₃₋₆ cycloalkyle substitué par 0 à 5 R^{e} ; et
R^{e} est un halo, CN, =O, C(=O)OH, un C₁₋₆ alkyle, -CH₂OH ou un -S(=O)₂C₁₋₃ alkyle.

11. Composé selon la revendication 1, présentant la formule (V) : 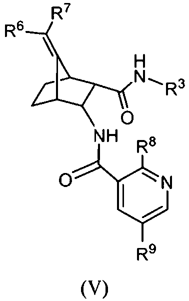 ou sel pharmaceutiquement acceptable de celui-ci, où :
R³ est un C₁₋₄ alkyle substitué par 0 à 3 R⁴, un -(CHR^{d})ₙ-C₃₋₆ carbocyclyle substitué par 0 à 3 R⁴ ;
R⁴ est un halo, CN ou un C₁₋₄ alkyle substitué par 0 à 5 substituants halo ;
R⁶ est un halo, un C₁₋₄ alkyle substitué par 0 à 3 R^{6a}, un C₃₋₆ cycloalkyle ou un hétérocyclyle de 5 ou 6 chaînons comprenant 1 à 3 hétéroatomes choisis parmi O, S et N et substitué par 0 à 5 R¹⁴ ;
R^{6a} est un halo ;
R⁷ est H ;
R⁸ est un -OC₁₋₃ alkyle ;
R⁹ est 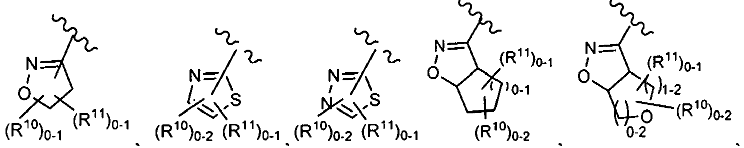 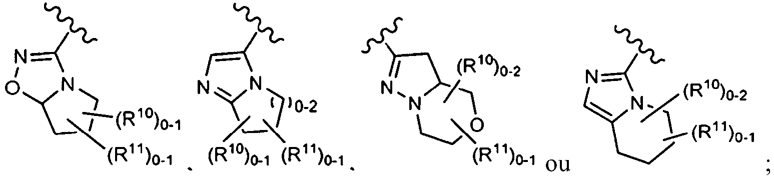
R¹⁰ est un halo, CN, un C₁₋₄ alkyle ou -OH ;
R¹¹ est un C₁₋₃ alkyle substitué par 0 à 3 R¹² et 0 à 2 R¹³, CN ou OR^{b} ;
R¹² est un halo ;
R¹³ est -OR^{b} ou un C₃₋₆ carbocyclyle ;
R¹⁴ est un halo, CN ou un C₁₋₄ alkyle substitué par 0 à 3 halo ;
R^{b} est H ou un C₁₋₃ alkyle substitué par 0 à 5 R^{e} ;
R^{d} est H ou un C₁₋₄ alkyle ;
R^{c} est un halo ou OH ; et
n vaut zéro ou 1.

12. Composé selon la revendication 1, présentant la formule (VI) : 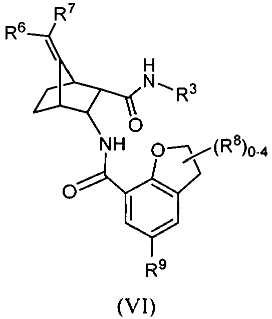 ou sel pharmaceutiquement acceptable de celui-ci, où :
R³ est un C₁₋₄ alkyle substitué par 0 à 3 R⁴, un -(CHR^{d})ₙ-C₃₋₆ carbocyclyle substitué par 0 à 3 R⁴ ;
R⁴ est un halo, CN ou un C₁₋₄ alkyle substitué par 0 à 5 substituants halo ;
R⁶ est un halo, un C₁₋₄ alkyle substitué par 0 à 3 R^{6a}, un C₃₋₆ cycloalkyle ou un hétérocyclyle de 5 ou 6 chaînons comprenant 1 à 3 hétéroatomes choisis parmi O, S et N et substitué par 0 à 5 R¹⁴ ;
R^{6a} est un halo ou -OH ;
R⁷ est H ;
R⁸ est un -OC₁₋₃ alkyle ;
R⁹ est 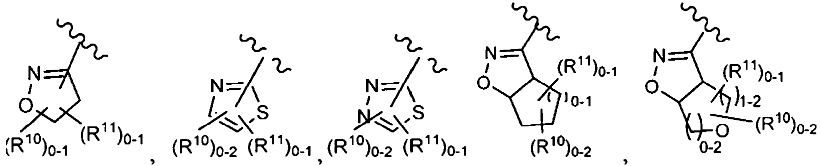 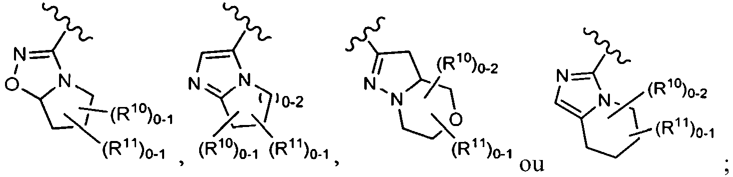
R¹⁰ est un halo, CN, un C₁₋₄ alkyle ou -OH ;
R¹¹ est un C₁₋₃ alkyle substitué par 0 à 3 R¹² et 0 à 2 R¹³, CN ou OR^{b} ;
R¹² est un halo ;
R¹³ est -OR^{b} ou un C₃₋₆ carbocyclyle ;
R¹⁴ est un halo, CN ou un C₁₋₄ alkyle substitué par 0 à 3 substituants halo ;
R^{b} est H ou un C₁₋₃ alkyle substitué par 0 à 5 R^{e} ;
R^{d} est H ou un C₁₋₄ alkyle ;
R^{e} est un halo ou OH ; et
n vaut zéro ou 1.

13. Composé selon la revendication 1, présentant la formule (VII) : 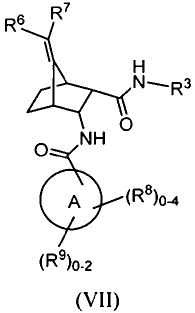 ou sel pharmaceutiquement acceptable de celui-ci, où : 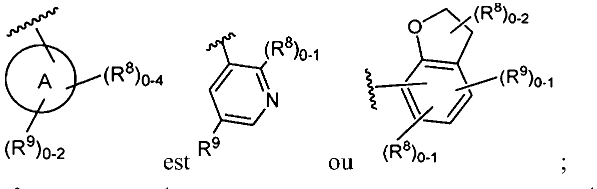
R³ est un -(CHR^{d})ₙ-C₃₋₁₀ carbocyclyle substitué par 0 à 5 R⁴ ;
R⁴ est un halo, CN, un C₁₋₄ alkyle substitué par 0 à 5 halo ou -OH, un -OC₁₋₄ alkyle substitué par 0 à 5 substituants halo ou -S(=O)ₚR^{c} ;
R⁶ est un C₁₋₄ alkyle substitué par 0 à 3 R^{6a} ou un C₃₋₆ cycloalkyle substitué par 0 à 5 R¹⁴ ;
R^{6a} est un halo ;
R⁷ est H ;
R⁸ est un C₁₋₃ alkyle ou un -OC₁₋₃ alkyle ;
R⁹ est -C(=O)NR¹⁷R¹⁷, un C₃₋₆ carbocyclyle substitué par 0 à 3 R¹⁰ et 0 à 2 R¹¹ ou un hétérocyclyle de 5 à 12 chaînons comprenant 1 à 5 hétéroatomes choisis parmi O, S(=O)p, N et NR^{11a} et substitué par 0 à 3 R¹⁰ et 0 à 2 R¹¹ ;
R¹⁰ est un halo, CN, un C₁₋₄ alkyle, =O, -OH ou un -OC₁₋₄ alkyle ;
R¹¹ est un C₁₋₄ alkyle substitué par 0 à 5 R¹² et 0 à 2 R¹³, -S(=O)ₚR^{c}, un C₃₋₆ cycloalkyle substitué par 0 à 5 R^{e} ;
R^{11a} est H, un C₁₋₄ alkyle substitué par 0 à 4 R^{11b}, -C(=O)R^{b}, -C(=O)OR^{b}, - C(=O)NR^{a}R^{a}, un C₃₋₆ cycloalkyle substitué par 0 à 5 R^{e}, un aryle substitué par 0 à 5 R^{e}, un hétérocyclyle de 4 à 6 chaînons comprenant 1 à 4 hétéroatomes choisis parmi O, S(=O)ₚ, N et NR¹⁵ et substitué par 0 à 5 R^{e} ;
R^{11b} est un halo, -OH, -C(=O)OH, un -C(=O)OC₁₋₄ alkyle ou un aryle ;
R¹² est un halo, -C(=O)OR^{b}, un C₁₋₄ alkyle substitué par 0 à 3 halo ou OH ou un C₃₋₆ cycloalkyle ;
R¹³ est -OR^{b}, -NR^{a}R^{a}, -OC(=O)NR^{a}R^{a} ;
R¹⁴ est un halo ;
R¹⁷ est H ou un C₁₋₄ alkyle substitué par 0 à 3 R¹⁰ et 0 à 2 R¹¹ ; ou R¹⁷ et R¹⁷ conjointement avec l'atome d'azote auquel ils sont tous les deux liés forment un hétérocyclyle de 3 à 9 chaînons comprenant 1 à 5 hétéroatomes choisis parmi O, S(=O)ₚ, N et NR^{11a} et substitué par 0 à 3 R¹⁰ et 0 à 2 R¹¹ ;
R^{a} est H, un C₁₋₆ alkyle substitué par 0 à 5 R^{e}, un C₃₋₁₀ carbocyclyle substitué par 0 à 5 R^{e} ; ou R^{a} et R^{a} conjointement avec l'atome d'azote auquel ils sont tous les deux liés forment un hétérocyclyle substitué par 0 à 5 R^{e} ;
R^{b} est H ou un C₁₋₄ alkyle substitué par 0 à 5 R^{e} ;
R^{c} est un C₁₋₄ alkyle ;
R^{d} est H ou un C₁₋₃ alkyle ;
R^{e} est un halo, CN, NO₂, =O, un C₁₋₆ alkyle substitué par 0 à 5 R^{g}, un C₃₋₆ cycloalkyle ou -S(=O)ₚR^{f} ;
R^{f} est H, un C₁₋₆ alkyle ;
R^{g} est un halo, CN, -OH ou un C₁₋₆ alkyle ;
n vaut zéro ou 1 ; et
p vaut zéro, 1 ou 2.

14. Composé selon la revendication 13, ou sel pharmaceutiquement acceptable de celui-ci, où :
R³ est un -CHR^{d}-C₃₋₆ cycloalkyle substitué par 0 à 2 R⁴ ou un phényle substitué par 0 à 2 R⁴ ;
R⁴ est F, CH₃ ou CF₃ ;
R⁶ est un C₁₋₄ alkyle substitué par 0 à 3 halo ou un C₃₋₆ cycloalkyle substitué par 0 à 3 substituants halo ;
R⁷ est H ;
R⁸ est -OCH₃ ;
R⁹ est -C(=O)NR¹⁷R¹⁷, 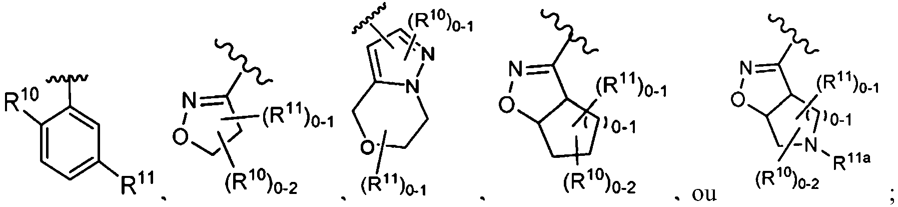
R¹⁰ est un halo ;
R¹¹ est un C₁₋₄ alkyle substitué par 0 à 3 R¹² et 0 à 2 R¹³, -S(=O)ₚR^{c} ou un C₃₋₆ cycloalkyle substitué par 0 à 3 R^{e} ;
R^{11a} est H ou un C₁₋₄ alkyle substitué par 0 à 3 R^{11b} ;
R^{11b} est -OH ;
R¹² est un C₁₋₄ alkyle substitué par 0 à 3 substituants halo ;
R¹³ est -OC(=O)NR^{a}R^{a} ;
R¹⁴ est un halo ;
R¹⁷ est H ou un C₁₋₄ alkyle substitué par 0 à 2 R¹⁰ et 0 à 2 R¹¹ ; ou R¹⁷ et R¹⁷ conjointement avec l'atome d'azote auquel ils sont tous les deux liés forment un hétérocyclyle de 3 à 9 chaînons comprenant 1 à 4 hétéroatomes choisis parmi O, S(=O)ₚ, N et NR^{11a} et substitué par 0 à 2 R¹⁰ et 0 à 2 R¹¹ ;
R^{a} est H, un C₁₋₄ alkyle substitué par 0 à 4 R^{e}, un C₃₋₁₀ carbocyclyle substitué par 0 à 4 R^{e} ; ou R^{a} et R^{a} conjointement avec l'atome d'azote auquel ils sont tous les deux liés forment un hétérocyclyle substitué par 0 à 4 R^{e} ;
R^{b} est H ou un C₁₋₃ alkyle ;
R^{c} est un C₁₋₃ alkyle ;
R^{d} est H ou un C₁₋₃ alkyle ;
R^{e} est un C₁₋₄ alkyle, un C₃₋₆ cycloalkyle ou -S(=O)ₚR^{f} ;
R^{f} est un C₁₋₄ alkyle ;
n vaut zéro ou 1 ; et
p vaut zéro, 1 ou 2.

15. Composition pharmaceutique comprenant un composé selon l'une quelconque des revendications 1 à 14, ou un sel pharmaceutiquement acceptable de celui-ci, et un véhicule pharmaceutiquement acceptable.

16. Composé selon l'une quelconque des revendications 1 à 14, ou composition pharmaceutique selon la revendication 15, pour une utilisation en thérapie.

17. Composé selon l'une quelconque des revendications 1 à 14, ou composition pharmaceutique selon la revendication 15, pour une utilisation dans le traitement d'une maladie choisie dans le groupe constitué par : angine de poitrine, angor instable, infarctus du myocarde, insuffisance cardiaque, maladie coronarienne aiguë, insuffisance cardiaque aiguë, insuffisance cardiaque chronique et dommage iatrogénique cardiaque.

18. Composé ou composition pour une utilisation selon la revendication 17, où la maladie est une insuffisance cardiaque.

19. Composé selon l'une quelconque des revendications 1 à 14, ou composition pharmaceutique selon la revendication 15, pour une utilisation dans le traitement d'une fibrose.
